(19) Europäisches Patentamt
European Patent Office
Office européen des brevets

(11) EP 2 331 552 B1

(12) # EUROPEAN PATENT SPECIFICATION

(45) Date of publication and mention of the grant of the patent:
**27.02.2013 Bulletin 2013/09**

(21) Application number: **09736718.9**

(22) Date of filing: **17.09.2009**

(51) Int Cl.:
***C07D 498/08*** *(2006.01)* ***A61K 31/407*** *(2006.01)*
***A61P 31/14*** *(2006.01)*

(86) International application number:
**PCT/US2009/057235**

(87) International publication number:
**WO 2010/036551 (01.04.2010 Gazette 2010/13)**

(54) **HEPATITIS C VIRUS INHIBITORS**

HEPATITIS C VIREN HEMMER

INHIBITEURS DU VIRUS DE L HÉPATITE C

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **29.09.2008 US 100907 P**
**14.09.2009 US 559179**

(43) Date of publication of application:
**15.06.2011 Bulletin 2011/24**

(73) Proprietor: **Bristol-Myers Squibb Company**
**Princeton NJ 08543-4000 (US)**

(72) Inventors:
- **HIEBERT, Sheldon**
**Manitoba, MB (CA)**
- **RAJAMANI, Ramkumar**
**Wallingford, CT 06492 (US)**
- **BOWSHER, Michael S.**
**Wallingford, Connecticut 06492 (US)**
- **LI, Rongti**
**Port Lavaca, TX 77979, (US)**
- **SCOLA, Paul Michael**
**Wallingford, Connecticut 06492 (US)**

(74) Representative: **Reitstötter - Kinzebach**
**Patentanwälte**
**Sternwartstrasse 4**
**81679 München (DE)**

(56) References cited:
**WO-A-2006/119061** **WO-A-2008/051475**
**WO-A-2008/057208** **WO-A-2008/057209**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 331 552 B1

## Description

The present disclosure is generally directed to antiviral compounds, and more specifically directed to compounds which inhibit the function of the NS3 protease (also referred to herein as "serine protease") encoded by Hepatitis C virus (HCV), compositions comprising such compounds, and methods for inhibiting the function of the NS3 protease.

HCV is a major human pathogen, infecting an estimated 170 million persons worldwide - roughly five times the number infected by human immunodeficiency virus type 1. A substantial fraction of these HCV infected individuals develop serious progressive liver disease, including cirrhosis and hepatocellular carcinoma.

Presently, the most effective HCV therapy employs a combination of alpha-interferon and ribavirin, leading to sustained efficacy in 40% of patients. Recent clinical results demonstrate that pegylated alpha-interferon is superior to unmodified alpha-interferon as monotherapy. However, even with experimental therapeutic regimens involving combinations of pegylated alpha-interferon and ribavirin, a substantial fraction of patients do not have a sustained reduction in viral load. Thus, there is a clear and unmet need to develop effective therapeutics for treatment of HCV infection.

HCV is a positive-stranded RNA virus. Based on a comparison of the deduced amino acid sequence and the extensive similarity in the 5' untranslated region, HCV has been classified as a separate genus in the Flaviviridae family. All members of the Flaviviridae family have enveloped virions that contain a positive stranded RNA genome encoding all known virus-specific proteins via translation of a single, uninterrupted, open reading frame.

Considerable heterogeneity is found within the nucleotide and encoded amino acid sequence throughout the HCV genome. Six major genotypes have been characterized, and more than 50 subtypes have been described. The major genotype of HCV differ in their distribution worldwide, and the clinical significance of the genetic heterogeneity of HCV remains elusive despite numerous studies of the possible effect of genotypes on pathogenesis and therapy.

The single strand HCV RNA genome is approximately 9500 nucleotides in length and has a single open reading frame (ORF) encoding a single large polyprotein of about 3000 amino acids. In infected cells, this polyprotein is cleaved at multiple sites by cellular and viral proteases to produce the structural and non- structural (NS) proteins. In the case of HCV, the generation of mature non-structural proteins (NS2, NS3, NS4A, NS4B, NS5A, and NS5B) is effected by two viral proteases. The first one cleaves at the NS2-NS3 junction; the second one is a serine protease contained within the *N*-terminal region of NS3 and mediates all the subsequent cleavages downstream of NS3, both in cis, at the NS3-NS4A cleavage site, and in trans, for the remaining NS4A- NS4B, NS4B-NS5A, NS5A-NS5B sites. The NS4A protein appears to serve multiple functions, acting as a co-factor for the NS3 protease and possibly assisting in the membrane localization of NS3 and other viral replicase components. The complex formation of the NS3 protein with NS4A is essential for efficient polyprotein processing, enhancing the proteolytic cleavage at all of the sites. The NS3 protein also exhibits nucleoside triphosphatase and RNA helicase activities. NS5B is a RNA-dependent RNA polymerase that is involved in the replication of HCV. Compounds inhibiting HCV NS 3 protease are disclosed in WO 2006/119061, WO 2008/057209, WO 2008/051475 and WO 2008/057208. They are therefore useful for treating or preventing HCV infections.

The present disclosure provides peptide compounds that can inhibit the functioning of the NS3 protease, e.g., in combination with the NS4A protease. Further, the present disclosure describes the administration of combination therapy to a patient whereby a compound in accordance with the present disclosure, which is effective to inhibit the HCV NS3 protease, can be administered with additional compounds having anti-HCV activity.

In its first aspect the present disclosure provides a compound of formula (I)

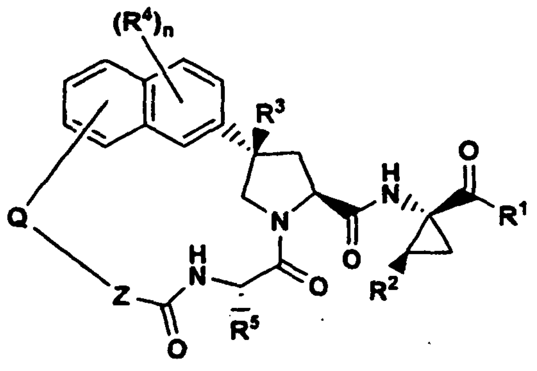

(I),

or a pharmaceutically acceptable salt thereof, wherein

nis0, 1,2,or3;

$R^1$ is selected from hydroxy and-$NHSO_2R^6$;
$R^2$ is selected from hydrogen, alkenyl, alkyl, and cycloalkyl, wherein the alkenyl, the alkyl, and the cycloalkyl are each optionally substituted with one, two, three, or four halo groups;
$R^3$ is selected from hydrogen, alkoxy, alkoxyalkoxy, alkylsulfanyl, alkylsulfonyl, alkylsulfoxyl, hydroxy, and $(NR^cR^d)$carbonyloxy;
each $R^4$ is independently selected from alkoxy, alkyl, cyano, dialkylamino, halo, haloalkyl, haloalkoxy, a monocyclic heterocycle, hydroxy, and phenyl; wherein the moncyclic heterocycle and the phenyl are each optionally substituted with one, two, three, four, or five substituents independently selected from alkoxy, alkyl, dialkylamino, halo, haloalkoxy, and haloalkyl;
$R^5$ is selected from hydrogen, alkenyl, alkyl, aryl, arylalkyl, cycloalkyl, (cycloalkyl)alkyl, heterocyclyl, and heterocyclylalkyl; wherein the alkyl and cycloalkyl are each optionally substituted with one group selected from alkoxy, haloalkoxy, halo, haloalkyl, cyano, and dialkylamino;
$R^6$ is selected from alkyl, aryl, arylalkyl, cycloalkyl, (cycloalkyl)alkyl, heterocyclyl, and -$NR^aR^b$; wherein the alkyl and cycloalkyl are each optionally substituted with one group selected from alkyl, alkoxy, halo, haloalkyl, cyano, cyanoalkyl, and haloalkoxy;
$R^a$ and $R^b$ are independently selected from hydrogen, alkoxy, alkyl, aryl, arylalkyl, cycloalkyl, (cycloalkyl)alkyl, haloalkyl, heterocyclyl, and heterocyclylalkyl;
$R^c$ and $R^d$ are independently selected from hydrogen and alkyl;
Q is a $C_{4-8}$ saturated or unsaturated chain, wherein the chain is optionally substituted with one, two, three, or four groups independently selected from alkyl, halo, and haloalkyl, wherein the alkyl and haloalkyl groups can optionally form a 3-7 membered ring with the carbon atom to which they are attached; and
Z is selected from $CH_2$, O, and $NR^z$; wherein $R^z$ is selected from hydrogen and alkyl.

In a first embodiment of the first aspect the present disclosure provides a compound of formula (I), or a pharmaceutically acceptable salt thereof, wherein $R^1$ is- $NHSO_2R^6$. In a second embodiment of the first aspect n is 0, 1, or 2. In a third embodiment of the first aspect Q is a $C_{4-8}$ saturated or unsubstituted chain optionally substituted with two alkyl groups which can optionally form a 3-membered ring with the carbon atom to which they are attached. In a fourth embodiment of the first aspect $R^3$ is selected from alkoxy, alkoxyalkoxy, hydroxy, and $(NR^cR^d)$carbonyloxy. In a fifth embodiment of the first aspect $R^2$ is selected from alkenyl, alkyl, and unsubstituted cycloalkyl; wherein the alkyl is optionally substituted with two halo groups. In a sixth embodiment of the first aspect $R^5$ is selected from alkyl and heterocyclyl.

In a seventh embodiment of the first aspect the present disclosure provides a ccompound of formula (I), or a pharmaceutically acceptable salt thereof, wherein
$R^1$ is -$NHSO_2R^6$;
$R^2$ is selected from alkenyl, alkyl, and unsubstituted cycloalkyl; wherein the alkyl is optionally substituted with two halo groups; and
$R^5$ is selected from alkyl and heterocyclyl.

In an eighth embodiment of the first aspect the present disclosure provides a compound of formula (I), or a pharmaceutically acceptable salt thereof, wherein
n is 0,1, or 2;
$R^1$ is -$NHSO_2R^6$; wherein $R^6$ is cycloalkyl;
$R^2$ is selected from alkenyl, alkyl, and unsubstituted cycloalkyl; wherein the alkyl is optionally substituted with two halo groups;
$R^3$ is alkoxy, alkoxyalkoxy, hydroxy, and $(NR^cR^d)$carbonyloxy;
each $R^4$ is selected from alkoxy, dialkylamino, and halo;
$R^5$ is selected from alkyl and heterocyclyl; and
Q is a $C_{4-8}$ saturated or unsubstituted chain optionally substituted with two alkyl groups which can optionally form a 3-membered ring with the carbon atom to which they are attached.

In a second aspect the present disclosure provides a compound of formula (II)

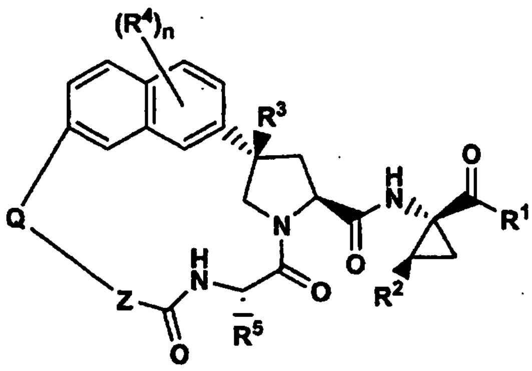

(II),

or a pharmaceutically acceptable salt thereof, wherein

n is 0, 1, 2 or 3;
$R^1$ is selected from hydroxy and-$NHSO_2R^6$;
$R^2$ is selected from hydrogen, alkenyl, alkyl, and cycloalkyl, wherein the alkenyl, the alkyl, and the cycloalkyl are each optionally substituted with one, two, three, or four halo groups;
$R^3$ is selected from hydrogen, alkoxy, alkylsulfanyl, alkylsulfonyl, alkylsulfoxyl, and hydroxy;
each $R^4$ is independently selected from alkoxy, alkyl, cyano, dialkylamino, halo, haloalkyl, haloalkoxy, a monocyclic heterocycle, hydroxy, and phenyl; wherein the moncyclic heterocycle and the phenyl are each optionally substituted with one, two, three, four, or five substituents independently selected from alkoxy, alkyl, dialkylamino, halo, haloalkoxy, and haloalkyl;
$R^5$ is selected from hydrogen, alkenyl, alkyl, aryl, arylalkyl, cycloalkyl, (cycloalkyl)alkyl, heterocyclyl, and heterocyclylalkyl; wherein the alkyl and cycloalkyl are each optionally substituted with one group selected from alkoxy, haloalkoxy, halo, haloalkyl, cyano, and dialkylamino;
$R^6$ is selected from alkyl, aryl, arylalkyl, cycloalkyl, (cycloalkyl)alkyl, heterocyclyl, and-$NR^aR^b$; wherein the alkyl and cycloalkyl are each optionally substituted with one group selected from alkyl, alkoxy, halo, haloalkyl, cyano, cyanoalkyl, and haloalkoxy,
$R^a$ and $R^b$ are independently selected from hydrogen, alkoxy, alkyl, aryl, arylalkyl, cycloalkyl, (cycloalkyl)alkyl, haloalkyl, heterocyclyl, and heterocyclylalkyl;
Q is a $C_{4-8}$ saturated or unsaturated chain, wherein the chain is optionally substituted with one, two, three, or four groups independently selected from alkyl, halo, and haloalkyl, wherein the alkyl and haloalkyl groups can optionally form a 3-7 membered ring with the carbon atom to which they are attached; and
Z is selected from $CH_2$, O, and $NR^z$; wherein $R^z$ is selected from hydrogen and alkyl.

In a third aspect the present disclosure provides a composition comprising the compound of formula (I), or a pharmaceutically acceptable salt thereof, and a pharmaceutically acceptable carrier. In a first embodiment of the third aspect the composition further comprises at least one additional compound having anti-HCV activity. In a second embodiment of the third aspect at least one of the additional compounds is an interferon or a ribavirin. In a third embodiment of the third aspect the interferon is selected from interferon alpha 2B, pegylated interferon alpha, consensus interferon, interferon alpha 2A, and lymphoblastiod interferon tau.

In a fourth embodiment of the third aspect the present disclosure provides a composition comprising the compound of formula (I), or a pharmaceutically acceptable salt thereof, a pharmaceutically acceptable carrier, and at least one additional compound having anti-HCV activity, wherein at least one of the additional compounds is selected from interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

In a fifth embodiment of the third aspect the present disclosure provides a composition comprising the compound of formula (I), or a pharmaceutically acceptable salt thereof, a pharmaceutically acceptable carrier, and at least one additional compound having anti-HCV activity, wherein at least one of the additional compounds is effective to inhibit the function of a target selected from HCV metalloprotease, HCV serine protease, HCV polymerase, HCV helicase, HCV NS4B protein, HCV entry, HCV assembly, HCV egress, HCV NS5A protein, and IMPDH for the treatment of an HCV infection.

In a fourth aspect the present disclosure provides a composition comprising a compound of formula (I), or a

pharmaceutically acceptable salt thereof, one, two, three, four, or five additional compounds having anti-HCV activity, and a pharmaceutically acceptable carrier. In a first embodiment of the fourth aspect the compsition comprises three or four additional compounds having anti-HCV activity. In a second embodiment of the fourth aspect the composition comprises one or two additional compounds having anti-HCV activity.

In a fifth aspect the present disclosure provides a compound of the invention for use in a method of treating an HCV infection in a patient, comprising administering to the patient a therapeutically effective amount of formula (I), or a pharmaceutically acceptable salt thereof. In a first embodiment of the fifth aspect the method further comprises administering at least one additional compound having anti-HCV activity prior to, after, or simultaneously with the compound of formula (I), or a pharmaceutically acceptable salt thereof. In a second embodiment of the fifth aspect at least one of the additional compounds is an interferon or a ribavirin. In a third embodiment of the fifth aspect the interferon is selected from interferon alpha 2B, pegylated interferon alpha, consensus interferon, interferon alpha 2A, and lymphoblastiod interferon tau.

In a fourth embodiment of the fifth aspect the present disclosure provides a compound of the invention for use in a method of treating an HCV infection in a patient, comprising administering to the patient a therapeutically effective amount of formula (I), or a pharmaceutically acceptable salt thereof, and at least one additional compound having anti-HCV activity prior to, after, or simultaneously with the compound of formula (I), or a pharmaceutically acceptable salt thereof, wherein at least one of the additional compounds is selected from interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

In a fifth embodiment of the fifth aspect the present disclosure provides a compound of the invention for use in a method of treating an HCV infection in a patient, comprising administering to the patient a therapeutically effective amount of formula (I), or a pharmaceutically acceptable salt thereof, and at least one additional compound having anti-HCV activity prior to, after, or simultaneously with the compound of formula (I), or a pharmaceutically acceptable salt thereof, wherein at least one of the additional compounds is effective to inhibit the function of a target selected from HCV metalloprotease, HCV serine protease, HCV polymerase, HCV helicase, HCV NS4B protein, HCV entry, HCV assembly, HCV egress, HCV NS5A protein, and IMPDH for the treatment of an HCV infection.

In a sixth aspect the present disclosure provides a compound of the invention for use in a method of treating an HCV infection in a patient, comprising administering to the patient a therapeutically effective amount of a compound of formula (I), or a pharmaceutically acceptable salt thereof and one, two, three, four, or five additional compounds having anti-HCV activity prior to, after, or simultaneously with the compound of formula (I), or a pharmaceutically acceptable salt thereof. In a first embodiment of the sixth aspect the method comprises administering three or four additional compounds having anti-HCV activity. In a second embodiment of the sixth aspect the method comprises administering one or two additional compounds having anti-HCV activity.

Other aspects of the present disclosure may include suitable combinations of embodiments disclosed herein.

Yet other aspects and embodiments may be found in the description provided herein.

The description of the present disclosure herein should be construed in congruity with the laws and principals of chemical bonding. In some instances it may be necessary to remove a hydrogen atom in order accommodate a substitutent at any given location.

It should be understood that the compounds encompassed by the present disclosure are those that are suitably stable for use as pharmaceutical agent.

It is intended that the definition of any substituent or variable at a particular location in a molecule be independent of its definitions elsewhere in that molecule. For example, when n is 2, each of the two $R^4$ groups may be the same or different.

All patents, patent applications, and literature references cited in the specification are herein incorporated by reference in their entirety. In the case of inconsistencies, the present disclosure, including definitions, will prevail.

As used herein, the singular forms "a", "an", and "the" include plural reference unless the context clearly dictates otherwise.

The term "alkenyl," as used herein, refers to a straight or branched chain group of two to ten carbon atoms containing at least one carbon-carbon double bond.

The term "alkoxy," as used herein, refers to an alkyl group attached to the parent molecular moiety through an oxygen atom.

The term "alkyl," as used herein, refers to a group derived from a straight or branched chain saturated hydrocarbon containing from one to ten carbon atoms.

The term "alkylsulfanyl," as used herein, refers to an alkyl group attached to the parent molecular moiety through a sulfur atom.

The term "alkylsulfonyl," as used herein, refers to an alkyl group attached to the parent molecular moiety through a sulfonyl group.

The term "alkylsulfoxyl," as used herein, refers to an alkyl group attached to the parent molecular moiety through

a sulfoxyl group.

The term "aryl," as used herein, refers to a phenyl group, or a bicyclic fused ring system wherein one or both of the rings is a phenyl group. Bicyclic fused ring systems consist of a phenyl group fused to a four- to six-membered aromatic or nonaromatic carbocyclic ring. The aryl groups of the present invention can be attached to the parent molecular moiety through any substitutable carbon atom in the group. Representative examples of aryl groups include, but are not limited to, indanyl, indenyl, naphthyl, phenyl, and tetrahydronaphthyl.

The term "arylalkyl," as used herein, refers to an alkyl group substituted with one, two, or three aryl groups.

The term "cyano," as used herein, refers to -CN.

The term "cyanoalkyl," as used herein, refers to an alkyl group substituted with one, two, or three cyano groups.

The term "cycloalkyl," as used herein, refers to a saturated monocyclic or bicyclic hydrocarbon ring system having three to seven carbon atoms and zero heteroatoms. Representative examples of cycloalkyl groups include, but are not limited to, cyclopropyl, cyclobutyl, and cyclopentyl.

The term "(cycloalkyl)alkyl," as used herein, refers to an alkyl group substituted with one, two, or three cycloalkyl groups.

The term "dialkylamino," as used herein, refers to $-NR^pR^q$, wherein $R^p$ and $R^q$ are alkyl groups. The alkyl groups may be the same or different.

The terms "halo" and "halogen," as used herein, refer to F, Cl, Br, and I.

The term "haloalkoxy," as used herein, refers to a haloalkyl group attached to the parent molecular moiety through an oxygen atom.

The term "haloalkyl," as used herein, refers to an alkyl group substituted with one, two, three, or four halogen atoms.

The term "heterocyclyl," as used herein, refers to a five-, six-, or seven-membered ring containing one, two, three, or four heteroatoms independently selected from nitrogen, oxygen, and sulfur. The five-membered ring has zero to two double bonds and the six- and seven-membered rings have zero to three double bonds. The term "heterocyclyl" also includes bicyclic groups in which the heterocyclyl ring is fused to a phenyl group, a monocyclic cycloalkenyl group, a monocyclic cycloalkyl group, or another monocyclic heterocyclyl group; and tricyclic groups in which a bicyclic system is fused to a phenyl group, a monocyclic cycloalkenyl group, a monocyclic cycloalkyl group, or another monocyclic heterocyclyl group. The heterocyclyl groups of the present invention can be attached to the parent molecular moiety through a carbon atom or a nitrogen atom in the group. Examples of heterocyclyl groups include, but are not limited to, benzothienyl, furyl, imidazolyl, indolinyl, indolyl, isothiazolyl, isoxazolyl, morpholinyl, oxazolyl, piperazinyl, piperidinyl, pyrazolyl, pyridinyl, pyrrolidinyl, pyrrolopyridinyl, pyrrolyl, thiazolyl, thienyl, and thiomorpholinyl.

The term "heterocyclylalkyl," as used herein, refers to an alkyl group substituted with one, two, or three heterocyclyl groups.

The term "hydroxy," as used herein, refers to -OH.

The term "$-NR^aR^b$," as used herein, refers to two groups, $R^a$ and $R^b$, which are attached to the parent molecular moiety through a nitrogen atom. $R^a$ and $R^b$ are independently selected from hydrogen, alkoxy, alkyl, aryl, arylalkyl, cycloalkyl, (cycloalkyl)alkyl, haloalkyl, heterocyclyl, and heterocyclylalkyl.

The term "$-NR^cR^d$," as used herein, refers to two groups, $R^c$ and $R^d$, which are attached to the parent molecular moiety through a nitrogen atom. $R^c$ and $R^d$ are independently selected from hydrogen and alkyl.

The term "$(NR^cR^d)$carbonyl," as used herein, refers to an $-NR^cR^d$ group attached to the parent moleclular moiety through a carbonyl group.

The term "$(NR^cR^d)$carbonyloxy;" as used herein, refers to an $(NR^cR^d)$carbonyl group attached to the parent molecular moiety through an oxygen atom.

The compounds of the present disclosure can exist as pharmaceutically acceptable salts. The term "pharmaceutically acceptable salt," as used herein, represents salts or zwitterionic forms of the compounds of the present disclosure which are water or oil-soluble or dispersible, which are, within the scope of sound medical judgment, suitable for use in contact with the tissues of patients without excessive toxicity, irritation, allergic response, or other problem or complication commensurate with a reasonable benefit/risk ratio, and are effective for their intended use. The salts can be prepared during the final isolation and purification of the compounds or separately by reacting a suitable basic functionality with a suitable acid. Representative acid addition salts include acetate, adipate, alginate, citrate, aspartate, benzoate, benzenesulfonate, bisulfate, butyrate, camphorate, camphorsulfonate; digluconate, glycerophosphate, hemisulfate, heptanoate, hexanoate, formate, fumarate, hydrochloride, hydrobromide, hydroiodide, 2-hydroxyethanesulfonate, lactate, maleate, mesitylenesulfonate, methanesulfonate, naphthylenesulfonate, nicotinate, 2-naphthalenesulfonate, oxalate, palmoate, pectinate, persulfate, 3-phenylproprionate, picrate, pivalate, propionate, succinate, tartrate, trichloroacetate, trifluoroacetate, phosphate, glutamate, bicarbonate, para-toluenesulfonate, and undecanoate. Examples of acids which can be employed to form pharmaceutically acceptable addition salts include inorganic acids such as hydrochloric, hydrobromic, sulfuric, and phosphoric, and organic acids such as oxalic, maleic, succinic, and citric.

Basic addition salts can be prepared during the final isolation and purification of the compounds by reacting

an acidic group with a suitable base such as the hydroxide, carbonate, or bicarbonate of a metal cation or with ammonia or an organic primary, secondary, or tertiary amine. The cations of pharmaceutically acceptable salts include lithium, sodium, potassium, calcium, magnesium, and aluminum, as well as nontoxic quaternary amine cations such as ammonium, tetramethylammonium, tetraethylammonium, methylamine, dimethylamine, trimethylamine, triethylamine, diethylamine, ethylamine, tributylamine, pyridine, *N,N*-dimethylaniline, *N*-methylpiperidine, *N*-methylmorpholine, dicyclohexylamine, procaine, dibenzylamine, *N,N*-dibenzylphenethylamine, and *N,N'*-dibenzylethylenediamine. Other representative organic amines useful for the formation of base addition salts include ethylenediamine, ethanolamine, diethanolamine, piperidine, and piperazine.

As used herein, the term "anti-HCV activity" means the compound is effective to treat the HCV virus.

The term "compounds of the disclosure", and equivalent expressions, are meant to embrace compounds of formula (I), and pharmaceutically acceptable enantiomers, diastereomers, and salts thereof. Similarly, references to intermediates, are meant to embrace their salts where the context so permits.

The term "patient" includes both human and other mammals.

The term "pharmaceutical composition" means a composition comprising a compound of the disclosure in combination with at least one additional pharmaceutical carrier, i.e., adjuvant, excipient or vehicle, such as diluents, preserving agents, fillers, flow regulating agents, disintegrating agents, wetting agents, emulsifying agents, suspending agents, sweetening agents, flavoring agents, perfuming agents, antibacterial agents, antifungal agents, lubricating agents and dispensing agents, depending on the nature of the mode of administration and dosage forms. Ingredients listed in Remington's Pharmaceutical Sciences, 18th ed., Mack Publishing Company, Easton, PA (1999) for example, may be used.

The phrase "pharmaceutically acceptable" is employed herein to refer to those compounds, materials, compositions, and/or dosage forms which are, within the scope of sound medical judgment, suitable for use in contact with the tissues of patients without excessive toxicity, irritation, allergic response, or other problem or complication commensurate with a reasonable risk/benefit ratio.

The term "sulfonyl," as used herein, refers to $-SO_2-$.

The term "sulfoxyl," as used herein, refers to -S(O)-.

The term "therapeutically effective amount" means the total amount of each active component that is sufficient to show a meaningful patient benefit, e.g., a sustained reduction in viral load. When applied to an individual active ingredient, administered alone, the term refers to that ingredient alone. When applied to a combination, the term refers to combined amounts of the active ingredients that result in the therapeutic effect, whether administered in combination, serially or simultaneously.

The terms "treat" and "treating" refers to: (i) preventing a disease, disorder or condition from occurring in a patient which may be predisposed to the disease, disorder and/or condition but has not yet been diagnosed as having it; (ii) inhibiting the disease, disorder or condition, i.e., arresting its development; and/or (iii) relieving the disease, disorder or condition, i.e., causing regression of the disease, disorder and/or condition.

Where used in naming compounds of the present disclosure, the designations P1', P1, P2, P2*, P3, and P4, as used herein, map the relative positions of the amino acid residues of a protease inhibitor binding relative to the binding of the natural peptide cleavage substrate. Cleavage occurs in the natural substrate between P1 and P1' where the nonprime positions designate amino acids starting from the C-terminus end of the peptide natural cleavage site extending towards the *N*-terminus; whereas, the prime positions emanate from the N-teminus end of the cleavage site designation and extend toward the C-terminus. For example, P1' refers to the first position away from the right hand end of the C-terminus of the cleavage site (i.e. *N*-terminus first position); whereas P1 starts the numbering from the left hand side of the C-terminus cleavage site, P2: second position from the C-terminus, etc.). (see Berger A. & Schechter I., Transactions of the Royal Society London series (1970), B257, 249-264].

Asymmetric centers exist in the compounds of the present disclosure. For example, the compounds may include P1 cyclopropyl element of formula

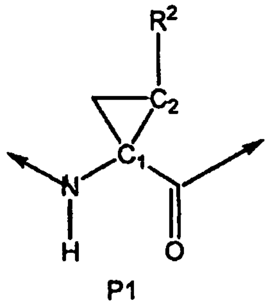

P1

wherein $C_1$ and $C_2$ each represent an asymmetric carbon atom at positions 1 and 2 of the cyclopropyl ring.

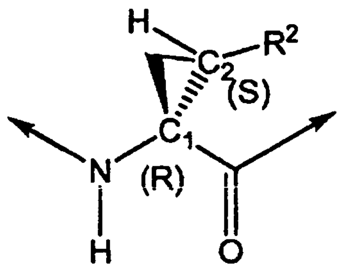

(1R, 2S)
$R^2$ is syn to carbonyl

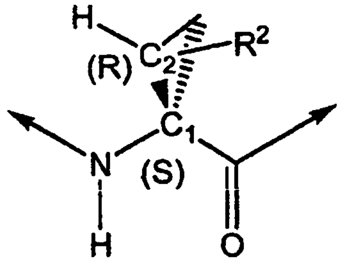

(1S, 2R)
$R^2$ is syn to carbonyl

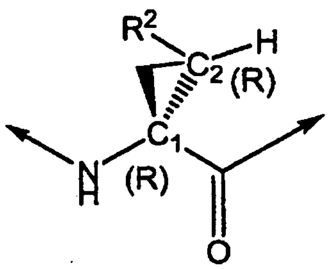

(1R, 2R)
$R^2$ is syn to amide

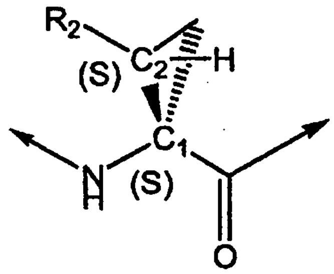

(1S, 2S)
$R^2$ is syn to amide

It should be understood that the disclosure encompasses all stereochemical forms, or mixtures thereof, which possess the ability to inhibit HCV protease.

Certain compounds of the present disclosure may also exist in different stable conformational forms which may be separable. Torsional asymmetry due to restricted rotation about an asymmetric single bond, for example because of steric hindrance or ring strain, may permit separation of different conformers. The present disclosure includes each conformational isomer of these compounds and mixtures thereof.

Certain compounds of the present disclosure may exist in zwitterionic form and the present disclosure includes each zwitterionic form of these compounds and mixtures thereof.

When it is possible that, for use in therapy, therapeutically effective amounts of a compound of formula (I), as well as pharmaceutically acceptable salts thereof, may be administered as the raw chemical, it is possible to present the active ingredient as a pharmaceutical composition. Accordingly, the disclosure further provides pharmaceutical compositions, which include therapeutically effective amounts of compounds of formula (I) or pharmaceutically acceptable salts thereof, and one or more pharmaceutically acceptable carriers, diluents, or excipients. The compounds of formula (I) and pharmaceutically acceptable salts thereof, are as described above. The carrier(s), diluent(s), or excipient(s) must be acceptable in the sense of being compatible with the other ingredients of the formulation and not deleterious to the recipient thereof. In accordance with another aspect of the disclosure there is also provided a process for the preparation of a pharmaceutical formulation including admixing a compound of formula (I), or a pharmaceutically acceptable salt thereof, with one or more pharmaceutically acceptable carriers, diluents, or excipients.

Pharmaceutical formulations may be presented in unit dose forms containing a predetermined amount of active ingredient per unit dose. Dosage levels of between about 0.01 and about 150 milligram per kilogram ("mg/kg") body weight per day, preferably between about 0.05 and about 100 mg/kg body weight per day of the compounds of the disclosure are typical in a monotherapy for the prevention and treatment of HCV mediated disease. Typically, the pharmaceutical compositions of this disclosure will be administered from about 1 to about 5 times per day or alternatively, as a continuous infusion. Such administration can be used as a chronic or acute therapy. The amount of active ingredient

that may be combined with the carrier materials to produce a single dosage form will vary depending on the condition being treated, the severity of the condition, the time of administration, the route of administration, the rate of excretion of the compound employed, the duration of treatment, and the age, gender, weight, and condition of the patient. Preferred unit dosage formulations are those containing a daily dose or sub-dose, as herein above recited, or an appropriate fraction thereof, of an active ingredient. Generally, treatment is initiated with small dosages substantially less than the optimum dose of the compound. Thereafter, the dosage is increased by small increments until the optimum effect under the circumstances is reached. In general, the compound is most desirably administered at a concentration level that will generally afford antivirally effective results without causing any harmful or deleterious side effects.

When the compositions of this disclosure comprise a combination of a compound of the disclosure and one or more additional therapeutic and/or prophylactic agent, both the compound and the additional agent can be present in a dose that is less than or equal to the dosage normally administered in a monotherapy regimen. The compositions of this disclosure may be co-formulated with one or more additional therapeutic or prophylactic agents, for example, in the form of a monolithic and/or bi/multi-layer tablet or may be administered separately from the therapeutic or prophylactic agent(s).

Pharmaceutical formulations may be adapted for administration by any appropriate route, for example by the oral (including buccal or sublingual), rectal, nasal, topical (including buccal, sublingual, or transdermal), vaginal, or parenteral (including subcutaneous, intracutaneous, intramuscular, intra-articular, intrasynovial, intrasternal, intrathecal, intralesional, intravenous, or intradermal injections or infusions) route. Such formulations may be prepared by any method known in the art of pharmacy, for example by bringing into association the active ingredient with the carrier(s) or excipient (s).

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil emulsions.

For instance, for oral administration in the form of a tablet or capsule, the active drug component can be combined with an oral, non-toxic pharmaceutically acceptable inert carrier such as ethanol, glycerol, water, and the like. Powders are prepared by comminuting the compound to a suitable fine size and mixing with a similarly comminuted pharmaceutical carrier such as an edible carbohydrate, as, for example, starch or mannitol. Flavoring, preservative, dispersing, and coloring agent can also be present.

Capsules are made by preparing a powder mixture, as described above, and filling formed gelatin sheaths. Glidants and lubricants such as colloidal silica, talc, magnesium stearate, calcium stearate, or solid polyethylene glycol can be added to the powder mixture before the filling operation. A disintegrating or solubilizing agent such as agar-agar, calcium carbonate, or sodium carbonate can also be added to improve the availability of the medicament when the capsule is ingested.

Moreover, when desired or necessary, suitable binders, lubricants, disintegrating agents, and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, and the like. Lubricants used in these dosage forms include sodium oleate, sodium chloride, and the like. Disintegrators include, without limitation, starch, methyl cellulose, agar, betonite, xanthan gum, and the like. Tablets are formulated, for example, by preparing a powder mixture, granulating or slugging, adding a lubricant and disintegrant, and pressing into tablets. A powder mixture is prepared by mixing the compound, suitable comminuted, with a diluent or base as described above, and optionally, with a binder such as carboxymethylcellulose, an aliginate, gelating, or polyvinyl pyrrolidone, a solution retardant such as paraffin, a resorption accelerator such as a quaternary salt and/or and absorption agent such as betonite, kaolin, or dicalcium phosphate. The powder mixture can be granulated by wetting with a binder such as syrup, starch paste, acadia mucilage, or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc, or mineral oil. The lubricated mixture is then compressed into tablets. The compounds of the present disclosure can also be combined with a free flowing inert carrier and compressed into tablets directly without going through the granulating or slugging steps. A clear or opaque protective coating consisting of a sealing coat of shellac, a coating of sugar or polymeric material, and a polish coating of wax can be provided. Dyestuffs can be added to these coatings to distinguish different unit dosages.

Oral fluids such as solution, syrups, and elixirs can be prepared in dosage unit form so that a given quantity contains a predetermined amount of the compound. Syrups can be prepared by dissolving the compound in a suitably flavored aqueous solution, while elixirs are prepared through the use of a non-toxic vehicle. Solubilizers and emulsifiers such as ethoxylated isostearyl alcohols and polyoxyethylene sorbitol ethers, preservatives, flavor additive such as peppermint oil or natural sweeteners, or saccharin or other artificial sweeteners, and the like can also be added.

Where appropriate, dosage unit formulations for oral administration can be microencapsulated. The formulation can also be prepared to prolong or sustain the release as for example by coating or embedding particulate material in polymers, wax, or the like.

The compounds of formula (I), and pharmaceutically acceptable salts thereof, can also be administered in the form of liposome delivery systems, such as small unilamellar vesicles, large unilamellar vesicles, and multilamellar vesicles. Liposomes can be formed from a variety of phopholipids, such as cholesterol, stearylamine, or phophatidylcholines.

The compounds of formula (I) and pharmaceutically acceptable salts thereof may also be delivered by the use of monoclonal antibodies as individual carriers to which the compound molecules are coupled. The compounds may also be coupled with soluble polymers as targetable drug carriers. Such polymers can include polyvinylpyrrolidone, pyran copolymer, polyhydroxyprppylmethacrylamidephenol, polyhydroxyethylaspartamidephenol, or polyethyleneoxidepolylysine substituted with palitoyl residues. Furthermore, the compounds may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates, and cross-linked or amphipathic block copolymers of hydrogels.

Pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6), 318 (1986).

Pharmaceutical formulations adapted for topical administration may be formulated as ointments, creams, suspensions, lotions, powders, solutions, pastes, gels, sprays, aerosols, or oils.

For treatments of the eye or other external tissues, for example mouth and skin, the formulations are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in oil base.

Pharmaceutical formulations adapted for topical administrations to the eye include eye drops wherein the active ingredient is dissolved or suspended in a suitable carrier, especially an aqueous solvent.

Pharmaceutical formulations adapted for topical administration in the mouth include lozenges, pastilles, and mouth washes.

Pharmaceutical formulations adapted for rectal administration may be presented as suppositories or as enemas.

Pharmaceutical formulations adapted for nasal administration wherein the carrier is a solid include a course powder which is administered in the manner in which snuff is taken, i.e., by rapid inhalation through the nasal passage from a container of the powder held close up to the nose. Suitable formulations wherein the carrier is a liquid, for administration as a nasal spray or nasal drops, include aqueous or oil solutions of the active ingredient.

Pharmaceutical formulations adapted for administration by inhalation include fine particle dusts or mists, which may be generated by means of various types of metered, dose pressurized aerosols, nebulizers, or insufflators.

Pharmaceutical formulations adapted for vaginal administration may be presented as pessaries, tampons, creams, gels, pastes, foams, or spray formulations.

Pharmaceutical formulations adapted for parenteral administration include aqueous and non-aqueous sterile injection solutions which may contain anti-oxidants, buffers, bacteriostats, and soutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules, and tablets.

It should be understood that in addition to the ingredients particularly mentioned above, the formulations may include other agents conventional in the art having regard to the type of formulation in question, for example those suitable for oral administration may include flavoring agents.

Table 1 below lists some illustrative examples of compounds that can be administered with the compounds of this disclosure. The compounds of the disclosure can be administered with other anti-HCV activity compounds in combination therapy, either jointly or separately, or by combining the compounds into a composition.

*Table 1*

| *Brand Name* | *Physiological Class* | *Type of Inhibitor or Target* | *Source Company* |
|---|---|---|---|
| NIM811 | | Cyclophilin Inhibitor | Novartis |
| Zadaxin | | Immunomodulator | Sciclone |
| Suvus | | Methylene blue | Bioenvision |
| Actilon (CPG10101) | | TLR9 agonist | Coley |

(continued)

| *Brand Name* | *Physiological Class* | *Type of Inhibitor or Target* | *Source Company* |
|---|---|---|---|
| Batabulin (T67) | Anticancer | β-tubulin inhibitor | Tularik Inc., South San Francisco, CA |
| ISIS 14803 | Antiviral | antisense | ISIS Pharmaceutical s Inc, Carlsbad, CA/Elan Phamaceuticals Inc., New York, NY |
| Summetrel | Antiviral | antiviral | Endo Pharmaceutical s Holdings Inc., Chadds Ford, PA |
| GS-9132 (ACH-806) | Antiviral | HCV Inhibitor | Achillion / Gilead |
| Pyrazolopyrimidine compounds and salts From WO-2005047288 26 May 2005 | Antiviral | HCV Inhibitors | Arrow Therapeutics Ltd. |
| Levovirin | Antiviral | IMPDH inhibitor | Ribapharm Inc., Costa Mesa, CA |
| Merimepodib (VX-497) | Antivirai | IMPDH inhibitor | Vertex Pharmaceutical s Inc., Cambridge, MA |
| XTL-6865 (XTL-002) | Antiviral | monoclonal antibody | XTL Biopharmaceuti cals Ltd., Rehovot, Isreal |
| Telaprevir (VX-950, LY-570310) | Antiviral | NS3 serine protease inhibitor | Vertex Pharmaceutical s Inc., Cambridge, MA/ Eli Lilly and Co. Inc., Indianapolis, IN |
| HCV-796 | Antiviral | NS5B Replicase Inhibitor | Wyeth/ Viropharma |
| NM-283 | Antiviral | NS5B Replicase Inhibitor | Idenix / Novartis |
| GL-59728 | Antiviral | NS5B Replicase Inhibitor | Gene Labs / Novartis |
| GL-60667 | Antiviral | NS5B Replicase Inhibitor | Gene Labs / Novartis |
| 2'C MeA | Antiviral | NS5B Replicase Inhibitor | Gilead |
| PSI 6130 | Antiviral | NS5B Replicase Inhibitor | Roche |
| R1626 | Antiviral | NS5B Replicase Inhibitor | Roche |
| 2'C Methyl adenosine | Antiviral | NS5B Replicase Inhibitor | Merck |
| JTK-003 | Antiviral | RdRp inhibitor | Japan Tobacco Inc., Tokyo, Japan |
| Levovirin | Antiviral | ribavirin | ICN Pharmaceutical s, Costa Mesa, CA |
| Ribavirin | Antiviral | ribavirin | Schering-Plough Corporation, Kenilworth, NJ |
| Viramidine | Antiviral | Ribavirin Prodrug | Ribapharm Inc., Costa Mesa, CA |
| Heptazyme | Antiviral | ribozyme | Ribozyme Pharmaceutical s Inc., Boulder, CO |
| BILN-2061 | Antiviral | serine protease inhibitor | Boehringer Ingelheim Pharma KG, Ingelheim, Germany |

(continued)

| *Brand Name* | *Physiological Class* | *Type of Inhibitor or Target* | *Source Company* |
|---|---|---|---|
| SCH 503034 | Antiviral | serine protease inhibitor | Schering Plough |
| Zadazim | Immune modulator | Immune modulator | SciClone Pharmaceutical s Inc., San Mateo, CA |
| Ceplene | Immunomodulator | immune modulator | Maxim Pharmaceutical s Inc., San Diego, CA |
| CellCept | Immunosuppressant | HCV IgG immunosuppressant | F. Hoffmann-La Roche LTD, Basel, Switzerland |
| Civacir | Immunosuppressant | HCV IgG immunosuppressant | Nabi Biopharmaceuti cals Inc., Boca Raton, FL |
| Albuferon - a | Interferon | albumin IFN-α2b | Human Genome Sciences Inc., Rockville, MD |
| Infergen A | Interferon | IFN alfacon-1 | InterMune Pharmaceutical s Inc., Brisbane, CA |
| Omega IFN | Interferon | IFN-ω | Intarcia Therapeutics |
| IFN-β and BMZ701 | Interferon | IFN-β and EMZ701 | Transition Therapeutics Inc., Ontario, Canada |
| Rebif | Interferon | IFN-β1a | Serono, Geneva, Switzerland |
| Roferon A | Interferon | IFN-α2a | F. Hoffmann-La Roche LTD, Basel, Switzerland |
| Intron A | Interferon | EFN-α2b | Schering-Plough Corporation, Kenilworth, NJ |
| Intron A and Zadaxin | Interferon | IFN-α2b/α1-thymosin | RegeneRx Biopharmiceuti cals Inc., Bethesda, MD/ SciClone Pharmaceutical s Inc, San Mateo, CA |
| Rebetron | Interferon | IFN-α2b/ribavirin | Schering-Plough Corporation, Kenilworth, NJ |
| Actimmune | Interferon | INF-γ | InterMune Inc., Brisbane, CA |
| Interferon-β | Interferon | Interferon-β-1a | Serono |
| Multiferon | Interferon | Long lasting IFN | Viragen/Valenti s |
| Wellferon | Interferon | lymphoblastoid IFN-αn1 | GlaxoSmithKli ne plc, Uxbridge, UK |
| Omniferon | Interferon | natural IFN-α | Viragen Inc., Plantation, FL |
| Pegasys | Interferon | PEGylated IFN-α2a | F. Hoffmann-La Roche LTD, Basel, Switzerland |
| Pegasys and Ceplene | Interferon | PEGylated IFN-α2a/ immune modulator | Maxim Pharmaceutical s Inc., San Diego, CA |
| Pegasys and Ribavirin | Interferon | PEGylated IFN-α2a/ribavirin | F. Hoffmann-La Roche LTD, Basel, Switzerland |
| PEG-Intron | Interferon | PEGylated IFN-α2b | Schering-Plough Corporation, Kenilworth, NJ |

(continued)

| Brand Name | Physiological Class | Type of Inhibitor or Target | Source Company |
|---|---|---|---|
| PEG-Intron / Ribavirin | Interferon | PEGylated IFN-$\alpha$2b/ribavirin | Schering-Plough Corporation, Kenilworth, NJ |
| IP-501 | Liver protection | antifibrotic | Indevus Pharmaceutical s Inc., Lexington, MA |
| IDN-6556 | Liver protection | caspase inhibitor | Idun Pharmaceutical s Inc., San Diego, CA |
| ITMN-191 (R-7227) | Antiviral | serine protease inhibitor | InterMune Pharmaceutical s Inc., Brisbane, CA |
| GL-59728 | Antiviral | NS5B Replicase Inhibitor | Genelabs |
| ANA-971 | Antiviral | TLR-7 agonist | Anadys |
| MK 78009 | Antiviral | serine protease inhibitor | Merck |
| TMC-435350 | Antiviral | serine protease inhibitor | Tibotec |

The compounds of the disclosure may also be used as laboratory reagents. Compounds may be instrumental in providing research tools for designing of viral replication assays, validation of animal assay systems and structural biology studies to further enhance knowledge of the HCV disease mechanisms. Further, the compounds of the present disclosure are useful in establishing or determining the binding site of other antiviral compounds, for example, by competitive inhibition.

The compounds of this disclosure may also be used to treat or prevent viral contamination of materials and therefore reduce the risk of viral infection of laboratory or medical personnel or patients who come in contact with such materials, e.g., blood, tissue, surgical instruments and garments, laboratory instruments and garments, and blood collection or transfusion apparatuses and materials.

This disclosure is intended to encompass compounds having formula (I) when prepared by synthetic processes or by metabolic processes including those occurring in the human or animal body (*in vivo)* or processes occurring *in vitro.*

The present disclosure will now be described in connection with certain embodiments which are not intended to limit its scope. On the contrary, the present disclosure covers all alternatives, modifications, and equivalents as can be included within the scope of the claims. Thus, the following examples, which include specific embodiments, will illustrate one practice of the present disclosure, it being understood that the examples are for the purposes of illustration of certain embodiments and are presented to provide what is believed to be the most useful and readily understood description of its procedures and conceptual aspects.

The abbreviations used in the present application, including particularly in the illustrative schemes and examples which follow, are well-known to those skilled in the art. Some of the abbreviations used are as follows: Me for methyl; DIBAL-H for diisobutylaluminum hydride; DCM for dichloromethane; Ph for phenyl; $Ph_3PMeBr$ for methyltriphenylphosphonium bromide; THF for tetrahydrofuran; h or hr or hrs for hours; EtOAc for ethyl acetate; min or mins for minutes; r.t. or RT for room temperature or retention time (context will dictate); Et for ethyl; $Et_3N$ or TEA for triethylamine; TMS for trimethylsilyl; TAS-F for tris(dimethylamino)sulfur(trimethylsilyl) difluoride; DMSO for N,N-dimethylsulfoxide; DMF for N, N-dimethylformamide; TFA for trifluoroacetic acid; HATU for for *O*-(7-azabenzotriazol-1-yl)-*N,N,N',N'*-tetramethyluronium phosphate; DCE for 1,2-dichloroethane; MeOH for methanol; DIEA for diisopropylethylamine; pTSA or TsOH for para-tolylsulfonic acid; MOMCl for methyl chloromethyl ether; TBAF for tetrabutylammonium fluoride; IBX for 2-iodoxybenzoic acid; Hex for hexanes; BuLi for butyllithium; Bu for butyl; NBS for N-bromosuccinimide; LAH for lithium aluminum hydride; $Et_2O$ for diethyl ether, and MsCl for methanesulfonyl chloride.

The starting materials useful to synthesize the compounds of the present disclosure are known to those skilled in the art and can be readily manufactured or are commercially available.

The following methods set forth below are provided for illustrative purposes and are not intended to limit the scope of the claims. It will be recognized that it may be necessary to prepare such a compound in which a functional group is protected using a conventional protecting group then to remove the protecting group to provide a compound of the present disclosure. The details concerning the use of protecting groups in accordance with the present disclosure are known to those skilled in the art.

*Preparation of Intermediate 1:*

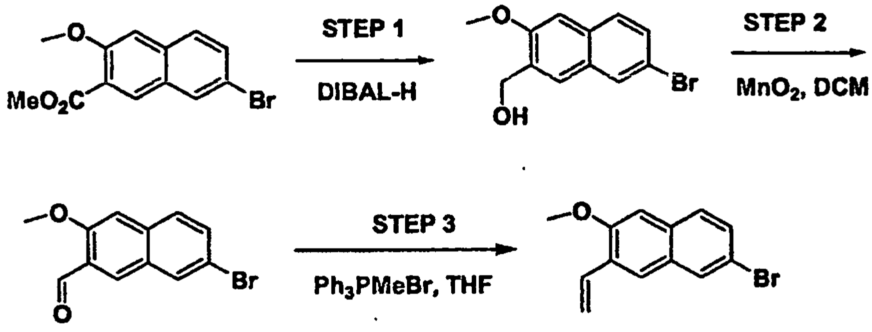


Intermediate 1

*Step 1:*

Diisobutylaluminum hydride (1.0M in hexanes) (112 mL, 112 mmol) was added slowly to a solution of methyl 7-bromo-3-methoxy-2-naphthoate (made in 3 steps from 3-hydroxy-2-naphthoic acid according to ref: J. Med. Chem. 1990, 33, 171) (11 g, 37.3 mmol) in THF (300 mL) at -40 °C (acetonitrile/dry ice). After the addition the reaction was stirred for 3 hrs. and then EtOAc (100 mL) was added and the ice bath removed. After 5 min. 1.0M HCl solution (200 mL) was added and stirred for 10min. The organics were washed with 1.0M HCl solution and brine and then dried with $MgSO_4$, filtered and concentrated to give (7-bromo-3-methoxynaphthalen-2-yl)methanol as a light yellow solid. $^1H$ NMR (500 MHz, CHLOROFORM-*d*) d ppm 2.34 (t, *J*=6.56 Hz, 1 H) 3.98 (s, 3 H) 4.83 (d, *J*=6.41 Hz, 2 H) 7.11 (s, 1 H) 7.51 (dd, *J*=8.70, 1.98 Hz, 1 H) 7.61 (d, *J*=8.85 Hz, 1 H) 7.66 (s, 1 H) 7.93 (d, *J*=1.83 Hz, 1 H).

*Step 2:*

Manganese dioxide (32.5 g, 374 mmol) was added to a solution of (7-bromo-3-methoxynaphthalen-2-yl)methanol (10 g, 37.3 mmol) in DCM (400 mL) and stirred at r.t. for 7 days. The reaction was filtered through diatomaceous earth (Celite®) and concentrated to give 7-bromo-3-methoxy-2-naphthaldehyde (9.2 g, 93% for the 2 steps) as a yellow solid. $^1H$ NMR (500 MHz, CHLOROFORM-*d*) d ppm 4.02 (s, 3 H) 7.16 (s, 1 H) 7.55 - 7.65 (m, 2 H) 8.01 (s, 1 H) 8.24 (s, 1 H) 10.57 (s, 1 H).

*Step 3:*

Sodium hydride (60% disp. in oil) (5.55 g, 139 mmol) was added to a solution of methyltriphenylphosphonium bromide (24.79 g, 69.4 mmol) in THF (100 mL) at 0 °C and stirred for 30 min. A solution of 7-bromo-3-methoxy-2-naphthaldehyde (9.2 g, 34.7 mmol) in THF (100 mL) was added and the reaction was allowed to warm to r.t. overnight. The reaction was diluted with ether and filtered through diatomaceous earth (Celite®). The filtrate was concentrated and purified by flash chromatography on the Biotage (1-5% EtOAc in hexanes) to give 6-bromo-2-methoxy-3-vinylnaphthalene (3.2 g, 35%) as a white solid. $^1H$ NMR (500 MHz, CHLOROFORM-*d*) d ppm 3.96 (s, 3 H) 5.40 (dd, *J*=10.99,1.53 Hz, 1 H) 5.90 (dd, *J*=17.55, 1.37 Hz, 1 H) 7.07 (s, 1 H) 7.13 (dd, *J*=17.55, 11.44 Hz, 1 H) 7.47 (dd, *J*=8.70, 1.98 Hz, 1 H) 7.58 (d, *J*=8.85 Hz, 1 H) 7.79 (s, 1 H) 7.92 (d, *J*=1.83 Hz, 1 H).

*Preparation of Intermediate 2:*

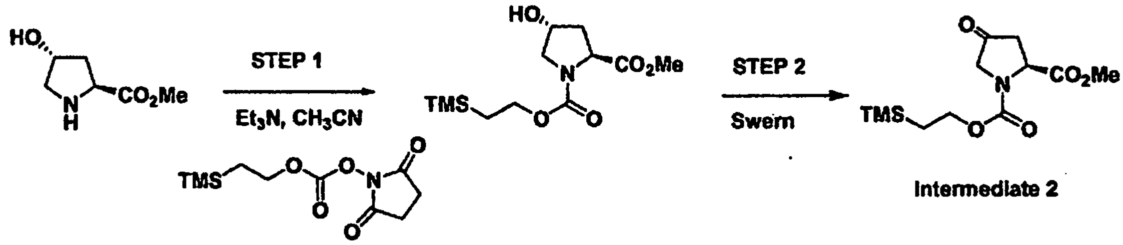


Intermediate 2

*Step 1:*

2,5-Dioxopyrrolidin-1-yl 2-(trimethylsilyl)ethyl carbonate (2.5 g, 9.64 mmol) was added to a solution of (2S,4R)-methyl 4-hydroxypyrrolidine-2-carboxylate, HCl salt (2.10 g, 11.6 mmol) and triethylamine (4.0 mL, 28.9 mmol) in acetonitrile (20 mL) and stirred at r.t. overnight. The reaction was quenched with water and ether. The organic layer was washed with 1.0M HCl (2x) and then brine. It was then dried with $MgSO_4$, filtered and concentrated to give crude (2S,4R)-2-methyl 1-(2-(trimethylsilyl)ethyl) 4-hydroxypyrrolidine-1,2-dicarboxylate which was used directly in the next step.

*Step 2:*

To solution of dimethyl sulfoxide (3.5 mL, 48.7 mmol) in DCM (100 mL) at - 78 °C was added oxalyl chloride (2.3 mL, 24.3 mmol) dropwise. The formed solution was stirred at this temperature for 30 min. A solution of (2S,4R)-2-methyl 1-(2-(trimethylsilyl)ethyl) 4-hydroxypyrrolidine-1,2-dicarboxylate (3.2 g, 11.6 mmol) in DCM (10 mL) was added at -78 °C. The formed slurry was stirred at -78 °C for 1 hr before addition of Hunig's base (9.6 mL, 55.3 mmol) dropwise. This solution was stirred at room temperature 30 min. and then washed with 1M HCl and brine, dried over $MgSO_4$, filtered and concentrated. The residual light brown oil was purified by flash chromatography on the Biotage (20-33% EtOAc in hexanes) to give (S)-2-methyl 1-(2-(trimethylsilyl)ethyl) 4-oxopyrrolidine-1,2-dicarboxylate (2.4 g, 76%) as a light yellow oil. $^1H$ NMR (400 MHz, DMSO-$d_6$) d ppm 0.03 (s, 9 H) 0.81 - 1.05 (m, 2 H) 2.52 = 2.73 (m, 1 H) 2.99 - 3.25 (m, 1 H) 3.66 (s, 3 H) 3.68 - 3.79 (m, 1 H) 3.81 - 3.98 (m, 1 H) 4.05 - 4.22 (m, 2 H) 4.62 - 4.80 (m, 1 H).

*Preparation of Intermediate 3*:

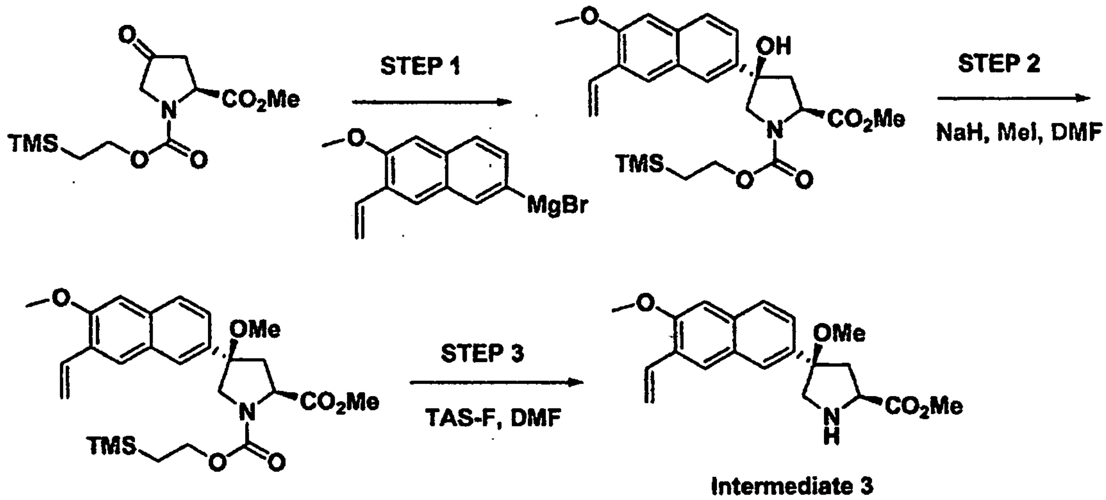


Intermediate 3

*Step 1:*

Magnesium (0.443 g, 18.24 mmol) was stirred in a round bottom flask under nitrogen ovenight. THF (4 mL) was added to the magnesium as well as a drop of 1,2-dibromoethane. This was heated to 60 °C and after 10 min. at this temperature a solution of 6-bromo-2-methoxy 3-vinylnaphthalene (Intermediate 1) (3.2 g, 12.16 mmol) in THF (20 mL) was added over 30 min. After the addition the reaction had turned light brown and stirring was continued at 70 °C for 2 hrs. The Grignard solution (20.8 mL, 10.40 mmol) was added to a solution of (S)-2-methyl 1-(2-(trimethylsilyl)ethyl)

4-oxopyrrolidine-1,2-dicarboxylate (Intermediate 2) (2.3 g, 8.00 mmol) in toluene (40 mL) at 0°C and stirred for 1 hr and then quenched with sat. $NH_4Cl$ solution. The aqueous layer was extracted with DCM and the combined organics were dried over $MgSO_4$, filtered and concentrated to give crude material. The crude material was purified by flash chromatography on the Biotage (20-40% EtOAc in hexanes) to give (2S,4R)-2-methyl 1-(2-(trimethylsilyl)ethyl) 4-hydroxy-4-(6-methoxy-7-vinylnaphthalen-2-yl)pyrrolidine-1,2-dicarboxylate (1.41 g, 37%) as a white solid. $^1$H NMR (400 MHz, CHLOROFORM-*d*) d ppm 0.05 (s, 9 H) 0.95 - 1.09 (m, 2 H) 2.36 - 2.55 (m, 1 H) 2.67 - 2.83 (m, 1 H) 3.79 - 3.87 (m, 4 H) 3.90 - 4.04 (m, 4 H) 4.16 - 4.30 (m, 2 H) 4.49 - 4.68 (m, 1 H) 5.38 (dd, *J*=11.04, 1.51 Hz, 1 H) 5.89 (dd, *J*=17.82, 1.51 Hz, 1 H) 7.08 (s, 1 H) 7.14 (dd, *J*=17.69, 11.17 Hz, 1 H) 7.47 (d, *J*=8.53 Hz, 1 H) 7.70 (d, *J*=8.53 Hz, 1 H) 7.88 (s, 2 H).

*Step 2:*

NaH (60% in oil) (0.229 g, 5.72 mmol) was added to a solution of (2S,4R)-2-methyl 1-(2-(trimethylsilyl)ethyl) 4-hydroxy-4-(6-methoxy-7-vinylnaphthalen-2-yl)pyrrolidine-1,2-dicarboxylate (1.5 g, 3.18 mmol) and methyl iodide (0.36 mL, 5.72 mmol) at 0°C in DMF (30 mL) and stirred at this temperature for 3 hrs. The reaction was then quenched with sat. $NH_4Cl$ solution and ether. The ether layer was washed with brine, dried over $MgSO_4$, filtered and concentrated to give crude material. The crude material was purified by flash chromatography on the Biotage (20-40% EtOAc in hexanes) to give (2S,4R)-2-methyl 1-(2-(trimethylsilyl)ethyl) 4-methoxy-4-(6-methoxy-7-vinylnaphthalen-2-yl)pyrrolidine-1,2-dicarboxylate (1.1 g, 71%) as a white foam. LCMS: r.t. = 1.956 min., $[M+Na]^+$ = 508 Phenomenex Luna C18 10u (3 x50 mm); Solvent A =10% acetonitrile - 90% water - 0.1% TFA, Solvent B = 90% acetonitrile - 10% water - 0.1% TFA; gradient = 0% to 100% solvent B over 2 min. and then hold for 1 min. ; 4 mL/min; inj. vol. = 5 ul; wavelength = 220 nm.

*Step 3:*

Tris(dimethylamino)sulfur(trimethylsilyl)difluoride (2.27 g, 8.24 mmol) was added to a solution of (2S,4R)-2-methyl 1-(2-(trimethylsilyl)ethyl) 4-methoxy-4-(6-methoxy-7-vinynaphthalen-2-yl)pyrrolidine-1,2-dicarboxylate (1 g, 2.06 mmol) in DMF (10 mL) at 0°C and then allowed to warm to r.t. overnight. The reaction was poured into sat. $NaHCO_3$ solution and extracted with ether and then DCM. The combined organics were washed with brine, dried over $MgSO_4$, filtered and concentrated to give (2S,4R)-methyl 4-methoxy-4-(6-methoxy-7-vinylnaphthalen-2-yl)pyrrolidine-2-carboxylate (520 mg, 74%) as a white foam. $^1$H NMR (500 MHz, CHLOROFORM-*d*) d ppm 2.49 (dd, *J*=13.43, 9.77 Hz, 1 H) 2.69 (d, *J*=13.43 Hz, 1 H) 2.81 (s, 3 H) 3.04 (d, *J*=11.90 Hz, 1 H) 3.53 (d, *J*=12.21 Hz, 1 H) 3.75 (s, 3 H) 3.89 (s, 3 H) 3.90 - 3.95 (m, 1 H) 5.31 (d, *J*=11.29 Hz, 1 H) 5.84 (d, *J*=17.70 Hz, 1 H) 7.04 (s, 1 H) 7.08 (dd, *J*=17.70, 10.99 Hz, 1 H) 7.35 (d, *J*=8.24 Hz, 1 H) 7.59-7.68 (m, 2 H) 7.83 (s, 1 H) 7.94 (s, 1 H).

*Preparation of Compound 1*

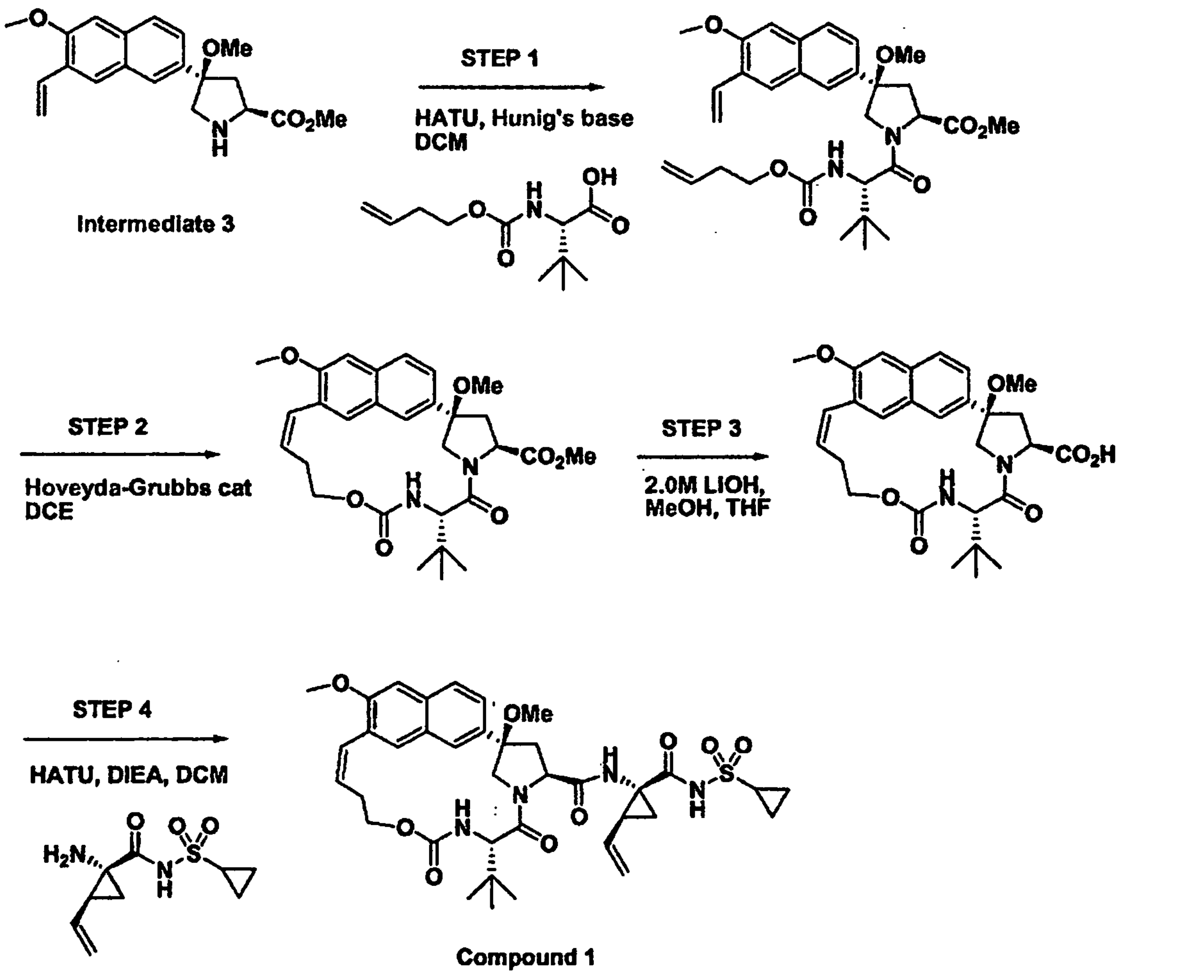


Compound 1

*Step 1:*

HATU (120 mg, 0.316 mmol) was added to a solution of (2S,4R)-methyl 4-methoxy-4-(6-methoxy-7-vinylnaphthalen-2-yl)pyrrolidine-2-carboxylate (90 mg, 0.264 mmol), (S)-2-((but-3-enyloxy)carbonylamino)-3,3-dimethylbutanoic acid (66 mg, 0.290 mmol) and Hunig's base (0.14 mL, 0.791 mmol) in DCM (4 mL) and stirred at r.t. overnight. The reaction was concentrated and purified by flash chromatography on the Biotage (10-40% EtOAc in hexanes) to give (2S,4R)-methyl 1-((S)-2-((but-3-enyloxy)carbonylamino)-3,3-dimethylbutanoyl)-4-methoxy-4-(6-methoxy-7-vinylnaphthalen-2-yl)pyrrolidine-2-carboxylate (94 mg, 0.17 mmol, 64.5% yield) as a colorless oil. $^1H$ NMR (400 MHz, CHLOROFORM-*d*) δ ppm 0.89 - 0.94 (m, 1 H) 0.95 - 0.99 (m, 1 H) 1.10 -1.19 (m, 7 H) 1.28 - 1.34 (m, 1 H) 2,30 - 2.40 (m, 2 H) 2.52 - 2.62 (m, 1 H) 2.83 - 2.97 (m, 4 H) 3.74 - 3.79 (m, 3 H) 3.93 - 4.07 (m, 4 H) 4.15 - 4.25 (m, 2 H) 4.28 - 4.41 (m, 1 H) 4.75 - 4.85 (m, 1 H) 5.01 - 5.16 (m, 2 H) 5.35 - 5.47 (m, 2 H) 5.69 - 5.84 (m, 1 H) 5.86 - 5.94 (m, 1 H) 7.08 - 7.19 (m, 2 H) 7.38 - 7.44 (m, 1 H) 7.65 - 7.76 (m, 2 H) 7.86 - 7.96 (m, 1 H). LCMS: r.t. = 2.11 min., $[M+H]^+$ = 553 Phenomenex Luna S10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. = 10 ul; wavelength = 220 nm.

*Step 2:*

A solution of (2S,4R)-methyl 1-((S)-2-((but-3-enyloxy)carbonylamino)-3,3-dimethylbutanoyl)-4-methoxy-4-(6-methoxy-7-vinylnaphthalen-2-yl)pyrrolidine-2-carboxylate (94 mg, 0.170 mmol) in DCE (50 mL) was sparged with nitrogen for 30 min. and then Hoveyda-Grubbs Catalyst 2nd Generation (11 mg, 0.417 mmol) was added and the reaction sealed and heated to 80°C overnight. The reaction was concentrated to give crude material. The crude material was purified by flash chromatography on the Biotage (10-40% EtOAc in hexanes) to give the product (12 mg, 0.023 mmol, 13% yield) as a white solid. $^1H$ NMR (500 MHz, CHLOROFORM-*d*) δ ppm 1.08 - 1.21 (m, 9 H) 2.33 - 2.49 (m, 1 H) 2.53 - 2.65 (m, 1 H) 2.70 (dd, *J*=12.05, 7.78 Hz, 1 H) 3.06 - 3.19 (m, 3 H) 3.67 - 3.80 (m, 5 H) 3.93 - 4.02 (m, 4 H) 4.02 - 4.11 (m, 1 H) 4.73 - 4.88 (m, 1 H) 4.98 - 5.10 (m, 1 H) 5.60 (d, *J*=10.07 Hz, 1 H) 5.78 - 5.93 (m, 1 H) 6.87 - 7.04 (m, 1H) 7.06

- 7.22 (m, 2 H) 7.48 - 7.56 (m, 1 H) 7.65 - 8.00 (m, 3 H). LCMS: r.t. = 2.02 min., $[M+H]^+$ = 525 Phenomenex Luna S10 (3 x50 mm); Solvent A = 95% water - 5% methanol-10mM ammonium acetate, Solvent B = 5% water - 95% methanol -10 mM ammonium acetate; gradient = 0% to 100% solvent B over 2 min. and then hold for 1 min.; 4 mL/min; inj. vol. = 10 ul; wavelength = 220 nm.

*Step 3:*

2.0M LiOH (0.04 mL, 0.076 mmol) was added to a solution of the product from Step 2 (8 mg, 0.015 mmol) in THF (0.5 mL) and MeOH (0.5 mL) and stirred at r.t. overnight. The reaction was quenched with sat. $NH_4Cl$ solution and ether. The organics were dried, filtered and concentrated to give the crude product (5 mg, 64%) which was used directly in the next step. LCMS: r.t. = 1.42 min., $[M+H]^+$ = 511 Phenomenex Luna C18 10u (3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 4:*

HATU (4 mg, 10.8 μmol) was added to a solution of (1R,2S)-1-amino-N-(cyclopropylsulfonyl)-2-vinylcyclopropanecarboxamide, pTSA salt (6 mg, 0.015 mmol), the product from Step 3 (5 mg, 9.79 μmol) and Hunig's base (5 μL, 0.029 mmol) in dichloromethane (0.5 mL) and stirred at r.t. for 4 hrs. The reaction was concentrated and then purified by prep. HPLC (Sunfire C 18 10u (30 x100 mm); flow = 42 mL/min; solvent gradient = 95:5 to 5:95 water/acetonitrile (with 10mM ammonium acetate)) to give Compound 1 (4 mg, 55%) as a white solid. $^1H$ NMR (500 MHz, CHLOROFORM-*d*) d ppm 0.95 - 1.05 (m, 2 H) 1.10 (br. s., 9 H) 1.26 - 1.47 (m, 3 H) 1.89 - 1.99 (m, 1 H) 1.99 - 2.05 (m, 1 H) 2.46 - 2.55 (m, 1 H) 2.57 - 2.68 (m, 2 H) 2.68 - 2.77 (m, 1 H) 2.82 - 2.97 (m, 1 H) 3.15 (s, 3 H) 3.60 - 3.69 (m, 1 H) 3.76 (d, *J*=10.38 Hz, 1 H) 3.97 (s, 3 H) 4.02 - 4.12 (m, 1 H) 4.86 (d, *J*=10.07 Hz, 1 H) 4.99 - 5.16 (m, 3 H) 5.20 (d, *J*=18.31 Hz, 1 H) 5.62 - 5.77 (m, 2 H) 5.77 - 5.91 (m, 1 H) 6.55 (br. s., 1 H) 6.90 (d, *J*=11.90 Hz, 1H) 7.09 (s, 1 H) 7.51 (d, *J*=7.63 Hz, 1 H) 7.71 - 7.84 (m, 2 H) 8.07 (br. s., 1 H). LCMS: r.t. = 1.64 min., $[M+Na]^+$ = 745 Phenomenex Luna C18 10u (3 x50 mm); Solvent A = 10% acetonitrile - 90% water - 0.1 % TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Preparation of Compound 2*

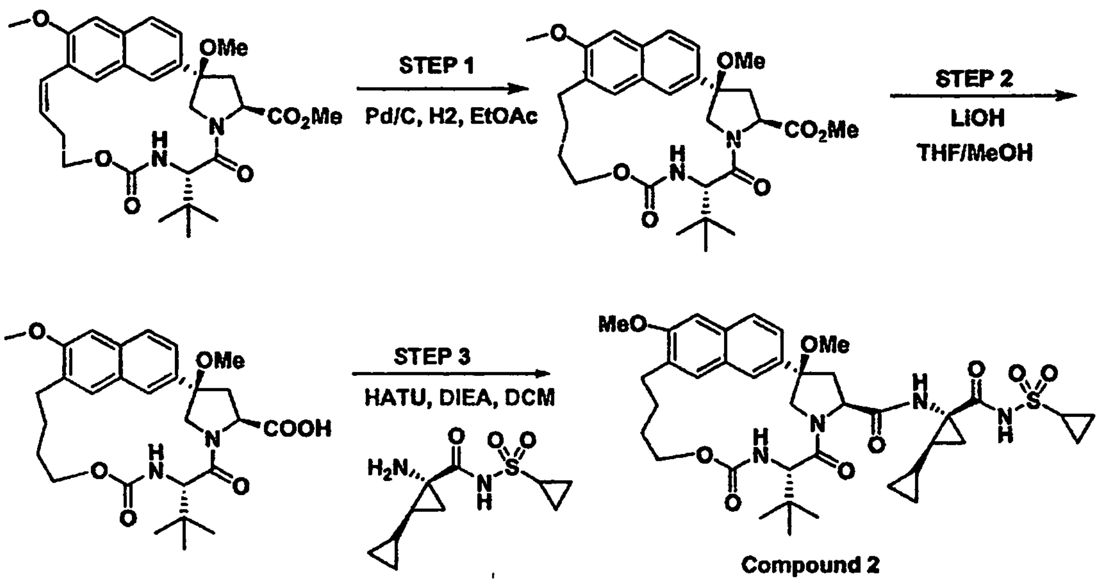


Compound 2

*Step 1:*

A solution of the product from Compound 1 Step 2 (12 mg, 0.023 mmol) and 10% Pd/C (3 mg, 2.3 μmol) in EtOAc (2 mL) was stirred under an atmosphere of hydrogen overnight. The reaction was filtered through a nylon frit and concentrated to give crude product (11 mg, 0.021 mmol, 91% yield) as a white solid. LCMS: r.t. = 2.02 min., $[M+H]^+$ =

527 Phenomenex Luna S10 (3 x50 mm); Solvent A = 95% water - 5% methanol-10mM ammonium acetate, Solvent B = 5% water - 95% methanol -10 mM ammonium acetate; gradient = 0% to 100% solvent B over 2 min. and then hold for 1 min.; 4 mL/min; inj. vol. = 10 ul; wavelength = 220 nm.

*Step 2:*

2.0 M lithium hydroxide (0.052 mL, 0.104 mmol) was added to a solution of the product from Step 1 (11 mg, 0.021 mmol) in THF (0.5 mL) and MeOH (0.5 mL) and stirred for 3 hours. The reaction was diluted with ether and 1.0 M HCl. The organic layer was dried, filtered and concentrated to give the crude product (10 mg, 0.02 mmol, 93% yield) as a white solid. LCMS: r.t. = 1.69 min., $[M+H]^+$ = 513 Phenomenex Luna S10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. = 10 ul; wavelength = 220 nm.

*Step 3:*

HATU (11.13 mg, 0.029 mmol) was added to a solution of (1S,2R)-2-amino-N-(cyclopropylsulfonyl)bi(cyclopropane)-2-carboxamide, HCl salt (6.5 mg, 0.023 mmol), the product from Step 2 (10 mg, 0.020 mmol) and Hunig's base (10 μL, 0.059 mmol) in dichloromethane (1 mL) and stirred at r.t. overnight. The reaction was concentrated and purified by HPLC (Xbridge C18 10u (30 x100 mm); flow = 42 mL/min; solvent gradient = 95:5 to 5:95 water/acetonitrile (with 10mM ammonium acetate)). The product fractions were concentrated to give Compound 2 (2.7 mg, 3.65 μmol, 19 % yield) as a white solid. $^1H$ NMR (400 MHz, MeOD) δ ppm 0.20 - 0.30 (m, 2 H) 0.41 - 0.57 (m, 2 H) 0.79 - 0.89 (m, 1 H) 0.91 - 1.03 (m, 3 H) 1.04 - 1.14 (m, 10 H) 1.15 - 1.22 (m, 2 H) 1.40 -1.54 (m, 1 H) 1.70 (dd, *J*=7.91, 5.14 Hz, 1 H) 2.02 - 2.09 (m, 1 H) 2.09 - 2.24 (m, 2 H) 2.34 - 2.44 (m, 1 H) 2.44 - 2.52 (m, I H) 2.56 - 2.69 (m, 1 H) 2.87 - 2.98 (m, 2 H) 3.14 - 3.19 (m, 3 H) 3.78 - 3.90 (m, 3 H) 3.93 (s, 3 H) 4.72 - 4.80 (m, 2 H) 4.94 - 5.01 (m, 2 H) 7.17 (s, 1 H) 7.35 (d, *J*=1.76 Hz, 1 H) 7.47 (s, 1 H) 7.51 (dd, *J*=8.66, 1.88 Hz, 1 H) 7.81 (d, *J*=8.53 Hz, 1 H). LCMS: r.t. =1.87 min., $[M-OMe]^+$ = 707 Phenomenex Luna S10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. = 10 ul; wavelength = 220 nm.

*Preparation of Compound 3*

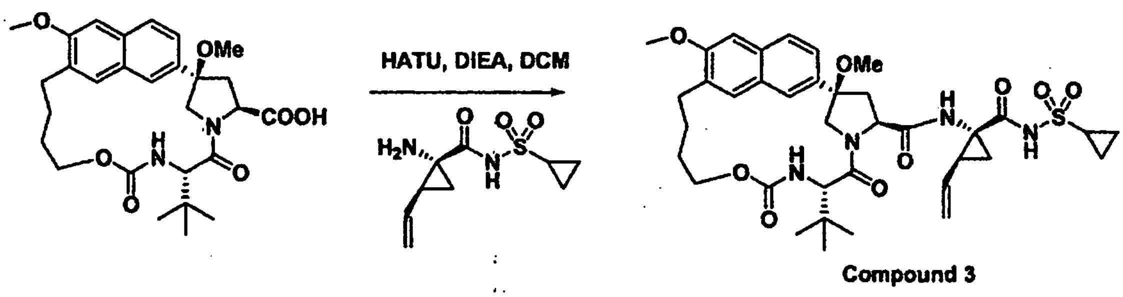


Compound 3

Compound 3 was made using the same procedure as Compound 2 Step 3 except using (1R,2S)-1-amino-N-(cyclopropylsulfonyl)-2-vinylcyclopropanecarboxamide, TsOH salt. $^1H$ NMR (400 MHz, CHLOROFORM-*d*) δ ppm 1.00 (d, *J*=8.03 Hz, 2 H) 1.05 - 1.13 (m, 9 H) 1.26 - 1.34 (m, 2 H) 1.41 (dd, *J*=9.29, 5.52 Hz, 1 H) 1.93 (dd, *J*=8.16, 5.65 Hz, 1 H) 1.97 - 2.05 (m, 1 H) 2.60 - 2.69 (m, 2 H) 2.87 (td, *J*=8.41, 4.02 Hz, 1 H) 2.93 - 2.98 (m, 1 H) 3.15 (s, 3 H) 3.50 (s, 3 H) 3.74 - 3.82 (m, 2 H) 3.87 (dd, *J*=10.92, 7.15 Hz, 1 H) 3.94 (s, 3 H) 4.76 - 4.87 (m, 3 H) 5.12 (dd, *J*=10.42, 1.38 Hz, 1 H) 5.21 (d, *J*=17.07 Hz, 1 H) 5.73 (ddd, *J*=17.13, 10.23, 8.53 Hz, 1 H) 5.81 (d, *J*=10.04 Hz, 1 H) 6.86 (br. s, 1H) 7.05 (s, 1 H) 7.32 (d, *J*=1.51 Hz, 1 H) 7.49 (dd, *J*=8.53, 2.0) Hz, 1 H) 7.53 (s, 1 H) 7.76 (d,*J*=8.78 Hz, 1 H). LCMS: r.t. = 4.23 min., $[M-OMe]^+$ = 731 Phenomenex Luna C18, 50 x 2, 3u; Solvent A = 10% acetonitrile - 90% water - 0.1 % TFA, Solvent B = 90% acetonitrile - 10% water - 0.1% TFA; gradient = 0% to 100% solvent B over 4 min. and then hold for 1 min.; 0.8 mL/min; inj. vol. = 3 ul; wavelength = 220 nm.

*Preparation of Compound 4*

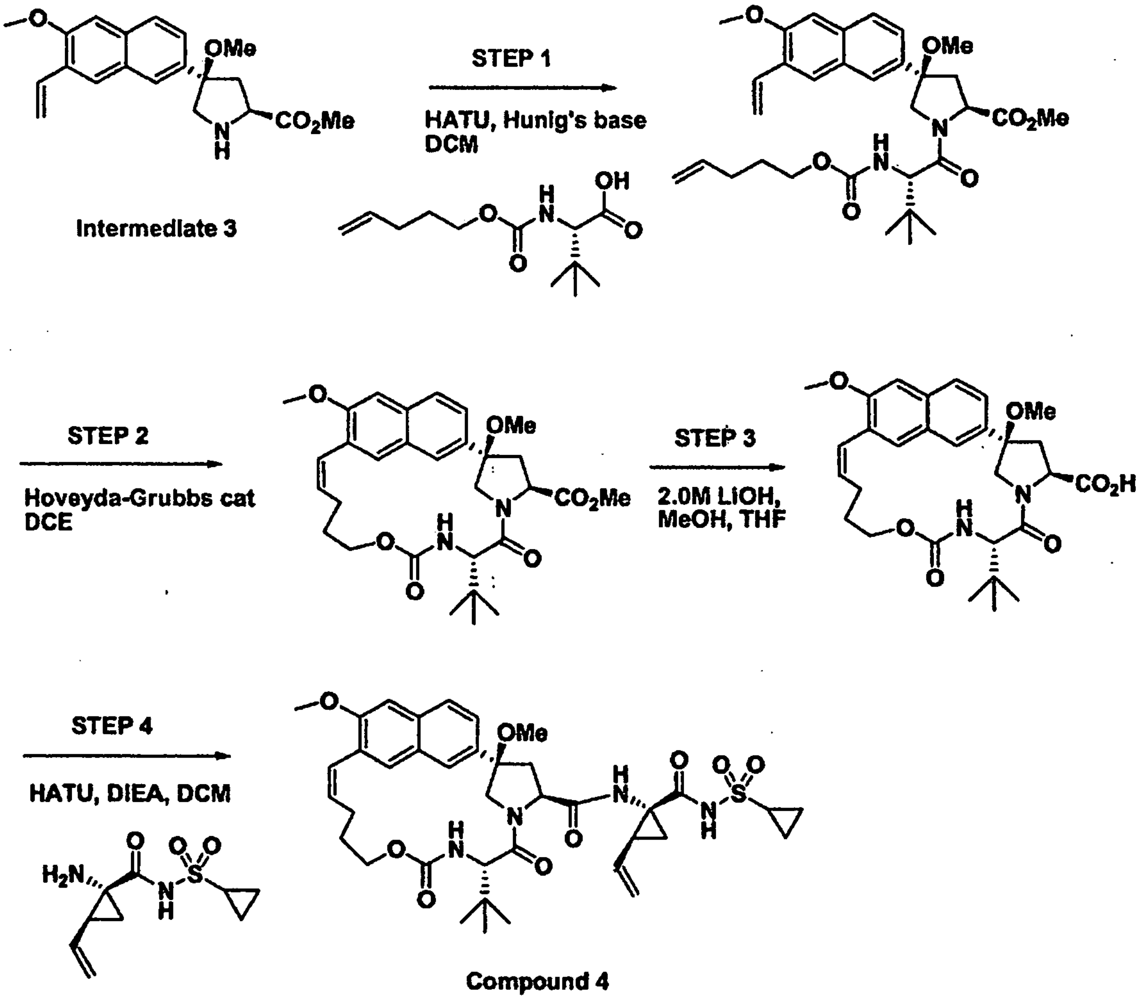


Compound 4

*Step 1:*

HATU (120 mg, 0.316 mmol) was added to a solution of (2S,4R)-methyl 4-methoxy-4-(6-methoxy-7-vinylnaphthalen-2-yl)pyrrolidine-2-carboxylate (90 mg, 0.264 mmol), (S)-3,3-dimethyl-2-((pent-4-enyloxy)carbonylamino)butanoic acid (70.6 mg, 0.290 mmol) and Hunig's base (0.138 mL, 0.791 mmol) in DCM (4 mL) and stirred at r.t. overnight. The reaction was concentrated and purified by flash chromatography on the Biotage (10-40% EtOAc in hexanes) to give (2S,4R)-methyl 1-((S)-3,3-dimethyl-2-((pent-4-enyloxy)carbonylamino)butanoyl)-4-methoxy-4-(6-methoxy-7-vinylnaphthalen-2-yl)pyrrolidine-2-carboxylate (95 mg, 0.168 mmol, 63.6% yield) as colorless oil. $^1$H NMR (400 MHz, CHLOROFORM-*d*) δ ppm 0.88 - 1.02 (m, 2 H) 1.10 -1.18 (m, 7 H) 1.26 -1.35 (m, 1 H) 1.63 -1.83 (m, 1 H) 2.05 - 2.19 (m, 2 H) 2.53 - 2.63 (m, 1 H) 2.83 - 2.99 (m, 4 H) 3.73 - 3.81 (m, 3 H) 3.94 - 4.04 (m, 1 H) 3.97 (s, 3 H) 4.04 - 4.12 (m, 1 H) 4.15 - 4.26 (m, 2 H) 4.38 (d, *J*=9.79 Hz, 1 H) 4.74 - 4.88 (m, 1 H) 4.92 - 5.07 (m, 2 H) 5.35 - 5.47 (m, 2 H) 5.71 - 5.86 (m, *J*=17.22, 10.32, 6.68, 6.68 Hz, 1 H) 5.87 - 5.98 (m, 1 H) 7.08 - 7.21 (m, 2 H) 7.38 - 7.46 (m, 1 H) 7.64 - 7.80 (m, 2 H) 7.94 (s, 1 H). LCMS: r.t. = 2.15 min., $[M+H]^+$ = 567 Phenomenex Luna S 10 (3 x50 mm); Solvent A = 95% water - 5% methanol-10mM ammonium acetate, Solvent B = 5% water - 95% methanol -10 mM ammonium acetate; gradient = 0% to 100% solvent B over 2 min. and then hold for 1 min.; 4 mL/min; inj. vol. = 10 ul; wavelength = 220 nm.

*Step 2:*

A solution of (2S,4R)-methyl 1-((S)-3,3-dimethyl-2-((pent-4-enyloxy)carbonylamino)butanoyl)-4-methoxy-4-(6-methoxy-7-vinylnaphthalen-2-yl)pyrrolidine-2-carboxylate (95 mg, 0.168 mmol) in DCE (50 mL) was sparged with nitrogen for 30 min. and then Hoveyda-Grubbs Catalyst 2nd Generation (10.5 mg, 0.017 mmol) was added and the reaction sealed and heated to 80°C overnight. The reaction was concentrated to give crude material. The crude material was purified by flash chromatography on the Biotage (10-40% EtOAc in hexanes) to give the purified product (46 mg, 0,085 mmol, 51% yield) as a white solid. $^1$H NMR (400 MHz, CHLOROFORM-*d*) δ ppm 1.09 - 1.18 (m, 9 H) 2.06 - 2.19 (m, 1 H) 2.35 - 2.47 (m, 1 H) 2.64 - 2.82 (m, 2 H) 3.02 - 3.15 (m, 3 H) 3.69 - 3.78 (m, 5 H) 3.95 (d, *J*=1.25 Hz, 3 H) 3.96 - 4.09

(m, 2 H) 4.72 - 4.87 (m, 2 H) 4.99 - 5.10 (m, 1 H) 5.61 (dd, *J*=9.79, 2.76 Hz, 1 H) 5.67 - 5.86 (m, 1 H) 6.66 - 6.89 (m, 1 H) 7.09 (d, *J*=9.03 Hz, 1 H) 7.41 - 7.56 (m, 2 H) 7.65 - 8.00 (m, 3 H). LCMS: r.t. = 2.04 min., $[M+H]^+$ = 539 Phenomenex Luna S10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. = 10 ul; wavelength = 220 nm.

*Step 3:*

2.0M LiOH (0.09 mL, 0.186 mmol) was added to a solution of the product from Step 2 (20 mg, 0.037 mmol) in THF (0.5 mL) and MeOH (0.5 mL) and stirred at r.t. overnight. The reaction was quenched with sat. $NH_4Cl$ solution and ether. The organics were dried, filtered and concentrated to give the crude product (17 mg, 87%) which was used directly in the next step. LCMS: r.t. = 1.42 min., $[M+Na]^+$ = 547 Phenomenex Luna C18 10u (3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 4:*

HATU (14 mg, 0.036 mmol) was added to a solution of (1R,2S)-1-amino-N-(cyclopropylsulfonyl)-2-vinylcyclopropanecarboxamide, pTSA salt (20 mg, 0.049 mmol), the product from Step 3 (17 mg, 0.032 mmol) and Hunig's base (0.017 mL, 0.097 mmol) in dichloromethane (0.5 mL) and stirred at r.t. for 4 hrs. The reaction was concentrated and then purified by prep. HPLC (Sunfire C18 10u (30 x100 mm); flow = 42 mL/min; solvent gradient = 95:5 to 5:95 water/ acetonitrile (with 10mM ammonium acetate)) to give Compound 4 (12 mg, 49%) as a white solid. $^1H$ NMR (500 MHz, CHLOROFORM-*d*) d ppm 0.94 - 1.04 (m, 2 H) 1.09 (s, 9 H) 1.21 - 1.36 (m, 2 H) 1.41 -1.49 (m, 1 H) 1.86 -1.98 (m, 2 H) 1.98 - 2.13 (m, 2 H) 2.17 (dd, *J*=12.97, 5.65 Hz, 1 H) 2.48 - 2.57 (m, 1 H) 2.62 (t, *J*=11.60 Hz, 1 H) 2.69 - 2.81 (m, 1 H) 2.82 - 2.94 (m, 1 H) 3.06 (s, 3 H) 3.67 - 3.74 (m, 1 H) 3.77 (d, *J*=10.68 Hz, 1 H) 3.86 - 3.94 (m, 1 H) 3.96 (s, 3 H) 4.68 - 4.83 (m, 2 H) 4.96 (d, *J*=10.38 Hz, 1 H) 5.10 (d, *J*=10.07 Hz, 1 H) 5.19 (d, *J*=16.79 Hz, 1 H) 5.65 - 5.82 (m, 3 H) 6.56 (br. s., 1 H) 6.69 (d, *J*=11.90 Hz, 1 H) 7.11 (s, 1 H) 7.42 - 7.56 (m, 2 H) 7.73 (s, 1 H) 7.77 (d, *J*=8.24 Hz, 1 H). LCMS: r.t. = 1.66 min., $[M+Na]^+$ = 759 Phenomenex Luna C18 10u (3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Preparation of Compound 5*

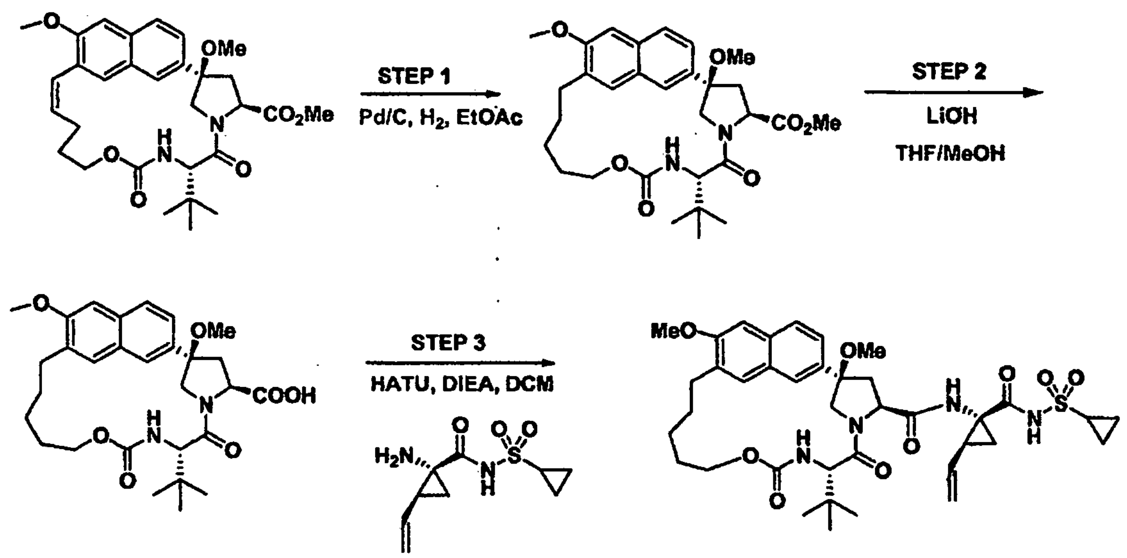


Compound 5

*Step 1:*

A solution of the product from Compound 4 Step 2 (46 mg, 0.085 mmol) and 10% Pd/C (9 mg, 8.54 μmol) in

ethyl acetate (5 mL) was stirred under an atmosphere of hydrogen overnight. The reaction was filtered through a nylon frit and concentrated to give crude product (40 mg, 0,074 mmol, 87% yield) as a white solid. LCMS: r.t. = 2.04 min., $[M+H]^+$ = 541 Phenomenex Luna S 10 (3 x50 mm); Solvent A = 95% water- 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. =10 ul; wavelength = 220 nm.

*Step 2:*

2.0 M lithium hydroxide (0.18 mL, 0.36 mmol) was added to a solution of the product from Step 1 (40 mg, 0.074 mmol) in THF (1 mL) and MeOH (1 mL) and stirred for 3 hours. The reaction was diluted with ether and 1.0 M HCl. The organic layer was dried, filtered and concentrated to give the crude product (38 mg, 0,072 mmol, 98% yield) as a white solid. LCMS: r.t. = 1.80 min., $[M+H]^+$ = 527 Phenomenex Luna S10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1 main.; 4 mL/min; inj. vol. = 10 ul; wavelength = 220 nm.

*Step 3:*

HATU (20 mg, 0.054 mmol) was added to a solution of (1R,2S)-1-amino-N-(cyclopropylsulfonyl)-2-vinylcyclopropanecarboxamide, TsOH salt (17 mg, 0.043 mmol), the product from Step 2 (19 mg, 0.036 mmol) and Hunig's base (0.019 mL, 0.108 mmol) in dichloromethane (1.5 mL) and stirred at r.t. overnight. The reaction was concentrated and purified by HPLC (Xbridge C18 10u (30 x100 mm); flow = 42 mL/min; solvent gradient = 95:5 to 5:95 water/acetonitrile (with 10mM ammonium acetate)). The product fractions were concentrated to give Compound 5 (4.3 mg, 5.82 μmol, 16 % yield) as a white solid. $^1$H NMR (400 MHz, MeOD) δ ppm 0.99 - 1.05 (m, 2 H) 1.10 (s, 9 H) 1.16 - 1.23 (m, 2 H) 1.23 - 1.32 (m, 1 H) 1.38 (dd,*J*=9.41, 5.40 Hz, 1 H) 1.70 (br. s., 1 H) 1.75 (d, *J*=9.54 Hz, 2 H) 1.84 (dd, *J*=8.16, 5.40 Hz, 1 H) 1.88 - 1.99 (m, 1 H) 2.13 (q, *J*=8.87 Hz, 1 H) 2.15 - 2.23 (m, 1 H) 2.39 (t, *J*=11.80 Hz, 1 H) 2.66 (dd, *J*=11.80, 6.78 Hz, 1 H) 2.78 - 2.87 (m, 2 H) 2.87 - 2.94 (m, 1 H) 3.07 - 3.12 (m, 3 H) 3.67 - 3.79 (m, 2 H) 3.80 - 3.89 (m, 1 H) 3.94 (s, 3 H) 4.98 (t, *J*=10.67 Hz, 1 H) 5.04 - 5.18 (m, 2 H) 5.23 (d, *J*=17.07 Hz, 1 H) 5.64 - 5.79 (m, 1 H) 7.20 (s, 1 H) 7.53 (dd, *J*=8.66, 1.38 Hz, 1 H) 7.58 (d, *J*=5.52 Hz, 2 H) 7.84 (d, *J*=8.53 Hz, 1 H). LCMS: r.t. = 1.93 min., $[M-OMe]^+$ = 707 Phenomenex Luna S10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. = 10 ul; wavelength = 220 nm.

*Compound 6*

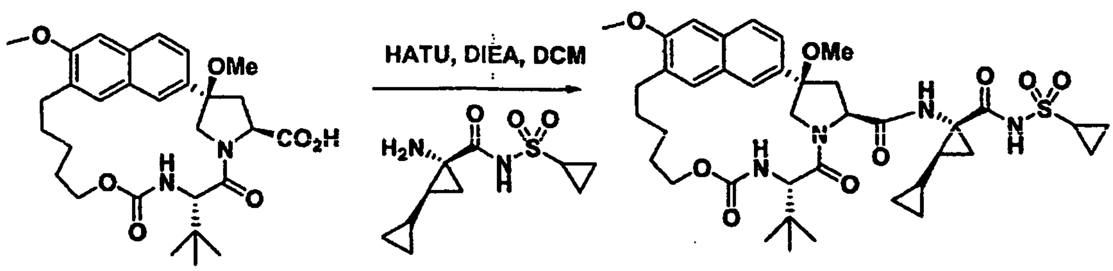


**Compound 6**

Compound 6 was made using the same procedure as for Compound 5 Step 3 except that (1S,2R)-2-amino-N-(cyclopropylsulfonyl)bi(cyclopropane)-2-carboxamide, HCl salt was used. $^1$H NMR (500 MHz, CHLOROFORM-*d*) d ppm 0.11 - 0.29 (m, 2 H) 0.45 - 0.59 (m, 2 H) 0.78 - 0.89 (m, 1 H) 0.93 (q, *J*=8.44 Hz, 1 H) 0.97 - 1.05 (m, 2 H) 1.08 (s, 9 H) 1.17 - 1.25 (m, 2 H) 1.30 - 1.41 (m, 2 H) 1.62 - 1.72 (m, 2 H) 1.73 -1.84 (m, 2 H) 1.84 - 1.98 (m, 1 H) 2.13 - 2.31 (m, 1 H) 2.50 - 2.59 (m, 1 H) 2.62 (t, *J*=11.44 Hz, 1 H) 2.76 - 2.88 (m, 2 H) 2.88 - 2.97 (m, 1 H) 3.06 (s, 3 H) 3.60 - 3.69 (m, 1 H) 3.74 (d, *J*=10.68 Hz, 1 H) 3.83 (ddd, *J*=11.06, 3.59, 3.36 Hz, 1 H) 3.94 (s, 3 H) 4.77 (d, *J*=10.07 Hz, 1 H) 4.94 (d, *J*=10.68 Hz, 1 H) 5.02 (t, *J*=10.53 Hz, 1 H) 5.62 (d, *J*=9.77 Hz, 1 H) 6.42 (br. s., 1 H) 7.05 (s, 1 H) 7.42 - 7.52 (m, 2 H) 7.56 (s, 1 H) 7.76 (d, *J*=8.55 Hz, 1 H) 9.91 (br. s., 1 H). LCMS: r.t. = 1.74 min., $[M+Na]^+$ = 775 Phenomenex Luna C18 10u (3 x50 mm); Solvent A =10% acetonitrile - 90% water - 0.1 % TFA, Solvent B = 90% acetonitrile - 10% water - 0.1 % TFA; gradient = 0% to 100% solvent B over 2 min. and then hold for 1 min.; 4 mL/min; inj. vol. = 5 ul; wavelength = 220 nm.

*Preparation of Compound 7*

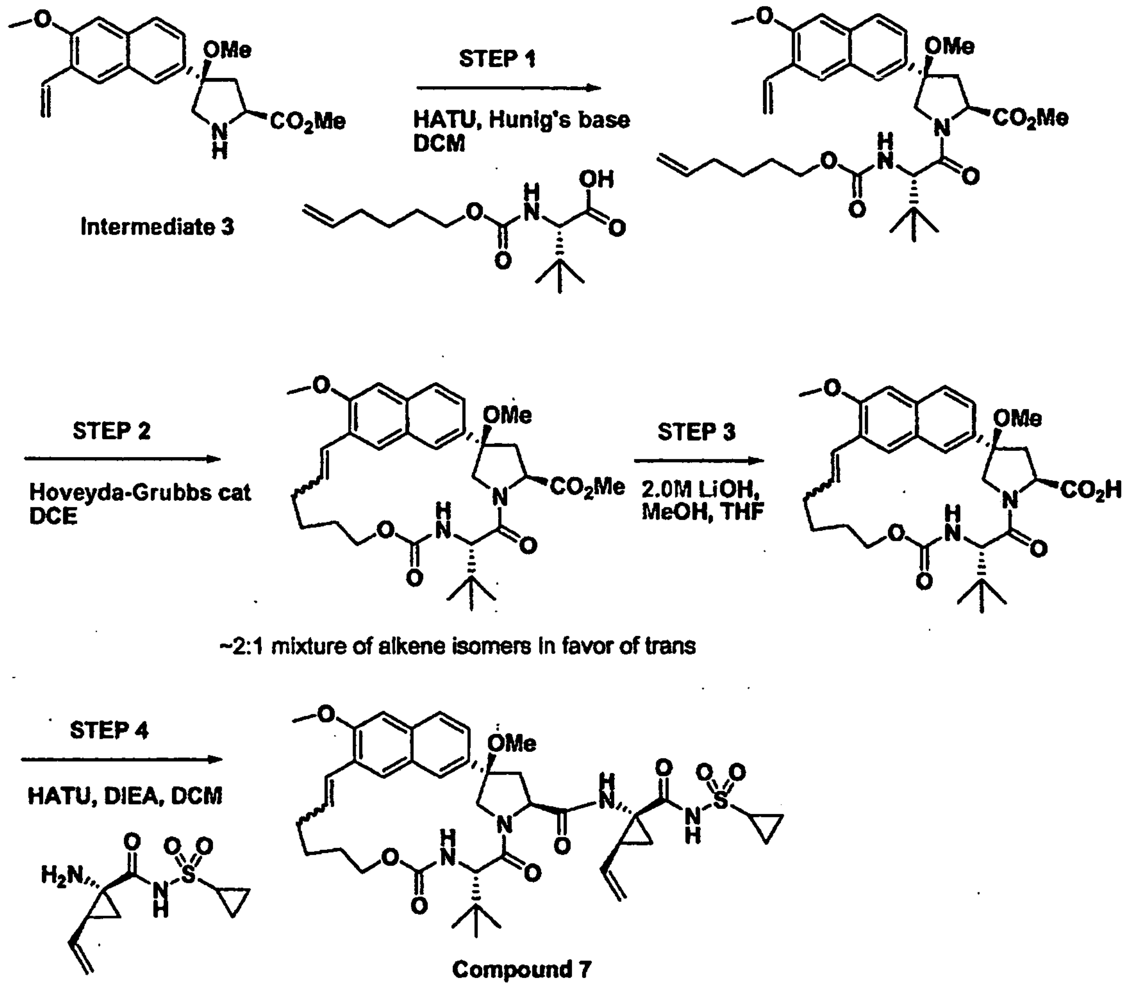


*Step 1:*

HATU (66 mg, 0.176 mmol) was added to a solution of (2S,4R)-methyl 4-methoxy-4-(6-methoxy-7-vinylnaphthalen-2-yl)pyrrolidine-2-carboxylate (50 mg, 0.146 mmol), (S)-2-((hex-5-enyloxy)carbonylamino)-3,3-dimethylbutanoic acid (56 mg, 0.22 mmol) and Hunig's base (0.077 mL, 0.439 mmol) in DCM (2 mL) and stirred at r.t. overnight. The reaction was concentrated and purified by flash chromatography on the Biotage (10-40% EtOAc in hexanes) to give (2S,4R)-methyl 1-((S)-2-((hex-5-enyloxy)carbonylamino)-3,3-dimethylbutanoyl)-4-methoxy-4-(6-methoxy-7-vinylnaphthalen-2-yl)pyrrolidine-2-carboxylate (49 mg, 58%) as a white foam. LCMS: r.t. = 1.90 min., $[M+Na]^+$ = 603 Phenomenex Luna C18 10u (3 x50 mm); Solvent A =10% acetonitrile - 90% water - 0.1 % TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 2:*

A solution of (2S,4R)-methyl 1-((S)-2-((hex-5-enyloxy)carbonylamino)-3,3-dimethylbutanoyl)-4-methoxy-4-(6-methoxy-7-vinylnaphthalen-2-yl)pyrrolidine-2-carboxylate (49 mg, 0.084 mmol) in DCE (20 mL) was sparged with nitrogen for 30 min. and then Hoveyda-Grubbs catalyst (5 mg, 8.4 μmol) was added and the reaction sealed and heated to 80°C for 3 hrs. The reaction was concentrated to give crude material. The crude material was purified by flash chromatography on the Biotage (20-60% EtOAc in hexanes) to give the purified product (34 mg, 73%) as a white foam. LCMS: r.t. = 1.73 min., $[M+H]^+$ = 553 Phenomenex Luna C18 10u (3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold

for 1 min.; 4 mL/min; inj. vol. = 5 ul; wavelength = 220 nm.

*Step 3:*

2.0M LiOH (0.15 mL, 0.31 mmol) was added to a solution of the product from Step 2 (34 mg, 0.062 mmol) in THF (1 mL) and MeOH (1 mL) and stirred at r.t. overnight. The reaction was quenched with sat. $NH_4Cl$ solution and ether. The organics were dried, filtered and concentrated to give the crude product (30 mg, 91%) which was used directly in the next step. LCMS: r.t. =1.53 min., $[M+H]^+$ = 539 Phenomenex Luna C18 10u (3 x50 mm); Solvent A = 10% acetonitrile - 90% water - 0.1 % TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 4:*

HATU (23 mg, 0.061 mmol) was added to a solution of (1R,2S)-1-amino-N-(cyclopropylsulfonyl)-2-vinylcyclopropanecarboxamide, pTSA salt (34 mg, 0.084 mmol), the product from Step 3 (30 mg, 0.056 mmol) and Hunig's base (0.029 mL, 0.167 mmol) in dichloromethane (0.5 mL) and stirred at r.t. for 4 hrs. The reaction was concentrated and then purified by prep. HPLC (Sunfire C18 10u (30 x100 mm); flow = 42 mL/min; solvent gradient = 95:5 to 5:95 water/acetonitrile (with 10mM ammonium acetate)) to give Compound 7 (21 mg, 50%) as a white solid. LCMS: r.t. = 1.73 min., $[M+Na]^+$ = 773 Phenomenex Luna C18 10u (3 x50 mm); Solvent A = 10% acetonitrile - 90% water - 0.1% TFA, Solvent B = 90% acetonitrile - 10% water - 0.1 % TFA; gradient = 0% to 100% solvent B over 2 min. and then hold for 1 min.; 4 mL/min; inj. vol. = 5 ul; wavelength = 220 nm.

*Preparation of Compound 8*

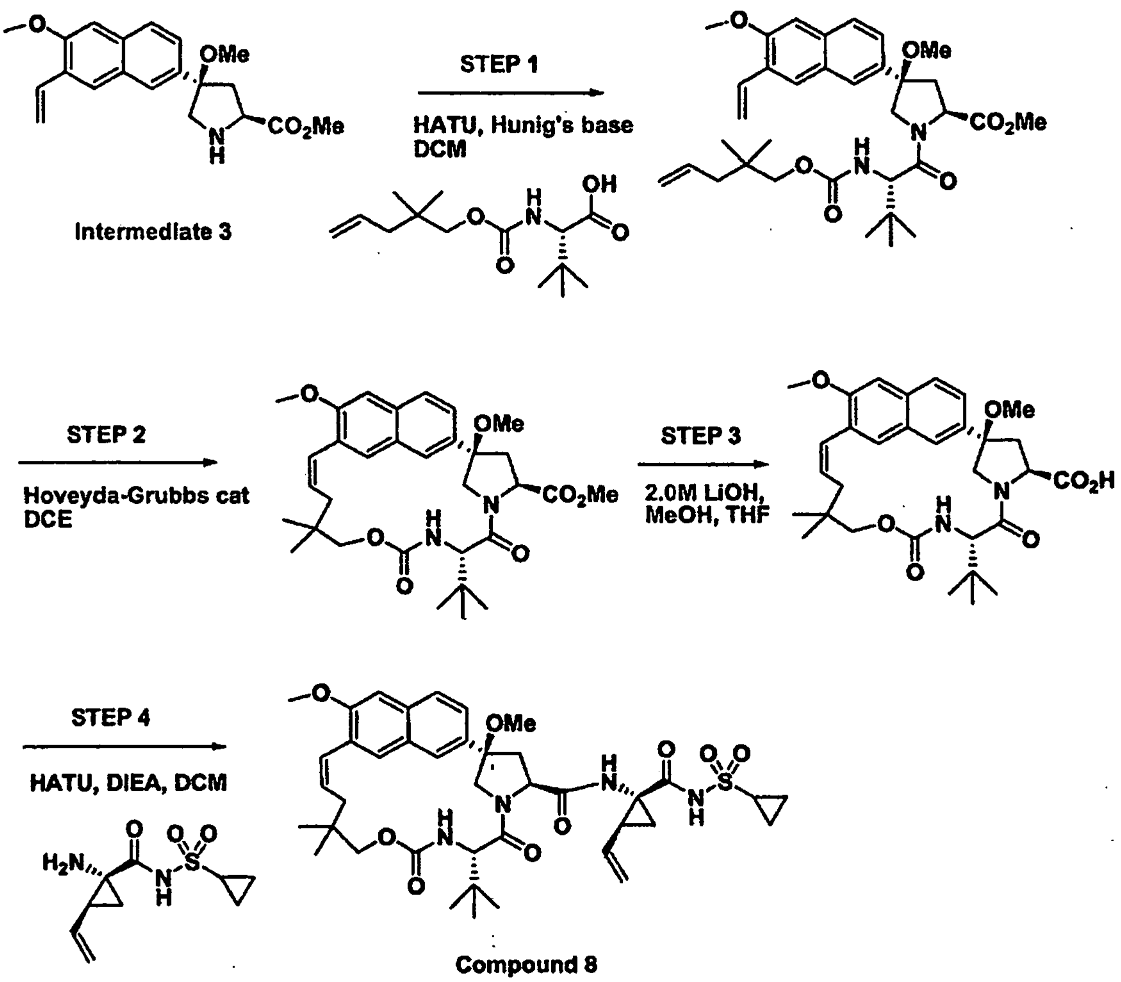


Compound 8

*Step 1:*

HATU (267 mg, 0.703 mmol) was added to a solution of (2S,4R)-methyl 4-methoxy-4-(6-methoxy-7-vinylnaphthalen-2-yl)pyrrolidine-2-carboxylate (200 mg, 0.586 mmol), (S)-2-((2,2-dimethylpent-4-enyloxy)carbonylamino)-3,3-dimethylbutanoic acid (238 mg, 0.879 mmol) and Hunig's base (0.31 mL, 1.76 mmol) in DCM (8 mL) and stirred at r.t. overnight. The reaction was concentrated and purified by flash chromatography on the Biotage (10-40% EtOAc in hexanes) to give (2S,4R)-methyl 1-((S)-2-((2,2-dimethylpent-4-enyloxy)carbonylamino)-3,3-dimethylbutanoyl)-4-methoxy-4-(6-methoxy-7-vinylnaphthalen-2-yl)pyrrolidine-2-carboxylate (242 mg, 70%) as a white foam. LCMS: r.t. =1.98 min., $[M+Na]^+$ = 617 Phenomenex Luna C 18 10u (3 x50 mm); Solvent A =10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 2:*

A solution (2S,4R)-methyl 1-((S)-2-((2,2-dimethylpent-4-enyloxy)carbonylamino)-3,3-dimethylbutanoyl)-4-methoxy-4-(6-methoxy-7-vinylnaphthalen-2-yl)pyrrolidine-2-carboxylate (242 mg, 0.407 mmol) in DCE (100 mL) was sparged with nitrogen for 30 min. and then Hoveyda-Grubbs catalyst (26 mg, 0.041 mmol) was added and the reaction sealed and heated to 80°C for 4 days. The reaction was concentrated to give crude material. The crude material was purified by flash chromatography on the Biotage (10-60% EtOAc in hexanes) to give the purified product as a lightly colored foam (65 mg, 28%). LCMS: r.t. = 1.83 min., $[M+H]^+$ = 567 Phenomenex Luna C18 10u (3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 3:*

2.0M LiOH (0.29 mL, 0.57 mmol) was added to a solution of the product from Step 2 (65 mg, 0.115 mmol) in THF (1 mL) and MeOH (1 mL) and stirred at r.t. overnight. The reaction was quenched with sat. $NH_4Cl$ solution and ether. The organics were dried, filtered and concentrated to give the crude product (62 mg, 98%) which was used directly in the next step. LCMS: r.t. =1.61 min., $[M+H]^+$ = 553 Phenomenex Luna C18 10u (3 x50 mm); Solvent A = 10% acetonitrile - 90% water - 0.1 % TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 4:*

HATU (9 mg, 0.024 mmol) was added to a solution of (1R,2S)-1-amino-N-(cyclopropylsulfonyl)-2-vinylcyclopropanecarboxamide, pTSA salt (13 mg, 0.033 mmol), the product from Step 3 (12 mg, 0.022 mmol) and Hunig's base (0.011 mL, 0.065 mmol) in dichloromethane (2 mL) and stirred at r.t.overnight. The reaction was concentrated and then purified by prep. HPLC (Sunfire C18 10u (30 x100 mm); flow = 42 mL/min; solvent gradient = 95:5 to 5:95 water/acetonitrile (with 10mM ammonium acetate)) to give Compound 8 (6 mg, 35%) as a white solid. $^1$H NMR (500 MHz, CHLOROFORM-*d*) d ppm 0.96 - 1.02 (m, 2 H) 1.04 (s, 6 H) 1.11 (s, 9 H) 1.28 - 1.37 (m, 2 H) 1.46 (dd *J*=9.16, 5.49 Hz, 1 H) 1.91 - 1.97 (m, 1 H) 1.99 - 2.05 (m, 1 H) 2.32 - 2.40 (m, 1 H) 2.54 (dd, *J*=11.29, 7.02 Hz, 1 H) 2.58 - 2.68 (m, 2 H) 2.81 - 2.93 (m, 1 H) 3.10 (s, 3 H) 3.41 (d, *J*=10.68 Hz, 1 H) 3.73 (dd, *J*=11.29, 7.02 Hz, 1 H) 3.77 (d, *J*=10.68 Hz, 1 H) 3.96 (s, 3 H) 4.70 (d, *J*=10.68 Hz, I H) 4.81 (d, *J* = 9.77 Hz, 1 H) 4.90 (d, *J*=10.68 Hz, 1 H) 5.12 (d, *J*=10.38 Hz, 1 H) 5.21 (d, *J*=17.40 Hz, 1 H) 5.70 (d, *J*=10.99 Hz, 1 H) 5.72 - 5.80 (m, 1 H) 5.94 (ddd, *J*=12.13, 7.71, 5.80 Hz, 1 H) 6.59 (br. s., 1 H) 6.79 (d, *J*=12.21 Hz, 1 H) 7.09 (s, 1 H) 7.37 (s, 1 H) 7.50 (dd, *J*=8.55, 1.83 Hz, I H) 7.76 (d, *J*=8.55 Hz, I H) 7.81 (s, 1 H). LCMS: r.t. = 1.78 min., $[M+Na]^+$ = 787 Phenomenex Luna C18 10u (3 x50 mm); Solvent A = 10% acetonitrile - 90% water - 0.1% TFA, Solvent B = 90% acetonitrile - 10% water - 0.1 % TFA; gradient = 0% to 100% solvent B over 2 min. and then hold for 1 min.; 4 mL/min; inj. vol. = 5 ul; wavelength = 220 nm.

*Preparation of Compound 9*

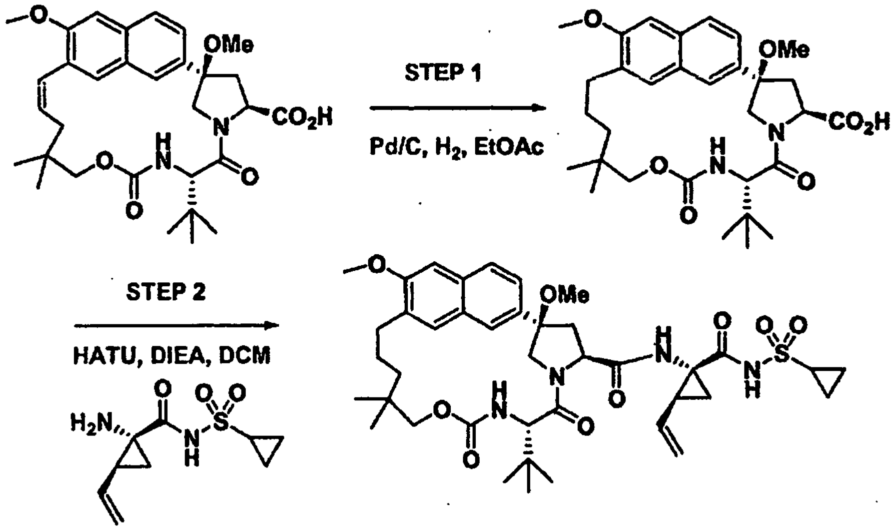


Compound 9

*Step 1:*

A solution of the product from Example 8, Step 3 (50 mg, 0.090 mmol) in EtOAc (2 mL) was passed through a Pd/C cartridge on the H-cube (flow =1 mL/min; full $H_2$ saturation; pressure =1 bar). The eluent was concentrated to give the crude product (45 mg, 90%) as a white foam which was used directly in the next step. LCMS: r.t. =1.70 min., $[M+H]^+$ = 555 Phenomenex Luna C18 10u (3 x50 mm); Solvent A = 10% acetonitrile - 90% water - 0.1% TFA, Solvent B = 90% acetonitrile - 10% water - 0.1 % TFA; gradient = 0% to 100% solvent B over 2 min. and then hold for 1 min.; 4 mL/min; inj. vol. = 5 ul; wavelength = 220 nm.

*Step 2:*

HATU (11 mg, 0.030 mmol) was added to a solution of (1R,2S)-1-amino-N-(cyclopropylsulfonyl)-2-vinylcyclopropanecarboxamide, pTSA salt (16 mg, 0.041 mmol), the product from Step 1 (15 mg, 0.027 mmol) and Hunig's base (0.014 mL, 0.081 mmol) in dichloromethane (1 mL) and stirred at r.t. overnight. The reaction was concentrated and then purified by prep. HPLC (Sunfire C18 10u (30 x100 mm); flow = 42 mL/min; solvent gradient = 95:5 to 5:95 water/acetonitrile (with 10mM ammonium acetate)) to give Compound 9 (2 mg, 10%) as a white solid. $^1$H NMR (500 MHz, CHLOROFORM-*d*) d ppm 0.87 - 0.95 (m, 1 H) 0.94 (s, 3 H) 1.00 (s, 3 H) 1.00 - 1.05 (m, 2 H) 1.11 (s, 9 H) 1.27 - 1.37 (m, 2 H) 1.47 (dd, *J*=9.31, 5.65 Hz, 1 H) 1.75 -1.92 (m, 2 H) 1.93 - 1.99 (m, 1 H) 1.99 - 2.09 (m, 2 H) 2.56 - 2.76 (m, 3 H) 2.88 - 2.92 (m, 2 H) 3.04 (s, 3 H) 3.28 (d, *J*=9.77 Hz, 1 H) 3.70 (dd, *J*=11.14, 7.17 Hz, 1 H) 3.74 (d, *J*=10.99 Hz, 1 H) 3.95 (s, 3 H) 4.82 (d, *J*=10.07 Hz, 1 H) 4.88 - 4.99 (m, 2 H) 5.12 (d, *J*=10.07 Hz, I H) 5.22 (d, *J*=16.79 Hz, 1 H) 5.65 (d, *J*=9.77 Hz, 1 H) 5.70 - 5.82 (m, 1 H) 6.53 (br. s., 1 H) 7.06 (s, 1 H) 7.43 (s, 1 H) 7.47 (dd, *J*=8.55, 1.83 Hz, 1 H) 7.52 (s, 1 H) 7.77 (d, *J*=8.55 Hz, 1 H). LCMS: r.t. = 1.86 min., $[M+Na]^+$ = 789 Phenomenex Luna C18 10u (3 x50 mm); Solvent A =10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Preparation of Compound 10*

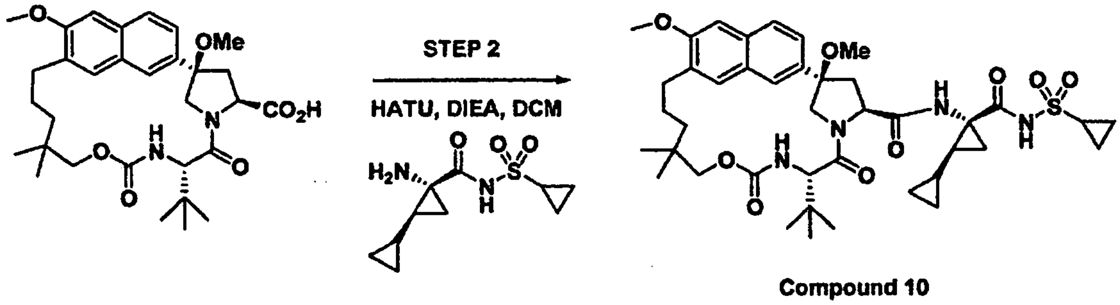


Compound 10

HATU (11 mg, 0.030 mmol) was added to a solution of (1S,2R)-2-amino-N-(cyclopropylsulfonyl)bi(cyclopropane)-2-carboxamide, HCl salt (11 mg, 0.041 mmol), the product from Example 9, Step 1 (15 mg, 0.027 mmol) and Hunig's base (0.014 mL, 0.081 mmol) in dichloromethane (1 mL) and stirred at r.t. overnight. The reaction was concentrated and then purified by prep. HPLC (Sunfire C18 10u (30 x100 mm); flow = 42 mL/min; solvent gradient = 95:5 to 5:95 water/acetonitrile (with 10mM ammonium acetate)) to give Compound 10 (9 mg, 42%) as a white solid. $^1$H NMR (500 MHz, CHLOROFORM-*d*) δ ppm 0.16 - 0.30 (m, 2 H) 0.43 - 0.60 (m, 2 H) 0.74 - 0.85 (m, 1 H) 0.86 - 0.92 (m, 1 H) 0.92 - 0.97 (m, 4 H) 0.97 - 1.05 (m, 5 H) 1.09 (s, 9 H) 1.22 (dd, *J*=9.46, 5.49 Hz, 1 H) 1.29 - 1.41 (m, 2 H) 1.71 - 1.91 (m, 3 H) 1.96 - 2.16 (m, 1 H) 2.56 - 2.76 (m, 3 H) 2.82 - 2.96 (m, 2 H) 3.03 (s, 3 H) 3.27 (d, *J*=10.38 Hz, 1 H) 3.72 (dt, *J*=10.61, 3.40 Hz, 2 H) 3.94 (s, 3 H) 4.81 (d, *J*=10.07 Hz, 1 H) 4.86 - 5.00 (m, 2 H) 5.66 (d, *J*=9.77 Hz, 1 H) 6.57 (br. s., 1 H) 7.05 (s, 1 H) 7.41 (s, 1 H) 7.46 (dd, *J*=8.55, 1.83 Hz, 1 H) 7.51 (s, 1 H) 7.76 (d, *J*=8.55 Hz, 1 H). LCMS: r.t. = 1.88 min., $[M+Na]^+$= 803 Phenomenex Luna C18 10u (3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Preparation of Compound 11*

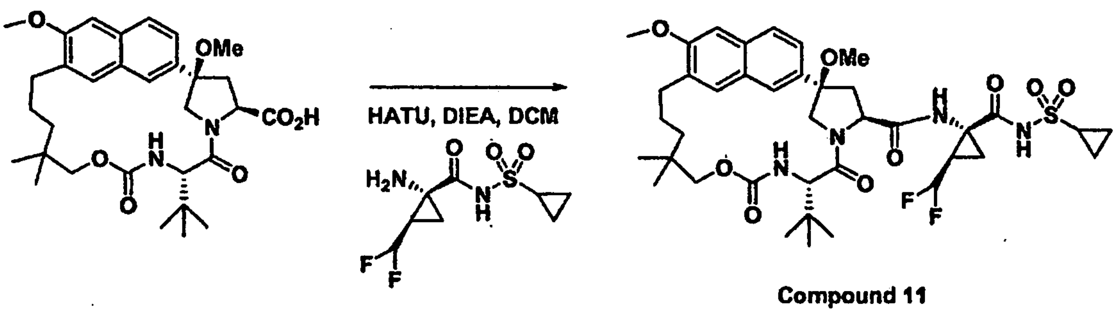


Compound 11

HATU (11 mg, 0.030 mmol) was added to a solution of (1R,2R)-1-amino-N-(cyclopropylsulfonyl)-2-(difluoromethyl)cyclopropanecarboxamide, HCl salt (12 mg, 0.041 mmol), the product from Example 9, Step 1 (15 mg, 0.027 mmol) and Hunig's base (0.014 mL, 0.081 mmol) in dichloromethane (1 mL) and stirred at r.t. overnight. The reaction was concentrated and then purified by prep. HPLC (Sunfire C18 10u (30 x100 mm); flow = 42 mL/min; solvent gradient = 95:5 to 5:95 water/acetonitrile (with 10mM ammonium acetate)) to give Compound 11 (13 mg, 61%) as a white solid. $^1$H NMR (500 MHz, CHLOROFORM-*d*) d ppm 0.84 - 1.03 (m, 9 H) 1.11 (s, 9 H) 1.21 - 1.35 (m, 2 H) 1.51 1.63 (m, 1 H) 1.75 - 1.94 (m, 3 H) 2.01 - 2.13 (m, 2 H) 2.43 - 2.63 (m, 2 H) 2.72 (dd, *J*=17.85, 12.05 Hz, 1 H) 2.83 (br. s., 1 H) 2.85 - 2.93 (m, 1 H) 3.04 (s, 3 H) 3.28 (d, *J*=10.38 Hz, 1 H) 3.64 (dd, *J*=10.53, 7.17 Hz, 1 H) 3.75 (d, *J*=10.68 Hz, 1 H) 3.93 (s, 3 H) 4.82 (d, *J*=10.07 Hz, 1 H) 4.95 (t, *J*=11.14 Hz, 2 H) 5.69 (d, *J*=9.77 Hz, 1 H) 5.91 (td, *J*=55.54, 7.02 Hz, 1 H) 6.62 (br. s., 1 H) 7.05 (s, 1 H) 7.40 - 7.48 (m, 2 H) 7.53 (s, 1 H) 7.76 (d, *J*=8.85 Hz, 1 H). LCMS: r.t. = 1.86 min., $[M+Na]^+$ = 813 Phenomenex Luna C 18 10u (3 x50 mm); Solvent A = 10% acetonitrile - 90% water - 0.1 % TFA, Solvent B = 90% acetonitrile - 10% water - 0.1 % TFA; gradient = 0% to 100% solvent B over 2 min. and then hold for 1 min.; 4 mL/min; inj. vol. = 5 ul; wavelength = 220 nm.

*Preparation of Compound 12*

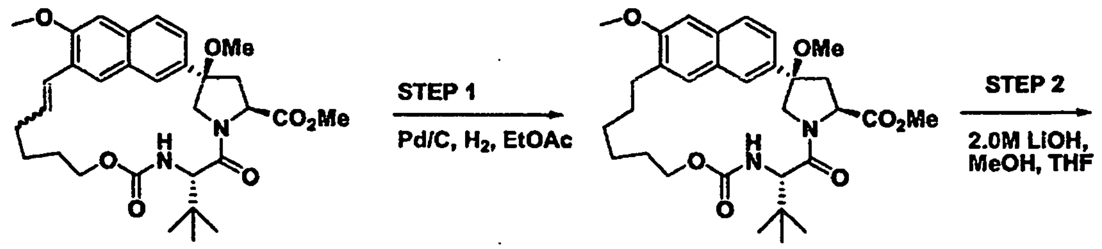


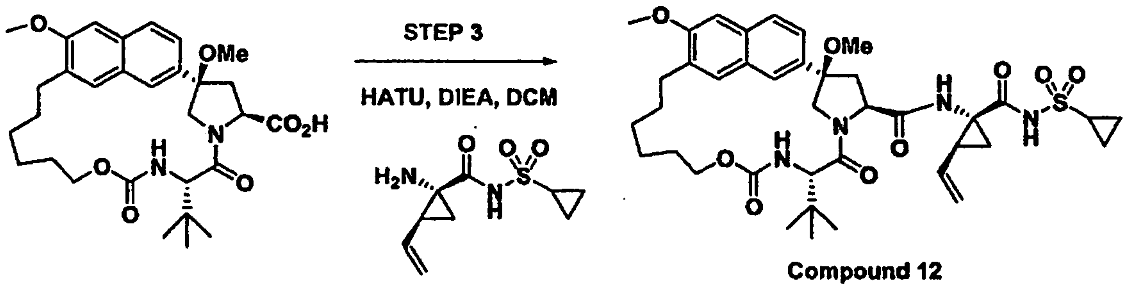


Compound 12

*Step 1:*

A solution of the product from Example 7, Step 2 (217 mg, 0.39 mmol) in EtOAc (8 mL) was passed through a Pd/C cartridge on the H-cube (flow =1 mL/min; full $H_2$ saturation; pressure = 1 bar). The eluent was concentrated to give the crude product (200 mg, 92%) as a white foam which was used directly in the next step. LCMS: r.t. = 1.83 min., $[M-OMe]^+$ = 523 Phenomenex Luna C18 10u (3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 2:*

2.0M LiOH (0.90 mL, 1.80 mmol) was added to a solution of the product from Step 1 (200 mg, 0.36 mmol) in THF (3 mL) and MeOH (3 mL) and stirred at r.t. overnight. The reaction was quenched with 1.0M HCl solution and ether. The organics were dried, filtered and concentrated to give the crude product. The crude material was purified by prep. HPLC (Sunfire C18 10u (30 x100 mm); flow = 42 mL/min; solvent gradient = 95:5 to 5:95 water/acetonitrile (with 0.1% TFA)) to give the product (120 mg, 63%) as a white solid. $^1H$ NMR (500 MHz, CHLOROFORM-*d*) d ppm 1.11 (s, 9 H) 1.19 - 1.28 (m, 1 H) 1.33 - 1.51 (m, 2 H) 1.57 - 1.76 (m, 3 H) 1.76-1.91 (m, 2 H) 2.52 (t, *J*=11.75 Hz, 1 H) 2.56 - 2.65 (m, 1 H) 2.68 - 2.80 (m, 1 H) 2.88 (ddd, *J*=14.57, 6.64, 3.81 Hz, 1 H) 3.16 (s, 3 H) 3.85 - 4.01 (m, 5 H) 4.16 (dd, *J*=11.29, 7.32 Hz, 1 H) 4.21 - 4.33 (m, 1 H) 4.69 (d, *J*=10.07 Hz, 1 H) 4.96 (d, *J*=10.38 Hz, 1 H) 6.16 (d, *J*=9.77 Hz, 1 H) 7.09 (s, 1 H) 7.43 (s, 1 H) 7.48 (s, 1 H) 7.51 (dd, *J*=8.55, 1.53 Hz, 1 H) 7.77 (d, *J*=8.55 Hz, 1 H).

*Step 3:*

HATU (62 mg, 0.163 mmol) was added to a solution of (1R,2S)-1-amino-N-(cyclopropylsulfonyl)-2-vinylcyclopropanecarboxamide, pTSA (89 mg, 0.222 mmol), the product from Step 3 (80 mg, 0.148 mmol) and Hunig'sBase (0.078 mL, 0.444 mmol) in DCM (5 mL) and stirred at r.t. for 4 hrs. The reaction was concentrated and then purified by prep. HPLC (Sunfire C18 10u (30 x100 mm); flow = 42 mL/min; solvent gradient = 95:5 to 5:95 water/acetonitrile (with 10mM ammonium acetate)). The product fractions were concentrated and then lyopholized to give Compound 12 (73 mg, 65%) a fluffy white solid. $^1H$ NMR (500 MHz, CHLOROFORM-*d*) d ppm 0.95 - 1.06 (m, 2 H) 1.13 (s, 9 H) 1.22 - 1.51 (m, 6 H) 1.65 -1.78 (m, 3 H) 1.79 - 1.89 (m, 2 H) 1.94 (dd, *J*=8.24, 5.49 Hz, 1 H) 2.02 (q, *J*=8.85 Hz, 1 H) 2.33 (dd, *J*=11.90, 6.10 Hz, 1 H) 2.59 (t, *J*=11.75 Hz, 1 H) 2.69 - 2.81 (m, 1 H) 2.81 - 2.94 (m, 2 H) 3.15 (s, 3 H) 3.77 (dd, *J*=11.44, 6.26 Hz, 1 H) 3.89 - 3.96 (m, 2 H) 3.94 (s, 3 H) 4.35 (ddd, *J*=10.22, 7.32, 7.17 Hz, 1 H) 4.61 (d, *J*=10.07 Hz, 1 H) 4.96 (d, *J*=10.07 Hz, 1 H) 5.10 (dd, *J*=10.38, 1.22 Hz, 1 H) 5.18 (dd, *J*=16.94, 1.07 Hz, 1 H) 5.60 (d, *J*=10.07 Hz, 1 H) 5.79 (ddd, *J*=17.01, 10.15, 8.85 Hz, 1 H) 6.41 (br. s., 1H) 7.08 (s, 1 H) 7.49 (s, 2 H) 7.52 (dd, *J*=8.55, 1.83 Hz, 1 H) 7.77 (d, *J*=8.85 Hz, 1 H) 9.96 (br. s., 1 H). LCMS: r.t. =1.79 min., $[M+Na]^+$ = 775 Phenomenex Luna C18 10u (3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Preparation of Compound 13*

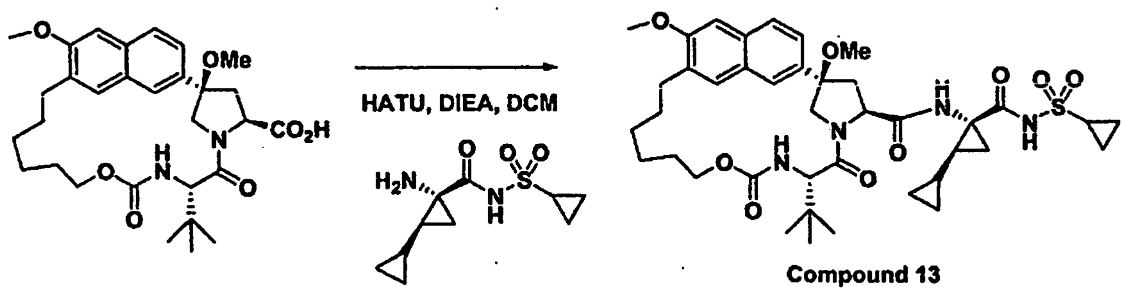


Compound 13

HATU (31 mg, 0.081 mmol) was added to a solution of (1S,2R)-2-amino-N-(cyclopropylsulfonyl)bi(cyclopropane)-2-carboxamide, HCl salt (31 mg, 0.111 mmol), the product from Example 12 Step 2 (40 mg, 0.074 mmol) and Hunig'sBase (0.039 mL, 0.222 mmol) in DCM (2 mL) and stirred at r.t for 4 hrs. The reaction was concentrated and then purified by prep. HPLC (Sunfire C18 10u (30 x100 mm); flow = 42 mL/min; solvent gradient = 95:5 to 5:95 water/acetonitrile (with 10mM ammonium acetate)). The product fractions were concentrated and then lyopholized to give Compound 13 (29 mg, 51%) as a fluffy white solid. $^1$H NMR (500 MHz, CHLOROFORM-*d*) d ppm 0.13-0.31 (m, 2 H) 0.47 - 0.59 (m, 2 H) 0.87 (td, *J*=8.32, 4.12 Hz, 1 H) 0.90 - 0.98 (m, 1 H) 0.98 - 1.06 (m, 2 H) 1.12 (s, 9 H) 1.26 (dd, *J*=9.61, 5.65 Hz, 2 H) 1.32 - -1.52 (m, 4 H) 1.62 - 1.76 (m, 3 H) 1.78 - 1.93 (m, 3 H) 2.32 (dd, *J*=11.90, 6.71 Hz, 1 H) 2.59 (t, *J*=11.60 Hz, 1 H) 2.71 - 2.81 (m, 1 H) 2.81 - 2.90 (m, 1 H) 2.90 - 2.98 (m, 1 H) 3.15 (s, 3 H) 3.76 (dd, *J*=11.44, 6.26 Hz, 1 H) 3.87 - 3.95 (m, 2 H) 3.94 (s, 3 H) 4.24 - 4.39 (m, 1 H) 4.60 (d, *J*=10.07 Hz, 1 H) 4.94 (d, *J*=10.38 Hz, 1H) 5.54 (d, *J*=10.07 Hz, 1 H) 6.27 (br. s., 1 H) 7.08 (s, 1 H) 7.44 - 7.48 (m, 2 H) 7.51 (dd, *J*=8.55, 1.83 Hz, 1 H) 7.76 (d, *J*=8.55 Hz, I H) 9.95 (br. s., 1H). LCMS: r.t. = 1.83 min., $[M+Na]^+$ = 789 Phenomenex Luna C18 10u (3 x50 mm); Solvent A =10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Preparation of Compound 14*

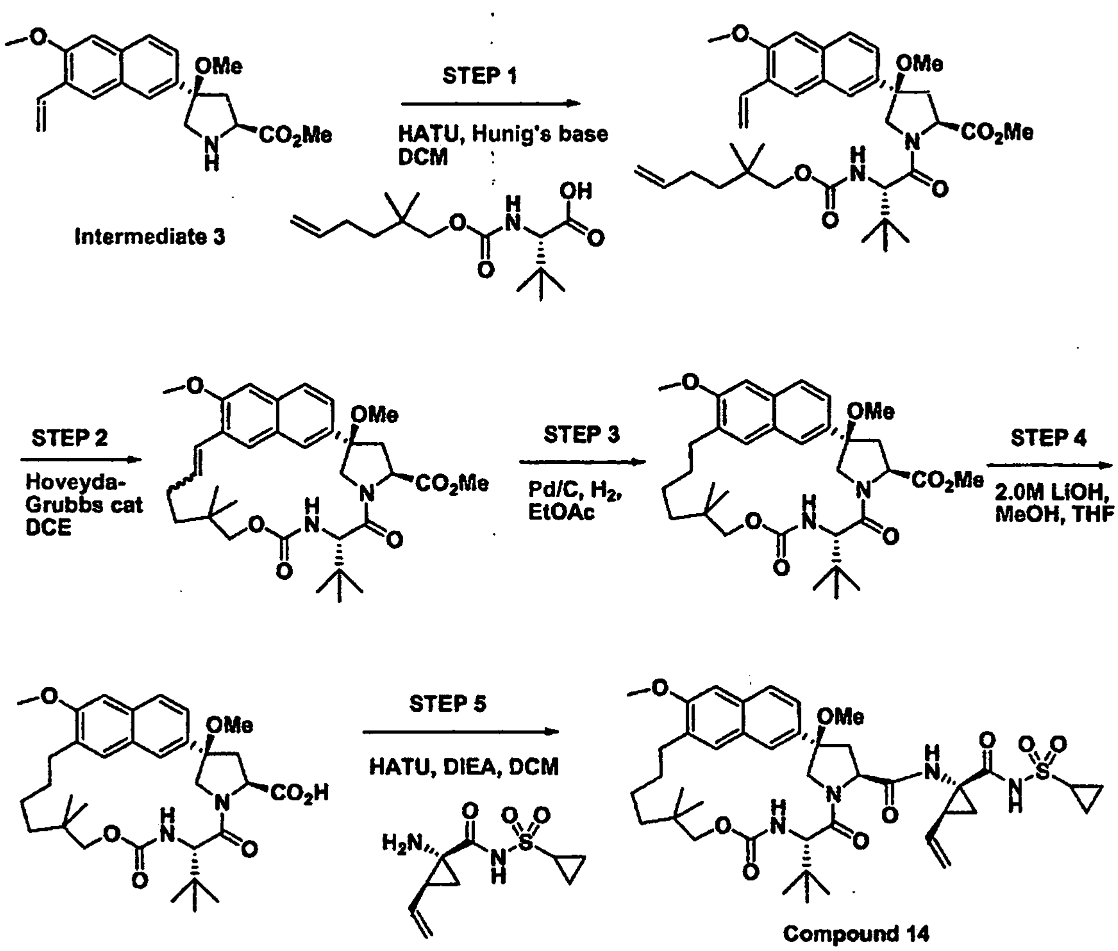


Compound 14

*Step 1:*

HATU (88 mg, 0.232 mmol) was added to a solution of (2S,4R)-methyl 4-methoxy-4-(6-methoxy-7-vinylnaphthalen-2-yl)pyrrolidine-2-carboxylate (Intermediate 3, 66 mg, 0.193 mmol), (S)-2-((2,2-dimethylhex-5-enyloxy)carbonylamino)-3,3-dimethylbutanoic acid (83 mg, 0.290 mmol) and Hunig'sBase (0.10 mL, 0.58 mmol) in DCM (5 mL) and stirred at r.t. for 4 hrs. The reaction was concentrated and purified by flash chromatography on the Biotage (10-40% EtOAc in hexanes) to give (2S,4R)-methyl 1-((S)-2-((2,2-dimethylhex-5-enyloxy)carbonylamino)-3,3-dimethylbutanoyl)-4-methoxy-4-(6-methoxy-7-vinylnaphthalen-2-yl)pyrrolidine-2-carboxylate (100 mg, 85%) as a colorless oil. LCMS: r.t. = 2.01 min., $[M+Na]^+$ = 631 Phenomenex Luna C18 10u (3 x50 mm); Solvent A = 10% acetonitrile - 90% water - 0.1% TFA, Solvent B = 90% acetonitrile - 10% water - 0.1 % TFA; gradient = 0% to 100% solvent B over 2 min. and then hold for 1 min.; 4 mL/min; inj. vol. = 5 ul; wavelength = 220 nm.

*Step 2:*

A solution of (2S,4R)-methyl 1-((S)-2-((2,2-dimethylhex-5-enyloxy)carbonylamino)-3,3-dimethylbutanoyl)-4-methoxy-4-(6-methoxy-7-vinylnaphthalen-2-yl)pyrrolidine-2-carboxylate (100 mg, 0.164 mmol) in DCE (50 mL) was sparged with nitrogen for 30 min. and then Hoveyda-Grubbs Catalyst 2nd Generation (10 mg, 0.016 mmol) was added and the reaction sealed and heated to 80°C overnight. The reaction was concentrated to give crude material. The crude material was purified by flash chromatography on the Biotage (20-50% EtOAc in hexanes) to give the purified product (71 mg, 74%) as a white foam. LCMS: r.t. = 1.89 min., $[M+H]^+$ = 581 Phenomenex Luna C18 10u (3 x50 mm); Solvent A =10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 3:*

Pd/C (13 mg, 0.012 mmol) was added to a solution of the product from Step 2 (71 mg, 0.122 mmol) in EtOAc

(3 mL) and stirred under an atmosphere of hydrogen at r.t. overnight. The reaction was filtered through a nylon frit and concentrated to give the product (71 mg, 100%) as a white foam. LCMS: r.t. = 2.29 min., $[M+H]^+$ = 583 Phenomenex Luna C18 10u (3 x50 mm); Solvent A = 10% methanol - 90% water - 0.1 % TFA, Solvent B = 90% methanol - 10% water - 0.1 % TFA; gradient = 0% to 100% solvent B over 2 min. and then hold for 1 min.; 5 mL/min; inj. vol. = 5 ul; wavelength = 220 nm.

*Step 4:*

2.0M LiOH (0.31 mL, 0.62 mmol) was added to a solution of the product from Step 3 (71 mg, 0.122 mmol) in THF (1 mL) and MeOH (1 mL) and stirred at r.t. for 4 hrs. The reaction was quenched with 1.0M HCl solution and ether. The organics were dried, filtered and concentrated to give the crude product (60 mg, 87%) which was used directly in the next step. $^1$H NMR (500 MHz, CHLOROFORM-*d*) d ppm 0.75 (s, 3 H) 0.96 (s, 3 H) 1.04 (s, 9 H) 1.09 -1.19 (m, 2 H) 1.23 - 1.34 (m, 1 H) 1.55 - 1.66 (m, 1 H) 1.68 -1.75 (m, 1 H) 2.34 - 2.40 (m, 2 H) 2.60 - 2.71 (m, 1 H) 2.76 - 2.86 (m, 1 H) 3.11 (s, 3 H) 3.47 (d, *J*=10.38 Hz, 1 H) 3.82 (d, *J*=10.07 Hz, 1 H) 3.85 (s, 3 H) 3.93 (d, *J*=10.38 Hz, 1 H) 4.03 (dd, *J*=10.83, 7.78 Hz, 1 H) 4.61 (d, *J*=10.38 Hz, 1 H) 4.77 (d, *J*=10.38 Hz, 1 H) 5.78 (d, *J*=10.38 Hz, 1 H) 6.99 (s, 1 H) 7.17 (d, *J*=1.22 Hz, 1 H) 7.27 (s, 1 H) 7.44 (dd, *J*=8.55, 1.83 Hz, 1 H) 7.67 (d, 1 H). LCMS: r.t. = 2.23 min., $[M+H]^+$ = 569 Phenomenex Luna C18 10u (3 x50 mm); Solvent A = 10% methanol - 90% water - 0.1% TFA, Solvent B = 90% methanol - 10% water - 0.1% TFA; gradient = 0% to 100% solvent B over 2 min. and then hold for 1 min.; 5 mL/min; inj. vol. = 5 ul; wavelength = 220 nm.

*Step 5:*

HATU (22 mg, 0.058 mmol) was added to a solution of (1R,2S)-1-amino-N-(cyclopropylsulfonyl)-2-vinylcyclopropanecarboxamide, pTSA salt (32 mg, 0.079 mmol), the product from Step 4 (30 mg, 0.053 mmol) and Hunig'sBase (0.028 mL, 0.158 mmol) in DCM (2 mL) and stirred at r.t. overnight. The reaction was concentrated and then purified by prep. HPLC (X-bridge C18 10u (30 x100 mm); flow = 42 mL/min; solvent gradient = 95:5 to 5:95 water/acetonitrile (with 10mM ammonium acetate)). The product fractions were concentrated to give Compound 14 (9 mg, 21%) as a white solid. $^1$H NMR (500 MHz, CHLOROFORM-*d*) d ppm 0.84 (s, 3 H) 1.04 (s, 3 H) 1.00 - 1.13 (m, 3 H) 1.14 (s, 9 H) 1.19 - 1.28 (m, 1 H) 1.28 - 1.45 (m, 3 H) 1.47 (dd, *J*=9.46, 5.49 Hz, 1 H) 1.65 - 1.76 (m, 1 H) 1.78 - 1.90 (m, 2 H) 1.93 - 1.98 (m, I H) 1.98 - 2.07 (m, 1 H) 2.25 (dd, *J*=11.75, 6.26 Hz, 1 H) 2.57 (t, *J*=11.75 Hz, 1 H) 2.70 - 2.80 (m, 1 H) 2.81 - 2.97 (m, 2 H) 3.20 (s, 3 H) 3.54 (d, *J*=10.38 Hz, 1 H) 3.77 (dd, *J*=11.60, 6.10 Hz, 1 H) 3.94 (d, *J*=9.77 Hz, 1 H) 3.94 (s, 3 H) 4.07 (d, *J*=10.38 Hz, 1 H) 4.67 (d, *J*=10.38 Hz, 1 H) 4.90 (d, *J*=10.07 Hz, 1 H) 5.11 (d, *J*=10.38 Hz, 1 H) 5.20 (dd, *J*=17.09, 0.92 Hz, 1 H) 5.62 (d, *J*=10.38 Hz, 1 H) 5.80 (ddd, *J*=17.17, 10.30, 8.55 Hz, 1 H) 6.35 (br. s., 1 H) 7.08 (s, 1 H) 7.28 (d, *J*=1.22 Hz, 1 H) 7.34 (s, 1 H) 7.53 (dd, *J*=8.55, 1.83 Hz, 1 H) 7.77 (d, *J*=8.55 Hz, 1 H) 9.85 (br. s., 1 H). LCMS: r.t. = 1.93 min., $[M+Na]^+$ = 803 Phenomenex Luna C 18 10u (3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Preparation of Compound 15*

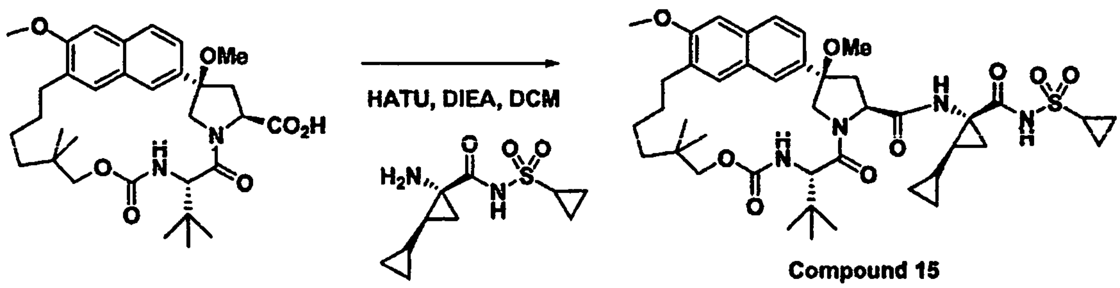


Compound 15

HATU (22 mg, 0.058 mmol) was added to a solution of (1S,2R)-2-amino-N-(cyclopropylsulfonyl)bi(cyclopropane)-2-carboxamide, HCl salt (22 mg, 0.079 mmol), the product from Example 14 Step 4 (30 mg, 0.053 mmol) and Hunig's Base (0.028 mL, 0.158 mmol) in DCM (2 mL) and stirred at r.t. overnight. The reaction was concentrated and then purified by prep. HPLC (X-bridge C18 10u (30 x100 mm); flow = 42 mL/min; solvent gradient = 95:5 to 5:95 water/acetonitrile (with 10mM ammonium acetate)). The product fractions were concentrated to give Compound 15 (14 mg,

33%) as a white solid. $^1$H NMR (500 MHz, CHLOROFORM-*d*) d ppm 0.15 - 0.29 (m, 2 H) 0.45 - 0.60 (m, 2 H) 0.83 (s, 3 H) 0.85 - 0.89 (m, 1 H) 0.91 - 0.97 (m, 1 H) 1.01 - 1.05 (m, 5 H) 1.07 - 1.11 (m, 1 H) 1.13 (s, 9 H) 1.15 - 1.18 (m, 1 H) 1.20 - 1.29 (m, 2 H) 1.34 - 1.46 (m, 3 H) 1.65 - 1.74 (m, 1 H) 1.77 - 1.89 (m, 3 H) 2.23 (dd, *J*=11.75, 6.26 Hz, 1 H) 2.57 (t, *J*=11.75 Hz, 1 H) 2.69 - 2.79 (m, 1 H) 2.81 - 2.91 (m, 1 H) 2.91 - 3.00 (m, 1 H) 3.19 (s, 3 H) 3.54 (d, *J*=10.38 Hz, 1 H) 3.78 (dd, *J*=11.29, 6.10 Hz, 1 H) 3.94 (s, 3 H) 3.91 - 3.95 (m, 1 H) 4.06 (d, *J*=10.38 Hz, I H) 4.66 (d, *J*=10.38 Hz, 1 H) 4.88 (d, *J*=10.07 Hz, I H) 5.60 (d, *J*=10.07 Hz, 1 H) 6.28 (br. s., 1 H) 7.07 (s, 1 H) 7.26 (d, *J*=1.53 Hz, 1 H) 7.33 (s, I H) 7.52 (dd, *J*=8.55, 1.83 Hz, 1 H) 7.76 (d, *J*=8.55 Hz, I H) 9.85 (br. s., 1 H). LCMS: r.t. =1.94 min., [M+Na]$^+$ = 817 Phenomenex Luna C18 10u (3 x50 mm); Solvent A =10% acetonitrile - 90% water - 0.1% TFA, Solvent B = 90% acetonitrile - 10% water - 0.1 % TFA; gradient = 0% to 100% solvent B over 2 min. and then hold for 1 min.; 4 mL/min; inj. vol. = 5 ul; wavelength = 220 nm.

*Preparation of Compound 16*

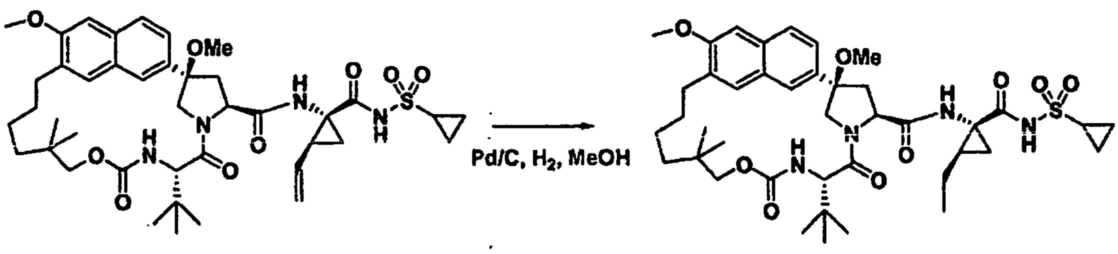


Compound 14

Compound 16

A solution of Compound 9 (10 mg, 0.013 mmol) in MeOH (2 mL) was passed through a Pd/C cartridge on the H-cube (flow = 1 mL/min; full $H_2$ saturation; pressure =1 bar). The eluent was concentrated to give the crude material which was purified by prep. HPLC (Sunfire C18 10u (30 x100 mm); flow = 42 mL/min; solvent gradient = 95:5 to 5:95 water/acetonitrile (with 10mM ammonium acetate)). The product fraction was concentrated and then lyopholized to give Compound 12 (2.5 mg, 25%) as a white solid. $^1$H NMR (500 MHz, CHLOROFORM-*d*) d ppm 0.84 (s, 3 H) 0.90 (t, *J*=7.32 Hz, 3 H) 1.01 - 1.06 (m, 5 H) 1.07 - 1.12 (m, 1 H) 1.15 (s, 9 H) 1.21 - 1.27 (m, 3 H) 1.30 - 1.37 (m, 2 H) 1.38 - 1.46 (m, 2 H) 1.51 - 1.65 (m, 2 H) 1.67 - 1.74 (m, 2 H) 1.79 - 1.89 (m, 2 H) 2.23 (dd, *J*=12.36, 6.56 Hz, 1 H) 2.57 (t, *J*=11.75 Hz, 1 H) 2.72 - 2.80 (m, 1 H) 2.81 - 2.90 (m, 1 H) 2.91 - 2.99 (m, 1 H) 3.20 (s, 3 H) 3.54 (d, *J*=10.07 Hz, 1 H) 3.77 (dd, *J*=11.60, 6.41 Hz, 1 H) 3.94 (s, 3 H) 3.94 (d, *J*=10.07 Hz, I H) 4.07 (d, *J*=10.38 Hz, 1 H) 4.68 (d, *J*=10.07 Hz, 1 H) 4.89 (d, *J*=9.77 Hz, 1 H) 5.62 (d, *J*=10.38 Hz, I H) 6.27 (br. s., 1 H) 7.07 (s, 1 H) 7.28 (d, *J*=1.83 Hz, I H) 7.34 (s, 1 H) 7.52 (dd, *J*=8.70, 1.98 Hz, 1 H) 7.76 (d, *J*=8.55 Hz, 1 H). LCMS: r.t. =1.94 min., [M+Na]$^+$ = 805 Phenomenex Luna C18 10u (3 x50 mm); Solvent A =10% acetonitrile - 90% water - 0.1% TFA, Solvent B = 90% acetonitrile - 10% water - 0.1 % TFA; gradient = 0% to 100% solvent B over 2 min. and then hold for 1 min.; 4 mL/min; inj. vol. = 5 ul; wavelength = 220 nm.

*Preparation of Compound 17*

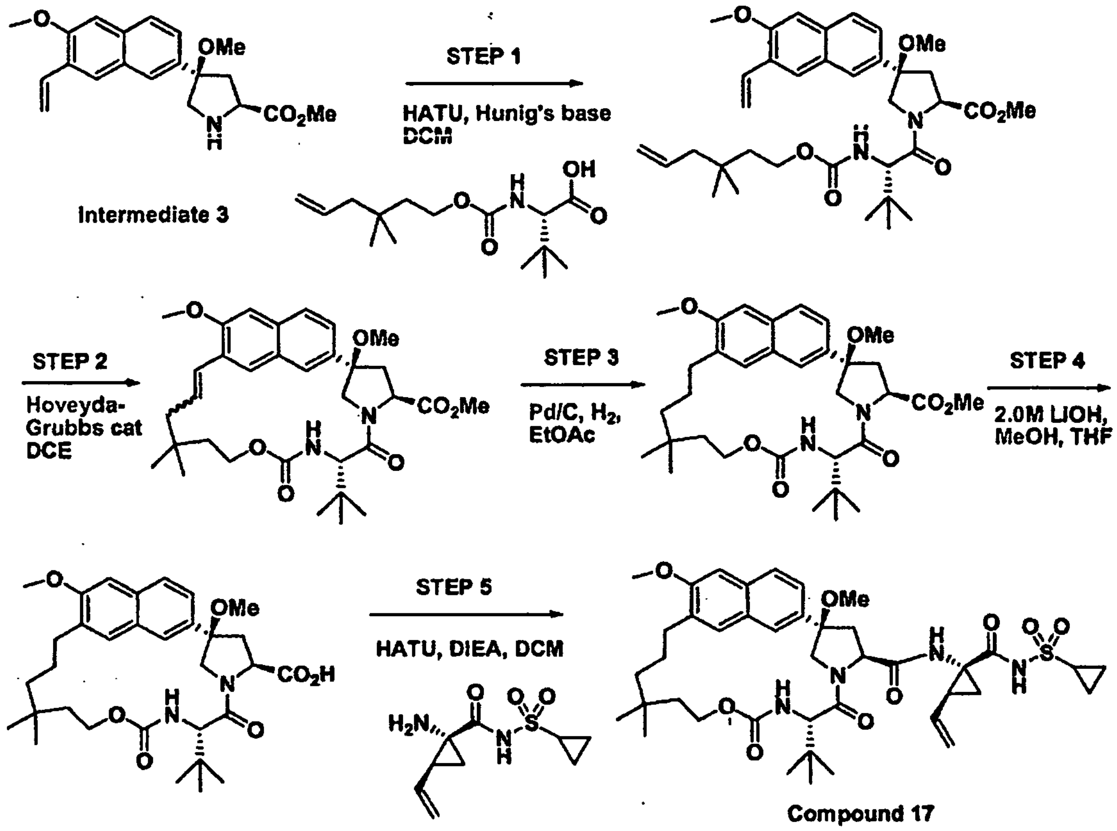


Intermediate 3

Compound 17

*Step 1:*

HATU (88 mg, 0.232 mmol) was added to a solution of (2S,4R)-methyl 4-methoxy-4-(6-methoxy-7-vinylnaphthalen-2-yl)pyrrolidine-2-carboxylate (Intermediate 3, 66 mg, 0.193 mmol), (S)-2-((3,3-dimethylhex-5-enyloxy)carbonylamino)-3,3-dimethylbutanoic acid (83 mg, 0.290 mmol) and Hunig'sBase (0.10 mL, 0.58 mmol) in DCM (5 mL) and stirred at r.t. overnight. The reaction was concentrated and purified by flash chromatography on the Biotage (10-40% EtOAc in hexanes) to give (2S,4R)-methyl 1-((S)-2-((3,3-dimethylhex-5-enyloxy)carbonylamino)-3,3-dimethylbutanoyl)-4-methoxy-4-(6-methoxy-7-vinylnaphthalen-2-yl)pyrrolidine-2-carboxylate (95 mg, 81%) as a colorless oil. LCMS: r.t. = 2.01 min., $[M+Na]^+$ = 631 Phenomenex Luna C18 10u (3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 2:*

A solution of (2S,4R)-methyl 1-((S)-2-((3,3-dimethylhex-5-enyloxy)carbonylamino)-3,3-dimethylbutanoyl)-4-methoxy-4-(6-methoxy-7-vinylnaphthalen-2-yl)pyrrolidine-2-carboxylate (95 mg, 0.156 mmol) in DCE (50 mL) was sparged with nitrogen for 30 min. and then Hoveyda-Grubbs Catalyst 2nd Generation (10 mg, 0.016 mmol) was added and the reaction sealed and heated to 80°C overnight. The reaction was concentrated to give crude material. The crude material was purified by flash chromatography on the Biotage (20-50% EtOAc in hexanes) to give the purified product (76 mg, 84%) as a colorless oil. LCMS: r.t. = 1.93 min., $[M+H]^+$ = 581 Phenomenex Luna C18 10u (3 x50 mm); Solvent A =10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step* 3:

Pd/C (14 mg, 0.013 mmol) was added to a solution of the product from Step 2 (76 mg, 0.131 mmol) in EtOAc (5 mL) and stirred under an atmosphere of hydrogen at r.t. for 4 hrs. The reaction was filtered through a nylon frit and concentrated to give the product (72 mg, 94%) as a white foam. LCMS: r.t. = 2.00 min., $[M+Na]^+$ = 605 Phenomenex Luna C18 10u (3 x50 mm); Solvent A =10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul;

wavelength = 220 nm. $^{1}$H NMR (500 MHz, CHLOROFORM-*d*) d ppm 0.90 (s, 3 H) 0.94 (s, 3 H) 1.13 (s, 9 H) 1.27 - -1.31 (m, 1 H) 1.44 (td, *J*=13.43, 3.66 Hz, 1 H) 1.70 (t, *J*=8.39 Hz, 2 H) 1.73 - 1.81 (m, 1 H) 1.82 - 1.94 (m, 1 H) 2.43 (t, *J*=11.60 Hz, 1 H) 2.62 (dd, *J*=11.75, 7.17 Hz, 1 H) 2.71 (ddd, *J*=16.71, 8.47, 4.12 Hz, 1 H) 2.84 - 2.94 (m, 1 H) 3.10 (s, 3 H) 3.71 (s, 3 H) 3.85 (d, *J*=10.07 Hz, 1 H) 3.93 (s, 3 H) 3.94 - 4.01 (m, 1 H) 4.10 - 4.15 (m, 1 H) 4.27 - 4.35 (m, 1 H) 4.53 (d, *J*=10.07 Hz, 1 H) 4.97 (d, *J*=10.07 Hz, 1 H) 5.40 (d, *J*=10.07 Hz, 1 H) 7.07 (s, 1 H) 7.51 (dd, *J*=8.55, 1.83 Hz, I H) 7.67 (d, *J*=1.22 Hz, 1 H) 7.70 (s, 1 H) 7.76 (d, *J*=8.55 Hz, 1 H).

*Step 4:*

2.0M LiOH (0.31 mL, 0.62 mmol) was added to a solution of the product from Step 3 (72 mg, 0.124 mmol) in THF (1 mL) and MeOH (1 mL) and stirred at r.t. overnight. The reaction was quenched with 1.0M HCl solution and ether. The organics were dried, filtered and concentrated to give the crude product (70 mg, 100%) which was used directly in the next step. LCMS: r.t. =1.79 min., [M+Na]$^{+}$ = 591 Phenomenex Luna C18 10u (3 x50 mm); Solvent A = 10% acetonitrile - 90% water - 0.1 % TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step* 5:

HATU (6.7 mg, 0.018 mmol) was added to a solution of (1R,2S)-1-amino-N-(cyclopropylsulfonyl)-2-vinylcyclopropanecarboxamide, pTSA salt (7.8 mg, 0.019 mmol), the product from Step 4 (10 mg, 0.018 mmol) and Hunig'sBase (0.009 mL, 0.053 mmol) in DCM (1 mL) and stirred at r.t. overnight. The reaction was concentrated and then purified by prep. HPLC (X-bridge C18 10u (30 x100 mm); flow = 42 mL/min; solvent gradient = 95:5 to 5:95 water/acetonitrile (with 10mM ammonium acetate)). The product fractions were concentrated to give Compound 17 (4 mg, 29%) as a white solid. $^{1}$H NMR (400 MHz, CHLOROFORM-*d*) d ppm 0.92 (s, 3H) 0.97 (s, 3 H) 0.99 - 1.06 (m, 2 H) 1.13 (s, 9 H) 1.23 - 1.41 (m, 6 H) 1.45 - 1.50 (m, 2 H) 1.71 - 1.78 (m, 2 H) 1.93 - 2.06 (m, 2 H) 2.39 (dd, *J*=12.05, 6.27 Hz, 1 H) 2.62 (t, *J*=11.42 Hz, 1 H) 2.69 - 2.78 (m, 1 H) 2.84 - 2.95 (m, 2 H) 3.14 (s, 3 H) 3.75 - 3.82 (m, 1 H) 3.89 (d, *J*=9.79 Hz, 1 H) 3.95 (s, 3 H) 3.97 - 4.04 (m, 1 H) 4.31 - 4.42 (m, 1 H) 4.53 (d, *J*=10.04 Hz, 1 H) 5.03 (d, *J*=10.04 Hz, 1 H) 5.10 (dd, *J*=10.54, 1.00 Hz, 1 H) 5.19 (d, *J*=16.06 Hz, 1 H) 5.43 (d, *J*=10.54 Hz, 1 H) 5.80 (ddd, *J*=17.19, 10.29, 8.41 Hz, 1 H) 6.32 (br. s., 1 H) 7.09 (s, 1 H) 7.52 (dd, *J*=8.53, 1.51 Hz, I H) 7.69 (s, 2 H) 7.78 (d, *J*=8.53 Hz, 1 H). LCMS: r.t. =1.96 min., [M+Na]$^{+}$ = 803 Phenomenex Luna C 18 10u (3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Preparation of Compound 18*

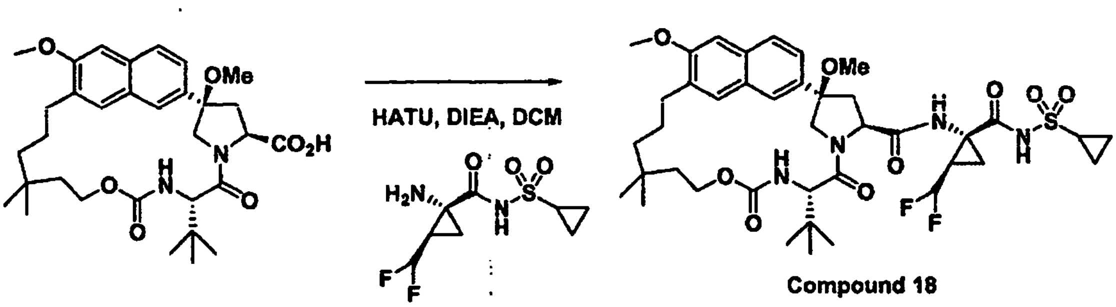


Compound 18

HATU (6.7 mg, 0.018 mmol) was added to a solution of (1R,2R)-1-amino-N-(cyclopropylsulfonyl)-2-(difluoromethyl)cyclopropanecarboxamide, HCl (5.6 mg, 0.019 mmol), the product from Example 17 Step 4 (10 mg, 0.018 mmol) and Hunig'sBase (9.2 μL, 0.053 mmol) in dichloromethane (1 mL) and stirred at r.t overnight. The reaction was concentrated and then purified by prep. HPLC (X-bridge C18 10u (30 x100 mm); flow = 42 mL/min; solvent gradient = 95:5 to 5:95 water/acetonitrile (with 10mM ammonium acetate)). The product fractions were concentrated to give Compound 18 (11 mg, 77%) as a white solid. $^{1}$H NMR (400 MHz, CHLOROFORM-d) d ppm 0.94 (s, 3 H) 0.98 (s, 3 H) 1.01 - 1.07 (m, 2 H) 1.14 (s, 9 H) 1.23 - 1.40 (m, 3 H) 1.46 - 1.67 (m, 4 H) 1.73 - 1.85 (m, 4 H) 1.87 - 1.98 (m, 1 H) 2.11 (t, *J*=7.28 Hz, 1 H) 2.36 (dd, *J*=11.92, 5.65 Hz, 1 H) 2.57 (t, *J*=11.80 Hz, 1 H) 2.66 - 2.77 (m, 1 H) 2.85 - 2.98 (m, 1 H) 3.13 (s, 3 H) 3.71 (dd, *J*=11.42, 6.40 Hz, 1 H) 3.89 (d, *J*=10.04 Hz, 1 H) 3.95 (s, 3 H) 3.99 - 4.09 (m, 1 H) 4.37 - 4.49 (m, 1 H) 4.56 (d, *J*=10.04 Hz, 1 H) 5.06 (d, *J*=10.04 Hz, 1 H) 5.47 (d, *J*=9.79 Hz, 1 H) 5.97 (td, *J*=55.71, 7.03 Hz, 1 H) 6.29

(br. s., 1 H) 7.08 (s, 1 H) 7.50 (dd, *J*=8.78, 1.76 Hz, 1 H) 7.71 (br. s., 1 H) 7.72 (br. s., 1 H) 7.78 (d, *J*=8.78 Hz, 1 H). LCMS: r.t. = 1.96 min., $[M+Na]^+$ = 826 Phenomenex Luna C18 10u (3 x50 mm); Solvent A = 10% acetonitrile - 90% water - 0.1% TEA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Preparation of Compound 19*

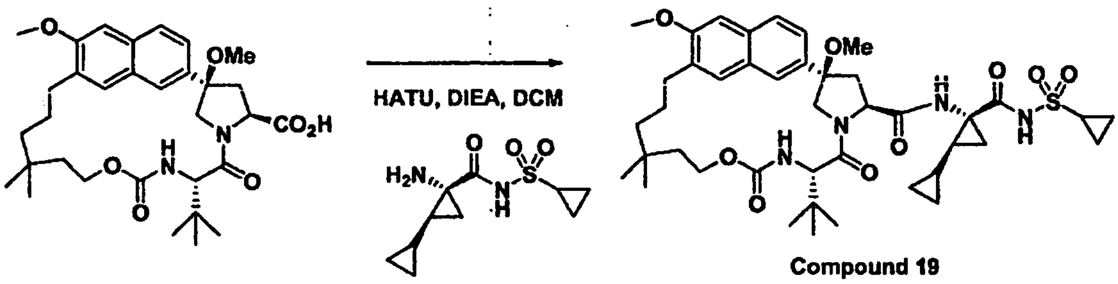


Compound 19

HATU (33.4 mg, 0.088 mmol) was added to a solution of (1S,2R)-2-amino-N-(cyclopropylsulfonyl)bi(cyclopropane)-2-carboxamide, TFA salt (34.7 mg, 0.097 mmol), the product from Example 17 Step 4 (50 mg, 0.088 mmol) and Hunig'sBase (46 µL, 0.264 mmol) in Dichloromethane (2 mL) and stirred at r.t. overnight. The reaction was concentrated and then purified by prep. HPLC (X-bridge C18 10u (30 x100 mm); flow = 42 mL/min; solvent gradient = 95:5 to 5:95 water/acetonitrile (with 10mM ammonium acetate)). The product fractions were concentrated to give Compound 19 (22 mg, 31 %) as a white solid. $^1$H NMR (400 MHz, CHLOROFORM-*d*) d ppm 0.14 - 0.28 (m, 2 H) 0.47 - 0.60 (m, 2 H) 0.82 - 0.90 (m, 1 H) 0.92 (s, 3 H) 0.97 (s, 3 H) 1.00 - 1.07 (m, 2 H) 1.12 (s, 9 H) 1.15 - 1.19 (m, 1 H) 1.22 - 1.32 (m, 3 H) 1.34 - 1.41 (m, 2 H) 1.44 - 1.51 (m, 1 H) 1.68 - 1.93 (m, 5 H) 2.38 (dd, *J*=12.55, 6.78 Hz, 1 H) 2.63 (t, *J*=11.67 Hz, 1 H) 2.68 - 2.78 (m, 1 H) 2.85 - 2.99 (m, 2 H) 3.13 (s, 3 H) 3.78 (dd, *J*=11.29, 6.53 Hz, 1 H) 3.87 (d, *J*=10.29 Hz, 1 H) 3.95 (s, 3 H) 3.96 - 4.03 (m, 1 H) 4.28 - 4.39 (m, 1 H) 4.51 (d, *J*=10.04 Hz, 1 H) 5.00 (d, *J*=10.29 Hz, 1 H) 5.40 (d, *J*=9.79 Hz, 1 H) 6.25 (s, 1 H) 7.08 (s, 1 H) 7.51 (dd, *J*=8.53, 1.76 Hz, 1 H) 7.68 (s, 2 H) 7.77 (d, *J*=8.53 Hz, 1 H). LCMS: r.t. = 1.98 min., $[M+Na]^+$ = 816 Phenomenex Luna C18 10u (3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Preparation of Compound 20*

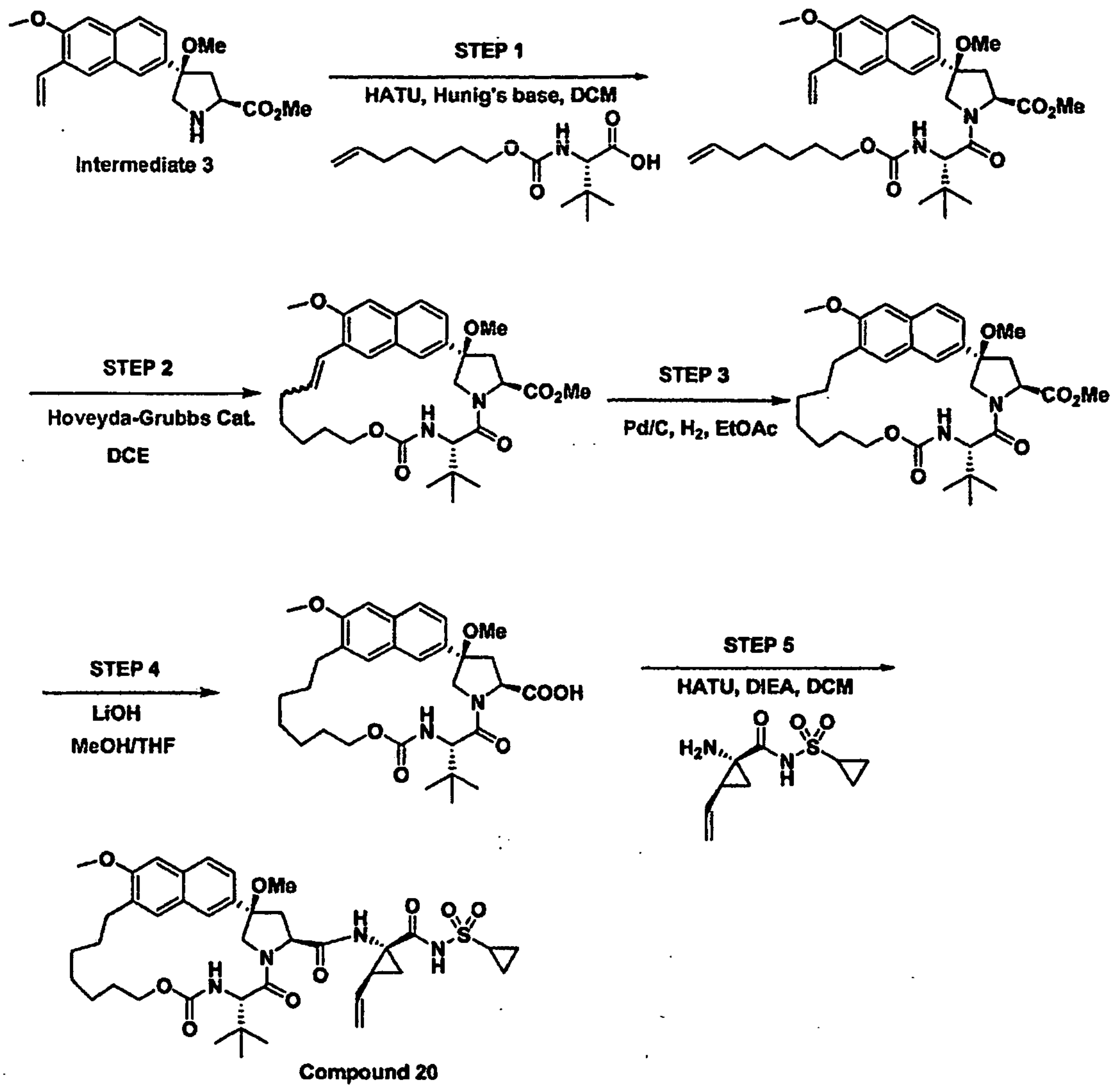


Intermediate 3

Compound 20

*Step 1:*

HATU (67 mg, 0.176 mmol) was added to a solution of (2S,4R)-methyl 4-methoxy-4-(6-methoxy-7-vinylnaphthalen-2-yl)pyrrolidine-2-carboxylate (50 mg, 0.146 mmol), (S)-2-((hept-6-enyloxy)carbonylamino)-3,3-dimethylbutanoic acid (44 mg, 0.161 mmol) and Hunig's base (0.077 mL, 0.439 mmol) in DCM (3 mL) and stirred at r.t. overnight. The reaction was concentrated and purified by flash chromatography on the Biotage (10-40% EtOAc in hexanes) to give (2S,4R)-methyl 1-((S)-2-((hept-6-enyloxy)carbonylamino)-3,3-dimethylbutanoyl)-4-methoxy-4-(6-methoxy-7-vinylnaphthalen-2-yl)pyrrolidine-2-carboxylate (50 mg, 0.084 mmol, 57% yield) as a whie solid. $^1H$ NMR (400 MHz, CHLOROFORM-d) δ ppm 0.88 - 0.95 (m, 1 H) 0.95 -1.01 (m, 1 H) 1.10 -1.21 (m, 7 H) 1.29 -1.50 (m, 5 H) 1.52 - 1.65 (m, 2 H) 2.00 - 2.12 (m, 1 H) 2.53 - 2.62 (m, 1 H) 2.83 - 2.98 (m, 4 H) 3.73 - 3.82 (m, 3 H) 3.92 - 4.01 (m, 4 H) 4.02 - 4.11 (m, 1 H) 4.15 - 4.26 (m, 2 H) 4.39 (d, *J*=9.79 Hz, 1 H) 4.74 - 4.84 (m, 1 H) 4.89 - 5.05 (m, 2 H) 5.34 - 5.45 (m, 2 H) 5.73 - 5.86 (m, 1 H) 5.86 - 5.96 (m, 1 H) 7.08 - 7.20 (m, 2 H) 7.39 - 7.45 (m, 1 H) 7.66 - 7.77 (m, 2 H) 7.94 (s, I H). LCMS: r.t. = 2.20 min., $[M+H]^+$ = 595 Phenomenex Luna S 10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. = 10 ul; wavelength = 220 nm.

*Step 2:*

A solution of (2S,4R)-methyl 1-((S)-2-((hept-6-enyloxy)carbonylamino)-3,3-dimethylbutanoyl)-4-methoxy-4-(6-methoxy-7-vinylnaphthalen-2-yl)pyrrolidine-2-carboxylate (50 mg, 0.084 mmol) in DCE (30 mL) was sparged with nitrogen for 30 min. and then Hoveyda-Grubbs Catalyst 2nd Generation (5 mg, 8.41 μmol) was added and the reaction sealed and heated to 80°C for 16 hours. The reaction was concentrated to give crude material. The crude material was

purified by flash chromatography on the Biotage (10-40% EtOAc in hexanes) to give the purified product (40 mg, 0.071 mmol, 84% yield) as a white solid. $^1$H NMR (400 MHz, CHLOROFORM-*d*) δ ppm 1.11 - 1.18 (m, 9 H) 1.50 - 1.60 (m, 2 H) 1.63 - 1.70 (m, 2 H) 2.39 - 2.52 (m, 2 H) 2.61 (d, *J*=7.28 Hz, 1 H) 3.02 - 3.10 (m, 3 H) 3.12 - 3.19 (m, 1 H) 3.69 - 3.74 (m, 4 H) 3.80 (d, *J*=10.54 Hz, 1 H) 3.86 - 3.93 (m, 1 H) 3.94 - 3.97 (m, 4 H) 4.04 (dd, *J*=11.29, 7.03 Hz, 1 H) 4.48 - 4.58 (m, 1 H) 4.67 (d, *J*=9.54 Hz, 1 H) 4.90 (d, *J*=10.04 Hz, 1 H) 5.52 (d, *J*=9.54 Hz, 1 H) 6.21 - 6.29 (m, 1 H) 6.80 (d, *J*=15.56 Hz, 1 H) 7.02 - 7.07 (m, 1 H) 7.46 - 7.56 (m, 2 H) 7.59 - 7.66 (m, 1 H) 7.75 (d, *J*=8.78 Hz, 1 H) 7.79 (s, 1 H). LCMS: r.t. = 2.20 min., $[M+H]^+$ = 567 Phenomenex Luna S10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. = 10 ul; wavelength = 220 nm.

*Step 3:*

A solution of the product from Step 2 (40 mg, 0.071 mmol) and 10% Pd/C (8 mg, 7.06 μmol) in EtOAc (2 mL) was stirred under an atmosphere of hydrogen for 20 hours. The reaction was filtered through a nylon frit and concentrated to give crude product (40 mg, 0.070 mmol, 100 % yield) as a white solid. LCMS: r.t. = 2.18 min., $[M+H]^+$ = 569 Phenomenex Luna S10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. = 10 ul; wavelength = 220 nm.

*Step 4:*

2.0 M lithium hydroxide (0.18 mL, 0.36 mmol) was added to a solution of the product from Step 3 (40 mg, 0.070 mmol) in THF (1 mL) and MeOH (1 mL) and stirred for 3 hours. The reaction was diluted with ether and 1.0 M HCl. The organic layer was dried, filtered and concentrated to give the crude product (35 mg, 0.063 mmol, 90 % yield). LCMS: r.t. = 1.97 min., $[M+H]^+$ = 555 Phenomenex Luna S10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. = 10 ul; wavelength = 220 nm.

*Step 5:*

HATU (36.0 mg, 0.095 mmol) was added to a solution of (1R,2S)-1-amino-N-(cyclopropylsulfonyl)-2-vinylcyclopropanecarboxamide, TsOH salt (30 mg, 0.076 mmol), the product from Step 4 (35 mg, 0.063 mmol) and Hunig's base (0.033 mL, 0.189 mmol) in dichloromethane (2 mL) and stirred at r.t. for 16 hours. The reaction was concentrated and purified by HPLC (Xbridge C 18 10u (30 x100 mm); flow = 42 mL/min; solvent gradient = 95:5 to 5:95 water/acetonitrile (with 10mM ammonium acetate)). The product fractions were concentrated to give Compound 20 (1.5 mg, 1.93 μmol, 3 % yield) as a white solid. $^1$H NMR (400 MHz, CHLOROFORM-d) 8 ppm 1.04 (br. s., 2 H) 1.11 (s, 9 H) 1.26 (br. s., 1 H) 1.11 - 1.14 (m, 1H) 1.30 - 1.47 (m, 6 H) 1.51 - 1.63 (m, 3 H) 1.68 - 1.86 (m, 5 H) 2.58 - 2.71 (m, 2 H) 2.78 (dd, *J*=12.55,10.29 Hz, 1 H) 2.85 - 2.95 (m, 1 H) 3.04 (s, 3 H) 3.79 - 3.88 (m, 2 H) 3.95 (s, 3 H) 4.12 (dd, *J*=10.04, 7.28 Hz, 1 H) 4.53 - 4.62 (m, 2 H) 4.82 (d, *J*=10.54 Hz, I H) 5.45 (d, *J*=9.79 Hz, 2 H) 5.91 (br. s, 1 H) 7.09 (s, 1 H) 7.50 - 7.57 (m, 2 H) 7.59 (s, 1 H) 7.78 (d, *J*=8.53 Hz, 1 H). LCMS: r.t. = 2.07 min., $[M-OMe]^+$ = 735 Phenomenex Luna S10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. = 10 ul; wavelength = 220 nm.

*Preparation of Compound 21:*

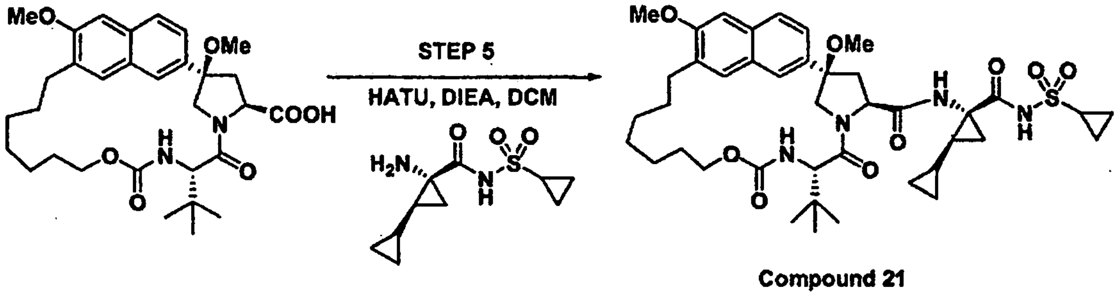


Compound 21

Compound 21 was prepared according to the preparation of compound 20 except using (1S,2R)-2-amino-N-(cyclopropylsulfonyl)bi(cyclopropane)-2-carboxamide, HCl salt in step 5 of the preparation. LCMS: r.t. = 2.11 min., $[M+Na]^+$ = 803 Phenomenex Luna C18 10u (3 x 50 mm); Solvent A = 10% methanol - 90% water - 0.1% TFA, Solvent B = 90% methanol - 10% water - 0.1% TFA; gradient = 0% to 100% solvent B over 2 min. and then hold for 1 min.; 4 mL/min; inj. vol. = 5 ul; wavelength = 220 nm. $^1$H NMR (500 MHz, MeOD) d ppm 0.25 (dddd, *J*=18.01, 8.93, 4.65, 4.43 Hz, 2 H) 0.37 - 0.50 (m, 1 H) 0.50 - 0.63 (m, 1 H) 0.72 - 0.87 (m, 1 H) 0.97 - 1.09 (m, 3 H) 1.12 (s, 9 H) 1.16 - 1.28 (m, 3 H) 1.28 - 1.39 (m, 3 H) 1.40 - 1.52 (m, 2 H) 1.55 - 1.69 (m, 2 H) 1.69 - 1.79 (m, 3 H) 1.85 (ddd, *J*=13.89, 7.17, 7.02 Hz, 1 H) 2.39 (t, *J*=11.75 Hz, 1 H) 2.55 (dd, *J*=11.75, 6.56 Hz, 1 H) 2.60 - 2.74 (m, 1 H) 2.85 - 2.99 (m, 2 H) 3.05 (s, 3 H) 3.78 - 3.89 (m, 3 H) 3.94 (s, 3 H) 4.51 - 4.71 (m, 2 H) 5.01 (d, *J*=10.68 Hz, 1 H) 7.08 (d, *J*=9.16 Hz, 1 H) 7.21 (s, 1 H) 7.53 (dd, *J*=8.85, 1.83 Hz, 1 H) 7.59 (s, 1 H) 7.73 (s, 1 H) 7.82 (d, *J*=8.54 Hz, 1 H).

*Preparation of Intermediate 4:*

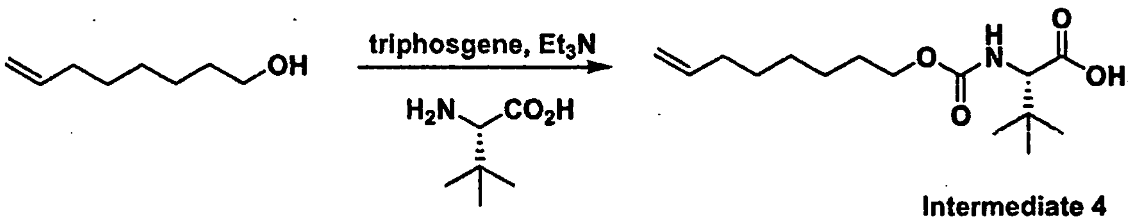


Intermediate 4

Hunig's base (1.331 mL, 7.62 mmol) was added dropwise to a 0 °C solution of oct-7-en-1-ol (1.075 g, 8.39 mmol) and triphosgene (1.131 g, 3.81 mmol) in dioxane (70 mL). The resulting white suspension was stirred for 5 min. at 0 °C, then allowed to warm up to room temperature for 1 hour. A solution of (S)-2-amino-3,3-dimethylbutanoic acid (1 g, 7.62 mmol) in dioxane (5 mL) along with IN NaOH (8 mL) was added. The reaction mixture was stirred at r.t. for 3 hrs and then diluted with ether and 1.0M HCl. The aqueous layer was extracted with ether two times. The combined organics were washed with brine, dried over $MgSO_4$, filtered and concentrated to give the crude product as light yellow oil which was taken into ether and extracted with sat. sodium bicarbonate three times. The combined aqueous layers were washed with ether and then acidified with conc. HCl and the product extracted with ether. The organics were dried with magnesium sulfate, filtered and concentrated to give (S)-3,3-dimethyl-2-((oct-7-enyloxy)carbonylamino)butanoic acid (Intermediate 4, 1.27 g, 4.46 mmol, 58.5% yield) as colorless oil. $^1$H NMR (500 MHz, CHLOROFORM-*d*) δ ppm 1.04 (s, 9 H) 1.31 - 1.44 (m, 6 H) 1.63 (qd, *J*=6.92, 6.71 Hz, 2 H) 2.02 - 2.08 (m, 2 H) 4.04 - 4.11 (m, 2 H) 4.19 (d, *J*=9.77 Hz, 1 H) 4.91 - 5.05 (m, 2 H) 5.21 (d, *J*=9.46 Hz, 1 H) 5.76 - 5.87 (m, *J*=17.05, 10.34, 6.60, 6.60 Hz, 1 H).

*Preparation of compound 22*

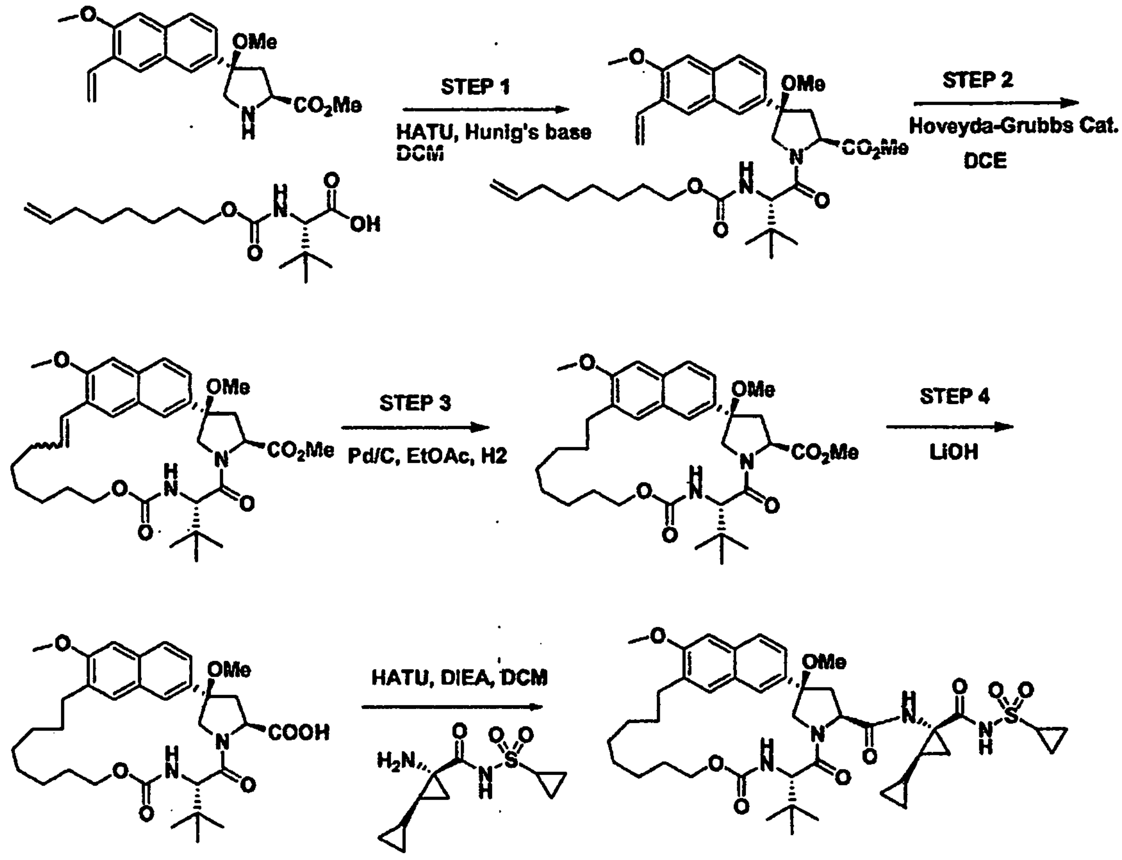


**Compound 22**

*Step 1:*

HATU (107 mg, 0.281 mmol) was added to a solution of (2S,4R)-methyl 4-methoxy-4-(6-methoxy-7-vinylnaphthalen-2-yl)pyrrolidine-2-carboxylate (80 mg, 0.234 mmol), (S)-3,3-dimethyl-2-((oct-7-enyloxy)carbonylamino)butanoic acid (73.6 mg, 0.258 mmol) and Hunig's base (0.123 mL, 0.703 mmol) in DCM (4 mL) and stirred at room temperture overnight. The reaction was concentrated and purified by flash chromatography on the Biotage (20-50% EtOAc in hexanes) to give (2S,4R)-methyl 1-((S)-3,3-dimethyl-2-((oct-7-enyloxy)carbonylamino)butanoyl)-4-methoxy-4-(6-methoxy-7-vinylnaphthalen-2-yl)pyrrolidine-2-carboxylate (87 mg, 0.143 mmol, 61% yield) as white foam. LCMS: r.t. = 4.59 min., $[M+H]^+$ = 609 Phenomenex Luna C 18, 50 x 2, 3u; Solvent A = 10% acetonitrile - 90% water - 0.1% TFA, Solvent B = 90% acetonitrile - 10% water - 0.1% TFA; gradient = 0% to 100% solvent B over 4 min. and then hold for 1 min.; 0.8 mL/min; inj. vol. = 3 ul; wavelength = 220 nm. $^1$H NMR (500 MHz, CHLOROFORM-*d*) 8 ppm 1.15 (s, 9 H) 1.25 - 1.43 (m, 7 H) 1.55 - 1.64 (m, 2 H) 1.99 - 2.06 (m, 2 H) 2.57 (dd, *J*=12.97, 8.70 Hz, 1 H) 2.85 - 2.91 (m, 1 H) 2.92 - 2.95 (m, 2 H) 3.73 - 3.78 (m, 3 H) 3.92 - 3.97 (m, 1 H) 3.98 (s, 3 H) 4.03 - 4.08 (m, 1 H) 4.16 - 4.24 (m, 2 H) 4.38 (d, *J*=9.77 Hz, 1 H) 4.82 (dd, *J*=8.55, 3.36 Hz, 1 H) 4.90 - 5.01 (m, 2 H) 5.37 - 5.43 (m, 2 H) 5.74 - 5.84 (m, 1 H) 5.88 - 5.94 (m, 1 H) 7.09 - 7.13 (m, 1 H) 7.13 - 7.19 (m, 1 H) 7.42 (dd, *J*=8.55,1.83 Hz, 1 H) 7.71 - 7.76 (m, 2 H) 7.94 (s, 1 H).

*Step 2:*

A solution of (2S,4R)-methyl 1-((S)-3,3-dimethyl-2-((oct-7-enyloxy)carbonylamino)butanoyl)-4-methoxy-4-(6-methoxy-7-vinylnaphthalen-2-yl)pyrrolidine-2-carboxylate (87 mg, 0.143 mmol) in DCE (50 mL) was sparged with nitrogen for 30 min. and then Hoveyda-Grubbs Catalyst (2nd generation) (9 mg, 0.014 mmol) was added and the reaction sealed and heated to 80°C for overnight. The reaction was concentrated to give crude material. The crude material was purified by flash chromatography on the Biotage (20-40% EtOAc in hexanes) to give the purified product (63 mg, 0.108 mmol, 76% yield) as white solid. LCMS: r.t. = 4.47 min., $[M+H]^+$ = 581 Phenomenex Luna C18, 50 x 2, 3u; Solvent A = 10% acetonitrile - 90% water - 0.1% TFA, Solvent B = 90% acetonitrile - 10% water - 0.1 % TFA; gradient = 0% to 100% solvent B over 4 min. and then hold for 1 min.; 0.8 mL/min; inj. vol. = 3 ul; wavelength = 220 nm. $^1$H NMR (500 MHz, CHLOROFORM-*d*) δ ppm 1.16 (br. s., 9 H) 1.36 - 1.54 (m, 2H) 1.77 (br. s., 2 H) 2.02 - 2.08 (m, 2 H) 2.32 (d, *J*=10.38 Hz, 1 H) 2.37 - 2.49 (m, 2 H) 2.50 - 2.58 (m, 1 H) 3.08 - 3.13 (m, 3 H) 3.69 - 3.73 (m, 3 H) 3.87 (d, *J*=7.63 Hz, 1 H) 3.96

(d, *J*=1.83 Hz, 3 H) 4.04 - 4.21 (m, 4 H) 4.54 (d, *J*=9.77 Hz, 1 H) 4.94 (d, *J*=7.93 Hz, 1 H) 5.39 (d, *J*=9.46 Hz, 1 H) 6.16 (dd, *J*=8.70, 5.95 Hz, 1 H) 6.77 (d, *J*=16.79 Hz, 1 H) 7.06 (br. s., 1 H) 7.52 (d, *J*=8.24 Hz, 2 H) 7.72 - 7.79 (m, 2 H) 7.82 (br. s., 1 H).

*Step 3:*

A solution of the product from Step 2 (63 mg, 0.108 mmol) and 10% Pd/C (12 mg, 10.85 μmol) in EtOAc (2 mL) was stirred under an atmosphere of hydrogen for overnight. The reaction was filtered through a nylon frit and concentrated to give crude product (62 mg, 0.106 mmol, 98% yield) as white solid. LCMS: r.t = 2.27 min., $[M-OMe]^+$ = 551 Phenomenex Luna S10 (3 x50 mm); Solvent A = 95% water- 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. = 10 ul; wavelength = 220 nm.

*Step 4:*

2.0 M lithium hydroxide (0.266 mL, 0.532 mmol) was added to a solution of the product from Step 3 (62 mg, 0.106 mmol) in THF (1 mL) and MeOH (1 mL) and stirred at room temperture for 3 hours. The reaction was quenched with 1.0M HCl solution and extracted with ether. The organics were dried, filtered and concentrated to give crude product (60 mg, 0.106 mmol, 99% yield) as a white foam. LCMS: r.t. = 1.99 min., $[M-OMe]^+$ = 537 Phenomenex Luna S10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. = 10 ul; wavelength = 220 nm.

*Step5:*

HATU (30.1 mg, 0.079 mmol) was added to a solution of (1S,2R)-2-amino-N-(cyclopropylsulfonyl)bi(cyclopropane)-2-carboxamide, HCl salt (17.77 mg, 0.063 mmol), the product from Step 4 (30 mg, 0.053 mmol) and Hunig's base (0.028 mL, 0.158 mmol) in dichloromethane (2 mL) and stirred at r.t. overnight. The reaction was concentrated and purified by HPLC (Xbridge C18 10u (30 x100 mm); flow = 42 mL/min; solvent gradient = 95:5 to 5:95 water/acetonitrile (with 10mM ammonium acetate)). The product fractions were concentrated to give Compound 22 (19.5 mg, 0.024 mmol, 46% yield) as a white solid. LCMS: r.t. = 2.13 min., $[M-OMe]^+$ = 764 Phenomenex Luna S10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. = 10 ul; wavelength = 220 nm. $^1H$ NMR (500 MHz, MeOD) δ ppm 0.19 - 0.29 (m, 2 H) 0.41 - 0.49 (m, 1 H) 0.50 - 0.57 (m, 1 H) 0.75 - 0.83 (m, 1 H) 0.96 - 1.03 (m, 1 H) 1.07 (ddd, *J*=7.78, 2.29, 2.14 Hz, 2 H) 1.13 (s, 9 H) 1.18 (dd, *J*=9.46,5.19 Hz, 1 H) 1.23 - 1.28 (m, 2 H) 1.35 - 1.63 (m, 8 H) 1.71 (dd, *J*=8.24, 5.19 Hz, 2 H) 1.77 - 1.86 (m, 2 H) 2.34 - 2.43 (m, 2 H) 2.69 - 2.75 (m, 2 H) 2.92 - 2.99 (m, 1 H) 3.13 (s, 3 H) 3.83 (dd, *J*=10.53, 7.17 Hz, 1 H) 3.89 (d, *J*=10.38 Hz, 1 H) 3.94 (s, 3 H) 4.08 (ddd, *J*=10.38, 8.09, 4.73 Hz, 1 H) 4.32 - 4.39 (m, 1 H) 4.54 - 4.58 (m, 1 H) 4.99 (d, *J*=10.38 Hz, 1 H) 7.19 - 7.24 (m, 2 H) 7.53 (dd, *J*=8.70, 1.98 Hz, 1 H) 7.59 (s, 1 H) 7.76 (s, 1 H) 7.82 (d, *J*=8.55 Hz, 1 H).

*Preparation of Compound 23:*

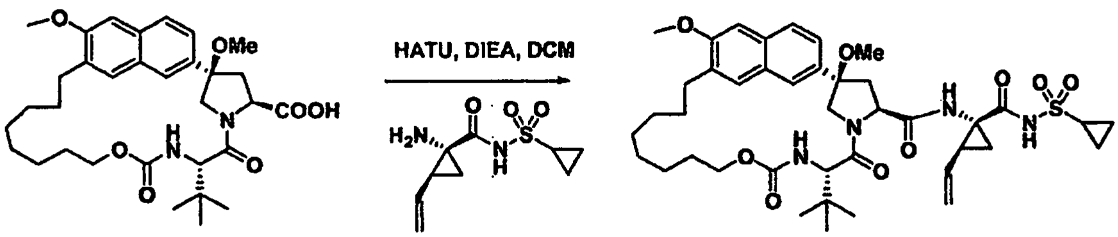


compound 23

Compound 23 was made using the same procedure as Compound 22 Step 5 except using (1R,2S)-1-amino-N-(cyclopropylsulfonyl)-2-vinylcyclopropanecarboxamide, TsOH salt. LCMS: r.t. = 2.08 min., $[M-OMe]^+$ = 749 Phenomenex Luna S10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1

min.; 4 mL/min; inj. vol. = 10 ul; wavelength = 220 nm. $^1$H NMR (500 MHz, MeOD) δ ppm 1.02 - 1.09 (m, 2 H) 1.14 (s, 9 H) 1.20 - 1.25 (m, 2 H) 1.38 (dd, *J*=9.31, 5.34 Hz, 2 H) 1.45 (br. s., 6 H) 1.49 - 1.76 (m, 4 H) 1.77 - 1.86 (m, 3 H) 2.12 (q, *J*=8.65 Hz, 1 H) 2.41 (d, *J*=9.16 Hz, 2 H) 2.68 - 2.77 (m, 2 H) 2.89 - 2.96 (m, 1 H) 3.13 (s, 3 H) 3.83 - 3.92 (m, 2 H) 3.94 (s, 3 H) 4.09 (ddd, *J*=10.15, 8.16, 4.88 Hz, 1 H) 4.32 - 4.39 (m, 1 H) 4.55 - 4.61 (m, 1 H) 4.99 (d, *J*=10.07 Hz, 1 H) 5.03 - 5.09 (m, 1 H) 5.22 (d, *J*=17.40 Hz, 1 H) 5.67 - 5.77 (m, 1 H) 7.19 - 7.25 (m, 2 H) 7.54 (dd, *J*=8.70,1.98 Hz, 1 H) 7.60 (s, 1 H) 7.77 (s, 1 H) 7.83 (d, *J*=8.55 Hz, 1 H).

*Preparation of Intermediate 5:*

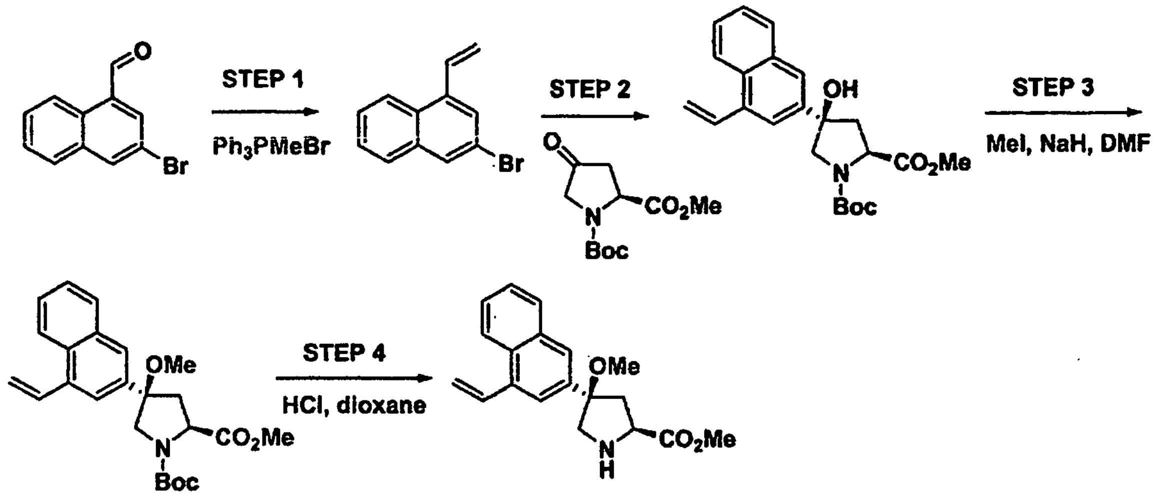


Intermediate 5

*Step 1:*

Sodium hydride (60% disp. in oil) (0.885 g, 22.12 mmol) was added to a solution of methyltriphenylphosphonium bromide (3.95 g, 11.06 mmol) in THF (25 mL) at 0°C and stirred for 30 min. A solution of 3-bromo-1-naphthaldehyde (made according to procedures outlined in WO 2004099143 (1.3 g, 5.53 mmol) in THF (25 mL) was added and the reaction was allowed to warm to r.t. overnight. The reaction was diluted with ether, quenched with water (2 mL), and filtered through diatomaceous earth (Celite®). The filtrate was concentrated and purified by flash chromatography on the Biotage (1-5% EtOAc in hexanes; 300 g) to give 3-promo-1-vinylnaphthalene (660 mg, 51%) as a colorless oil. $^1$H NMR (500 MHz, CHLOROFORM-*d*) d ppm 5.54 (dd, *J*=10.99, 1.22 Hz, 1 H) 5.82 (dd, *J*=17.09, 1.22 Hz, 1 H) 7.41 (dd, *J*=17.24, 10.83 Hz, 1 H) 7.48 - 7.57 (m, 2 H) 7.71 (d, *J*=2.14 Hz, 1 H) 7.73 - 7.80 (m, 1 H) 7.95 (d, *J*=1.83 Hz, 1 H) 8.03 - 8.11 (m, 1 H).

*Step 2:*

Magnesium (0.102 g, 4.18 mmol) was stirred in a round bottom. THF (2 mL) was added to the magnesium as well as a drop of 1,2-dibromoethane. This was heated to 60°C and after 10 min. at this temperature a solution of 3-bromo-1-vinylnaphthalene (0.65 g, 2.79 mmol) in THF (3 mL) was added. After the addition the reaction was continued at 70 °C for 2 hrs and then cooled to r.t. A solution of (S)-1-tert-butyl 2-methyl 4-oxopyrrolidine-1,2-dicarboxylate (0.6 g, 2.467 mmol) in toluene (10 mL) was added and stirred for 1 hr. The reaction was quenched with sat. ammonium chloride solution. The aqueous layer was extracted with ether and the combined organics were dried, filtered and concentrated to give crude material. The crude material was purified by flash chromatography on the Biotage (10-40% EtOAc in hexanes) to give (2S,4R)-1-tert-butyl 2-methyl 4-hydroxy-4-(4-vinylnaphthalen-2-yl)pyrrolidine-1,2-dicarboxylate (310 mg, 32%) as a white solid. $^1$H NMR (500 MHz, CHLOROFORM-*d*) d ppm 1.46 - 1.53 (m, 9 H) 2.32 - 2.50 (m, 1 H) 2.83 (ddd, *J*= 3.81, 9.99, 1.83 Hz, 1 H) 3.82 - 3.90 (m, 3 H) 3.86 - 4.09 (m, 2 H) 4.15 - 4.51 (m, 1 H) 4.52 - 4.66 (m, 1 H) 5.40 - 5.58 (m, 1 H) 5.82 (ddd, *J*=17.24, 12.21, 1.37 Hz, 1 H) 7.46 (ddd, *J*=11.37, 5.57, 5.34 Hz, 1 H) 7.49 - 7.59 (m, 2 H) 7.70 (d, *J*=2.44 Hz, 1 H) 7.86 (td, *J*=6.18, 2.29 Hz, 1 H) 7.96 (d, *J*=4.58 Hz, 1 H) 8.05 - 8.16 (m, 1 H).

*Step 3:*

NaH (60% in oil) (54 mg, 1.36 mmol) was added to a solution of (2S,4R)-1-tert-butyl 2-methyl 4-hydroxy-4-(4-vinylnaphthalen-2-yl)pyrrolidine-1,2-dicarboxylate (300 mg, 0.755 mmol) and iodomethane (0.085 mL, 1.359 mmol) in DMF (5 mL) at 0°C and stirred for 2 hrs. The reaction was quenched with sat. ammonium chloride and extracted with ether. The combined organics were dried, filtered and concentrated to give the crude material. The crude material was purified by flash chromatography on the Biotage (5-50% EtOAc in hexanes) to give (2S,4R)-1-tert-butyl 2-methyl 4-methoxy-4-(4-vinylnaphthalen-2-yl)pyrrolidine-1,2-dicarboxylate (280 mg, 90%) as a white foam. $^{1}$H NMR (500 MHz, CHLOROFORM-*d*) d ppm 1.44 - 1.54 (m, 9 H) 2.64 (ddd, *J*=17.40, 12.97, 8.70 Hz, 1 H) 2.77 - 2.92 (m, 1 H) 2.96 - 3.03 (m, 3 H) 3.69 - 3.82 (m, 4 H) 3.92 - 4.05 (m, 1 H) 4.38 - 4.68 (m, 1 H) 5.52 (td, *J*=10.07,1.22 Hz, 1 H) 5.82 (ddd, *J*=17.17, 8.93, 1.37 Hz, 1 H) 7.41 - 7.58 (m, 3 H) 7.60 - 7.66 (m, 1 H) 7.69 (s, 1 H) 7.81 - 7.87 (m, 1 H) 8.10 (dd, *J*=7.02, 2.14 Hz, 1 H).

*Step 4:*

4.0M HCl in dioxane (3.4 mL, 13.6 mmol) was added to a solution of (2S,4R)-1-tert-butyl 2-methyl 4-methoxy-4-(4-vinylnaphthalen-2-yl)pyrrolidine-1,2-dicarboxylate (280 mg, 0.680 mmol) in Dioxane (3 mL) and stirred for 2 hrs. The reaction was concentrated on the rotovap keeping the bath temperature below r.t. to give the HCl salt of (2S, 4R)-methyl 4-methoxy-4-(4-vinylnaphthalen-2-yl)pyrrolidine-2-carboxylate (Intermediate 5) as a white foam. $^{1}$H NMR (500 MHz, CHLOROFORM-*d*) d ppm 2.81 (dd, *J*=13.89, 9.61 Hz, 1 H) 3.01 (s, 3 H) 3.11 (d, *J*=13.73 Hz, 1 H) 3.83 (d, *J*=11.90 Hz, 1 H) 3.95 (s, 3 H) 4.20 (d, *J*=10.99 Hz, 1 H) 4.82 (d, *J*=9.46 Hz, 1 H) 5.56 (dd, *J*=10.99, 1.22 Hz, 1 H) 5.81 (dd, *J*=17.40, 1.22 Hz, 1 H) 7.45 (dd, *J*=17.40, 10.99 Hz, 1 H) 7.50 - 7.61 (m, 3 H) 7.69 (s, 1 H) 7.81 - 7.89 (m, 1 H) 8.11 (d, *J*=7.93 Hz, 1 H).

*Preparation of Compound 24*

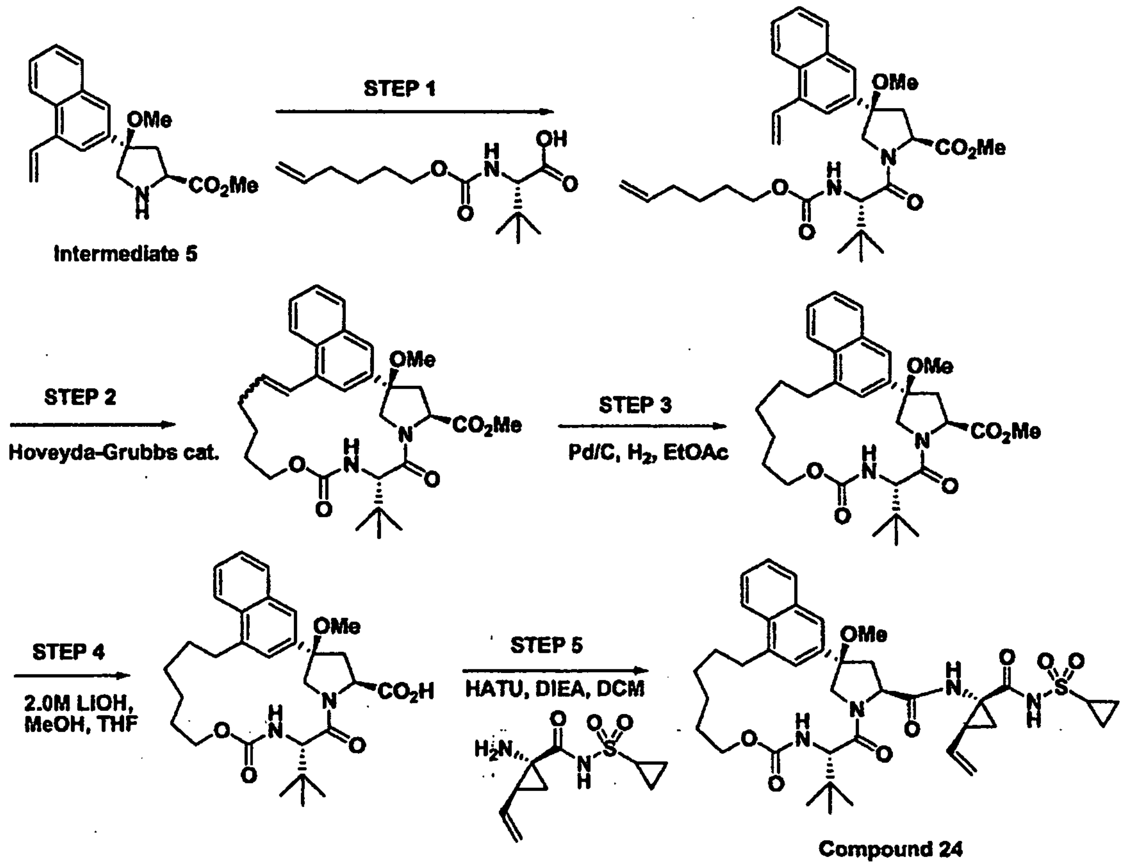


Compound 24

*Step 1:*

HATU (131 mg, 0.345 mmol) was added to a solution of (2S,4R)-methyl 4-methoxy-4-(4-vinylnaphthalen-2-yl) pyrrolidine-2-carboxylate, HCl salt (100 mg, 0.287 mmol), (S)-2-((hex-5-enyloxy)carbonylamino)-3,3-dimethylbutanoic acid (111 mg, 0.431 mmol) and Hunig'sBase (0.151 mL, 0.862 mmol) in DCM (5 mL) and stirred at r.t. for 3 hrs. The reaction was concentrated and purified by flash chromatography on the Biotage (10-40% EtOAc in hexanes) to give the product (95 mg, 60%) as a white foam. LCMS: r.t. = 1.76 min., $[M-H]^-$ = 549 Luna C18 10u (3 x 50 mm); Solvent A = 5% acetonitrile - 95% water - 10 mM ammonium acetate, Solvent B = 95% acetonitrile - 5% water - 10 mM ammonium acetate; gradient = 0% to 100% solvent B over 2 min. and then hold for 1 min.; 5 mL/min; imj. vol. = 10 ul; wavelength = 220 nm.

*Step 2:*

A solution of (2S,4R)-methyl 1-((S)-2-(((hex-5-enyloxy)carbonylamino)-3,3-dimethylbutanoyl)-4-methoxy-4-(4-vinylnaphthalen-2-yl)pyrrolidine-2-carboxylate (95 mg, 0.173 mmol) in DCE (30 mL) was sparged with nitrogen for 30 min. and then Hoveyda-Grubbs catalyst (2nd Generation) (10.8 mg, 0.017 mmol) was added and the reaction was sealed and heated to 90°C overnight. The reaction was concentrated and purified by flash chromatography on the Biotage (10-50% EtOAc in hexanes) to give the product (82 mg, 91%) as a light yellow foam. LCMS: r.t. = 1.75 min., $[M+Na]^+$ = 545 Phenomenex Luna C 18 10u (3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 3*:

10% Pd/C (17 mg, 0.016 mmol) was added to a solution of the product from Step 2 (82 mg, 0.157 mmol) in EtOAc (3 mL) and stirred under an atmosphere of hydrogen overnight. The reaction was filtered through a nylon frit and concentrated to give the crude product (73 mg, 89%) as a white foam. LCMS: r.t. = 1.73 min., $[M+Na]^+$ = 547 Phenomenex Luna C18 10u (3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 4:*

2.0M LiOH (0.35 mL, 0.70 mmol) was added to a solution of the product from Step 3 (73 mg, 0.139 mmol) in THF (1 mL) and MeOH (1 mL) and stirred at r.t. overnight. The reaction was diluted with EtOAc and 1.0M HCl. The organics were dried, filtered and concentrated to give the crude product (72 mg, 100%) as a white foam. LCMS: r.t. = 1.51 min., $[M+Na]^+$ = 533 Phenomenex Luna C18 10u (3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 5:*

HATU (16 mg, 0.043 mmol) was added to a solution of (1R,2S)-1-amino-N-(cyclopropylsulfonyl)-2-vinylcyclopropanecarboxamide, pTSA salt (17 mg, 0.043 mmol), the product from Step 4 (20 mg, 0.039 mmol) and Hunig's base (0.021 mL, 0.118 mmol) in dichloromethane (1 mL) and stirred at r.t. overnight. The reaction was concentrated and the crude material was purified by prep. HPLC (Sunfire C18 10u (30 x100 mm); flow = 42 mL/min; solvent gradient = 95:5 to 5:95 water/acetonitrile (with 0.1% TFA)). The product fractions were concentrated to give Compound 24 (12 mg, 42%) as a white solid. $^1H$ NMR (500 MHz, CHLOROFORM-*d*) d ppm 0.94 - 1.13 (m, 11 H) 1.17 - 1.74 (m, 9 H) 1.75 - 2.15 (m, 6 H) 2.35 - 2.99 (m, 3 H) 3.01 - 3.25 (m, 4 H) 3.28 - 4.36 (m, 4 H) 4.49 - 4.80 (m, 2 H) 5.04 - 5.35 (m, 2 H) 5.45 - 5.86 (m, 2 H) 6.64 - 7.09 (m, 1 H) 7.46 - 7.62 (m, 2 H) 7.64 - 7.94 (m, 2 H) 8.04 (d, *J*=5.80 Hz, 1 H) 9.96 (br. s., 1 H). LCMS: r.t. = 1.76 min., $[M+Na]^+$ = 745 Phenomenex Luna C18 10u (3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Preparation of Compound 25*

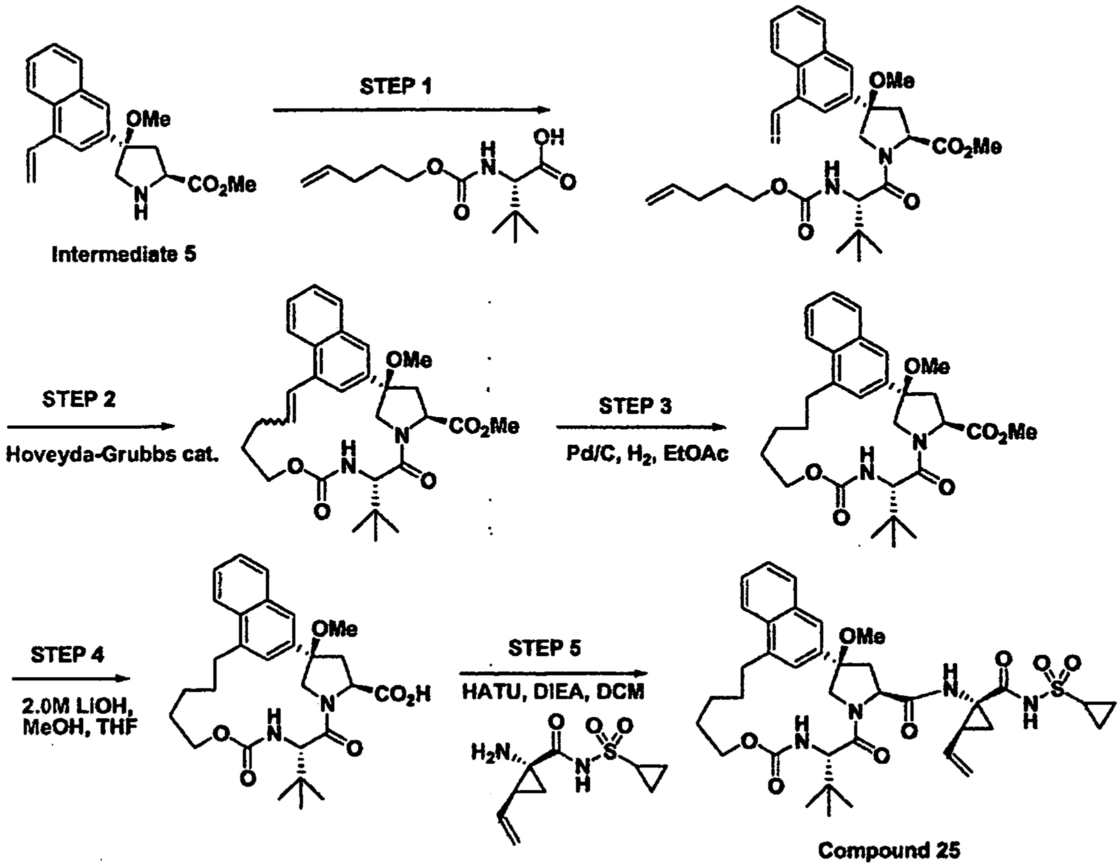


Intermediate 5

Compound 25

*Step 1:*

HATU (131 mg, 0.345 mmol) was added to a solution of (2S,4R)-methyl 4-methoxy-4-(4-vinylnaphthalen-2-yl) pyrrolidine-2-carboxylate, HCl salt (100 mg, 0.287 mmol), (S)-2-((pent-4-enyloxy)carbonylamino)-3,3-dimethylbutanoic acid (105 mg, 0.431 mmol) and Hunig's base (0.151 mL, 0.862 mmol) in DCM (5 mL) and stirred at r.t. for 3 hrs. The reaction was concentrated and purified by flash chromatography on the Biotage (10-40% EtOAc in hexanes) to give the product (85 mg, 55%) as a white foam. LCMS: r.t. =1.69 min., $[M-H]^-$ = 535 Luna C18 10u (3 x 50 mm); Solvent A = 5% acetonitrile - 95% water -10 mM ammonium acetate, Solvent B = 95% acetonitrile - 5% water - 10 mM ammonium acetate; gradient = 0% to 100% solvent B over 2 min. and then hold for 1 min.; 5 mL/min; inj. vol. =10 ul; wavelength = 220 nm.

*Step 2:*

A solution of (2S,4R)-methyl 1-((S)-2-((pent-4-enyloxy)carbonylamino)-3,3-dimethylbutanoyl)-4-methoxy-4-(4-vinylnaphthalen-2-yl)pyrrolidine-2-carboxylate (85 mg, 0.158 mmol) in DCE (30 mL) was sparged with nitrogen for 30 min. and then Hoveyda-Grubbs catalyst (2nd Generation) (10 mg, 0.016 mmol) was added and the reaction was sealed and heated to 90°C overnight. The reaction was concentrated and purified by flash chromatography on the Biotage (10-50% EtOAc in hexanes) to give the product (80 mg, 99%) as a light yellow foam. LCMS: r.t. =1.65 min., $[M+Na]^+$ = 531 Phenomenex Luna C18 10u (3 x50 mm); Solvent A =10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 3:*

10% Pd/C (17 mg, 0.016 mmol) was added to a solution of the product from Step 2 (80 mg, 0.157 mmol) in EtOAc (3 mL) and stirred under an atmosphere of hydrogen overnight. The reaction was filtered through a nylon frit and concentrated to give the crude product (73 mg, 91%) as a white foam. LCMS: r.t. =1.64 min., $[M+Na]^+$ = 533 Phenomenex Luna C18 10u (3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul;

wavelength = 220 nm.

*Step 4:*

2.0M LiOH (0.36 mL, 0.72 mmol) was added to a solution of the product from Step 3 (73 mg, 0.143 mmol) in THF (1 mL) and MeOH (1 mL) and stirred at r.t. overnight. The reaction was diluted with EtOAc and 1.0M HCl. The organics were dried, filtered and concentrated to give the crude product (72 mg, 100%) as a white foam. LCMS: r.t. =1.42 min., $[M+Na]^+$ = 519 Phenomenex Luna C18 10u (3 x50 mm); Solvent A = 10% acetonitrile - 90% water - 0.1% TFA, Solvent B = 90% acetonitrile - 10% water - 0.1 % TFA; gradient = 0% to 100% solvent B over 2 min. and then hold for 1 min.; 4 mL/min; inj. vol. = 5 ul; wavelength = 220 nm.

*Step 5:*

HATU (17 mg, 0.044 mmol) was added to a solution of (1R,2S)-1-amino-N-(cyclopropylsulfonyl)-2-vinylcyclopropanecarboxamide, pTSA salt (18 mg, 0.044 mmol), the product from Step 4 (20 mg, 0.039 mmol) and Hunig's base (0.021 mL, 0.118 mmol) in dichloromethane (1 mL) and stirred at r.t. overnight. The reaction was concentrated and the crude material was purified by prep. HPLC (Sunfire C18 10u (30 x 100 mm); flow = 42 mL/min; solvent gradient = 95:5 to 5:95 water/acetonitrile (with 0.1% TFA)). The product fractions were concentrated to give Compound 25 (8 mg, 28%) as a white solid. $^1H$ NMR (500 MHz, CHLOROFORM-d) d ppm 0.85 - 0.95 (m, 9 H) 0.96 - 1.10 (m, 2 H) 1.14 - 1.65 (m, 9 H) 1.63 -1.97 (m, 4H) 2.48 - 2.84 (m, 2 H) 2.85 - 2.93 (m, 1 H) 2.97 - 3.08 (m, 3 H) 3.11 - 3.62 (m, 1 H) 3.64 - 3.78 (m, 1 H) 4.07 (t, *J*=9.46 Hz, 1 H) 4.13 - 4.24 (m, 1 H) 4.31 - 4.47 (m, 2 H) 4.51 - 4.75 (m, 1 H) 5.15 (t, *J*=9.46 Hz, 1 H) 5.22 - 5.36 (m, 1 H) 5.47-5.86 (m, 2H) 7.17-7.35 (m, 1 H) 7.44 - 7.60 (m, 2 H) 7.60 - 7.76 (m, 1 H) 7.88 (dd, *J*=16.17, 7.63 Hz, 1 H) 8.03 (t, *J*=9.00 Hz, 1 H) 9.72 - 10.12 (m, 1 H) LCMS: r.t. =1.67 min., $[M+Na]^+$ = 731 Phenomenex Luna C18 10u (3 x50 mm); Solvent A =10% acetonitrile - 90% water - 0.1% TFA, Solvent B = 90% acetonitrile - 10% water - 0.1% TFA; gradient = 0% to 100% solvent B over 2 min.and then hold for 1 min.; 4 mL/min; inj. vol. = 5 ul; wavelength = 220 nm.

*Preparation of Compound 26*

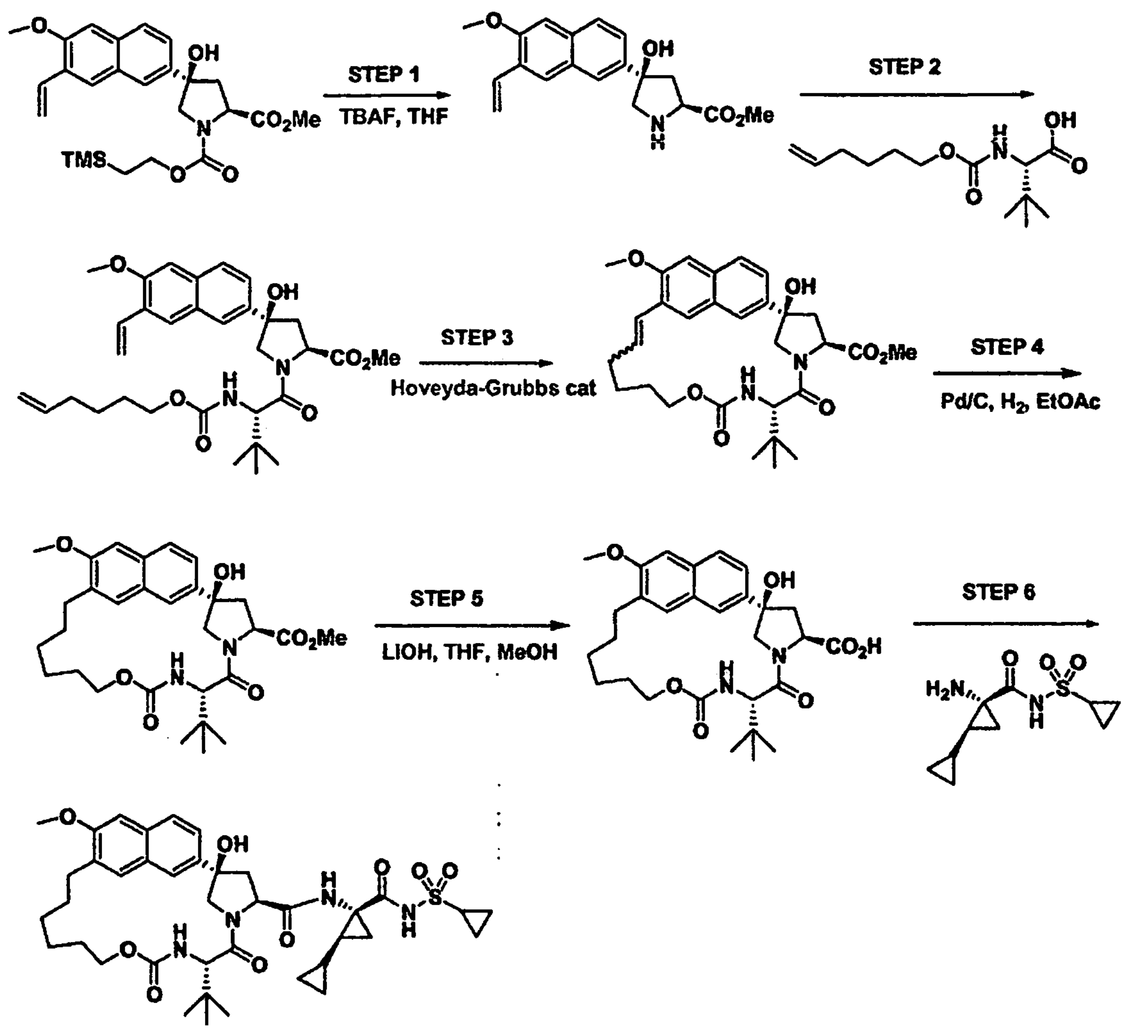


**Compound 26**

*Step 1:*

TBAF (1.0M in THF) (4.24 mL, 4.24 mmol) was added to a solution of (2S,4R)-2-methyl 1-(2-(trimethylsilyl)ethyl) 4-hydroxy-4-(6-methoxy-7-vinylnaphthalen-2-yl)pyrrolidine-1,2-dicarboxylate (the product from Step 1 in the synthesis of Intermediate 3) (0.5 g, 1.06 mmol) in THF (10 mL) at r.t. and stirred for 3 hrs. The reaction was diluted with water and ether. The water layer was extracted with ether and the combined organics were washed with brine, dried, filtered and concentrated to give the crude product which was used directly in the next step.

*Step 2:*

HATU (334 mg, 0.880 mmol) was added to a solution of (2S,4R)-methyl 4-hydroxy-4-(6-methoxy-7-vinylnaphthalen-2-yl)pyrrolidine-2-carboxylate (240 mg, 0.733 mmol), (S)-2-((hex-5-enyloxy)carbonylamino)-3,3-dimethylbutanoic acid (283 mg, 1.100 mmol) and Hunig's base (0.384 mL, 2.199 mmol) in DCM (10 mL) and stirred at r.t. for 3 days. The reaction was concentrated and purified by flash chromatography on the Biotage (10-40% EtOAc in hexanes) to give (2S,4R)-methyl 1-((S)-2-((hex-5-enyloxy)carbonylamino)-3,3-dimethylbutanoyl)-4-hydroxy-4-(6-methoxy-7-vinylnaphthalen-2-yl)pjrrolidine-2-carboxylate (100 mg, 24%) as a white foam. LCMS: r.t. =1.66 min., $[M+Na]^+$ = 589 Phenomenex Luna C18 10u (3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 3:*

A solution of (2S,4R)-methyl 1-((S)-2-((hex-5-enyloxy)carbonylamino)-3,3-dimethylbutanoyl)-4-hydroxy-4-(6-methoxy-7-vinylnaphthalen-2-yl)pyrrolidine-2-carboxylate (100 mg, 0.176 mmol) in DCE (40 mL) was sparged with nitrogen for 30 min. and then Hoveyda-Grubbs catalyst (2nd Generation) (11 mg, 0.018 mmol) was added and the reaction sealed and heated to 80°C overnight. The reaction was concentrated to give crude material. The crude material was purified by flash chromatography on the Biotage (20-50% EtOAc in hexanes) to give the purified product (48 mg, 50%) as a white foam. LCMS: r.t. =1.44 min., $[M+Na]^+$ = 561 Phenomenex Luna C18 10u (3 x50 mm); Solvent A = 10% acetonitrile - 90% water - 0.1 % TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 4:*

10% Pd/C (10 mg, 8.91 μmol) was added to a solution of the product from Step 3 (48 mg, 0.089 mmol) in EtOAc (2 mL) and stirred under an atmoshpere of hydrogen for 3 hrs. The reaction was filtered through a nylon frit and concentrated to give the crude product (48 mg, 100%) as a lightly colored foam. LCMS: r.t. = 1.53 min., $[M+Na]^+$ = 563 Phenomenex Luna C18 10u (3 x50 mm); Solvent A =10% acetonitrile - 90% water - 0.1 % TFA, Solvent B = 90% acetonitrile - 10% water - 0.1 % TFA; gradient = 0% to 100% solvent B over 2 min. and then hold for 1 min.; 4 mL/min; inj. vol. = 5 ul; wavelength = 220 nm.

*Step 5:*

2.0M LiOH (0.22 mL, 0.44 mmol) was added to a solution of the product from Step 4 (48 mg, 0.089 mmol) in THF (1 mL) and MeOH (1 mL) and stirred at r.t for 2 hrs. The reaction was diluted with 1.0M HCl and ether. The organics were dried, filtered and concentrated to give the crude product (45 mg, 96%). LCMS: r.t. = 1.35 min., $[M+Na]^+$ = 549 Phenomenex Luna C18 10u(3 x50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 6:*

HATU (48 mg, 0.128 mmol) was added to a solution of (1 S,2R)-2-amino-N-(cyclopropylsulfonyl)bi(cyclopropane)-2-carboxamide, HCl salt (29 mg, 0.103 mmol), the product from Step 5 (45 mg, 0.085 mmol) and Hunig's base (0.045 mL, 0.256 mmol) in dichloromethane (2 mL) and stirred at r.t. for 4 hrs. The reaction was concentrated and then purified by prep. HPLC (Sunfire C18 10u (30 x100 mm); flow = 42 mL/min; solvent gradient = 95:5 to 5:95 water/acetonitrile (with 10mM ammonium acetate)). The product fractions were concentrated to give Compound 26 (22 mg, 34%) as a white solid. $^1H$ NMR (500 MHz, CHLOROFORM-*d*) d ppm 0.15 - 0.29 (m, *J*=13.81, 9.42, 4.84, 4.73, 4.73 Hz, 2 H) 0.42 - 0.62 (m, 2 H) 0.76 - 0.85 (m, 1 H) 0.90 (q, *J*=8.44 Hz, 1 H) 0.96 - 1.16 (m, 3 H) 1.08 (s, 9 H) 1.17 - 1.28 (m, 1 H) 1.31 - 1.44 (m, 4 H) 1.56 - 1.72 (m, 3 H) 1.75 (dd, *J*=7.93, 5.80 Hz, I H) 1.79 - 1.90 (m, 2 H) 2.40 (dd, *J*=11.90, 6.71 Hz, 1 H) 2.61 (1, *J*=11.29 Hz, 1 H) 2.65 - 2.74 (m, 1H) 2.81 - 3.01 (m, 3 H) 3.86 - 4.00 (m, 6 H) 4.13 - 4.31 (m, 1 H) 4.53 (d, *J*=10.07 Hz, 1 H) 4.84 (d, *J*=10.68 Hz, 1 H) 5.59 (d, *J*=10.07 Hz, 1 H) 6.61 (s, 1 H) 7.05 (s, 1 H) 7.43 (s, 1 H) 7.47 (s, 1 H) 7.65 (d, *J*=8.55 Hz, 1 H) 7.73 (d, *J*=8.55 Hz, 1 H) 9.91 (br. s., 1 H). LCMS: r.t. = 1.59 min., $[M+Na]^+$ = 775 Phenomenex Luna C18 10u (3 x50 mm); Solvent A = 10% acetonitrile - 90% water - 0.1% TFA, Solvent B = 90% acetonitrile - 10% water - 0.1 % TFA; gradient = 0% to 100% solvent B over 2 min. and then hold for 1 min.; 4 mL/min; inj. vol. = 5 ul; wavelength = 220 nm.

*Preparation of Compound 27*

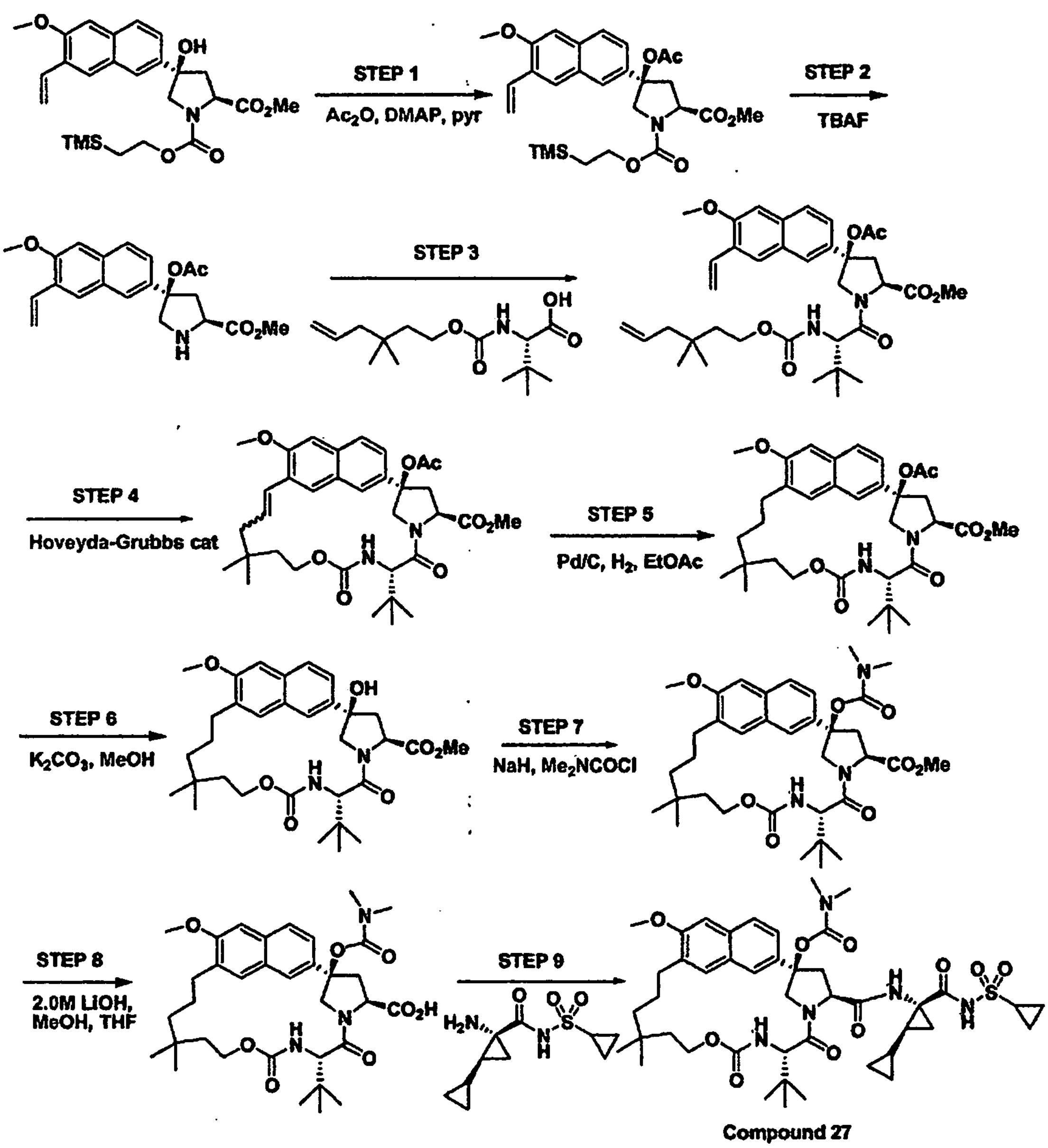


Compound 27

*Step 1:*

Acetic anhydride (0.75 mL, 7.95 mmol) was added to a solution of (2S,4R)-2-methyl 1-(2-(trimethylsilyl)ethyl) 4-hydroxy-4-(6-methoxy-7-vinylnaphthalen-2-yl)pyrrolidine-1,2-dicarboxylate (the product from Step 1 in the synthesis of Intermediate 3) (2.5 g, 5.30 mmol), DMAP (0.032 g, 0.265 mmol) and pyridine (4.29 mL, 53.0 mmol) in DCM (25 mL) at r.t. and heated to 40°C for 3 days. The reaction was quenched with sat. ammonium chloride solution and the aqueous layer extracted with EtOAc. The combined organics were dried with magnesium sulfate, filtered and concentrated to give the crude product. The crude was purified by flash chromatography on the Biotage (20-40% EtOAc in hexanes) to give (2S,4R)-2-methyl 1-(2-(trimethylsilyl)ethyl) 4-acetoxy-4-(6-methoxy-7-vinylnaphthalen-2-yl)pyrrolidine-1,2-dicarboxylate (1.05 g, 39%) as a yellow foam. $^1$H NMR (400 MHz, CHLOROFORM-*d*) $\delta$ ppm -0.01 - 0.18 (m, 9 H) 0.90 - 1.02 (m, 1 H) 1.02 - 1.13 (m, 1 H) 1.94 - 2.05 (m, 3 H) 2.72 - 2.91 (m, 1 H) 2.98 - 3.17 (m, 1 H) 3.80 (s, 3 H) 3.96 (s, 3 H) 4.10 - 4.35 (m, 3 H) 4.46 - 4.60 (m, 1 H) 5.38 (dd, *J*=11.17, 1.38 Hz, 1 H) 5.89 (dd, *J*=17.69, 1.13 Hz, 1 H) 7.08 (s, 1 H) 7.13 (dd, *J*= 17.69, 11.17 Hz, 1 H) 7.40 (t, *J*=7.15 Hz, 1 H) 7.69 (d, *J*=8.53 Hz, 1 H) 7.71 - 7.80 (m, 1 H) 7.87 (s, 1 H).

*Step 2:*

TBAF (1.0M in THF) (4.1 mL, 4.1 mmol) was added to a solution of (2S,4R)-2-methyl 1-(2-(trimethylsilyl)ethyl) 4-acetoxy-4-(6-methoxy-7-vinylnaphthalen-2-yl)pyrrolidine-1,2-dicarboxylate (1.05 g, 2.044 mmol) in THF (20 mL) and

stirred overnight at r.t. The reaction was quenched with water and extracted with EtOAc. The combined organics were dried with magnesium sulfate, filtered and concentrated to give the crude product (740 mg, 98%) as a brown foam. LCMS: r.t. =1.31 min., $[M-OAc]^+$ = 310 Waters Sunfire C18 5u (4.6 x 30 mm); Solvent A =10% methanol - 90% water - 0.1% TFA, Solvent B = 90% methanol - 10% water - 0.1% TFA; gradient = 0% to 100% solvent B over 2 min. and then hold for 1 min.; 5 mL/min; inj. vol. = 5 ul; wavelength = 220 nm.

*Step 3:*

HATU (618 mg, 1.624 mmol) was added to a solution of (2S,4R)-methyl 4-acetoxy-4-(6-methoxy-7-vinylnaphthalen-2-yl)pyrrolidine-2-carboxylate (600 mg, 1.624 mmol), (S)-2-((3,3-dimethylhex-5-enyloxy)carbonylamino)-3,3-dimethylbutanoic acid (421 mg, 1.477 mmol) and Hunig's base (0.774 mL, 4.43 mmol) in DCM (10 mL) and stirred at r.t. overnight. The reaction was concentrated and purified by flash chromatography on the Biotage (10-40% EtOAc in hexanes) to give (2S,4R)-methyl 4-acetoxy-1-((S)-2-((3,3-dimethylhex-5-enyloxy)carbonylamino)-3,3-dimethylbutanoyl)-4-(6-methoxy-7-vinylnaphthalen-2-yl)pyrrolidine-2-carboxylate (380 mg; 40%) as a light yellow foam. $^1H$ NMR (400 MHz, CHLOROFORM-d) d ppm 0.90 (s, 6 H) 1.13 (s, 9 H) 1.55 (t, *J=7.65* Hz, 1 H) 1.58 - 1.66 (m, 1 H) 1.96 (s, 3 H) 1.95 - 2.00 (m, 2H) 2.70 (dd, *J*=13.55, 9.29 Hz, 1 H) 3.02 (dd, *J*= 13.55, 4.27 Hz, 1 H) 3.78 (s, 3 H) 3.96 (s, 3 H) 3.99 - 4.09 (m, 1 H) 4.15 - 4.25 (m, 1 H) 4.43 (d, *J*=9.54 Hz, 1 H) 4.58 - 4.65 (m, 2 H) 4.72 (dd, *J*=9.03, 4.02 Hz, 1 H) 4.95 - 5.10 (m, 2 H) 5.31 - 5.44 (m, 2 H) 5.71 - 5.86 (m, 1 H) 5.91 (dd, *J*=17.57, 1.51 Hz, 1 H) 7.07 (s, 1 H) 7.13 (dd, *J*=17.69, 11.17 Hz, 1H) 7.38 - 7.45 (m, 1 H) 7.70 (d, *J*=8.53 Hz, 1 H) 7.83 (s, 1 H) 7.96 (s, 1 H).

*Step 4:*

A solution of (2S,4R)-methyl 4-acetoxy-1-((S)-2-((3,3-dimethylhex-5-enyloxy)carbonylamino)-3,3-dimethylbutanoyl)-4-(6-methoxy-7-vinylnaphthalen-2-yl)pyrrolidine-2-carboxylate (380 mg, 0.597 mmol) in DCE (100 mL) was sparged with nitrogen for 30 min. and then Hoveyda-Grubbs catalyst (2nd Generation) (37.5 mg, 0.060 mmol) was added and the reaction sealed and heated to 80°C overnight. The reaction was concentrated to give crude material. The crude material was purified by flash chromatography on the Biotage (10-40% EtOAc in hexanes) to give the purified product as a lightly colored foam.

*Step 5:*

10% Pd/C (54 mg, 0.051 mmol) was added to a solution of the product from Step 4 (310 mg, 0.509 mmol) in EtOAc and stirred under an atmosphere of hydrogen overnight. The reaction was filtered through a nylon frit and concentrated to give crude product (310 mg, 100%) as a white foam. LCMS: r.t. =1.88 min., $[M+H]^+$ = 611 Phenomenex Luna C18 10u (3 x 50 mm); Solvent A =10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 6*:

Potassium carbonate (351 mg, 2.54 mmol) was added to a solution of the product from Step 5 (310 mg, 0.508 mmol) in MeOH (5 mL) and stirred at r.t. for 3 hrs. The reaction was diluted with water and EtOAc. The combined organics were dried with magnesium sulfate, filtered and concentrated to give the crude product (270 mg, 94%) as a white foam. LCMS: r.t. =1.68 min., $[M+H]^+$ = 569 Phenomenex Luna C18 10u (3 x 50 mm); Solvent A = 10% acetonitrile - 90% water - 0.1% TFA, Solvent B = 90% acetonitrile - 10% water - 0.1% TFA; gradient = 0% to 100% solvent B over 2 min. and then hold for 1 min.; 4 mL/min; inj. vol. = 5 ul; wavelength = 220 nm.

*Step 7*:

NaH (60% in oil) (5 mg, 0.127 mmol) was added to a solution of the product from Step 6 (40 mg, 0.070 mmol) and dimethylcarbamyl chloride (0.012 mL, 0.127 mmol) at 0°C in DMF and stirred at this temperature for 3 hrs. The reaction was then quenched with sat. ammonium chloride solution and ether. The ether layer was washed with brine, dried, filtered and concentrated to give crude material. The crude material was purified by flash chromatography on the Biotage (20-60% EtOAc in hexanes) to give the product as a white foam. $^1H$ NMR (400 MHz, CHLOROFORM-*d*) d ppm 0.92 (s, 3 H) 0.93 (s, 3 H) 1.12 (s, 9 H) 1.28 - 1.45 (m, 2 H) 1.61 - 1.74 (m, 3 H) 1.88 (ddd, *J*=12.92, 8.28, 4.89 Hz, I H) 2.59 (dd, *J*=12.30, 11.04 Hz, 1 H) 2.69 (ddd, *J*=16.44, 8.03, 4.39 Hz, 1 H) 2.78 - 2.92 (m, 5 H) 3.03 (br. s., 3 H) 3.73 (s, 3 H) 3.92 (s, 3 H) 3.95 - 4.05 (m, 2 H) 4.15 - 4.35 (m, 2 H) 4.56 (d, *J*=9.79 Hz, 1H) 5.28 (d, *J*=9.79 Hz, 1 H) 5.53 (d, *J*=11.29 Hz, 1H) 7.03 (s, 1 H) 7.38 (dd, *J*=8.53, 2.01 Hz, 1H) 7.71 (d, *J*=8.53 Hz, 1 H) 7.79 (s, 1H) 7.86 (s, 1H).

*Step 8:*

2.0M LiOH (0.047 mL, 0.094 mmol) was added to a solution of the product from Step 7 (12 mg, 0.019 mmol) in THF (0.25 mL) and MeOH (0.25 mL) and stirred at r.t. overnight. The reaction was quenched with 1.0M HCl and diluted with ether. The combined organics were dried with magnesium sulfate, filtered and concentrated to give the crude product (12 mg, 100%) as a white solid. LCMS: r.t. = 1.84 min., $[M-H]^-$ = 624 Phenomenex Luna C18 10u (3 x 50 mm); Solvent A = 5% methanol - 95% water - 10 mM ammonium acetate, Solvent B = 95% methanol - 5% water - 10 mM ammonium acetate; gradient = 0% to 100% solvent B over 2 min. and then hold for 1 min.; 4 mL/min; inj. vol. = 5 ul; wavelength = 220 nm.

*Step 9:*

HATU (9 mg, 0.023 mmol) was added to a solution of (1S,2R)-2-amino-N-(cyclopropylsulfonyl)bi(cyclopropane)-2-carboxamide, TFA salt (10 mg, 0.029 mmol), the product from Step 8 (12 mg, 0.019 mmol) and Hunig's base (10 μL, 0.058 mmol) in dichloromethane (0.5 mL) and stirred at r.t. for 4 hrs. The reaction was concentrated to give crude material. The crude material was purified by prep. HPLC (YMC C18 10u (19 x100 mm); flow = 20 mL/min; solvent gradient = 95:5 to 5:95 water/acetonitrile (with 10mM ammonium acetate)) to give Compound 27 (7 mg, 43%) as a white solid. $^1$H NMR (500 MHz, CHLOROFORM-d) d ppm 0.17 - 0.28 (m, 2 H) 0.46 - 0.61 (m, 2 H) 0.77 - 0.86 (m, 1 H) 0.92 (s, 3 H) 0.94 (s, 3 H) 0.96 - 1.07 (m, 3 H) 1.10 (s, 9 H) 1.21- 1.32 (m, 3 H) 1.34 - 1.40 (m, 2 H) 1.40 - 1.49 (m, 1 H) 1.66 - 1.74 (m, 2 H) 1.74 - 1.79 (m, 1 H) 1.82 (dd, *J*=8.24, 5.80 Hz, 1 H) 1.84 - 1.94 (m, 1 H) 2.56 (dd, *J*=12.21, 6.71 Hz, 1H) 2.69 (ddd, *J*=12.67, 8.55, 4.43 Hz, 1 H) 2.74 - 2.81 (m, 1 H) 2.81 (s, 3 H) 2.83 - 2.91 (m, 1 H) 2.91 - 2.99 (m, 1 H) 3.04 (s, 3 H) 3.78 (dd, *J*=11.29,6.41 Hz, 1 H) 3.92 (s, 3 H) 3.95 - 4.06 (m, 2 H) 4.28 - 4.41 (m, 1 H) 4.54 (d, *J*=9.46 Hz, 1 H) 5.33 (d, *J*=9.77 Hz, 1 H) 5.61 (d, *J*=11.29 Hz, 1 H) 6.39 (s, 1 H) 7.03 (s, 1 H) 7.36 (dd, *J*=8.55, 2.14 Hz, 1 H) 7.71 (d, *J*=8.85 Hz, 1 H) 7.76 (s, 1 H) 7.87 (s, 1 H). LCMS: r.t. =1.92 min., $[M-H]^-$ = 850 Phenomenex Luna C18 10u (3 x 50 mm); Solvent A = 5% methanol - 95% water-10 mM ammonium acetate, Solvent B = 95% methanol - 5% water - 10 mM ammonium acetate; gradient = 0% to 100% solvent B over 2 min. and then hold for 1 min.; 4 mL/min; inj. vol. = 5 ul; wavelength = 220 nm.

*Preparation of Compound 28*

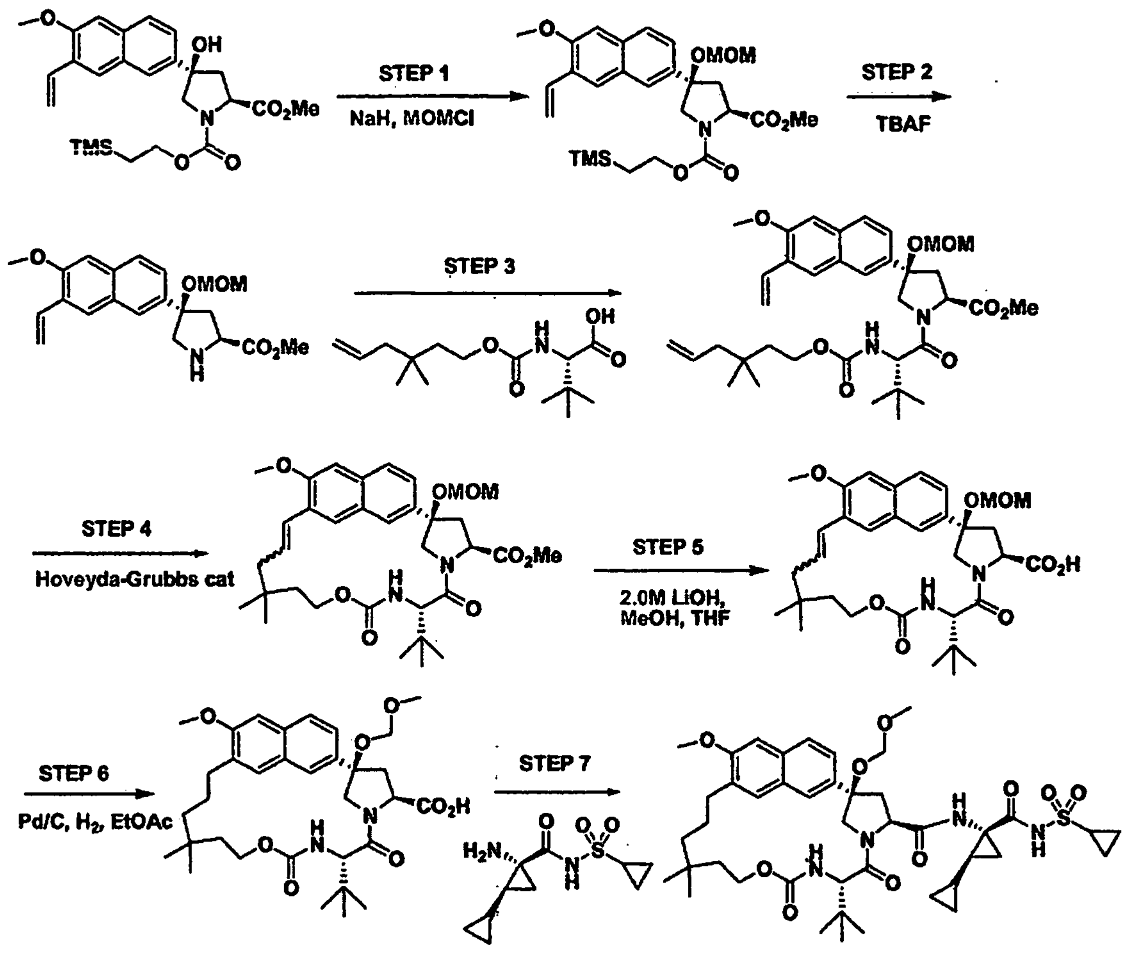


Compound 28

*Step 1:*

NaH (60% in oil) (68.7 mg, 1.717 mmol) was added to a solution of (2S,4R)-2-methyl 1-(2-(trimethylsilyl)ethyl) 4-hydroxy-4-(6-methoxy-7-vinylnaphthalen-2-yl)pyrrolidine-1,2-dicarboxylate (450 mg, 0.954 mmol) and MOM-Cl (0.13 mL, 1.717 mmol) at 0°C in DMF and stirred at this temperature for 3 hrs. The reaction was quenched with EtOAc and saturated ammonium chloride solution. The combined organics were dried with magnesium sulfate, filtered and concentrated to give the crude product. The crude material was purified by flash chromatography on the Biotage (10-40% EtOAc in hexanes) to give (2S,4R)-2-methyl 1-(2-(trimethylsilyl)ethyl) 4-(6-methoxy-7-vinylnaphthalen-2-yl)-4-(methoxymethoxy)pyrrolidine-1,2-dicarboxylate (320 mg, 65%) as a white foam. $^1$H NMR (400 MHz, CHLOROFORM-*d*) d ppm 0.05 (d, *J*=19.83 Hz, 9 H) 0.87 - 1.01 (m, 1 H) 1.04 - 1.15 (m, 1 H) 2.59 - 2.76 (m, 1 H) 2.82 (dd, *J*=12.80,3.76 Hz, 1 H) 3.31 (d, *J*=4.77 Hz, 3 H) 3.79 (d, *J*=5.27 Hz, 3 H) 3.96 (s, 3 H) 4.10 - 4.31 (m, 4 H) 4.35 - 4.52 (m, 3 H) 5.38 (dd, *J*=11.17,1.38 Hz, 1 H) 5.90 (dd, *J*=17.69,2.13 Hz, 1 H) 7.04 - 7.23 (m, 2 H) 7.35 - 7.49 (m, 1 H) 7.64 - 7.77 (m, 2 H) 7.87 (d, *J*=6.78 Hz, 1 H).

*Step 2:*

TBAF (1.0M in THF) (1.86 mL, 1.86 mmol) was added to a solution of (2S,4R)-2-methyl 1-(2-(trimethylsilyl)ethyl) 4-(6-methoxy-7-vinylnaphthalen-2-yl)-4-(methoxymethoxy)pyrrolidine-1,2-dicarboxylate (320 mg, 0.621 mmol) in THF (5 mL) and stirred at r.t. for 4 hrs. The reaction was diluted with brine and EtOAc. The combined organics were dried with magnesium sulfate, filtered and concentrated to give the crude product as a thick oil. LCMS: r.t. =1.35 min., $[M+H]^+$ = 372 Phenomenex Luna C18 10u (3 x 50 mm); Solvent A =10% methanol - 90% water - 0.1% TFA, Solvent B = 90% methanol - 10% water - 0.1% TFA; gradient = 0% to 100% solvent B over 2 min. and then hold for 1 min.; 4 mL/min; inj. vol. = 5 ul; wavelength = 220 nm.

*Step 3:*

HATU (259 mg, 0.682 mmol) was added to a solution of (2S,4R)-methyl 4-(6-methoxy-7-vinylnaphthalen-2-yl)-4-(methoxymethoxy)pyrrolidine-2-carboxylate (230 mg, .62 mmol), (S)-2-((3,3-dimethylhex-5-enyloxy)carbonylamino)-3,3-dimethylbutanoic acid (177 mg, 0.620 mmol) and Hunig's base (0.325 mL, 1.860 mmol) in DCM (10 mL) and stirred at r.t. overnight. The reaction was concentrated and purified by flash chromatography on the Biotage (5-40% EtOAc in hexanes) to give the product (290 mg, 73%) as a thick oil. $^1$H NMR (400 MHz, CHLOROFORM-*d*) d ppm 0.82 - 0.95 (m, 6 H) 1.14 (s, 9 H) 1.44 - 1.63 (m, 2 H) 1.86-2.00 (m, 2H) 2.61 2.81 (m, 2 H) 3.30 (s, 3 H) 3.75 (s, 3 H) 3.95 (s, 3 H) 3.96 - 4.07 (m, 1 H) 4.15 - 4.31 (m, 2 H) 4.31 - 4.44 (m, 2 H) 4.44 - 4.64 (m, 3 H) 4.92 - 5.10 (m, 2 H) 5.31 - 5.42 (m, 2 H) 5.67 - 5.84 (m, 1 H) 5.92 (dd, *J*=17.57,1.51 Hz, 1 H) 7.07 (s, 1 H) 7.12 (dd, *J*=17.82, 11.29 Hz, 1 H) 7.45 (d, *J*=8.38, 1 H) 7.71 (d, *J*=8.53 Hz, 1 H) 7.80 (s, 1 H) 7.99 (s, 1H).

*Step 4:*

A solution of (2S,4R)-methyl 1-((S)-2-((3,3-dimethylhex-5-enyloxy)carbonylamino)-3,3-dimethylbutanoyl)-4-(6-methoxy-7-vinylnaphthalen-2-yl)-4-(methoxymethoxy)pyrrolidine-2-carboxylate (290 mg, 0.454 mmol) in DCE (100 mL) was sparged with nitrogen for 30 min. and then Hoveyda-Grubbs catalyst (2nd Generation) (28 mg, 0.045 mmol) was added and the reaction sealed and heated to 80°C overnight. The reaction was concentrated to give crude material. The crude material was purified by flash chromatography on the Biotage (10-60% EtOAc in hexanes) to give the purified product (210 mg, 76%) as a white foam.

*Step 5:*

2.0M LiOH (1.19 mL, 2.38 mmol) was added to a solution of the product from Step 4 (290 mg, 0.475 mmol) in THF (2 mL) and MeOH (2 mL) and stirred at r.t. overnight. The reaction was diluted with 1.0M HCl and EtOAc. The combined organics were dried with magnesium sulfate, filtered and concentrated to give the crude product (210 mg, 74%) as a white foam. LCMS: r.t. = 2.05 min., $[M+H]^+$ = 597 Phenomenex Luna C18 10u (3 x 50 mm); Solvent A =10% methanol - 90% water - 0.1 % TFA, Solvent B = 90% methanol - 10% water - 0.1 % TFA; gradient = 0% to 100% solvent B over 2 min. and then hold for 1 min.; 4 mL/min; inj. vol. = 5 ul; wavelength = 220 nm.

*Step 6:*

10% Pd/C (38 mg, 0.035 mmol) was added to a solution of the product from Step 5 (210 mg, 0.352 mmol) in EtOAc (5 mL) and stirred under an atmosphere of hydrogen overnight. The reaction was filtered through a nylon frit and concentrated to give crude product (180 mg, 85%) as a white foam. LCMS: r.t. =1.89 min., $[M+H]^+$ = 599 Phenomenex Luna C18 10u (3 x 50 mm); Solvent A = 5% methanol - 95% water -10 mM ammonium acetate, Solvent B = 95% methanol - 5% water - 10 mM ammonium acetate; gradient = 0% to 100% solvent B over 2 min. and then hold for 1 min.; 4 mL/min; inj. vol. = 5 ul; wavelength = 220 nm.

*Step 7:*

HATU (171 mg, 0.451 mmol) was added to a solution of (1S,2R)-2-amino-N-(cyclopropylsulfonyl)bi(cyc1opropane)-2-carboxamide, HCl salt (101 mg, 0.361 mmol), the product from Step 6 (180 mg, 0.301 mmol) and Hunig's base (0.16 mL, 0.902 mmol) in dichloromethane (2 mL) and stirred at r.t. for 4 hrs. The reaction was concentrated and then purified by prep. HPLC (Sunfire C18 10u (30 x100 mm); flow = 42 mL/min; solvent gradient = 95:5 to 5:95 water/acetonitrile (with 10mM ammonium acetate)). The product fractions were concentrated to give Compound 28 (66 mg, 24%) as a white solid. $^1$H NMR (500 MHz, CHLOROFORM-d) $\delta$ ppm 0.11 - 0.28 (m, 2 H) 0.42 - 0.60 (m, 2 H) 0.76 - 0.86 (m, 1 H) 0.92 (s, 3 H) 0.94 (s, 3 H) 0.95 - 1.03 (m, 3 H) 1.09 (s, 9 H) 1.21 - 1.27 (m, 1 H) 1.28 - 1.37 (m, 3 H) 1.39 - 1.48 (m, 1 H) 1.60 - 1.80 (m, 5 H) 1.84 - 1.97 (m, 1 H) 2.52 - 2.60 (m, 1 H) 2.60 - 2.70 (m, 2 H) 2.85 - 3.01 (m, 2 H) 3.40 (s, 3 H) 3.65 (dd, *J*=10.99, 6.71 Hz, 1 H) 3.87 (d, *J*=10.99 Hz, 1 H) 3.93 (s, 3 H) 3.99 (td, *J*=11.06, 5.65 Hz, 1 H) 4.33 - 4.45 (m, 3 H) 4.55 (d, *J*=10.07 Hz, 1 H) 5.03 (d, *J*=10.99 Hz, 1 H) 5.47 (d, *J*=10.07 Hz, 1 H) 6.46 (br. s., 1H) 7.05 (s, I H) 7.44 (dd, *J*=8.55, 1.83 Hz, 1 H) 7.73 (d, *J*=8.55 Hz, 1 H) 7.77 - 7.87 (m, 2 H). LCMS: r.t. = 2.06 min., $[M-H]^-$ = 823 Phenomenex Luna C18 10u (3 x 50 mm); Solvent A = 5% methanol - 95% water - 10 mM ammonium acetate, Solvent B = 95% methanol - 5% water - 10 mM ammonium acetate; gradient = 0% to 100% solvent B over 2 min. and then hold for 1 min.; 4 mL/min; inj. vol. = 5 ul; wavelength = 220 nm.

*Preparation of Intermediate 6:*

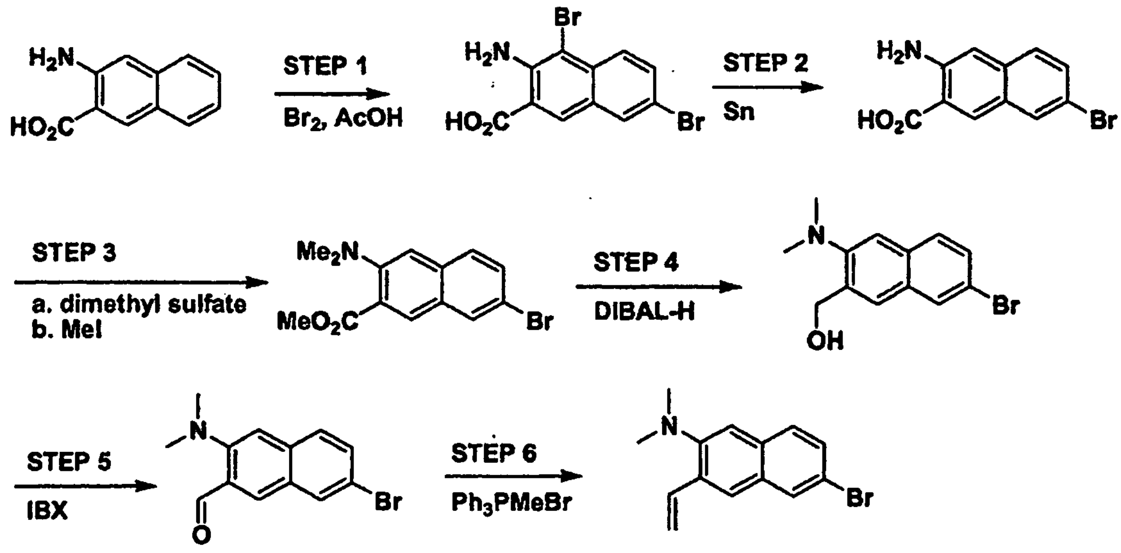


**Intermediate 6**

*Step 1:*

A solution of bromine (10.32 mL, 200 mmol) in acetic acid (50 mL) was added dropwise to a solution of 3-amino-2-naphthoic acid (15 g, 80 mmol) in acetic acid (100 mL) over 30 min. After the addition the reaction was heated to reflux for 1 hr, cooled and poured into ice water. The solid product was filtered, washed with water and air dried to give the product as a yellow solid. $^1$H NMR (500 MHz, CHLOROFORM-*d*) $\delta$ ppm 7.62 (dd, *J*=9.16, 1.83 Hz, 1 H) 7.88 (d, *J*=9.16 Hz, 1 H) 7.91 (d, *J*=2.14 Hz, 1 H) 8.49 (s, 1 H).

*Step 2:*

Tin (9.50 g, 80 mmol) was added to a suspension of 3-amino-4,7-dibromo-2-naphthoic acid (27.6 g, 80 mmol) in acetic acid (250 mL) and conc. HCl (50 mL, 80 mmol) and heated to reflux for 2 hrs. The reaction was poured into water and the resulting precipitate was filtered and dried under vacuum overnight to give 3-amino-7-bromo-2-naphthoic acid (19 g, 89 % yield) as a light yellow solid. $^1$H NMR (400 MHz, DMSO-$d_6$) $\delta$ ppm 7.03 (s, 1 H) 7.41- 7.48 (m, 1 H) 7.48 - 7.55 (m, 1 H) 8.05 (d, *J*=1.51 Hz, 1 H) 8.42 (s, 1 H).

*Step 3:*

Dimethyl sulfate (21.15 mL, 221 mmol) was added to a mixture of 3-amino-7-bromo-2-naphthoic acid (19 g, 71.4 mmol) and potassium carbonate (49.3 g, 357 mmol) in acetone (400 mL) and heated to reflux overnight. The reaction was cooled and water (10 mL) was added and continued stirring for 1 hr to destroy excess dimethyl sulfate. The reaction mixture was filtered and concentrated and then the residue taken up in DCM and washed with water. The organics were dried, filtered and concentrated to give the crude material. The crude material contained a mixture of mono and bis methylated products. The product mixture and iodomethane (4.46 mL, 71.4 mmol) in DMF (300 mL) were cooled to 0 °C and sodium hydride (2.86 g, 71.4 mmol) was added portion-wise. The reaction was allowed to warm up to r.t. overnight. The reaction was diluted with water and extracted with ether. The ether layer was washed with brine, collected, dried over $MgSO_4$, filtered and concentrated to give methyl 7-bromo-3-(dimethylamino)-2-naphthoate (22.5 g, 102 % yield) as an orange oil. $^1$H NMR (500 MHz, CHLOROFORM-*d*) $\delta$ ppm 2.90 (s, 6 H) 3.91 - 4.02 (m, 3 H) 7.16 (s, 1 H) 7.49 - 7.53 (m, 1 H) 7.54 - 7.58 (m, 1 H) 7.89 (d, *J*=1.22 Hz, 1 H) 8.06 (s, 1 H).

*Step 4:*

Diisobutylaluminum hydride (1.0M in DCM) (219 mL, 219 mmol) was added slowly to a solution of methyl 7-bromo-3-(dimethylamino)-2-naphthoate (22.5 g, 73.0 mmol) in THF (300 mL) at -40°C (acetonitrile/dry ice). After the

addition the reaction was stirred for 3 hrs. and EtOAc (100 mL) was added and the ice bath removed. After 5 min. 1.0M HCl solution (200 mL) was added and stirred for 10min. The organics were washed with 1.0M HCl solution and brine and then dried, filtered and concentrated to give the crude product. The crude material was purified on the Biotage (5-25% EtOAc:Hex) to give (7-bromo-3-(dimethylamino)naphthalen-2-yl)methanol (11 g, 53.8 % yield) as an orange thin oil. $^1$H NMR (500 MHz, CHLOROFORM-*d*) $\delta$ ppm 2.83 (s, 6 H) 4.95 (s, 2 H) 7.50 (s, 1H) 7.51 (dd, 1H) 7.58 (s, 1 H) 7.62 (d, *J*=8.55 Hz, 1 H) 7.91 (d, *J*=1.53 Hz, 1 H).

*Step 5:*

IBX (14.29 g, 51.0 mmol) was added to a solution of (7-bromo-3-(dimethylamino)naphthalen-2-yl)methanol (11 g, 39.3 mmol) in DMSO (150 mL) and heated to 45°C. After complete reaction the reaction was diluted with water and extracted with ether (2x). The organic layer was washed with brine, dried over $MgSO_4$, filtered and concentrated to give 7-bromo-3-(dimethylamino)-2-naphthaldehyde (10 g, 92 % yield) as a dark orange solid. $^1$H NMR (400 MHz, CHLOROFORM-d) $\delta$ ppm 2.95 (s, 6 H) 7.30 (s, 1 H) 7.51 - 7.66 (m, 2 H) 8.01 (d, J=1.26 Hz, 1 H) 8.21 (s, 1 H) 10.39 (s, 1H).

*Step 6:*

BuLi (28.8 mL, 71.9 mmol) was added to a solution of methyltriphenylphosphonium bromide (25.7 g, 71.9 mmol) in THF (165 mL) at 0 °C. The mixture was stirred at 0°C for 1 hr under nitrogen. A solution of 7-bromo-3-(dimethylamino)-2-naphthaldehyde (10 g, 36.0 mmol) in THF (165 mL) was added dropwise at 0°C. The mixture was stirred overnight from 0°C to r.t. The reaction was filtered and concentrated. The residue was diluted with diethyl ether and separated with water. The ether layer was collected and washed with brine, dried ($MgSO_4$), filtered and concentrated to give the crude material. The product was purified on the Biotage (5-25% EtOAc:Hex) to give 6-bromo-N,N-dimethyl-3-vinylnaphthalen-2-amine (8.4 g, 85 % yield) as a light orange solid. LCMS: rt = 1.29 min. $[M+H]^+$ = 277; Phenomenex-Luna C-18 (5$\mu$) (3.0x50mm); Solvent A = 10% acetonitrile-90% water - 0.1 % TFA, Solvent B = 90% acetonitrile - 10% water - 0.1 % TFA; gradient 0% to 100% solvent B over 2 min. and then hold for 1 min.; 4mL/min; inj. vol. = 5uL; wavelength = 220. $^1$H NMR (500 MHz, CHLOROFORM-*d*) $\delta$ ppm 2.77 - 2.85 (m, 6 H) 5.31 - 5.42 (m, 1 H) 5.79 - 5.92 (m, 1 H) 7.04 - 7.19 (m, 1H) 7.19 - 7.29 (m, 1H) 7.39 - 7.50 (m, 1H) 7.50 - 7.63 (m, 1H) 7.77 (d, *J*=13.12 Hz, 1H) 7.89 (d, *J*=11.60 Hz, 1H).

*Preparation of Intermediate 7:*

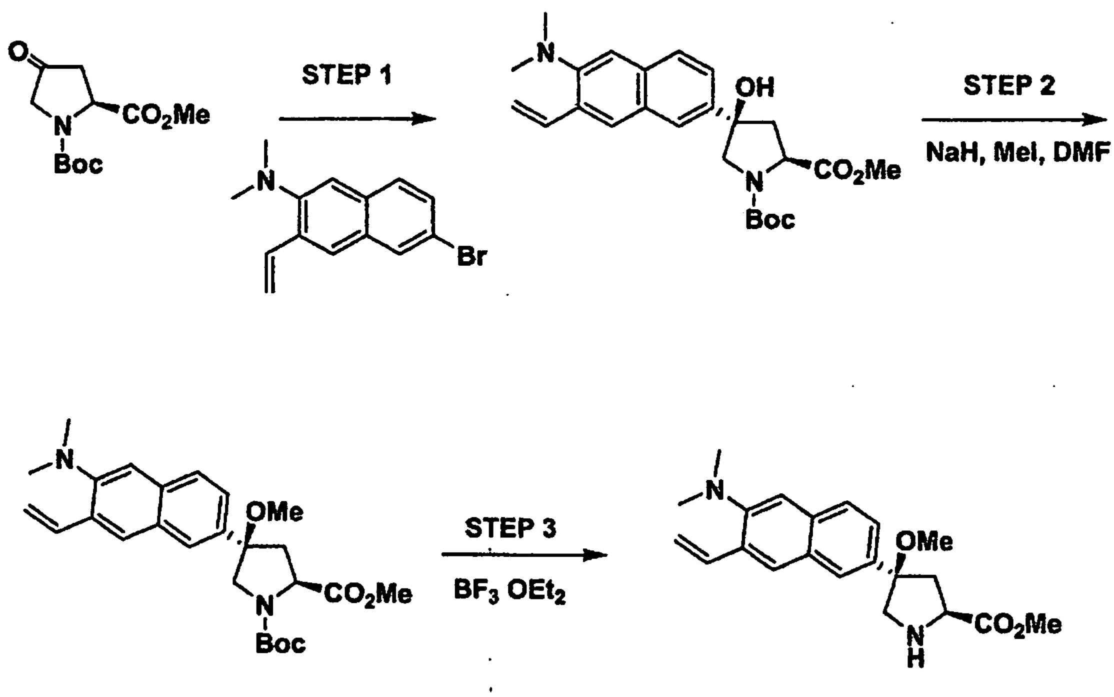


Intermediate 7

*Step 1:*

Magnesium (0.388 g, 15.97 mmol) was stirred in a round bottom flask under nitrogen for 1 hr. to cause scratching of the surface of the magnesium turnings. 10mL of THF was added to the magnesium turnings and stirred for an additional 30 min. 6-bromo-N,N-dimethyl-3-vinylnaphthalen-2-amine (Intermediate 6, 4.2 g, 15.21 mmol) in THF (110 mL), was added in dropwise at reflux with vigorous stirring. The resulting solution was added to a solution of (S)-1-tert-butyl 2-methyl 4-oxopyrrolidine-1,2-dicarboxylate (3.70 g, 15.21 mmol) in toluene (110 mL) at 0°C and stirred for 1 hr and then quenched with sat. $NH_4Cl$ solution. The aqueous layer was extracted with DCM and the combined organics were dried, filtered and concentrated to give crude material. The crude material was purified by flash chromatography on the Biotage (10-40% EtOAc:Hex) to give (2S,4R)-1-tert-butyl 2-methyl 4-(6-(dimethylamino)-7-vinylnaphthalen-2-yl)-4-hydroxypyrrolidine-1,2-dicarboxylate (2.43 g, 36 % yield) as a yellow foam. LCMS: rt =1.29 min. $[M+H]^+$ = 441; Phenomenex-Luna C-18 (5μ) (3.0x50mm); Solvent A = 10% acetonitrile-90% water - 0.1% TFA, Solvent B = 90% acetonitrile - 10% water - 0.1 % TFA; gradient 0% to 100% solvent B over 2 min. and then hold for 1 min.; 4mL/min; inj. vol. = 5uL; wavelength = 220. $^1$H NMR (500 MHz, CHLOROFORM-d δ ppm 1.48 (d, 9 H) 2.40 (dd, *J*=18.01, 14.04 Hz, 1H) 2.71 - 2.81 (m, 1H) 2.82 (d, 6 H) 3.85 (d, 3H) 3.99 - 4.05 (m, 1H) 4.42 - 4.62 (m, 1H) 5.35 (dd, *J*=10.83, 1.37 Hz, 1H) 5.85 (dd, *J*=17.70, 1.22 Hz, 1H) 7.13 (dd, *J*=17.55, 10.83 Hz, 1H) 7.28 (s, I H) 7.48 (ddd, *J*=8.24, 5.95, 1.68 Hz, 1H) 7.72 (d, *J*=8.55 Hz, 1H) 7.82 - 7.98 (m, 2 H).

*Step* 2:

NaH (60% in oil) (0.397 g, 9.93 mmol) was added to a solution of (2S,4R)-1-tert-butyl 2-methyl 4-(6-(dimethylainino)-7-vinyinaphthalen-2-yl)-4-hydroxypyrrolidine-1,2-dicarboxylate, (2.43 g, 5.52 mmol) and methyl iodide (0.62 mL, 9.93 mmol) at 0°C in DMF and stirred at this temperature for 3 hrs. The reaction was then quenched with sat. ammonium chloride solution and ether. The ether layer was washed with brine, dried, filtered and concentrated to give crude material. The crude material was purified by flash chromatography on the Biotage (5-35% EtOAc in hexanes) to give (2S,4R)-1-tert-butyl 2-methyl 4-(6-(dimethylamino)-7-vinylnaphthalen-2-yl)-4-methoxypyrrolidine-1 ,2-dicarboxylate (2.18 g, 87% yield) as a yellow foam. LCMS: rt =1.26 min. $[M+H]^+$ = 455; Phenomenex-Luna C-18 (5μ) (3.0x50mm); Solvent A =10% acetonitrile-90% water- 0.1% TFA, Solvent B = 90% acetonitrile - 10% water - 0.1% TFA; gradient 0% to 100% solvent B over 2 min. and then hold for 1 min.; 4mL/min; inj. vol. = 5uL; wavelength = 220.

*Step 3*:

A mixture of (2S,4R)-1-tert-butyl 2-methyl 4-(6-(dimethylamino)-7-vinylnaphthalen-2-yl)-4-methoxypyrrolidine-1,2-dicarboxylate (2.18 g, 4.80 mmol) and 4A molecular seives (2 g) in DCM (50 mL)were stirred at r.t. for one half hour. The reaction was cooled to 0°C and $BF_3.OEt_2$ (3.0 mL, 23.98 mmol) was added slowly. The reaction was then allowed to warm up to r.t. The reaction was quenched with saturated $NaHCO_3$ solution and diluted with EtOAc. The organics were washed with brine, dried, filtered and concentrated to give crude (2S,4R)-methyl 4-(6-(dimethylamino)-7-vinylnaphthalen-2-yl)-4-methoxypyrrolidine-2-carboxylate (1.48 g, 87 % yield) as an orange oil. LCMS: rt = 0.72 min. $[M+H]^+$ = 377; Phenomenex-Luna C-18 (5μ) (3.0x50mm); Solvent A =10% acetonitrile-90% water - 0.1% TFA, Solvent B = 90% acetonitrile - 10% water - 0.1 % TFA; gradient 0% to 100% solvent B over 2 min. and then hold for 1 min.; 4mL/min; inj. vol. = 5uL; wavelength = 220. $^1$H NMR (400 MHz, CHLOROFORM-*d* δ ppm 2.57 (dd, *J*=13.55, 9.54 Hz, 1 H) 2.75 (dt, *J*=13.55, 2.5 Hz, 1H) 2.83 (s, 6 H) 2.95 (s, 3 H) 3.12 (d, *J*=12.05 Hz, 1 H) 3.60 (dd, *J*=11.92, 1.88 Hz, 1H) 3.82 (s, 3 H) 4.00 (dd, *J*=9.54, 3.01 Hz, 1H) 5.36 (dd, *J*=11.04, 1.51 Hz, 1H) 5.86 (dd, *J*=17.57, 1.51 Hz, 1H) 7.14 (dd, *J*=17.69, 10.92 Hz, 1H) 7.29 (s, 1H) 7.41 (dd, *J*=8.53,1.76 Hz, 1H) 7.62 - 7.76 (m, 2 H) 7.90 (s, 1H)

*Preparation of Intermediate 8:*

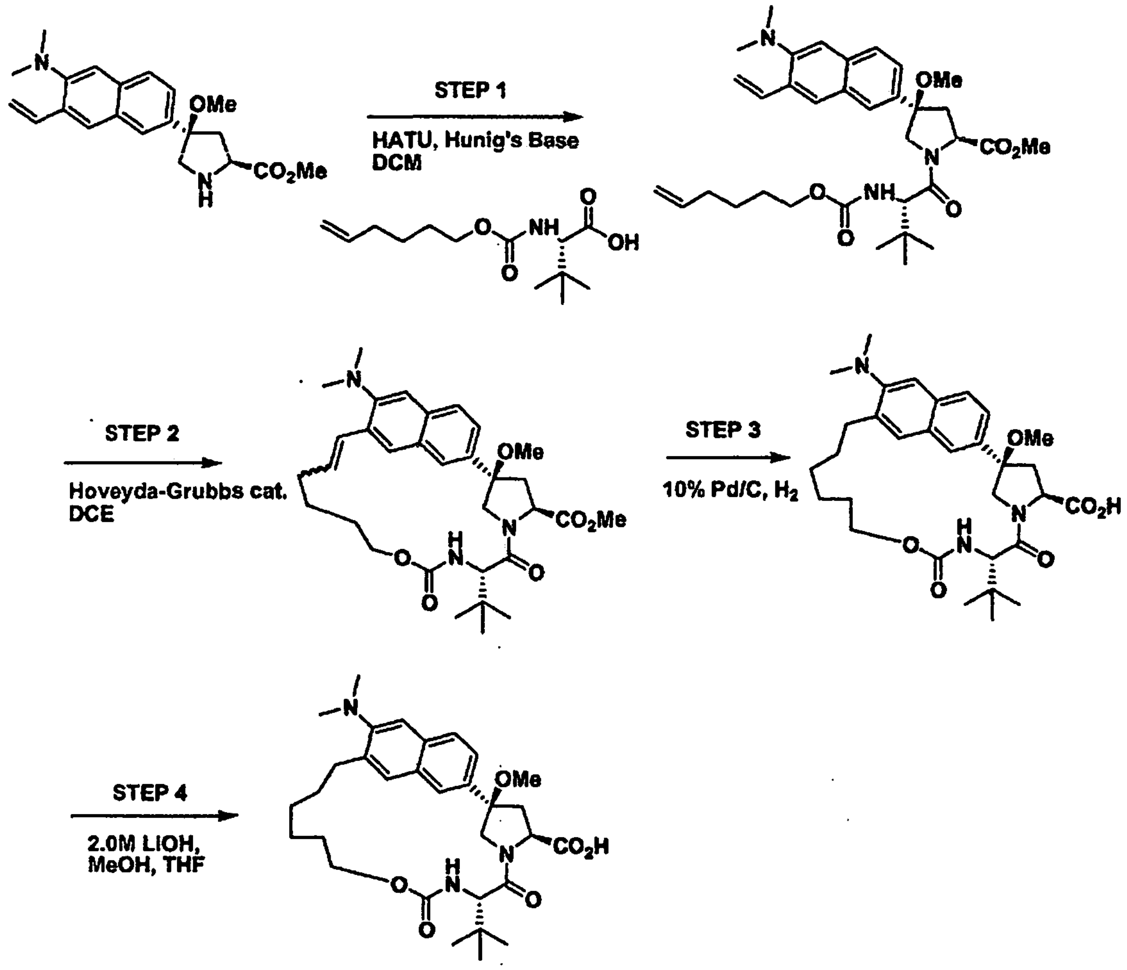


**Intermediate 8**

*Step 1:*

(S)-2-((hex-5-enyloxy)carbonylamino)-3,3-dimethylbutanoic acid (0.806 g, 3.13 mmol), DIEA (1.094 mL, 6.26 mmol) and HATU (0.873 g, 2.297 mmol) were added to a solution of (2S,4R)-methyl 4-(6-(dimethylamino)-7-vinylnaphthalen-2-yl)-4-methoxypyrrolidine-2-carboxylate (Intermediate 7, 0.74 g, 2.088 mmol) in DCM (20 mL). The reaction was stirred at r.t. overnight. The reaction was concentrated and purified on the Biotage (10-40% EtOAc:Hex) to give (2S, 4R)-methyl 4-(6-(dimethylamino)-7-vinylnaphthalen-2-yl)-1-((S)-2-((hex-5-enyloxy)carbonylamino)-3,3-dimethylbutanoyl)-4-methoxypyrrolidine-2-carboxylate (1.24 g, 100 % yield) as a white foam. LCMS: rt =1.48. $[M+Na]^+$ =616, Phenomenex-Luna C-18 (5μ) (3.0x50mm); Solvent A = 10% acetonitrile-90% water - 0.1% TFA, Solvent B = 90% acetonitrile - 10% water - 0.1 % TFA; gradient 0% to 100% solvent B over 2 min. and then hold for 1 min.; 4mL/min; inj. vol. = 5uL; wavelength = 220.

*Step 2:*

(2S,4R)-methyl 4-(6-(dimethylamino)-7-vinylnaphthalen-2-yl)-1-((S)-2-((hex-5-enyloxy)carbonylamino)-3,3-dimethylbutanoyl)-4-methoxypyrrolidine-2-carboxylate (800 mg, 1.347 mmol) was dissolved in 2mL of diethyl ether and $BF_3$-$OEt_2$ (0.171 mL, 1.347 mmol) was added and stirred at r.t. for 15 min. After 15 min. the reaction was concentrated and the white chalky residue taken up in DCE (135 mL) and bubbled with nitrogen gas for 15 min. Hoveyda-Grubbs catalyst (85 mg, 0.135 mmol) was added and heated in an oil bath at 80 °C until completion. The reaction was then partially concentrated and taken up in DCM. The organic layer was washed with saturated sodium bicarbonate then brine, dried over $MgSO_4$, filtered and concentrated. The crude material was purified on the Biotage (5-50% EtOAc: Hex) to give the product (470 mg, 0.831 mmol, 61.7 % yield) as a yellow foam. LCMS: rt =1.18 min. $[M+H]^+$ =566 Phenomenex-Luna C-18 (5μ) (3.0x50mm); Solvent A = 10% acetonitrile-90% water - 0.1% TFA, Solvent B = 90% acetonitrile - 10% water - 0.1 % TFA; gradient 0% to 100% solvent B over 2 min. and then hold for 1 min.; 4mL/min; inj. vol. = 5uL;

wavelength = 220.

*Step 3:*

10% Palladium on carbon (88 mg, 0.083 mmol) was added to a solution of the product from Step 2 (470 mg, 0.831 mmol) in ethyl acetate (8 mL) and stirred under a balloon of hydrogen overnight. The reaction was filtered through a Millipore millex-HV 0.45um frit and concentrated to give the product (455 mg, 96 % yield). LCMS: rt =1.16 min. $[M+H]^+$ = 568 Phenomenex-Luna C-18 (5μ) (3.0x50mm); Solvent A =10% acetonitrile-90% water - 0.1% TFA, Solvent B = 90% acetonitrile - 10% water - 0.1% TFA; gradient 0% to 100% solvent B over 2 min. and then hold for 1 min.; 4mL/min; inj. vol. = 5uL; wavelength = 220.

*Step 4:*

2.0M Lithium hydroxide (1.2 mL, 2.4 mmol) was added to a solution of the product from Step 3 (455 mg, 0.801 mmol) in THF (4 mL) and MeOH (4 mL) and was stirred at r.t. overnight. The reaction was diluted with EtOAc and washed with 1M HCl, then brine. The organic layer was collected, dried over $MgSO_4$, filtered and concentrated to give Intermediate 8 (339 mg, 76 % yield). LCMS: rt =1.03 min. $[M+H]^+$ = 554 Phenomenex-Luna C-18 (5μ) (3.0x50mm); Solvent A = 10% acetonitrile-90% water - 0.1% TFA, Solvent B = 90% acetonitrile - 10% water - 0.1% TFA; gradient 0% to 100% solvent B over 2 min. and then hold for 1 min.; 4mL/min; inj. vol. =5uL; wavelength = 220.

*Preparation of Compound 29:*

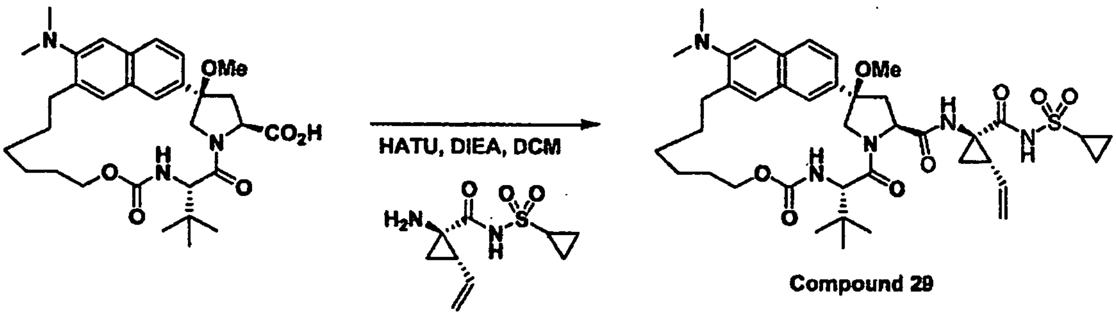


Compound 29

DIEA (0.161 mL, 0.921 mmol), HATU (128 mg, 0.338 mmol), and (1R,2S)-1-amino-N-(cyclopropylsulfonyl)-2-vinylcyclopropanecarboxamide, pTSA salt (124 mg, 0.307 mmol) were added to a solution of Intermediate 8 (170 mg, 0.307 mmol) in DCM (3 mL). The reaction was stirred at r.t. for 2hrs. The crude product was concentrated and purified by prep HPLC (Sunfire Prep C18 OBD 5u (30x100mm); flow = 42mL/min; solvent gradient 70:30 to 5:95 water/acetonitrile (with 10mM ammonium acetate)) to give Compound 29 (11.8 mg, 5 % yield) as a white solid: LCMS: rt =1.26 min. $[M+H]^+$ =766. Phenomenex-Luna C-18 (5μ) (3.0x50mm); Solvent A =10% acetonitrile-90% water - 0.1% TFA, Solvent B = 90% acetonitrile - 10% water - 0.1% TFA; gradient 0% to 100% solvent B over 2 min. and then hold for 1 min.; 4mL/min; inj. vol. = 5uL; wavelength = 220. $^1H$ NMR (500 MHz, CHLOROFORM-*d*) δ ppm 0.96 - 1.06 (m, 2 H) 1.11 (s, 9 H) 1.28 - 1.42 (m, 4 H) 1.47 (dd, *J*=9.46, 5.49 Hz, 2 H) 1.59 (dd, *J*=19.38, 13.28 Hz, 2 H) 1.66 - 1.72 (m, 1 H) 1.72 - 1.77 (m, 1 H) 1.94 (dd, *J*=8.24, 5.49 Hz, 1H) 1.97 - 2.05 (m, 1H) 2.36 (d, *J*=6.41 Hz, 1H) 2.60 (t, *J*=11.75 Hz, 1H) 2.84 - 2.91 (m, 2 H) 2.93 (s, 6 H) 3.05 - 3.17 (m, 3 H) 3.74 (dd, *J*=11.29, 6.10 Hz, 1H) 3.83 - 3.95 (m, 2H) 4.35 - 4.49 (m, 1 H) 4.55 (d, *J*=9.77 Hz, 1 H) 4.94 (d, *J*=10.07 Hz, 1 H) 5.08 (dd, *J*=10.22, 1.37 Hz, 1 H) 5.16 (d, *J*=17.40 Hz, 1H) 5.50 (d, *J*=10.07 Hz, 1 H) 5.78 (ddd, *J*=17.24, 10.22, 8.85 Hz, 1H) 6.36 (s, 1H) 7.51 (s, 1H) 7.54 (dd, *J*=8.70, 1.68 Hz, 1H) 7.65 (s, 1H) 7.71 (s, 1H) 7.80 (d, *J*=8.55 Hz, 1H) 10.01 (br. s., 1H).

*Preparation of Compound 30:*

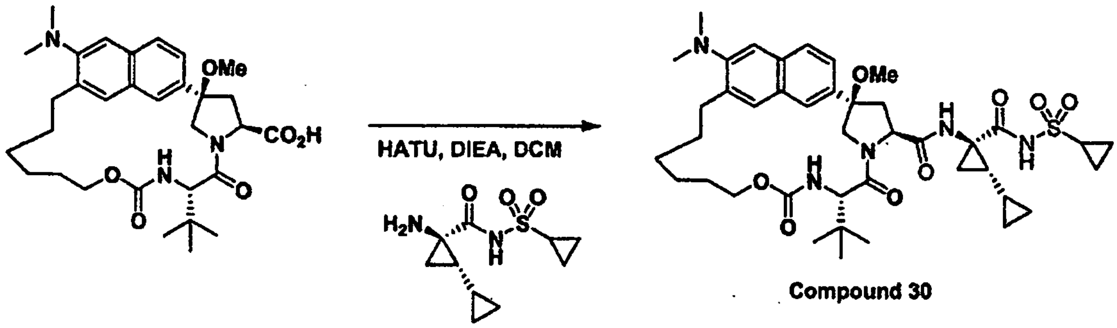


Compound 30

DIEA (0.161 mL, 0.921 mmol), HATU (128 mg, 0.338 mmol), and (1S,2R)-2-amino-N-(cyclopropylsulfonyl)bi (cyclopropane)-2-carboxamide, HCl salt (86 mg, 0.307 mmol) were added to a solution of Intermediate 8 (170 mg, 0.307 mmol) in DCM (3 mL). The reaction was stirred at r.t. for 2hrs. The crude product was concentrated and purified by prep HPLC (Sunfire Prep C18 OBD 5u (30x100mm); flow = 42mL/min; solvent gradient 70:30 to 5:95 water/acetonitrile (with 10mM ammonium acetate)) to give Compound 30 (12 mg, 5 % yield) as a white solid. LCMS: rt =1.28 min. $[M+H]^+$ =780 [M+Na] = 802. Phenomenex-Luna C-18 (5μ) (3.0x50mm); Solvent A = 10% acetonitrile-90% water - 0.1% TFA, Solvent B = 90% acetonitrile - 10% water - 0.1% TFA; gradient 0% to 100% solvent B over 2 min. and then hold for 1 min.; 4mL/min; inj. vol. = 5uL; wavelength =220. $^1$H NMR (500 MHz, CHLOROFORM-*d*) δ ppm 0.10 - 0.19 (m, 1 H) 0.23 (dt, *J*=9.46, 4.73 Hz, 1H) 0.49 (dd, *J*=8.09, 4.12 Hz, 1H) 0.51 - 0.57 (m, 1H) 0.78 - 0.88 (m, 1H) 0.92 (t, *J*=8.70 Hz, 1H) 0.99 - 1.06 (m, 2 H) 1.06 - 1.10 (m, 1H) 1.10 (s, 9 H) 1.25 (dd, *J*=9.46, 5.80 Hz, 1H) 1.37 (td, *J*=8.93, 4.43 Hz, 3 H) 1.43 - 1.51 (m, 1H) 1.53 - 1.64 (m, 2 H) 1.91 - 2.01 (m, 1H) 2.34 (dd, *J*=12.05, 6.26 Hz, 1H) 2.60 (t, *J*=11.75 Hz, 1 H) 2.89 (s, 6 H) 2.80 - 3.00 (m, 4 H) 3.10 (s, 3 H) 3.73 (dd, *J*=11.29, 6.41 Hz, 1H) 3.83 - 3.93 (m, 2 H) 4.33 - 4.48 (m, 1H) 4.53 (d, *J*=9.77 Hz, 1H) 4.91 (d, *J*=9.77 Hz, 1H) 5.47 (d, *J*=9.46 Hz, 1 H) 6.24 (s, 1 H) 7.49 (s, 1H) 7.52 (d, 1H) 7.62 (s, 1H) 7.69 (s, 1H) 7.78 (d, *J*=8.55 Hz, 1H) 9.97 - 10.03 (m, 1H).

*Preparation of Compound 31:*

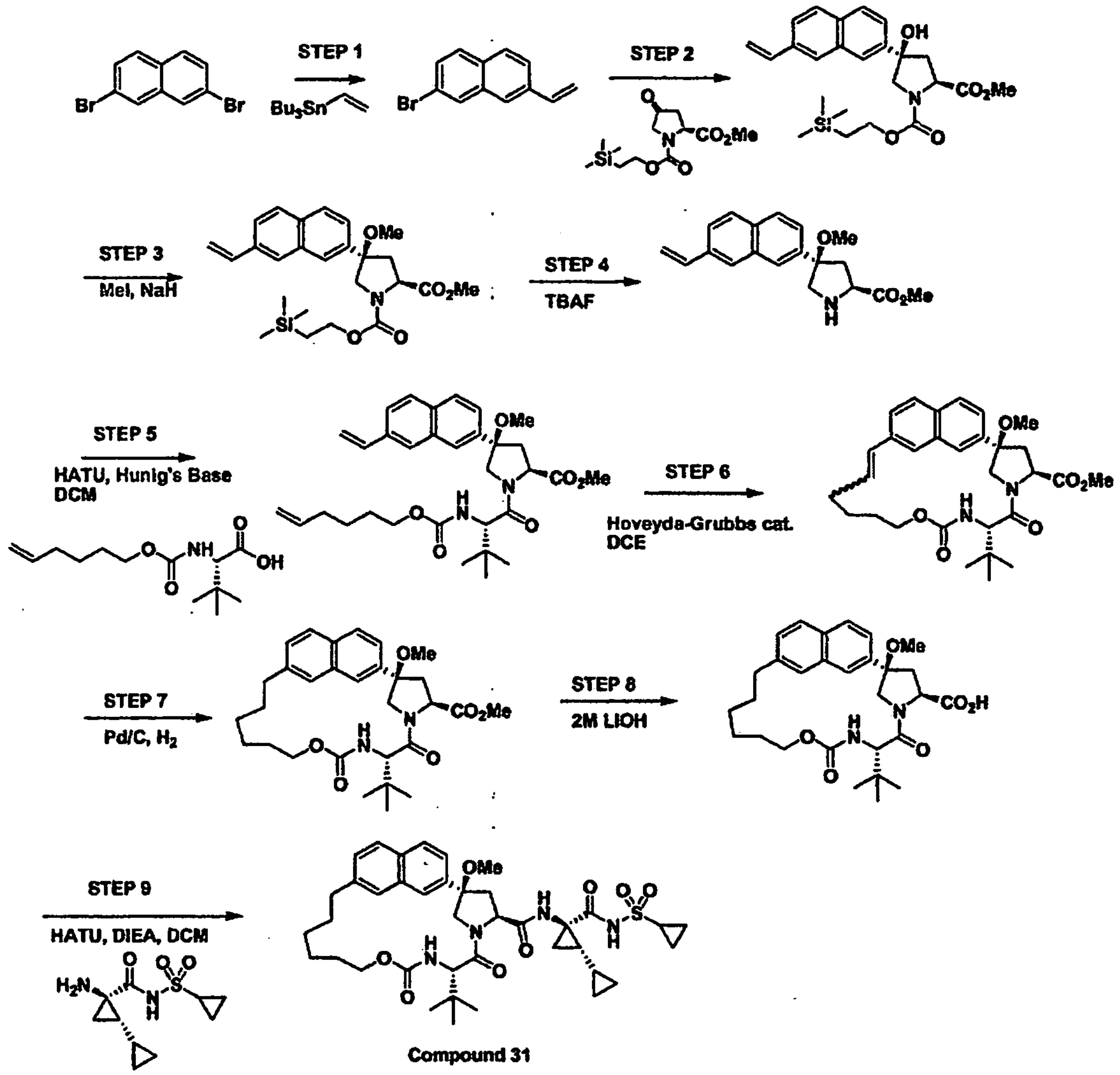


Compound 31

*Step 1:*

2,7-dibromonaphthalene (2.73 g, 9.55 mmol) and tributyl(vinyl)stannane (2.79 mL, 9.55 mmol) were added to toluene (75 mL) in a sealable vessel and sparged with nitrogen for 10 minutes. $Pd(Ph_3P)_4$ (0.552 g, 0.477 mmol) was added and sealed and heated at 90 °C for 4 hours. The reaction was cooled and concentrated onto celite and purified on the Biotage (100% Hex) to give 2-bromo-7-vinylnaphthalene (1.5 g, 67.4 % yield) as a white solid. $^1H$ NMR (400 MHz, CHLOROFORM-*d*) d ppm 5.39 (d, *J*=10.79 Hz, 1H) 5.90 (d, *J*=17.57 Hz, 1H) 6.88 (dd, *J*=17.69, 10.92 Hz, 1H) 7.52 (dd, *J*=8.66,1.88 Hz, 1H) 7.63 - 7.72 (m, 3 H) 7.77 (d, *J*=8.03 Hz, 1H) 7.98 (d, *J*=1.51 Hz, 1H).

*Step 2:*

Magnesium (0.336 g, 13.83 mmol) was stirred in a round bottom flask under nitrogen for 15 min. to cause scratching of the surface of the magnesium turnings. 10mL of THF was added to the magnesium turnings and stirred for an additional 30 min. 2-bromo-7-vinylnaphthalene (3.07 g, 13.17 mmol) in THF (90 mL), was added dropwise at reflux with vigorous stirring. This solution was added to a solution of (S)-2-methyl 1-(2-(trimethylsilyl)ethyl) 4-oxopyrrolidine-1,2-dicarboxyiate (3.78 g, 13.17 mmol) in DCM (110 mL) at r.t. and stirred for 1 hr then quenched with sat. $NH_4Cl$ solution. The aqueous layer was extracted with DCM and the combined organics were dried, filtered and concentrated to give crude material. The crude material was purified by flash chromatography on the Biotage (10-40% EtOAc:Hex) to give (2S,4R)-2-methyl 1-(2-(trimethylsilyl)ethyl) 4-hydroxy-4-(7-vinylnaphtlxalen-2-yl)pyrrolidine-1,2-dicarboxylate (1.55 g, 27 % yield) as a white foam. LCMS: rt = 1.90 min. $[M+Na]^+$ = 464. Phenomenex-Luna C-18 (5$\mu$) (3.0x50mm); Solvent A = 10% acetonitrile-90% water - 0.1 % TFA, Solvent B = 90% acetonitrile - 10% water - 0.1% TFA; gradient 0% to 100% solvent B over 2 min. and then hold for 1 min.; 4mL/min; inj. vol. = 5uL; wavelength =220.

*Step 3:*

NaH (60% in oil) (0.186 g, 4.65 mmol) was added to a solution of (2S,4R)-2-methyl 1-(2-(trimethylsilyl)ethyl) 4-hydroxy-4-(7-vinylnaphthalen-2-yl)pyrrolidine-1,2-dicarboxylate (1.14 g, 2.58 mmol) and methyl iodide (0.291 mL, 4.65 mmol) at 0°C in DMF and stirred at this temperature for 3 hrs. The reaction was then quenched with saturated $NH_4Cl$ solution and ether. The ether layer was washed with brine, dried, filtered and concentrated to give crude material. The crude material was purified by flash chromatography on the Biotage (20-40% EtOAc in hexanes) to give (2S,4R)-2-methyl 1-(2-(trimethylsilyl)ethyl) 4-methoxy-4-(7-vinylnaphthalen-2-yl)pyrxolidine-1,2-dicarboxylate (845 mg, 72 % yield) as a clear oil. LCMS: rt = 2.09 min. $[M+Na]^+$ = 478. Phenomenex-Luna C-18 (5μ) (3.0x50mm); Solvent A = 10% acetonitrile-90% water - 0.1% TFA, Solvent B = 90% acetonitrile - 10% water - 0.1 % TFA; gradient 0% to 100% solvent B over 2 min. and then hold for 1 min.; 4mL/min; inj. vol. = 5uL; wavelength =220.

*Step 4:*

TBAF (7.42 mL, 7.42 mmol) was added to a solution of (2S,4R)-2-methyl 1-(2-(trimethylsilyl)ethyl) 4-methoxy-4-(7-vinylnaphthalen-2-yl)pyrrolidine-1,2-dicarboxylate (0.845 g, 1.855 mmol) in THF (20 mL) at r.t. and stirred at this temperature overnight. The reaction was diluted with EtOAc and water. The organic layer was collected and washed with brine, dried over $MgSO_4$, filtered and concentrated to give (2S,4R)-methyl 4-methoxy-4-(7-vinylnaphthalen-2-yl) pyrrolidine-2-carboxylate (0.6 g, 104 % yield) as a light brown oil. LCMS: rt = 1.10 min. $[M+Na]^+$=334. Phenomenex-Luna C-18 (5μ) (3.0x50mm); Solvent A = 10% acetonitrile-90% water - 0.1% TFA, Solvent B = 90% acetonitrile - 10% water - 0.1% TFA; gradient 0% to 100% solvent B over 2 min. and then hold for 1 min.; 4mL/min; inj. vol. = 5uL; wavelength =220.

*Step 5:*

(S)-2-((hex-5-enyloxy)carbonylamino)-3,3-dimethylbutanoic acid (0.744 g, 2.89 mmol), DIEA (1.010 mL, 5.78 mmol) and HATU (0.806 g, 2.120 mmol) were added to a solution of (2S,4R)-methyl 4-methoxy-4-(7-vinylnaphthalen-2-yl)pyrrolidine-2-carboxylate (0.6 g, 1.927 mmol) in DCM (20 mL). The reaction was stirred at r.t. overnight. The reaction was concentrated and purified on the Biotage (10-40% EtOAc:Hex) to give (2S,4R)-methyl 1-((S)-2-((hex-5-enyloxy) carbonylamino)-3,3-dimethylbutanoyl)-4-methoxy-4-(7-vinylnaphthalen-2-yl)pyrrolidine-2-carboxylate (0.7 g, 66 % yield) as a white foam. LCMS: rt = 2.05 min. $[M+Na]^+$ = 573. Phenomenex-Luna C-18 (5μ) (3.0x50mm); Solvent A = 10% acetonitrile-90% water - 0.1% TFA, Solvent B = 90% acetonitrile - 10% water - 0.1% TFA; gradient 0% to 100% solvent B over 2 min. and then hold for 1 min.; 4mL/min; inj. vol. = 5uL; wavelength =220.

*Step 6:*

(2S,4R)-methyl 1-((S)-2-((hex-5-enyloxy)carbonylamino)-3,3-dimethylbutanoyl)-4-methoxy-4-(7-vinylnaphthalen-2-yl)pyrrolidine-2-carboxylate (0.7 g, 1.271 mmol) was dissolved in DCE (130 mL) and bubbled with nitrogen gas for 15 min. Hoveyda-Grubbs catalyst (0.080 g, 0.127 mmol) was added and the reaction sealed and heated in an oil bath at 70 °C for 3hrs. The reaction was cooled, concentrated and purified on the Biotage (10-50%EtOAc/Hex) to give the product (91 mg, 14 % yield) as a white solid. LCMS: rt = 1.96 min. $[M+Na]^+$ = 545. Phenomenex-Luna C-18 (5μ) (3.0x50mm); Solvent A = 10% acetonitrile-90% water - 0.1% TFA, Solvent B = 90% acetonitrile - 10% water - 0.1% TFA; gradient 0% to 100% solvent B over 2 min. and then hold for 1 min.; 4mL/min; inj. vol. = 5uL; wavelength =220.

*Step 7:*

10% Palladium on carbon (19 mg, 0.017 mmol) was added to a solution of the product from Step 6 (91 mg, 0.174 mmol) in ethyl acetate (3 mL) and stirred under a balloon of hydrogen overnight. The reaction was filtered through a Millipore millex-HV 0.45um frit and concentrated to give the product (90 mg, 99 % yield). LCMS: rt = 2.00 min. $[M+Na]^+$ = 547. Phenomenex-Luna C-18 (5μ) (3.0x50mm); Solvent A = 10% acetonitrile-90% water - 0.1% TFA, Solvent B = 90% acetonitrile - 10% water - 0.1% TFA; gradient 0% to 100% solvent B over 2 min. and then hold for 1 min.; 4mL/min; inj. vol. = 5uL; wavelength =220.

*Step 8:*

2.0M Lithium hydroxide (0.25 mL, 0.50 mmol) was added to a solution of the product from Step 7 (90 mg, 0.172 mmol) in THF (1.5 mL) and MeOH (1.5 mL) and was stirred at r.t. overnight. The reaction was diluted with 1M HCl and extracted with EtOAc. The organic layer was washed with brine, dried over $MgSO_4$, filtered and concentrated to give

the product (70 mg, 80 % yield). LCMS: rt =1.77 min. $[M+Na]^+$ = 533. Phenomenex-Luna C-18 (5μ) (3.0x50mm); Solvent A = 10% acetonitrile-90% water - 0.1% TFA, Solvent B = 90% acetonitrile - 10% water - 0.1% TFA; gradient 0% to 100% solvent B over 2 min. and then hold for 1 min.; 4mL/min; inj. vol. = 5uL; wavelength =220.

*Step 9:*

DIEA (0.072 mL, 0.411 mmol), HATU (57.3 mg, 0.151 mmol), and (1S,2R)-2-amino-N-(cyclopropylsulfonyl)bi (cyclopropane)-2-carboxamide, HCl salt (43 mg, 0.151 mmol) were added to a solution of the product from Step 8 (70 mg, 0.137 mmol) in DCM (2.5 mL). The reaction was stirred at r.t. for 2hrs. The crude product was concentrated and purified by prep HPLC (Sunfire Prep C18 OBD 5u (30x 100mm); flow = 42mL/min; solvent gradient 70:30 to 5:95 water/ acetonitrile (with 10mM ammonium acetate)) to give Compound 31 (24 mg, 24 % yield) as a white solid. LCMS: rt = 1.89 min. $[M-H]^-$= 735. Phenomenex-Luna C-18 (5μ) (3.0x50mm); Solvent A = 10% acetonitrile-90% water - 0.1% ammonium acetate, Solvent B = 90% acetonitrile - 10% water - 0.1 % ammonium acetate; gradient 0% to 100% solvent B over 2 min. and then hold for 1 min.; 4mL/min; inj. vol. = 5uL; wavelength =220. $^1$H NMR (500 MHz, CHLOROFORM-*d* δ ppm 0.12 - 0.27 (m, 2 H) 0.43 - 0.62 (m, 2 H) 0.77 - 0.87 (m, 1H) 0.87 - 0.97 (m, I H) 0.97 - 1.04 (m, 2 H) 1.05 - 1.15 (m, 9 H) 1.17 - 1.28 (m, 3 H) 1.28 - 1.54 (m, 5 H) 1.73 (br. s., 2 H) 1.76 - 1.85 (m, 3 H) 2.33 (dd, *J*=12.05,6.26 Hz, 1H) 2.60 (t, *J*=11.75 Hz, 1H) 2.71 - 2.88 (m, 2 H) 2.88 - 2.98 (m, 1H) 3.14 (s, 3 H) 3.77 (dd, *J*=11.29, 6.41 Hz, 1H) 3.84 - 3.96 (m, 2 H) 4.30 (dt, *J*=10.61, 7.21 Hz, 1H) 4.58 (d, *J*=10.07 Hz, 1H) 4.93 (d, *J*=10.38 Hz, 1H) 5.54 (d, *J*=10.07 Hz, 1H) 6.35 (s, 1H) 7.31 (dd, J=8.55, 1.53 Hz, 1 H) 7.45 - 7.59 (m, 3 H) 7.73 (d, *J*=8.24 Hz, 1H) 7.84 (d, *J*=8.55 Hz, 1H).

*Preparation of Compound 32:*

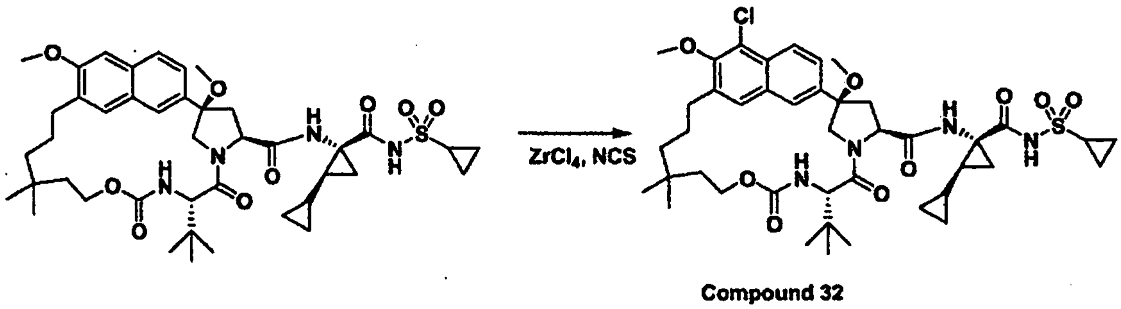


**Compound 32**

Zirconium(IV) chloride (0.15 mg, 0.629 μmol) and N-chlorosuccinimide (1.7 mg, 0.013 mmol) were added to a solution of compound 19 (10mg, 0.013 mmol) in DCM (0.5 mL) and stirred at r.t. overnight. The reaction was concentrated and purified by prep HPLC (Sunfire Prep C18 OBD 5u (30x100mm); flow = 42mL/min; solvent gradient 70:30 to 5:95 water/acetonitrile (with 10mM ammonium acetate)) to give Compound 32 (6 mg, 54 % yield) as a white solid. LCMS: rt = 2.72 min. $[M-MeOH]^+$ = 797. Phenomenex-Luna C-18 (3μ) (30x2mm); Solvent A = 10% acetonitrile-90% water - 0.1% TFA, Solvent B = 90% acetonitrile - 10% water - 0.1% TFA; gradient 0% to 100% solvent B over 2 min. and then hold for 1 min.; 4mL/min; inj. vol. = 5uL; wavelength =220. $^1$H NMR (500 MHz, CHLOROFORM-*d*) δ ppm 0.12 (d, *J*=2.44 Hz, 2 H) 0.55 (br. s., 2 H) 0.87 (d, *J*=7.32 Hz, 2 H) 0.93 (d, *J*=11.60 Hz, 6 H) 1.01 (d, *J*=10.07 Hz, 2 H) 1.11 (s, 9 H) 1.68 (br. s., 6 H) 1.75 - 1.95 (m, 3 H) 2.38 (br. s., 1H) 2.62 (br. s., 1H) 2.80 (br. s., 1H) 2.93 (br. s., 2 H) 3.10 (d, *J*=2.14 Hz, 3 H) 3.73 (br. s., 1H) 3.86 (br. s., 1H) 3.90 (d, *J*=2.44 Hz, 3 H) 3.93 - 4.05 (m, 1H) 4.33 (br. s., 1H) 4.47 (d, *J*=9.77 Hz, 1 H) 4.98 (br. s., 1H) 5.37 (br. s., 1H) 6.21 (br. s., 1H) 7.63 (d, *J*=9.16 Hz, 1H) 7.79 (br. s., 2 H) 8.22 (dd, *J*=8.55,2.14 Hz, 1 H) 9.99 (br. s., 1H).

*Preparation of Compound 33:*

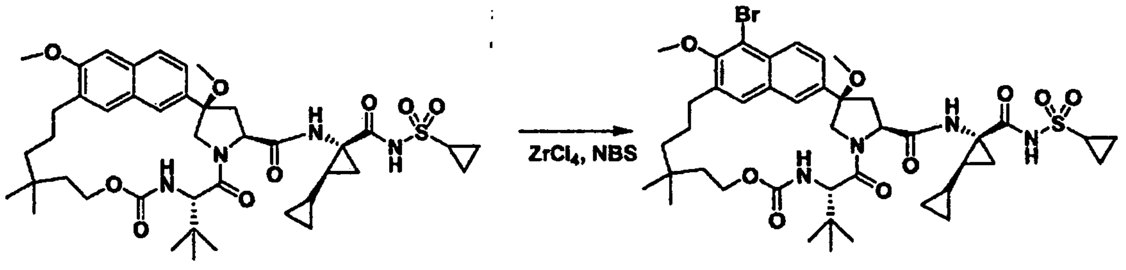


**Compound 33**

Zirconium(IV) chloride (0.73 mg, 3.14 μmol) and N-bromosuccinimide (11 mg, 0.063 mmol) were added to a solution of Compound 19 (50 mg, 0.063 mmol) in DCM (1 mL) and stirred at r.t. for overnight. The reaction was concentrated and purified on prep HPLC (Sunfire Prep C18 OBD 5u (30x100mm); flow = 42mL/min; solvent gradient 70:30 to 5:95 water/acetonitrile (with 10mM ammonium acetate)) to give Compound 33 (28 mg, 50.4 % yield) as a white solid LCMS: rt = 2.56 min. $[M-MeOH]^+$ = 843. Phenomenex-Luna C-18 (3μ) (30x2mm); Solvent A = 10% acetonitrile-90% water - 0.1% TFA, Solvent B = 90% acetonitrile - 10% water - 0.1% TFA; gradient 0% to 100% solvent B over 2 min. and then hold for 1 min.; 4mL/min; inj. vol. = 5uL; wavelength =220. $^1$H NMR (500 MHz, CHLOROFORM-*d*) δ ppm 0.14 (d, *J*=8.24 Hz, 1H) 0.23 (d, *J*=4.58 Hz, 1H) 0.43 - 0.62 (m, 2 H) 0.88 (d, *J*=11.90 Hz, 2H) 0.93 (d, *J*=10.07 Hz, 6 H) 0.97 - 1.07 (m, 3 H) 1.12 (s, 9 H) 1.26 (d, *J*=5.80 Hz, 1 H) 1.28 (s, 1H) 1.33 - 1.41 (m, 2 H) 1.68 (s, 2 H) 2.39 (s, 1H) 2.62 (t, *J*=11.75 Hz, 1H) 2.74 - 2.86 (m, 1H) 2.89 - 2.95 (m, 1H) 2.96 (s, 1H) 3.10 (s, 3 H) 3.73 (d, *J*=6.10 Hz, 1H) 3.89 (s, 3 H) 3.98 (s, 1H) 4.34 (s, 1H) 4.47 (d, *J*=10.07 Hz, 1H) 4.98 (s, 1H) 5.37 (d, *J*=9.46 Hz, 1H) 6.18 (s, 1H) 7.58 - 7.66 (m, 1H) 7.79 (d, *J*=2.14 Hz, 1H) 8.22 (d, *J*=8.85 Hz, 1H) 10.00 (s, 1H).

*Preparation of Intermediate 9:*

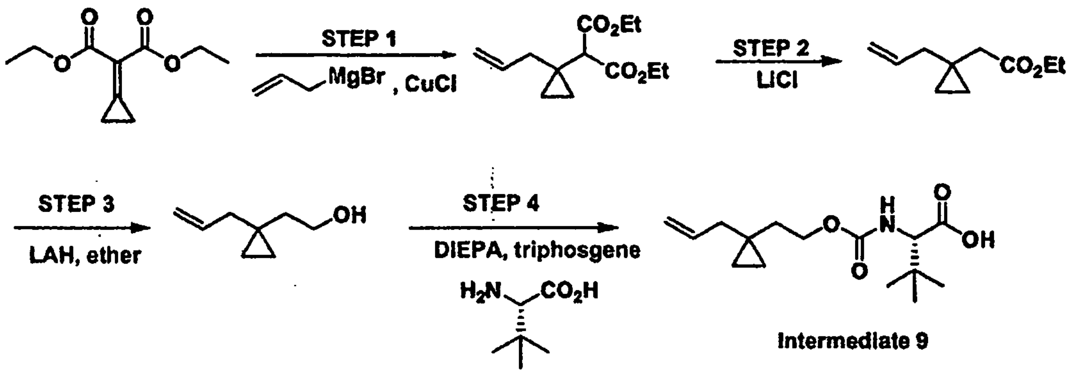


**Intermediate 9**

*Step 1:*

Allyl magnesium bromide (1.0M in ether) (9.4 mL, 9.4 mmol) was added to copper(I) chloride (42 mg, 0.43 mmol) in ether (100 mL) and cooled to -30°C. Diethyl 2-cyclopropylidenemalonaxe (1.7 g, 8.58 mmol) (made as described in Eur. J. Org. Chem. 2004, p. 3992) was added and the reaction allowed to warm to r.t. for 1 hr. The reaction was quenched with sat. ammonium chloride solution and this was stirred for 10 min. The organic layer was separated, dried, filtered and concentrated to give diethyl 2-(1-allylcyclopropyl)malonate (1.6 g, 78%) as a light yellow oil. $^1$H NMR (500 MHz, CHLOROFORM-*d*) d ppm 0.50 - 0.62 (m, 4 H) 1.27 (t, *J*=7.17 Hz, 6 H) 2.30 (d, *J*=7.02 Hz, 2 H) 3.22 (s, 1H) 4.18 (q, *J*=7.02 Hz, 4 H) 4.95 - 5.10 (m, 2 H) 5.62 - 5.81 (m, 1H).

*Step 2:*

Lithium chloride (0.529 g, 12.48 mmol) was added to a solution of diethyl 2-(1-allylcyclopropyl)malonate (1.5 g, 6.24 mmol) in dmso (15 mL) and water (0.15 mL) and heated to 170°C for 2 hrs. The reaction was cooled and diluted with brine and ether. The aqueous layer was extracted with ether and then the combined organics were washed with brine, dried, filtered and concentrated to give 2-(1-allylcyclopropyl)acetate (1g, 95%) as light brown oil. $^1$H NMR (500 MHz, CHLOROFORM-*d*) d ppm 0.36 - 0.50 (m, 4 H) 1.26 (t, *J*=7.17 Hz, 3 H) 2.11 (d, J=7.02 Hz, 2 H) 2.23 (s, 2H) 4.13

(q, J=7.12 Hz, 2 H) 5.01 - 5.10 (m, 2 H) 5.69 - 5.88 (m, J=17.09, 10.07, 7.17, 7.17 Hz, 1H).

*Step 3:*

Lithium aluminum hydride (2.0M in THF) (3.0 mL, 6.0 mmol) was added portion-wised to a solution of ethyl 2-(1-allylcyclopropyl)acetate (1 g, 5.94 mmol) in ether (50 mL) and this was stirred at r.t. overnight. The reaction was quenched with water (0.6 mL) then 3.0M NaOH solution (1.2 mL) and then water (1.8 mL) and stirred vigorously until all the aluminum salts had precipitated. The mixture was dried with magnesium sulfate and then filtered through celite to give 2-(1-allylcyclopropyl)ethanol (650 mg, 87%). $^{1}$H NMR (500 MHz, CHLOROFORM-*d*) d ppm 0.24 - 0.43 (m, 4 H) 1.55 (t, *J*=7.02 Hz, 2 H) 2.02 (d, *J*=6.71 Hz, 2 H) 3.73 (t, *J*=7.02 Hz, 2 H) 4.96 - 5.14 (m, 2 H) 5.72 - 5.89 (m, 1H).

*Step 4:*

Hunig's base (0.93 mL, 5.34 mmol) was added dropwise to a 0 °C solution of 2-(1-allylcyclopropyl)ethanol (741 mg, 5.87 mmol) and triphosgene (792 mg, 2.67 mmol) in dioxane (10 mL). The resulting white suspension was stirred for 5 min. at 0 °C, then allowed to warm up to r.t. for 1 hour. A solution of (S)-2-amino-3,3-dimethylbutanoic acid (700 mg, 5.34 mmol) in dioxane (20 mL) and 3M NaOH (1.78 mL, 5.34 mmol) was added. The reaction mixture was stirred at r.t. overnight. The reaction was diluted with ether and 1.0M HCl and extracted with ether (2x). The combined organics were dried ($MgSO_4$), filtered and concentrated. This material was taken up in ether and extracted with sat. sodium bicarbonate (3x). The combined aqueous layers were washed with ether and then acidified with conc. HCl and the product extracted with ether. The organics were dried with magnesium sulfate, filtered and concentrated to give (S)-2-((2-(1-allylcyclopropyl)ethoxy)carbonylamino)-3,3-dimethylbutanoic acid (Intermediate 9,815 mg, 54%) as a colorless oil. $^{1}$H NMR (400 MHz, CHLOROFORM-*d*) d ppm 0.25 - 0.40 (m, 4 H) 1.03 (s, 9 H) 1.60 (t, *J*=7.15 Hz, 2 H) 2.03 (d, *J*=7.03 Hz, 2 H) 4.10 - 4.19 (m, 3 H) 4.97 - 5.14 (m, 2 H) 5.21 (d, *J*=9.29 Hz, 1H) 5.68 - 5.89 (m, *J*=17.07, 10.04, 7.03, 7.03 Hz, 1H).

*Preparation of Compound 34*

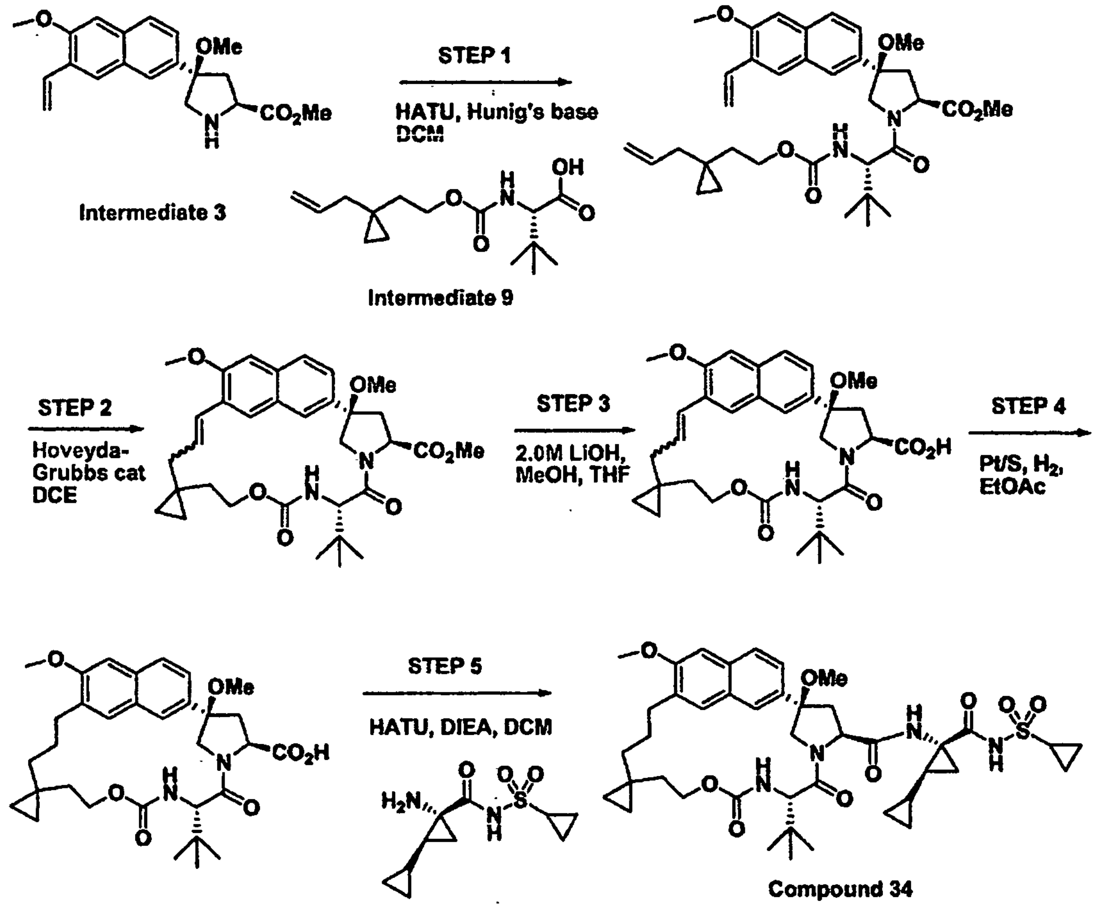


Intermediate 3

Intermediate 9

Compound 34

*Step 1:*

HATU (245 mg, 0.644 mmol) was added to a solution of (2S,4R)-methyl 4-methoxy-4-(6-methoxy-7-vinylnaphthalen-2-yl)pyrrolidine-2-carboxylate (Intermediate 3, 200 mg, 0.586 mmol), (S)-2-((2-(1-allylcyclopropyl)ethoxy)carbonylamino)-3,3-dimethylbutanoic acid (Intermediate 9, 199 mg, 0.703 mmol) and Hunig's base (0.31 mL, 1.76 mmol) in DCM (10 mL) and stirred at r.t. for 4 hrs. The reaction was concentrated and purified by flash chromatography on the Biotage (5-40% EtOAc in hexanes) to give (2S,4R)-methyl 1-((S)-2-((2-(1-allylcyclopropyl)ethoxy)carbonylamino)-3,3-dimethylbutanoyl)-4-methoxy-4-(6-methoxy-7-vinylnaphthalen-2-yl)pyrrolidine-2-carboxylate (200 mg, 56%) as a thick oil. LCMS: r.t. = 2.19 min., $[M-OMe]^+$ = 575 Phenomenex Luna C18 10u (3 x 50 mm); Solvent A = 10% methanol - 90% water - 0.1% TFA, Solvent B = 90% methanol - 10% water - 0.1 % TFA; gradient = 0% to 100% solvent B over 2 min. and then hold for 1 min.; 4 mL/min; inj. vol. = 5 ul; wavelength = 220 nm.

*Step 2:*

A solution of (2S,4R)-methyl 1-((S)-2-((2-(1-allylcyclopropyl)ethoxy)carbonylamino)-3,3-dimethylbutanoyl)-4-methoxy-4-(6-methoxy-7-vinylnaphthalen-2-yl)pyrrolidine-2-carboxylate (200 mg, 0.330 mmol) in DCE (75 mL) was sparged with nitrogen for 30 min. and then Hoveyda-Grubbs Catalyst (2nd Generation) (21 mg, 0.033 mmol) was added and the reaction sealed and heated to 80°C overnight. The reaction was concentrated to give crude material. The crude material was purified by flash chromatography on the Biotage (20-50% EtOAc in hexanes) to give the purified product (180 mg, 94%) as a white foam. LCMS: r.t. = 2.11 min., $[M+H]^+$ = 579 Phenomenex Luna C18 10u (3 x 50 mm); Solvent A = 10% methanol - 90% water - 0.1% TFA, Solvent B = 90% methanol - 10% water - 0.1% TFA; gradient = 0% to 100% solvent B over 2 min. and then hold for 1 min.; 4 mL/min; inj. vol. = 5 ul; wavelength = 220 nm.

*Step 3:*

2.0M LiOH (0.78 mL, 1.56 mmol) was added to a solution of the product from Step 2 (180 mg, 0.311 mmol) in THF (2 mL) and MeOH (2 mL) and stirred at r.t. overnight. The reaction was diluted with ether and 1.0M HCl solution. The organics were dried with magnesium sulfate, filtered and concentrated to give the crude product (160 mg, 91%) as

a white foam. LCMS: r.t. = 2.05 min., $[M+H]^+$ = 565 Phenomenex Luna C 18 10u (3 x 50 mm); Solvent A = 10% methanol - 90% water - 0.1% TFA, Solvent B = 90% methanol - 10% water - 0.1% TFA; gradient = 0% to 100% solvent B over 2 min. and then hold for 1 min.; 4 mL/min; inj. vol. = 5 ul; wavelength = 220 nm.

*Step 4:*

Platinum, sulfided, 5 wt. % on carbon, reduced, dry (111 mg, 0.028 mmol) was added to a solution of the product from Step 2 (160 mg, 0.283 mmol) in EtOAc (5 mL) and stirred under an atmosphere of hydrogen for 2 hrs. The reaction was filtered through a Millipore millex-HV 0.45um nylon frit and concentrated to give the crude product (140 mg, 87%). LCMS: r.t. = 2.15 min., $[M-OMe]^+$ = 535 Phenomenex Luna C18 10u (3 x 50 mm); Solvent A = 10% methanol - 90% water - 0.1 % TFA, Solvent B = 90% methanol - 10% water - 0.1 % TFA; gradient = 0% to 100% solvent B over 2 min. and then hold for 1 min.; 4 mL/min; inj. vol. = 5 ul; wavelength = 220 nm.

*Step 5:*

HATU (122 mg, 0.321 mmol) was added to a solution of (1S,2R)-2-amino-N-(cyclopropylsulfonyl)bi(cyclopropane)-2-carboxamide, HCl salt (90 mg, 0.321 mmol), the product from Step 4 (140 mg, 0.247 mmol) and Hunig's base (0.13 mL, 0.741 mmol) in dichloromethane (2 mL) and stirred at r.t. overnight. The reaction was concentrated and then purified by prep. HPLC (Sunfire C18 10u (30 x100 mm); flow = 42 mL/min; solvent gradient = 95:5 to 5:95 water/acetonitrile (with 10mM ammonium acetate)). The product fractions were concentrated on the speed vac to give Compound 34 (77mg, 39%) as a white solid $^1H$ NMR (500 MHz, CHLOROFORM-*d*) d ppm 0.17 (ddd, *J*=9.31, 4.58, 4.43 Hz, 1 H) 0.23 (dq, J=9.42, 4.69 Hz, 1 H) 0.28 - 0.37 (m, 4 H) 0.45 - 0.59 (m, 2 H) 0.83 (qd, *J*=8.19, 4.12 Hz, 1 H) 0.90 - 0.97 (m, 1 H) 0.97 - 1.06 (m, 2 H) 1.12 (s, 9 H) 1.20 - 1.29 (m, 2 H) 1.29 - 1.41 (m, 2 H) 1.42 - 1.53 (m, 1 H) 1.64 - 1.84 (m, 3 H) 1.88 - 2.00 (m, 1 H) 2.00 - 2.15 (m, 1 H) 2.41 (dd, *J*=11.75, 6.26 Hz, 1 H) 2.60 (t, *J*=11.60 Hz, 1 H) 2.70 - 2.87 (m, 2 H) 2.87 - 2.96 (m, 1 H) 3.08 (s, 3 H) 3.78 (dd, *J*=11.29, 6.41 Hz, 1 H) 3.87 (d, *J*=10.07 Hz, 1 H) 3.93 (s, 3 H) 4.10 (td, *J*=11.06, 5.65 Hz, 1 H) 4.41 (td, *J*=11.37, 5.04 Hz, 1 H) 4.46 (d, *J*=9.77 Hz, 1 H) 5.04 (d, *J*=10.07 Hz, 1 H) 5.47 (d, *J*=9.46 Hz, 1 H) 6.42 (s, 1 H) 7.07 (s, 1 H) 7.50 (dd, *J*=8.55, 1.53 Hz, 1 H) 7.74 (d, *J*=8.85 Hz, 1 H) 7.79 (s, 1 H) 7.91 (s, 1 H) 10.04 (s, 1 H).

*Preparation of Intermediate 10:*

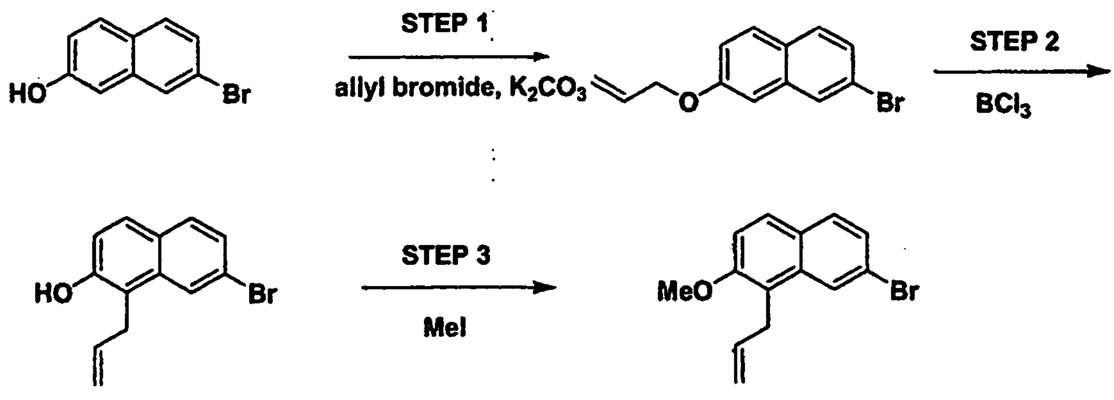


Intermediate 10

*Step 1:*

Allyl bromide (1.9 mL, 22.41 mmol) was added to a solution of 7-bromonaphthalen-2-ol (5 g, 22.41 mmol) and potassium carbonate (12.39 g, 90 mmol) in acetone (90 mL) and heated to reflux for 18 hours. The reaction was partially concentrated and taken up in water and ether. The organics were collected, dried over $MgSO_4$, filtered and concentrated to give 2-(allyloxy)-7-bromonaphthalene (6 g, 22.80 mmol, 102 % yield). LCMS: rt = 2.30min. $[M+H]^+$ = 264 and 262 ; Phenomenex-Luna C-18 (5μ) (30x2); Solvent A = 10% acetonitrile-90% water - 0.1% TFA, Solvent B = 90% acetonitrile - 10% water - 0.1% TFA; gradient 0% to 100% solvent B over 2 min. and then hold for 1 min.; 4mL/min; inj. vol. = 5uL; wavelength = 220. $^1H$ NMR (400 MHz, CHLOROFORM-*d*) d ppm 4.66 (dt, *J*=5.27, 1.38 Hz, 2 H) 5.35 (dd, *J*=10.54, 1.25 Hz, 1 H) 5.43 - 5.55 (m, 1 H) 6.03 - 6.20 (m, 1 H) 7.05 (d, *J*=2.51 Hz, 1 H) 7.19 (dd, *J*=8.91, 2.38 Hz, 1 H) 7.41 (dd,

$J$=8.66, 1.88 Hz, 1 H) 7.57 - 7.67 (m, 1 H) 7.72 (d, $J$=9.03 Hz, 1 H) 7.89 (d, $J$=1.76 Hz, 1 H)

*Step 2:*

Boron trichloride (23.5 mL, 23.56 mmol) was added to a solution of 2-(allyloxy)-7-bromonaphthalene (6.2 g, 23.56 mmol) in DCM (150 mL) at 0 °C and stirred for 20 min. before warming up to r.t. The reaction was stirred at r.t. for 20 min. and quenched with sat. ammonium chloride. The organic layer was washed with brine, dried over $MgSO_4$, filtered and concentrated to give the final product 1-allyl-7-bromonaphthalen-2-ol (6.1 g, 23.18 mmol, 98 % yield) as a brown oil. $^1$H NMR (400 MHz, CHLOROFORM-*d*) d ppm 3.78 (dt, $J$=5.77, 1.76 Hz, 2 H) 5.01 - 5.22 (m, 2 H) 5.98 - 6.15 (m, $J$=17.10, 10.26, 5.77, 5.77 Hz, 1 H) 7.11 (d, $J$=8.78 Hz, 1 H) 7.42 (dd, $J$=8.78,1.76 Hz, 1 H) 7.65 (d, $J$=9.03 Hz, 2 H) 8.05 (d, $J$=1.76 Hz, 1 H).

*Step 3*:

Iodomethane (1.7 mL, 27.8 mmol) was added to a solution of 1-allyl-7-bromonaphthalen-2-ol (6.1 g, 23.18 mmol) and cesium carbonate (9.06 g, 27.8 mmol) in DMF (110 mL) and stirred at r.t. for 18 hours. The reaction was diluted with water and extracted with diethyl ether (3x). The combined organics were washed with brine, dried over $MgSO_4$, filtered and concentrated to give 1-allyl-7-bromo-2-methoxynaphthalene (Intermediate 10, 5.87g, 91%). $^1$H NMR (500 MHz, CHLOROFORM-*d*) $\delta$ ppm 3.81 (dt, $J$=5.80, 1.68 Hz, 2 H) 3.96 (s, 3 H) 4.97 (dq, $J$=17.09, 1.83 Hz, 1 H) 5.00 - 5.07 (m, 1 H) 5.95 - 6.09 (m, 1 H) 7.30 (d, $J$=9.16 Hz, 1 H) 7.41 (dd, $J$=8.70,1.98 Hz, 1 H) 7.65 (d, $J$=8.85 Hz, 1 H) 7.73 (d, $J$=8.85 Hz, 1 H) 8.08 (d, $J$=1.83 Hz, 1 H)

*Preparation of Intermediate 11:*

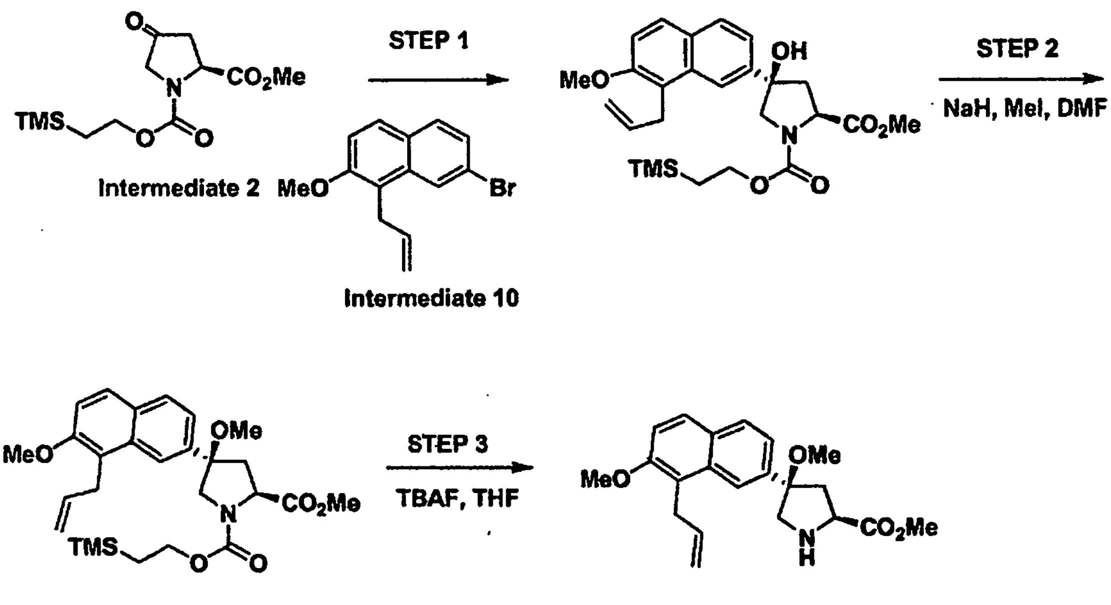


Intermediate 2

Intermediate 10

Intermediate 11

*Step 1:*

Magnesium (0.541 g, 22.24 mmol) was stirred in a round bottom flask under nitrogen for 30 min. to cause scratching of the surface of the magnesium turnings. 10mL of THF was added to the magnesium turnings and stirred for an additional 30 min. 1-allyl-7-bromo-2-methoxynaphthalene (Intermediate 10) (5.87 g, 21.18 mmol) in THF (75 mL), was added dropwise at reflux with vigorous stirring. This Grignard solution (85 mL) was added to a solution of (S)-2-methyl 1-(2-(trimethylsilyl)ethyl) 4-oxopyrrolidine-1,2-dicarboxylate (6.08 g, 21.16 mmol) in DCM (75 mL) at r.t. and stirred for 1 hr and then quenched with sat. ammonium chloride solution. The aqueous layer was extracted with DCM and the combined organics were dried over $MgSO_4$, filtered and concentrated to give crude material. The crude material was purified by flash chromatography on the Biotage (5-40% EtOAc:Hex) to give (2S,4R)-2-methyl 1-(2-(trimethylsilyl)ethyl) 4-(8-allyl-7-methoxynaphthalen-2-yl)-4-hydroxypyrrolidine-1,2-dicarboxylate (4.34 g, 8.94 mmol, 42 % yield) as a yellow oil. $^1$H NMR (500 MHz, CHLOROFORM-*d*) δ ppm -0.01 - 0.12 (m, 9 H) 1.04 (dd, *J*=9.16, 7.93 Hz, 2 H) 2.38 - 2.55 (m, 1 H) 2.73 - 2.86 (m, 1 H) 3.73 - 3.79 (m, 1 H) 3.82 - 3.87 (m, 3 H) 3.88 (br. s., 1 H) 3.91 - 3.94 (m, 1 H) 3.96 (s, 3 H) 3.97 - 4.08 (m, 2 H) 4.17 - 4.31 (m, 2 H) 4.52 - 4.70 (m, 1 H) 4.89 - 5.07 (m, 2 H) 5.96 - 6.15 (m, 1 H) 7.28 - 7.33 (m, 1 H) 7.41 (d, J=8.85 Hz, 1 H) 7.75 (d, *J*=9.16 Hz, 1 H) 7.78 - 7.83 (m, 1 H) 8.04 - 8.12 (m, 1 H).

*Step 2:*

NaH (60% in oil) (0.643 g, 16.09 mmol) was added to a solution of (2S,4R)-2-methyl 1-(2-(trimethylsilyl)ethyl) 4-(8-allyl-7-methoxynaphthalen-2-yl)-4-hydroxypyrrolidine-1,2-dicarboxylate (4.34 g, 8.94 mmol) and methyl iodide (1.0 mL, 16.09 mmol) at 0°C in DMF and stirred at this temperature for 3 hrs. The reaction was then quenched with sat. $NH_4Cl$ solution and ether. The ether layer was washed with brine, dried over $MgSO_4$, filtered and concentrated to give crude material. The crude material was purified by flash chromatography on the Biotage (5-45% EtOAc in hexanes) to give (2S,4R)-2-methyl 1-(2-(trimethylsilyl)ethyl) 4-(8-allyl-7-methoxynaphthalen-2-yl)-4-methoxypyrrolidine-1,2-dicarboxylate (2.97 g, 5.94 mmol, 66 % yield) as a yellow oil. LCMS: rt = 2.44min. $[M+H]^4$ = 500 ; Phenomenex-Luna C-18 (5μ.); Solvent A = 10% acetonitrile-90% water - 0.1% TFA, Solvent B = 90% acetonitrile - 10% water - 0.1% TFA; gradient 0% to 100% solvent B over 2 min. and then hold for 1 min.; 4mL/min; inj. vol. = 5uL; wavelength = 220. $^1$H NMR (400 MHz, CHLOROFORM-*d*) δ ppm -0.01 - 0.12 (m, 9 H) 0.89 - 1.14 (m, 2 H) 1.56 - 1.78 (m, 1 H) 2.62 (ddd, *J*=12.74, 9.10, 3.01 Hz, 1 H) 2.87 (t, *J*=13.30 Hz, 1 H) 2.95 (d, *J*=4.77 Hz, 3 H) 3.73 - 3.83 (m, 3 H) 3.84 - 3.89 (m, 2 H) 3.94 - 3.99 (m, 3 H) 4.01 - 4.11 (m, 1 H) 4.17 - 4.34 (m, 2 H) 4.45 - 4.71 (m, I H) 4.92 - 5.08 (m, 2 H) 5.93 - 6.17 (m, 1 H) 7.29 - 7.42 (m, 2 H) 7.69 - 7.91 (m, 3 H).

*Step 3:*

TBAF (1.0M THF, 23.8 mL, 23.78 mmol) was added to a solution of (2S,4R)-2-methyl 1-(2-(trimethylsilyl)ethyl) 4-(8-allyl-7-methoxynaphthalen-2-yl)-4-methoxypyrrolidine-1,2-dicarboxylate (2.97 g, 5.94 mmol) in THF (20 mL) and stirred at r.t. overnight. The reaction was diluted with DCM and washed with water and then brine. The organic layer was collected, dried over $MgSO_4$, filtered and concentrated to give crude (2S,4R)-methyl 4-(8-allyl-7-methoxynaphthalen-2-yl)-4-methoxypyrrolidine-2-carboxylate (Intermediate 11, 2.28 g, 6.41 mmol, 108 % yield) as a yellow oil. LCMS: rt = 1.35min. $[M+H]^+$ = 356 ; Phenomenex-Luna C-18 (5μ) (30x2); Solvent A = 10% acetonitrile-90% water - 0.1 % TFA, Solvent B = 90% acetonitrile - 10% water - 0.1% TFA; gradient 0% to 100% solvent B over 2 min. and then hold for 1 min.; 4mL/min; inj. vol. = 5uL; wavelength = 220.

*Prepartion of Compound 35:*

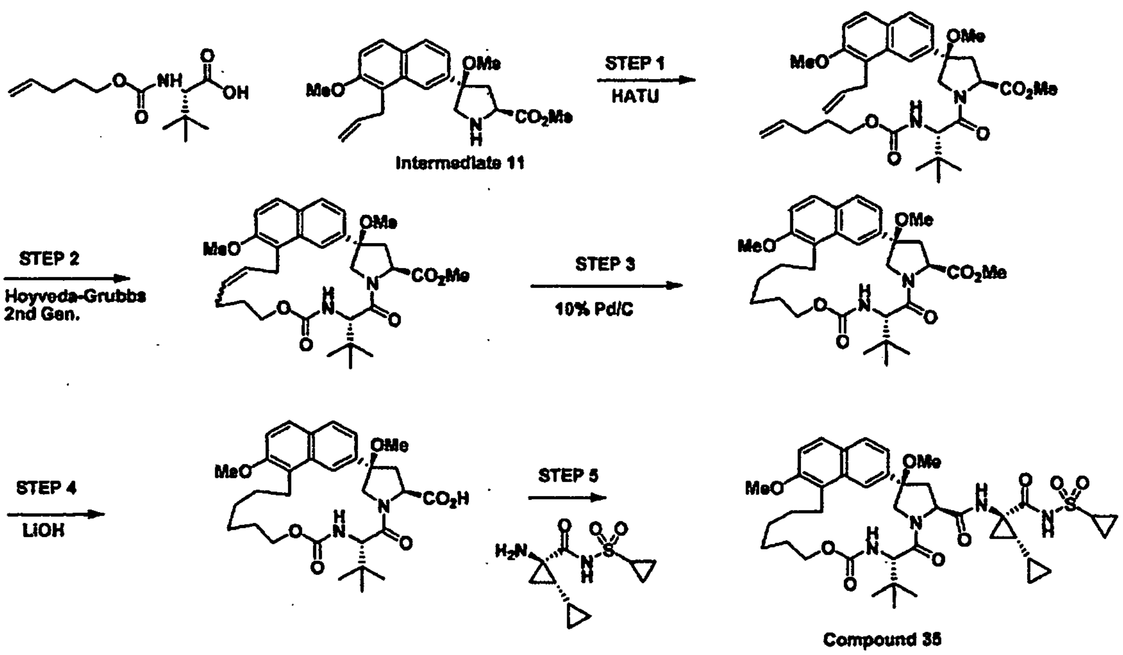

Intermediate 11

Compound 35

*Step 1:*

(S)-3,3-dimethyl-2-((pent-4-enyloxy)carbonylamino)butanoic acid (205 mg, 0.844 mmol), DIEA (0.393 mL, 2.251 mmol) and HATU (235 mg, 0.619 mmol) were added to a solution of (2S,4R)-methyl 4-(8-allyl-7-methoxynaphthalen-2-yl)-4-methoxypyrrolidine-2-carboxylate (Intermediate 11, 200 mg, 0.563 mmol) in DCM (5 mL). The reaction was stirred at r.t. for 18 hours. The reaction was concentrated and purified by flash chromatography on Biotage (10-40% EtOAc:Hex) to give (2S,4R)-methyl 4-(8-allyl-7-methoxynaphthalen-2-yl)-1-((S)-3,3-dimethyl-2-((pent-4-enyloxy)carbonylamino)butanoyl)-4-methoxypyrrolidine-2-carboxylate (300 mg, 0.517 mmol, 92 % yield) as a white sticky foam. LCMS: rt = 2.32min. $[M+H]^+$ 581 ; Phenomenex-Luna C-18 (5μ); Solvent A = 10% acetonitrile-90% water - 0.1% TFA, Solvent B = 90% acetonitrile - 10% water - 0.1% TFA; gradient 0% to 100% solvent B over 2 min. and then hold for 1 min.; 4mL/min; inj. vol. = 5uL; wavelength = 220.

***Step 2*:**

(2S,4R)-methyl 4-(8-allyl-7-methoxynaphthalen-2-yl)-1-((S)-3,3-dimethyl-2-((pent-4-enyloxy)carbonylamino)butanoyl)-4-methoxypyrrolidine-2-carboxylate (300 mg, 0.517 mmol) was dissolved in DCE (53 mL) and bubbled with nitrogen gas for 15 min. Hoyveda-Grubbs 2nd generation catalyst (32.5 mg, 0.052 mmol) was added and heated in an oil bath at 80 °C for 2 hours. The crude product was purified on the Biotage (5-50% EtOAc/Hex) to give the product (189 mg, 0.342 mmol, 66 % yield) as a greenish brown foam. LCMS: rt = 2.06min. $[M+H]^+$ = 553 ; Phenomenex-Luna C-18 (5μ) (30x2); Solvent A = 10% acetonitrile-90% water - 0.1% TFA, Solvent B = 90% acetonitrile - 10% water - 0.1 % TFA; gradient 0% to 100% solvent B over 2 min. and then hold for 1 min.; 4mL/min; inj. vol. = 5uL; wavelength = 220.

*Step 3:*

The product from Step 2 (140 mg, 0.253 mmol) in ethyl acetate (3 mL) was treated with 10% palladium on carbon (27.0 mg, 0.025 mmol) and hydrogenated under a balloon of hydrogen overnight. The reaction was filtered through a Millipore millex-HV 0.45um plug and concentrated to give the product (131 mg, 0.236 mmol, 93 % yield). LCMS: rt = 2.15min. $[M+H]^+$ = 555 ; Phenomenex-Luna C-18 (5μ) (30x2); Solvent A = 10% acetonitrile-90% water - 0.1% TFA, Solvent B = 90% acetonitrile - 10% water - 0.1% TFA; gradient 0% to 100% solvent B over 2 min. and then hold for 1 min.; 4mL/min; inj. vol. = 5uL; wavelength = 220.

*Step 4:*

2.0M Lithium hydroxide (0.354 mL, 0.709 mmol) was added to a solution of the product from Step 3 (131 mg, 0.236 mmol) in THF (1.5 mL) and MeOH (1.5 mL) and was stirred at r.t. overnight. The reaction was diluted with 1M HCl and extracted with EtOAc. The organic layer was washed with brine, dried over $MgSO_4$, filtered and concentrated to give the product (122 mg, 0.226 mmol, 96 % yield) as a white foam. LCMS: rt = 1.91min. $[M+H]^+$ = 541 ; Phenomenex-Luna C-18 (5μ) (30x2); Solvent A = 10% acetonitrile-90% water - 0.1% TFA, Solvent B = 90% acetonitrile - 10% water - 0.1% TFA; gradient 0% to 100% solvent B over 2 min. and then hold for 1 min.; 4mL/min; inj. vol. = 5uL; wavelength = 220.

*Step 5:*

DIEA (0.12 mL, 0.677 mmol), HATU (94 mg, 0.248 mmol), and (1S,2R)-2-amino-N-(cyclopropylsulfonyl)bi(cyclopropane)-2-carboxamide, HCl salt (70 mg, 0.248 mmol) were added to a solution of product from Step 4 (122 mg, 0.226 mmol) in DCM (2.5 mL). The reaction was stirred at r.t. for 2 hrs. The crude product was concentrated and purified on prep HPLC to give Compound 35 (40.4 mg, 0.050 mmol, 22 % yield) as a white solid. LCMS: rt = 2.22min. $[M-OMe]^+$ = 735 ; Phenomenex-Luna C-18 (5μ) (30x2); Solvent A = 10% acetonitrile-90% water - 0.1% TFA, Solvent B = 90% acetonitrile - 10% water - 0.1% TFA; gradient 0% to 100% solvent B over 2 min. and then hold for 1 min.; 4mL/min; inj. vol. = 5uL; wavelength = 220. $^1H$ NMR (500 MHz, CHLOROFORM-*d*) δ ppm 0.33 (td, *J*=7.71, 4.73 Hz, 2 H) 0.48 - 0.66 (m, 2 H) 0.92 (d, *J*=7.63 Hz, 1 H) 0.97 - 1.06 (m, 2 H) 1.04 - 1.08 (m, 2 H) 1.09 (s, 9 H) 1.16 - 1.30 (m, 1 H) 1.34 - 1.46 (m, 2 H) 1.52 (d, 2 H) 1.64 - 1.73 (m, 3 H) 1.85 (dd, *J*=7.78, 5.95 Hz, 1 H) 2.40 (dd, *J*=13.73,10.07 Hz, 1 H) 2.63 - 2.93 (m, 1 H) 2.95 - 3.07 (m, 6 H) 3.95 (s, 3H) 4.18 - 4.38 (m, 3 H) 4.45 (d, *J*=9.77 Hz, 1 H) 4.49 - 4.63 (m, 1 H) 4.63 - 4.78 (m, 1 H) 5.29 (d, *J*=9.16 Hz, 1 H) 6.62 - 7.11 (m, 1 H) 7.27 - 7.35 (m, 1 H) 7.38 - 7.49 (m, 1 H) 7.63 - 7.77 (m, 2 H) 7.79 - 7.90 (m, 1 H) 9.75 (br. s., 1 H).

*Preparation of Compound 36:*

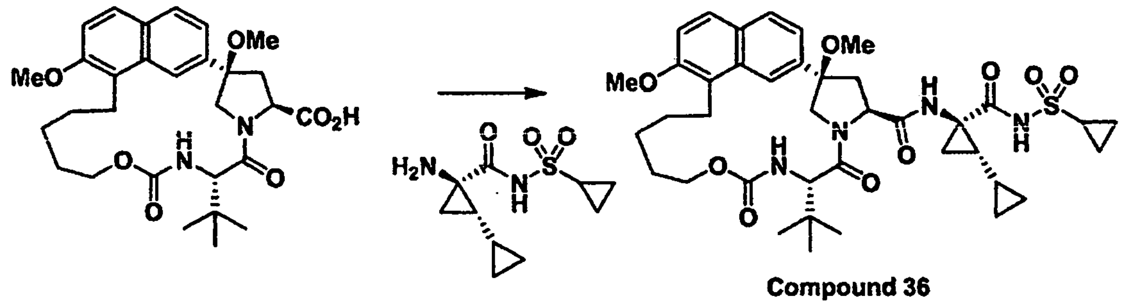

Compound 36

Compound 36 was prepared according to the preparation of compound 35 except using (S)-2-((but-3-enyloxy) carbonylamino)-3,3-dimethylbutanoic acid in step I of the preparation. LCMS: rt = 2.15min. $[M-OMe]^+$ = 721 ; Phenomenex-Luna C-18 (5μ) (30x2); Solvent A = 10% acetonitrile-90% water - 0.1% TFA, Solvent B = 90% acetonitrile - 10% water - 0.1 % TFA; gradient 0% to 100% solvent B over 2 min. and then hold for 1 min.; 4mL/min; inj. vol. = 5uL; wavelength = 220. $^1$H NMR (500 MHz, CHLOROFORM-*d*) δ ppm 0.33 (td, *J*=7.71, 4.73 Hz, 2 H) 0.48 - 0.66 (m, 2 H) 0.92 (d, *J*=7.63 Hz, 1 H) 0.97 - 1.06 (m, 2 H) 1.04 - 1.08 (m, 2 H) 1.09 (s, 9 H) 1.16 - 1.30 (m, 1 M 1.34 - 1.46 (m, 2 H) 1.64 - 1.73 (m, 3 H) 1.85 (dd, *J*=7.78, 5.95 Hz, 1 H) 2.40 (dd, *J*=13.73, 10.07 Hz, 1 H) 2.63 - 2.93 (m, 1 H) 2.95 - 3.07 (m, 6 H) 3.95 (s, 3 H) 4.18 - 4.38 (m, 3 H) 4.45 (d, *J*=9.77 Hz, 1 H) 4.49 - 4.63 (m, 1 H) 4.63 - 4.78 (m, 1 H) 5.29 (d, *J*=9.16 Hz, 1 H) 6.62 - 7.11 (m, 1 H) 7.27 - 7.35 (m, 1 H) 7.38 - 7.49 (m, 1 H) 7.63 - 7.77 (m, 2 H) 7.79 - 7.90 (m, 1 H) 9.75 (br. s., 1 H).

*Preparation of Compound 37:*

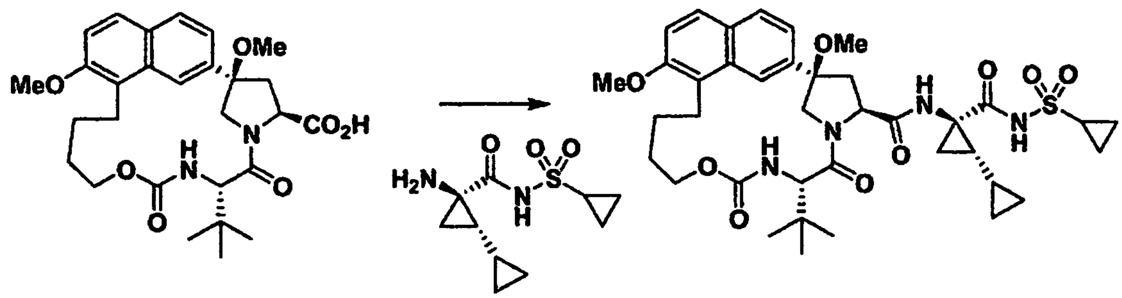

Compound 37

Compound 37 was prepared according to the preparation of compound 35 except using (S)-2-(allyloxycarbonylamino)-3,3-dimethylbutanoic acid in step 1 of the preparation. LCMS: rt = 2.13nim $[M-OMe]^+$ = 707 ; Phenomenex-Luna C-18 (5μ) (30x2); Solvent A = 10% acetonitrile-90% water - 0.1% TFA, Solvent B = 90% acetonitrile - 10% water - 0.1% TFA; gradient 0% to 100% solvent B over 2 min. and then hold for 1 min.; 4mL/min; inj. vol. = 5uL; wavelength = 220. $^1$H NMR (500 MHz, CHLOROFORM-*d*) δ ppm 0.13 - 0.29 (m, 2 H) 0.51 - 0.60 (m, 2 H) 0.77 - 0.89 (m, 1 H) 0.92 - 0.99 (m, 1 H) 1.02 (d, *J*=7.93 Hz, 2 H) 1.04 - 1.06 (m, 1 H) 1.06 - 1.15 (m, 9 H) 1.16 - 1.27 (m, 1 H) 1.29 - 1.43 (m, 3 H) 1.79 (dd, *J*=8.09, 5.65 Hz, 2 H) 1.98 (d, *J*=12.82 Hz, 1 H) 2.40 (dd, *J*=12.05,6.56 Hz, 1 H) 2.65 (t, *J*=11.29 Hz, 1 H) 2.87 (t, *J*=11.60 Hz, 1 H) 2.91 - 2.98 (m, 1 H) 3.00 - 3.17 (m, 3 H) 3.50 (td, *J*=12.36, 7.02 Hz, 1 H) 3.65 (dd, *J*=10.38, 6.41 Hz, 1 H) 3.80 - 3.91 (m, 1H) 3.91 - 3.95 (m, 3 H) 3.97 (s, 1 H) 4.57 (d, *J*=10.68 Hz, 1 H) 5.02 - 5.18 (m, 2 H) 5.43 (d, *J*=10.68 Hz, 1 H) 6.17 (br, s., 1 H) 7.30 (d, *J*=8.85 Hz, 1 H) 7.43 (dd, *J*=8.55, 1.22 Hz, 1 H) 7.70 (d, *J*=9.16 Hz, 1 H) 7.77 (s, 1 H) 7.83 (d, *J*=8.55 Hz, 1 H) 9.88 (br. s., 1 H).

*Preparation of Compound 38*

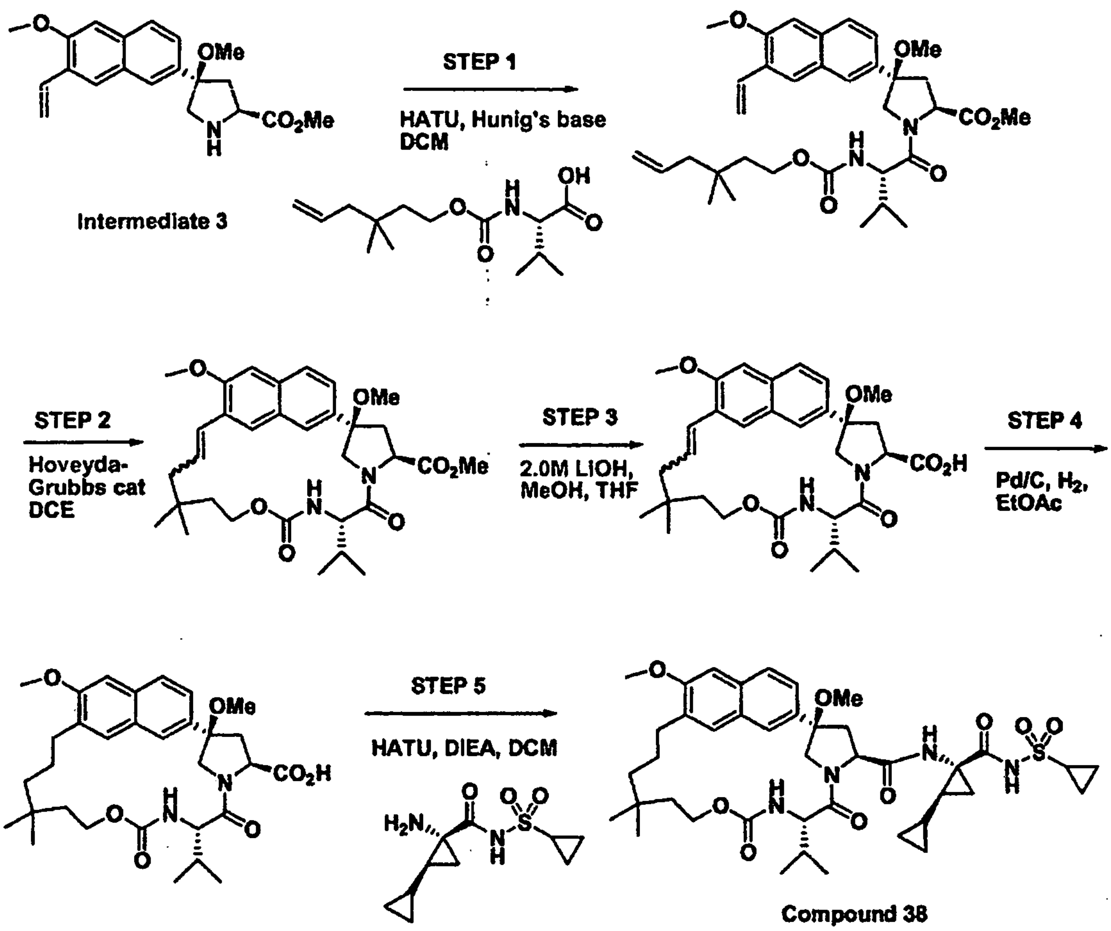


Compound 38

*Step 1:*

HATU (160 mg, 0.422 mmol) was added to a solution of (2S,4R)-methyl 4-methoxy-4-(6-methoxy-7-vinylnaphthalen-2-yl)pyrrolidine-2-carboxylate (Intermediate 3, 120 mg, 0.351 mmol), (S)-2-((3,3-dimethylhex-5-enyloxy)carbonylamino)-3-methylbutanoic acid (143 mg, 0.527 mmol) and Hunig's base (0.18 mL, 1.054 mmol) in DCM (4 mL) and stirred at r.t. overnight. The reaction was concentrated and purified by flash chromatography on the Biotage (10-40% EtOAc in hexanes) to give (2S,4R)-methyl 1-((S)-2-((3,3-dimethylhex-5-enyloxy)carbonylamino)-3-methylbutanoyl)-4-methoxy-4-(6-methoxy-7-vinylnaphthalen-2-yl)pyrrolidine-2-carboxylate (156 mg, 75%) as a white foam. LCMS: r.t. = 1.44 min., $[M-OMe]^+$ = 563 Phenomenex Luna C18 10u (3 x 50 mm); Solvent A = 10% methanol - 90% water - 0.1% TFA, Solvent B = 90% methanol - 10% water - 0.1 % TFA; gradient = 0% to 100% solvent B over 2 min. and then hold for 1 min.; 4 mL/min; inj. vol. = 5 ul; wavelength = 220 nm.

*Step 2:*

A solution of (2S,4R)-methyl 1-((S)-2-((3,3-dimethylhex-5-enyloxy)carbonylamino)-3-methylbutanoyl)-4-methoxy-4-(6-methoxy-7-vinylnaphthalen-2-yl)pyrrolidine-2-carboxylate (156 mg, 0.262 mmol) in DCE (50 mL) was sparged with nitrogen for 30 min. and then Hoveyda-Grubbs Catalyst (2nd generation) (16.5 mg, 0.026 mmol) was added and the reaction sealed and heated to 80°C overnight. The reaction was concentrated and purified by flash chromatography on the Biotage (10-40% EtOAc in hexanes) to give the product (105 mg, 71%) as a white foam. LCMS: r.t. = 2.01 min., $[M+H]^+$ = 567 Phenomenex Luna C18 10u (3 x 50 mm); Solvent A = 10% methanol - 90% water - 0.1% TFA, Solvent B = 90% methanol - 10% water - 0.1% TFA; gradient = 0% to 100% solvent B over 2 min. and then hold for 1 min.; 4 mL/min; inj. vol. = 5 ul; wavelength = 220 nm.

*Step 3:*

2.0M LiOH (0.46 mL, 0.92 mmol) was added to a solution of the product from Step 2 (105 mg, 0.185 mmol) in McOH (1 mL) and THF (1 mL) and stirred at r.t. for 3 days. The reaction was quenched with 1.0M HCl and diluted with

EtOAc. The combined organics were dried with magnesium sulfate, filtered and concentrated to give the crude product (100 mg, 98%) as a white solid. LCMS: r.t. = 2.05 min., $[M+H]^+$ = 553 Phenomenex Luna C18 10u (3 x 50 mm); Solvent A = 10% methanol - 90% water - 0.1% TFA, Solvent B = 90% methanol - 10% water - 0.1% TFA; gradient = 0% to 100% solvent B over 2 min. and then hold for 1 min.; 4 mL/min; inj. vol. = 5 ul; wavelength = 220 nm.

*Step 4:*

10% Pd/C (19 mg, 0.018 mmol) was added to a solution of the product from Step 3 (100 mg, 0.181 mmol) in MeOH (2 mL) and stirred under an atmosphere of hydrogen for 4 hrs. The reaction was filtered through a Millipore millex-HV 0.45um nylon frit and concentrated to give the crude product (100 mg, 100%) as a white foam. LCMS: r.t. = 2.01 min., $[M+H]^+$ = 555 Phenomenex Luna C18 10u (3 x 50 mm); Solvent A = 10% methanol - 90% water - 0.1% TFA, Solvent B = 90% methanol - 10% water - 0.1% TFA; gradient = 0% to 100% solvent B over 2 min. and then hold for 1 min.; 4 mL/min; inj. vol. = 5 ul; wavelength = 220 nm.

*Step 5:*

HATU (103 mg, 0.270 mmol) was added to a solution of (1S,2R)-2-amino-N-(cyclopropylsulfonyl)bi(cyclopropane)-2-carboxamide, HCl salt (76 mg, 0.270 mmol), the product from Step 4 (100 mg, 0.180 mmol) and Hunig's base (0.094 mL, 0.541 mmol) in dichloromethane (2 mL) and stirred at r.t. overnight. The reaction was concentrated and then purified by prep. HPLC (Sunfire C18 10u (30 x100 mm); flow = 42 mL/min; solvent gradient = 95:5 to 5:95 water/acetonitrile (with 10mM ammonium acetate)). The product fractions were concentrated on the speed vac to give Compound 38 (69 mg, 49%) a white solid. LCMS: r.t. = 2.13 min., $[M+Na]^+$ = 803 Phenomenex Luna C18 10u (3 x 50 mm); Solvent A = 10% methanol - 90% water - 0.1% TFA, Solvent B = 90% methanol - 10% water - 0.1 % TFA; gradient = 0% to 100% solvent B over 2 min. and then hold for 1 min.; 4 mL/min; inj. vol. = 5 ul; wavelength = 220 nm. $^1$H NMR (500 MHz, CHLOROFORM-*d*) d ppm 0.01 - 0.15 (m, 1 H) 0.17 - 0.31 (m, *J*=9.54, 4.84, 4.84, 4.58 Hz, 1 H) 0.42 - 0.61 (m, 2 H) 0.84 - 0.90 (m, 2 H) 0.90 (s, 3 H) 0.99 (s, 3 H) 1.01 - 1.05 (m, 2 H) 1.08 (t, *J*=6.71 Hz, 6 H) 1.16 - 1.27 (m, 3 H) 1.36 - 1.47 (m, 1 H) 1.56 (td, *J*=13.20, 4.73 Hz, 1 H) 1.69 - 1.88 (m, 5 H) 2.06 - 2.20 (m, 1 H) 2.29 (dd, *J*=11.90, 6.10 Hz, 1 H) 2.63 (t, *J*=11.60 Hz, 1 H) 2.71 - 2.81 (m, 1 H) 2.82 - 2.90 (m, 1 H) 2.91 - 3.01 (m, 1 H) 3.15 (s, 3 H) 3.73 (dd, *J*=11.29, 6.10 Hz, 1 H) 3.90 (d, *J*=10.07 Hz, 1 H) 3.94 (s, 3 H) 4.01 (td, *J*=10.99, 6.10 Hz, 1 H) 4.29 (t, *J*=9.61 Hz, 1 H) 4.41 (td, *J*=11.22, 4.43 Hz, 1 H) 5.16 (d, *J*=10.07 Hz, 1 H) 5.57 (d, *J*=9.46 Hz, 1 H) 6.39 (s, 1 H) 7.07 (s, 1 H) 7.51 (dd, *J*=8.55, 1.83 Hz, 1 H) 7.63 (s, 1 H) 7.71 (s, 1 H) 7.76 (d, *J*=8.55 Hz, 1 H) 10.19 (s, 1 H).

*Preparation of Intermediate 12:*

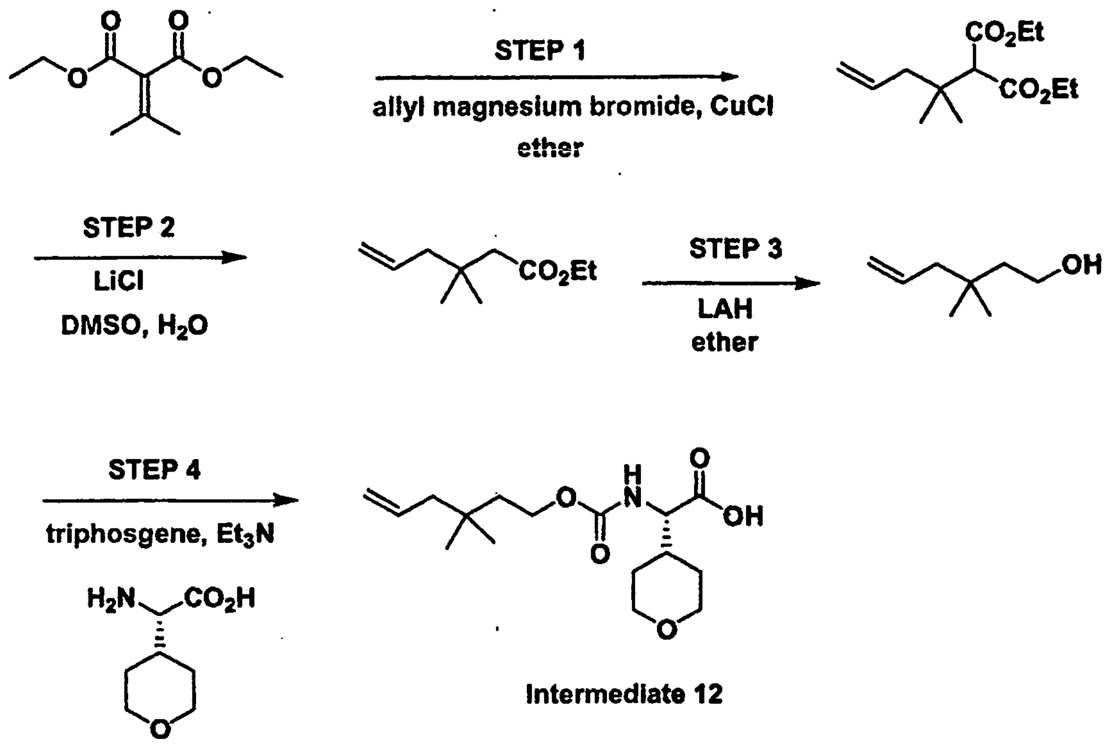


Intermediate 12

*Step 1:*

Allyl magnesium bromide (1.0M in ether) (137 mL, 137 mmol) was added to copper (I) chloride (6.24 mmol) in ether (250 mL) and cooled to -30°C. Diethyl isopropylidenemalonate (24.5 mL, 125 mmol) was added and the reaction allowed to warm to r.t. for 2 hrs. The reaction was quenched with sat. $NH_4Cl$ solution and this was stirred for 30 min. The organic layer was separated, dried, filtered and concentrated to give diethyl 2-(2-methylpent-4-en-2-yl)malonate (30.3 g, 128 mmol, 100% yield) as light yellow oil. $^1H$ NMR (400 MHz, CHLOROFORM-*d*) δ ppm 1.12 (s, 6 H) 1.27 (t, *J*=7.15 Hz, 6 H) 2.23 (d, *J*=7.53 Hz, 2 H) 3.31 (s, 1 H) 4.18 (q, *J*=7.11 Hz, 4 H) 4.98 - 5.12 (m, 2 H) 5.70 - 5.92 (m, 1 H).

*Step 2:*

Lithium chloride (10.50 g, 248 mmol) was added to a solution of diethyl 2-(2-methylpent-4-en-2-yl)malonate (30 g, 124 mmol) in dmso (250 mL) and water (2.5 mL) and heated to 160°C for 48 hours. The reaction was cooled and diluted with brine and ether. The aqueous layer was extracted with ether (3 x 200 mL) and then the combined organics were washed with brine (2 x 200 mL), dried, filtered and concentrated to give 3,3-dimethylhex-5-enoate (21 g, 123 mmol, 100% yield) as brown oil. $^1H$ NMR (500 MHz, CHLOROFORM-*d*) d ppm 1.01-1.06 (m, 6 H) 1.23 - 1.30 (m, 3 H) 2.06 - 2.13 (m, 2 H) 2.16 - 2.20 (m, 2 H) 4.08 - 4.16 (m, 2 H) 5.01 - 5.11 (m, 2 H) 5.77 - 5.89 (m, *J*=17.13, 10.03, 7.48,7.48 Hz, 1 H). LCMS: r.t. = 3.52 min., $[M+H]^+$ = 170, Phenomenex Luna C18, 50 x 2, 3u; Solvent A = 10% acetonitrile - 90% water - 0.1% TFA, Solvent B = 90% acetonitrile - 10% water - 0.1% TFA; gradient = 0% to 100% solvent B over 4 min. and then hold for 1 min.; 0.8 mL/min; inj. vol. = 3 ul; wavelength = 220 nm.

*Step 3:*

To a suspension of lithium aluminum hydride (4.90 g, 129 mmol) in dry $Et_2O$ (300 mL) was added dropwise a solution of ethyl 3,3-dimethylhex-5-enoate (22 g, 129 mmol) in ether (30 mL) at 0 °C. The mixture was stirred for 30 min at the same time, and then the reaction was warmed to the room temperature and stirred overnight. To the mixture were successively added 20 mL of water, 20 mL of 0.1 N NaOH aq and 40 mL of water with care. After stirring for 1 hour, the resulting mixture was filtered through celite pad. The filtrate was cincentrated in vacuo, and the residue was purified by flash chromatography on the Biotage (5-30% EtOAc in Hexanes) to give 3,3-dimethylhex-5-en-1-ol (10.03 g, 78 mmol, 61% yield) as light yellow oil. $^1H$ NMR (400 MHz, CHLOROFORM-*d*) d ppm 0.89 - 0.96 (m, 6 H) 1.07 - 1.13 (m, 1 H) 1.49 - 1.55 (m, 2 H) 1.99 (d, *J*=7.28 Hz, 2 H) 3.72 (td, *J*=7.65, 5.27 Hz, 2 H) 4.97 - 5.09 (m, 2 H) 5.76 - 5.89 (m, 1 H).

*Step 4:*

Hunig's base (0.274 mL, 1.571 mmol) was added dropwise to a solution of 3,3-dimethylhex-1-en-1-ol (0.583 g, 1.728 mmol) and triphosgene (0.233 g, 0.785 mmol) in dioxane (5 mL) at 0 °C. The resulting white suspension was stirred for 5 min. at 0 °C, then allowed to warm up to room temperature for 1 hour. A solution of (S)-2-amino-2-(tetrahydro-2H-pyran-4-yl)acetic acid (0.25 g, 1.571 mmol) in dioxane (2 mL) along with IN NaOH (1.6 mL) was added. The reaction mixture was stirred at r.t. for 3 hrs and then diluted with ether and 1.0M HCl. The aqueous layer was extracted with ether (2 x 30 mL). The combined organics were washed with brine, dried over $MgSO_4$, filtered and concentrated to give the crude product as a light yellow oil which was purified by flash chromatography on the Biotage (50-100% EtOAc in Hexanes and then 10 % MeOH in DCM)) to give (S)-2-((3,3-dimethylhex-5-enyloxy)carbonylamino)-2-(tetrahydro-2H-pyran-4-yl)acetic acid (Intermediate 12, 233 mg, 0.743 mmol, 47.3% yield) as white solid. LCMS: r.t. = 2.65 min., $[M+H]^+$ = 314, Phenomenex Luna C 18, 50 x 2, 3u; Solvent A = 10% acetonitrile - 90% water - 0.1% TFA, Solvent B = 90% acetonitrile - 10% water - 0.1% TFA; gradient = 0% to 100% solvent B over 4 min. and then hold for 1 min.; 0.8 mL/min; inj. vol. = 3 ul; wavelength = 220 nm. $^1$H NMR (400 MHz, CHLOROFORM-$_d$) d ppm 0.88 - 0.99 (m, 6 H) 1.52 (br. s., 2 H) 1.52 - 1.65 (m, 4 H) 1.99 (d, *J*=7.53 Hz, 2 H) 2.07 (d, *J*=12.80 Hz, 1 H) 3.37 (t, *J*=7.03 Hz, 2 H) 3.98-4.03 (m., 2 H) 4.11 (br. s., 1 H) 4.17 (d, *J*=8.03 Hz, 1 H) 4.23 (br. s., 1 H) 4.47 (br. s., 2 H) 4.98 - 5.11 (m, 2 H) 5.74 - 5.88 (m, *J*=17.07, 9.98, 7.43, 7.43 Hz, 1 H).

*Preparetion of Compound 39*

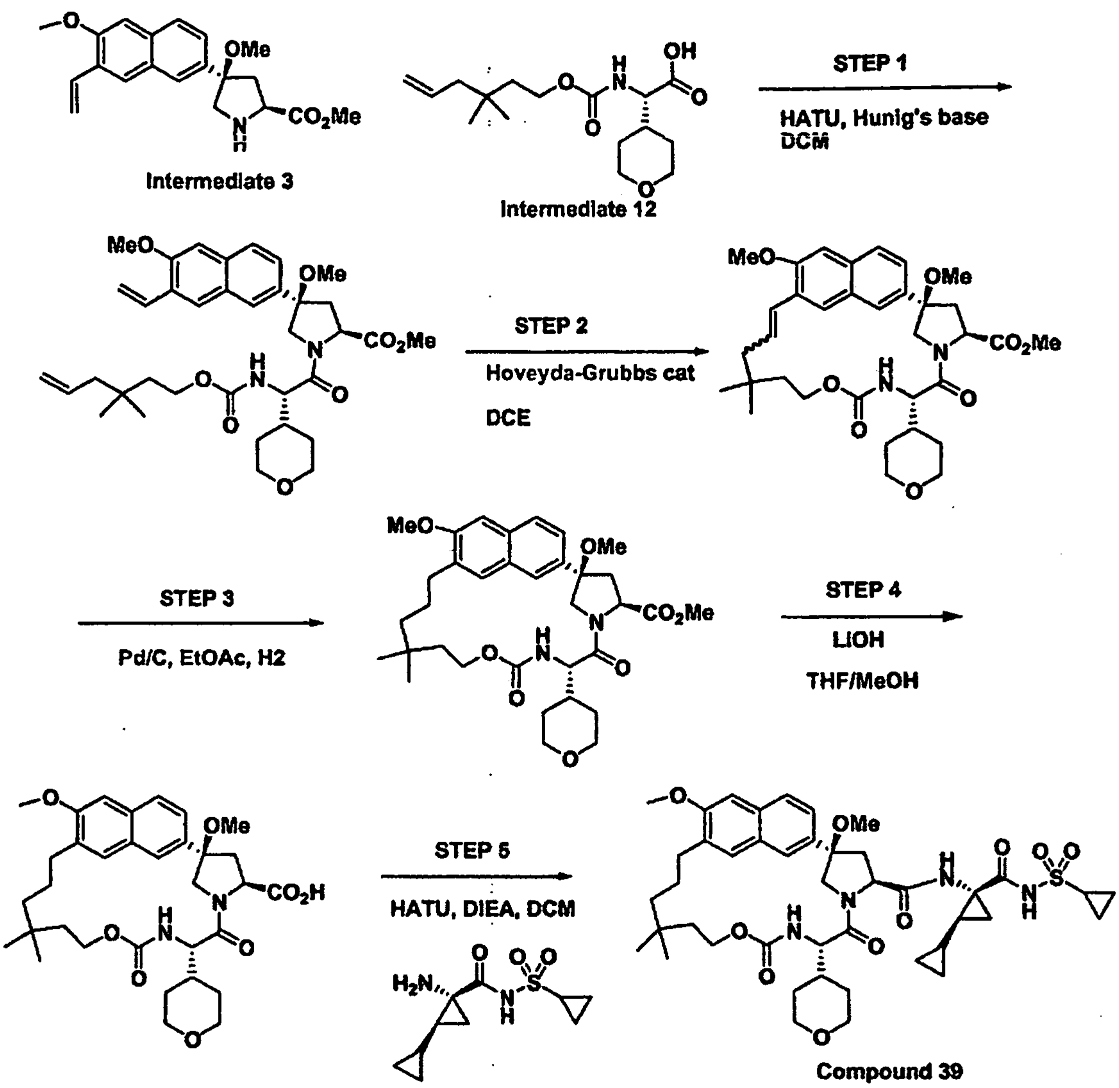


Intermediate 3

Intermediate 12

Compound 39

*Step 1:*

HATU (339 mg, 0.892 mmol) was added to a solution of (2S,4R)-methyl 4-methoxy-4-(6-methoxy-7-vinylnaphthalen-2-yl)pyrrolidine-2-carboxylate (Intermediate 3,279 mg, 0.818 mmol), (S)-2-((3,3-dimethylhex-5-enyloxy)carbonylamino)-2-(tetrahydro-2H-pyran-4-yl)acetic acid (Intermediate 12, 233 mg, 0.743 mmol) and Hunig's base (0.39 mL, 2.23 mmol) in DCM (8 mL) and stirred at r.t. overnight. The reaction was concentrated and purified by flash chromatography on the Biotage (20-80% EtOAc in hexanes) to give the product (200 mg, 0.314 mmol, 42% yield) as light yellow foam. LCMS: r.t. = 4.08 min., $[M+H]^+$ = 637, Phenomenex Luna C 18, 50 x 2, 3u; Solvent A = 10% acetonitrile - 90% water - 0.1% TFA, Solvent B = 90% acetonitrile - 10% water - 0.1 % TFA; gradient = 0% to 100% solvent B over 4 min. and then hold for 1 min.; 0.8 mL/min; inj. vol. = 3 ul; wavelength = 220 nm. $^1$H NMR (500 MHz, CHLOROFORM-*d*) d ppm 0.88 (s, 6 H) 0.90 - 0.94 (m, 2 H) 1.51 (t, *J*=7.63 Hz, 3 H) 1.75 (d, *J*=13.12 Hz, 1 H) 1.95 (d, *J*=7.32 Hz, 2 H) 2.60 (dd, *J*=13.12, 8.85 Hz, 1 H) 2.92 (dd, *J*=13.28, 1.98 Hz, 1 H) 2.95 (s, 3 H) 3.40 - 3.47 (m, 2 H) 3.76 (s, 3 H) 3.98 (s, 3 H) 4.02 - 4.08 (m, 3 H) 4.10 - 4.16 (m, 4H) 4.38 (dd, *J*=8.85, 7.63 Hz, 1 H) 4.87 (dd, *J*=8.55, 2.44 Hz, 1 H) 4.98 - 5.06 (m, 2 H) 5.30 - 5.34 (m, 1 H) 5.40 (dd, *J*=11.14, 1.37 Hz, 1 H) 5.72 - 5.83 (m, 1 H) 5.92 (dd, *J*=17.70, 1.53 Hz, 1 H) 7.11 (s, 1 H) 7.12 - 7.19 (m, 1 H) 7.41 (dd, *J*=8.55, 1.83 Hz, 1 H) 7.69 (s, 1 H) 7.75 (d, *J*=8.55 Hz, 1 H) 7.92 (s, 1 H).

*Step 2:*

A solution of (2S,4R)-methyl 1-((S)-2-((3,3-dimethylhex-5-enyloxy)carbonylamino)-2-(tetrahydro-2H-pyran-4-yl)acetyl)-4-methoxy-4-(6-methoxy-7-vinylnaphthalen-2-yl)pyrrolidine-2-carboxylate (200 mg, 0.314 mmol) in DCE (100 mL) was sparged with nitrogen for 30 min. and then Hoveyda-Grubbs Catalyst (2nd generation) (19.74 mg, 0.031 mmol) was added and the reaction sealed and heated to 80°C for 3 days. The reaction was concentrated to give crude material. The crude material was purified by flash chromatography on the Biotage (30-70% EtOAc in hexanes) to give the purified product (155 mg, 0.255 mmol, 81% yield) as a white solid. LCMS: r.t. = 3.92 min., $[M+H]^+$ = 609, Phenomenex Luna C18, 50 x 2, 3u; Solvent A = 10% acetonitrile - 90% water - 0.1% TFA, Solvent B = 90% acetonitrile - 10% water - 0.1% TFA; gradient = 0% to 100% solvent B over 4 min. and then hold for 1 min.; 0.8 mL/min; inj. vol. = 3 ul; wavelength = 220 nm. $^1$H NMR (500 MHz, CHLOROFORM-*d*) d ppm 1.00 (d, *J*=3.05 Hz, 6 H) 1.47 - 1.57 (m, 1 H) 1.62 - 1.76 (m, 3 H) 1.77 - 1.83 (m, 1 H) 1.93 (d, *J*=12.51 Hz, 1 H) 2.01 (ddd, *J*=11.98, 8.01, 4.43 Hz, 1 H) 2.14 - 2.24 (m, 2 H) 2.38 - 2.44 (m, 1 H) 2.56 - 2.61 (m, 1 H) 3.13 (s, 3 H) 3.39 - 3.48 (m, 2 H) 3.69 - 3.72 (m, 4 H) 3.82 (d, *J*=10.38 Hz, 1 H) 3.96 (s, 3 H) 3.96 - 4.00 (m, 1 H) 4.05 - 4.10 (m, 3 H) 4.71 (td, *J*=11.29, 4.27 Hz, 1 H) 4.77 (dd, *J*=9.46, 7.93 Hz, 1 H) 4.85 (d, *J*=10.07 Hz, 1 H) 5.40 (d, *J*=9.46 Hz, 1 H) 6.51 (ddd, *J*=15.79, 8.24, 8.01, 1 H) 6.75 (d, *J*=15.87 Hz, 1 H) 7.04 (s, 1 H) 7.48 (dd, *J*=8.70, 1.98 Hz, 1 H) 7.61 (d, *J*=1.83 Hz, 1 H) 7.74 (d, *J*=8.55 Hz, 1 H) 7.97 (s, 1 H).

*Step 3:*

A solution of the product from Step 2 (150 mg, 0.246 mmol) in ethyl acetate (3 mL) was stirred under an atmosphere of hydrogen for overnight. The reaction was filtered through a nylon frit and concentrated to give crude product as white solid (122 mg, 0.20 mmol, 81% yield). LCMS: r.t. = 4.04 min., $[M+H]^+$ = 611, Phenomenex Luna C18, 50 x 2, 3u; Solvent A = 10% acetonitrile - 90% water - 0.1% TFA, Solvent B = 90% acetonitrile - 10% water - 0.1% TFA; gradient = 0% to 100% solvent B over 4 min. and then hold for 1 min.; 0.8 mL/min; inj. vol. = 3 ul; wavelength = 220 nm. $^1$H NMR (400 MHz, CHLOROFORM-*d*) d ppm 0.90 (s, 3 H) 0.97 (s, 3 H) 1.46 -1.55 (m, 3 H) 1.64 -1.79 (m, 2 H) 1.82 -1.89 (m, 3 H) 1.91 - 1.96 (m, I H) 1.99 - 2.03 (m, 1 H) 2.45 (t, *J*=11.67 Hz, 1 H) 2.55 - 2.61 (m, 1 H) 2.73 - 2.90 (m, 2 H) 3.14 (s, 3 H) 3.38 - 3.46 (m, 2 H) 3.71 (s, 3 H) 3.87 - 3.91 (m, 1 H) 3.94 (s, 3 H) 3.98 - 4.02 (m, 1 H) 4.04 - 4.07 (m, 2 H) 4.32 (td, *J*=10.92, 5.02 Hz, 2 H) 4.28 - 4.36 (m, 1 H) 4.49 (t, *J*=9.79 Hz, 1 H) 5.08 (d, *J*=10.04 Hz, 1 H) 5.22 (d, *J*=9.79 Hz, 1 H) 7.09 (s, 1 H) 7.52 (dd, *J*=8.66, 1.88 Hz, 1 H) 7.63 - 7.65 (m, 2 H) 7.77 (d, *J*=8.78 Hz, 1 H).

*Step 4:*

2.0 M lithium hydroxide (0.499 mL, 0.999 mmol) was added to a solution of the product from Step 3 (122 mg, 0.200 mmol) in THF (1 mL) and MeOH (1 mL) and stirred at r.t. for 3 hour. The reaction was quenched with 1.0M HCl solution and extracted with ether. The organics were dried, filtered and concentrated to give crude product (119 mg, 0.199 mmol. 100% yield) as white foam. LCMS: r.t. = 3.67 min., $[M+H]^+$ = 597, Phenomenex Luna C18, 50 x 2, 3u; Solvent A =10% acetonitrile - 90% water - 0.1% TFA, Solvent B = 90% acetonitrile - 10% water - 0.1% TFA; gradient = 0% to 100% solvent B over 4 min. and then hold for 1 min.; 0.8 mL/min; inj. vol. = 3 ul; wavelength = 220 nm. $^1$H NMR (500 MHz, CHLOROFORM-*d*) d ppm 0.88 (s, 3 H) 0.95 (s, 3 H) 1.17 - 1.24 (m, 1 H) 1.43 - 1.55 (m, 3 H) 1.62 - 1.76 (m, 2 H) 1.76 - 1.91 (m, 4 H) 1.98 - 2.05 (m, 1 H) 2.54 (t, *J*=11.75 Hz, 1 H) 2.58 - 2.64 (m, 1 H) 2.72 - 2.80 (m, 1 H) 2.81 - 2.89 (m, 1 H) 3.12 (s, 3 H) 3.34 - 3.42 (m, 2 H) 3.89 (d, *J*=10.07 Hz, 1 H) 3.95 (s, 3 H) 3.96 - 4.06 (m, 3 H) 4.06 - 4.10 (m, 1 H) 4.30 (td, *J*=10.99, 4.58 Hz, 1 H) 4.52 (t, *J*=9.77 Hz, 1 H) 5.14 (d, *J*=9.77 Hz, 1 H) 5.98 (d, *J*=9.16 Hz, 1 H) 7.08

(s, 1 H) 7.52 (dd, *J*=8.70, 1.68 Hz, 1 H) 7.66 (d, *J*=7.93 Hz, 2 H) 7.77 (d, *J*=8.55 Hz, 1 H).

*Step 5:*

HATU (114 mg, 0.299 mmol) was added to a solution of (1S,2R)-2-amino-N-(cyclopropylsulfonyl)bi(cyclopropane)-2-carboxamide, HCl salt (67.2 mg, 0.239 mmol), the product from Step 4 (119 mg, 0.199 mmol) and Hunig's base (0.10 mL, 0.59 mmol) in dichloromethane (5 mL) and stirred at r.t. overnight. The reaction was concentrated and purified by HPLC (Xbridge C18 10u (30 x100 mm); flow = 42 mL/min; solvent gradient = 95:5 to 5:95 water/acetonitrile (with 10mM ammonium acetate)). The product fractions were concentrated to give Compound 39 (102.1 mg, 0.123 mmol, 62%) as white solid. LCMS: r.t. = 4.13 min., $[M+Na]^+$ = 845, Phenomenex Luna C18, 50 x 2, 3u; Solvent A = 10% acetonitrile - 90% water - 0.1% TFA, Solvent B = 90% acetonitrile - 10% water - 0.1% TFA; gradient = 0% to 100% solvent B over 4 min. and then hold for 1 min.; 0.8 mL/min; inj. vol. = 3 ul; wavelength = 220 nm. $^1$H NMR (500 MHz, MeOD) d ppm 0.20 (dt, *J*=9.46, 4.73 Hz, 1 H) 0.27 (dt, *J*=9.46, 4.73 Hz, 1 H) 0.42 - 0.48 (m, 1 H) 0.52 - 0.58 (m, 1 H) 0.78 - 0.85 (m, 1 H) 0.91 (s, 3 H) 0.95 - 0.99 (m, 1 H) 1.00 (s, 3 H) 1.07 - 1.11 (m, 2 H) 1.12 - 1.16 (m, 1 H) 1.21 - 1.28 (m, 3 H) 1.36 - 1.56 (m, 3 H) 1.67 - 1.91 (m, 7 H) 2.14 - 2.23 (m, 1 H) 2.31 - 2.36 (m, 1 H) 2.44 (t, *J*=11.60 Hz, 1 H) 2.81 (d, *J*=4.27 Hz, 2 H) 2.96 - 3.02 (m, 1 H) 3.15 (s, 3 H) 3.39 - 3.47 (m, 2 H) 3.84 (dd, *J*=11.29, 6.41 Hz, 1 H) 3.91 - 3.93 (m, 1 H) 3.94 (s, 3 H) 3.96 - 4.04 (m, 3 H) 4.34 - 4.40 (m, 1 H) 4.42 (d,*J*=10.38 Hz, 1 H) 5.26 (d,*J*=10.38 Hz, 1 H) 7.21 (s, 1 H) 7.53 (dd, *J=8.55,* 1.83 Hz, 1 H) 7.65 (s, 1 H) 7.78 (s, 1 H) 7.82 (d, *J*=8.85 Hz, 1 H).

*Preparation of intermediate 13:*

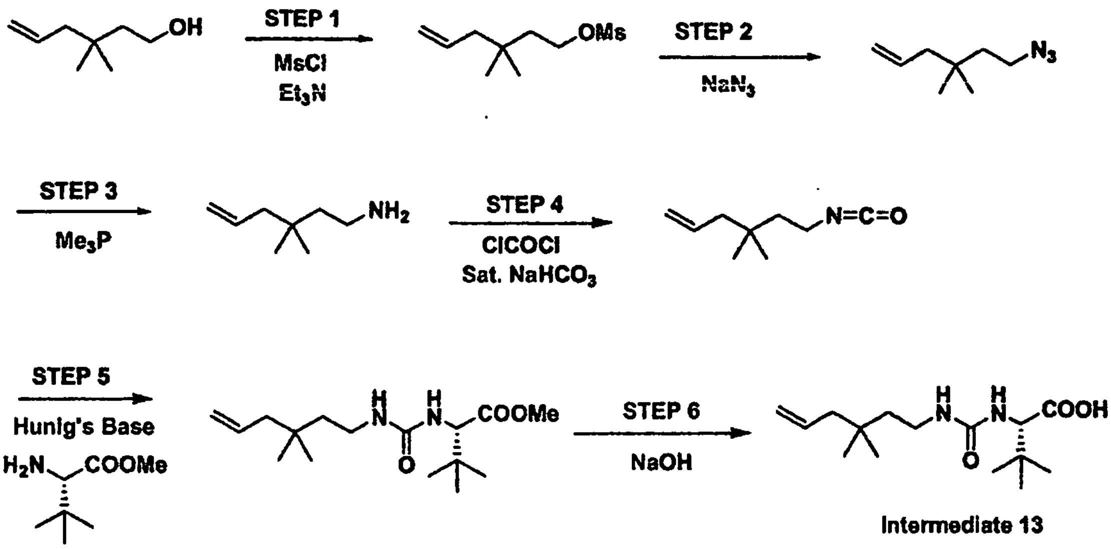


Intermediate 13

*Step 1:*

Methanesulfonyl chloride (2.67 mL, 34.3 mmol) was added to a solution of 3,3-dimethylhex-5-en-1-ol (2 g, 15.60 mmol) and triethylamine (6.5 mL, 46.8 mmol) in DCM (60 mL) at 0°C and stirred for 30 min. The reaction was quenched with water. The organic layer was dried over $MgSO_4$, filtered, and concentrated to give crude product. The crude residue was purified by flash chromatography on the Biotage (5-20% EtOAc in hexanes) to give the purified product (3.22 g 15.61 mmol, 100% yield) as colorless oil. $^1$H NMR (500 MHz, CHLOROFORM-*d*) $\delta$ ppm 0.95 (s, 6 H) 1.67 - 1.73 (m, 2 H) 2.00 (d, *J*=7.32 Hz, 2 H) 3.01 (s, 3 H) 4.27 - 4.32 (m, 2 H) 5.01 - 5.10 (m, 2H) 5.75 - 5.85 (m, 1H).

*Step 2:*

Sodium azide (6.09 g, 94 mmol) was added to a solution of 3,3-dimethylhex-5-enyl methanesulfonate (3.22 g, 15.61 mmol) in dry DMF (50 mL) at room temperature and stirred for 24 hours. Water was added and the mixture was extracted with $Et_2O$ three times, the combined organic layers were washed with brine and dried over $MgSO_4$ and concentrated. The residue was purified by flash chromatography on the Biotage (1-5% EtOAc in hexanes) to give the purified product (1.567 g, 10.23 mmol. 65% yield) as colorless oil. $^1$H NMR (400 MHz, CHLOROFORM-*d*) $\delta$ ppm 0.94

(s, 6 H) 1.51 - 1.56 (m, 2 H) 1.95 - 2.02 (m, 2 H) 3.23 - 3.31 (m, 2 H) 5.00 - 5.10 (m, 2 H) 5.73 - 5.87 (m, I H).

*Step 3:*

Trimethylphosphine (7.78 g, 102 mmol) was added to a solution of 6-azido-4,4-dimethylhex-1-ene (1.567 g, 10.23 mmol) in THF (100 mL) at 0 °C. The reaction was warmed to room temperature and stirred overnight. 15 g of SCX resin was added to the reaction mixture, filtered and washed with MeOH. The product amine was then eluted with 2.0 M $NH_3$ in MeOH and removal of solvent gave 3,3-dimethylhex-5-en-1-amine (0.69 g, 5.42 mmol, 53% yield) as colorless oil. $^1$H NMR (500 MHz, CHLOROFORM-*d*) δ ppm 0.79 - 0.94 (m, 6 H) 1.31 - 1.42 (m, 2 H) 1.47 - 1.64 (m, 2 H) 1.90 - 2.02 (m, 2 H) 2.61 - 2.77 (m, 2 H) 4.94 - 5.08 (m, 2 H) 5.72 - 5.89 (m, 1 H).

*Step 4:*

Sat. $NaHCO_3$ (7 mL, 5.42 mmol) was added to a solution of 3,3-dimethylhex-5-en-1-amine (0.69 g, 5.42 mmol) in DCM (10 mL). 2.0 M phosgene in toluene (6.78 mL, 13.56 mmol) was added into the bottom DCM layer with syringe. The resulting mixture was stirred for 10 min at room temperature. The DCM layer was separated. The aqueous layer was extracted with DCM. The combined organic layers were dried over $MgSO_4$, filtered and concentrated to give crude 6-isocyanato-4,4-dimethylhex-1-ene (0.83 g, 5.42 mmol, 100% yield).

*Step 5:*

6-isocyanato-4,4-dimethylhex-1-ene (0.83 g, 5.42 mmol) was added to a solution of (S)-methyl 2-amino-3,3-dimethylbutanoate (0.787 g, 5.42 mmol) in THF (50 mL), and stirred overnight at room temperature. After removal of solvent, the residue was purified by flash chromatography on the Biotage (20-40% EtOAc in Hexanes) to give (S)-methyl 2-(3-(3,3-dimethylhex-5-enyl)ureido)-3,3-dimethylbutanoate (0.645 g, 2.19 mmol, 40 % yield) as colorless oil. LCMS: r.t. = 3.25 min., $[M+H]^+$ = 299 Phenomenex Luna C18, 50 x 2, 3u; Solvent A = 10% acetonitrile - 90% water - 0.1% TFA, Solvent B = 90% acetonitrile - 10% water - 0.1% TFA; gradient = 0% to 100% solvent B over 4 min. and then hold for 1 min.; 0.8 mL/min; inj. vol. = 3 ul; wavelength = 220 nm. $^1$H NMR (500 MHz, CHLOROFORM-*d*) δ ppm 0.91 (s, 6 H) 0.97 (s, 9 H) 1.37 - 1.44 (m, 2 H) 1.62 (s, 2 H) 1.97 (d, *J*=7.32 Hz, 2 H) 3.15 - 3.23 (m, 2 H) 3.72 (d, *J*=1.22 Hz, 3 H) 4.32 (d, *J*=8.55 Hz, 1 H) 4.89 (d, *J*=9.46 Hz, 1 H) 4.98 - 5.08 (m, 2 H) 5.75 - 5.87 (m, 1 H).

*Step 6:*

3M sodium hydroxide (1.25 mL, 3.75 mmol) was added to a solution of (S)-methyl 2-(3-(3,3-dimethylhex-5-enyl)ureido)-3,3-dimethylbutanoate (0.2 g, 0.67 mmol) in EtOH (8 mL) and heated to 60 °C for 48 hours. The reaction was quenched with 1.0M HCl solution and extracted with ether. The organics were dried, filtered and concentrated to give (S)-2-(3-(3,3-dimethylhex-5-enyl)ureido)-3,3-dimethylbutanoic acid (Intermediate 13, 0.128 g, 0.45 mmol, 60% yield) as colorless oil. LCMS: r.t. = 2.79 min., $[M+H]^+$ = 285 Phenomenex Luna C18, 50 x 2, 3u; Solvent A =10% acetonitrile - 90% water - 0.1% TFA, Solvent B = 90% acetonitrile - 10% water - 0.1% TFA; gradient = 0% to 100% solvent B over 4 min. and then hold for 1 min.; 0.8 mL/min; inj. vol. = 3 ul; wavelength = 220 nm. $^1$H NMR (500 MHz, CHLOROFORM-*d*) δ ppm 0.86 - 0.96 (m, 6 H) 1.00 - 1.07 (m, 9 H) 1.13 (br s, 1 H) 1.20 - 1.25 (m, 1 H) 1.29 (s, 1 H) 1.39 - 1.51 (m, 2 H) 1.93 - 2.02 (m, 2 H) 3.14 (br s, 1 H) 3.44 - 3.54 (m, 1 H) 3.68 (s, 1 H) 4.97 - 5.09 (m, 2 H) 5.75 - 5.88 (m, 1 H) 6.50 (s, 1H).

*Preparation of Compound 40*

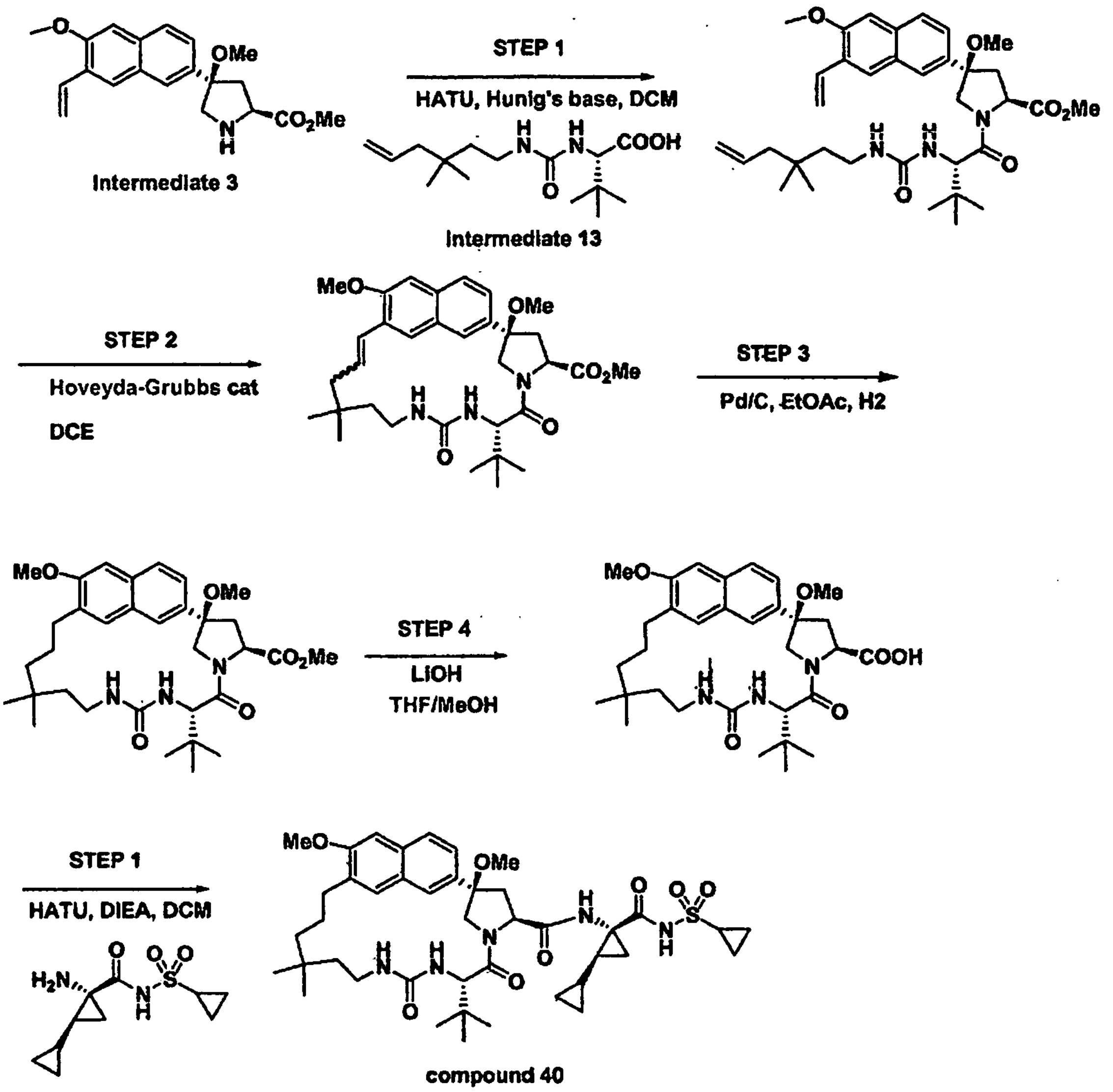


Intermediate 3

Intermediate 13

compound 40

*Step 1:*

HATU (107 mg, 0.281 mmol) was added to a solution of (2S,4R)-methyl 4-methoxy-4-(6-methoxy-7-vinylnaphthalen-2-yl)pyrrolidine-2-carboxylate (Intermediate 3, 80 mg, 0.234 mmol), (S)-2-(3-(3,3-dimethylhex-5-enyl)ureido)-3,3-dimethylbutanoic acid (Intermediate 13, 66.6 mg, 0.234 mmol) and Hunig's base (0.123 mL, 0.703 mmol) in DCM (4 mL) and stirred at r.t. overnight. The reaction was concentrated and purified by flash chromatography on the Biotage (20-50% EtOAc in hexanes) to give the product (43 mg, 0.071 mmol, 30% yield) as white foam. LCMS: r.t. = 4.22 min., $[M+Na]^+$ = 630 Phenomenex Luna C18, 50 x 2, 3u; Solvent A = 10% acetonitrile - 90% water - 0.1% TFA, Solvent B = 90% acetonitrile- 10% water - 0.1% TFA; gradient = 0% to 100% solvent B over 4 min. and then hold for 1 min.; 0.8 mL/min; inj. vol. = 3 ul; wavelength = 220 nm. $^1$H NMR (500 MHz, CHLOROFORM-*d*) $\delta$ ppm 0.85 - 0.98 (m, 10 H) 0.99 - 1.17 (m, 6 H) 1.32 - 1.46 (m, 2 H) 1.91 - 2.01 (m, 2 H) 2.51 - 2.65 (m, 1 H) 2.85 - 2.96 (m, 4 H) 3.04 - 3.13 (m, 1 H) 3.15 - 3.27 (m, 1 H) 3.72 - 3.82 (m, 2 H) 3.92 - 4.00 (m, 4 H) 4.16 - 4.35 (m, 1 H) 4.48 - 4.62 (m, 2 H) 4.79 (ddd, *J*=15.49, 8.77, 2.59 Hz, 1 H) 4.95 - 5.06 (m, 2 H) 5.30 (t, *J*=9.92 Hz, 1 H) 5.36 - 5.44 (m, 1 H) 5.73 - 5.86 (m, 1 H) 5.88 - 5.96 (m, 1 H) 7.06 - 7.20 (m, 2 H) 7.38 - 7.45 (m, 1 H) 7.62 - 7.77 (m, 2 H) 7.86 - 7.94 (m, 1 H).

*Step 2:*

A solution of the product from Step 1 (43 mg, 0.071 mmol) in DCB (40 mL) was sparged with nitrogen for 30 min. and then Hoveyda-Grubbs Catalyst (2nd generation) (4.5 mg, 7.07 $\mu$mol) was added and the reaction sealed and heated to 80°C for overnight. The reaction was concentrated to give crude material. The crude material was purified by flash chromatography on the Biotage (20-50% EtOAc in hexanes) to give the purified product (41 mg, 0,071 mmol, 100%

yield) as white solid. LCMS: r.t. = 4.01 min., $[M+H]^+$ = 580 Phenomenex Luna C18, 50 x 2, 3u; Solvent A = 10% acetonitrile - 90% water - 0.1% TFA, Solvent B = 90% acetonitrile - 10% water - 0.1% TFA; gradient = 0% to 100% solvent B over 4 min. and then hold for 1 min.; 0.8 mL/min; inj. vol. = 3 ul; wavelength = 220 nm. $^1$H NMR (500 MHz, CHLOROFORM-*d*) δ ppm 0.82 - 0.93 (m, 9 H) 1.01 - 1.08 (m, 6 H) 1.32 - 1.37 (m, 2 H) 1.88 - 1.97 (m, 1 H) 2.09 - 2.17 (m, 1 H) 2.55 - 2.62 (m, 1 H) 3.04 - 3.09 (m, 3 H) 3.69 - 3.74 (m, 3 H) 3.76 - 3.85 (m, 2 H) 3.90 - 4.00 (m, 4 H) 4.05 (dd, *J*=10.99, 7.32 Hz, 1 H) 4.83 - 4.89 (m, 1 H) 4.90 - 5.02 (m, 2 H) 5.60 (d, *J*=9.77 Hz, 1 H) 6.44 - 6.53 (m, 1 H) 6.63 - 6.69 (m, 1 H) 6.67 (d, *J*=15.87 Hz, 1 H) 7.01 - 7.06 (m, 1 H) 7.43 - 7.48 (m, 1 H) 7.69 - 7.74 (m, 1 H) 7.75 - 7.80 (m, 1 H) 8.18 (s, 1 H).

*Step 3:*

A solution of the product from Step 2 (41 mg, 0.071 mmol) and 10% Pd/C (7.5 mg, 7.07 μmol) in EtOAc (2 mL) was stirred under an atmosphere of hydrogen overnight. The reaction was filtered through a nylon frit and concentrated to give crude product (35 mg, 0,060 mmol, 85% yield) as white solid. LCMS: r.t. = 2.14 min., $[M+H]^+$ = 582 Phenomenex Luna S10 (3 x50 mm); Solvent A = 95% water - 5% methanol-10mM ammonium acetate, Solvent B = 5% water - 95% methanol -10 mM ammonium acetate; gradient = 0% to 100% solvent B over 2 min. and then hold for 1 min.; 4 mL/min; inj. vol. = 10 ul; wavelength = 220 nm.

*Step 4:*

2.0 M lithium hydroxide (0.150 mL, 0.301 mmol) was added to a solution of the product from Step 3 (35 mg, 0.060 mmol) in THF (1 mL) and MeOH (1 mL) and stirred at room temperature for 3 hours. The reaction was quenched with 1.0M HCl solution and extracted with ether. The organics were dried, filtered and concentrated to give crude product (34 mg, 0.060mmol, 100% yield) as a white foam. LCMS: r.t. = 2.23 min., $[M+H]^+$ = 568 Phenomenex Luna S 10 (3 x50 mm); Solvent A = 90% water - 10% methanol-0.1% TFA, Solvent B = 10% water - 90% methanol - 0.1% TFA; gradient = 0% to 100% solvent B over 2 min. and then hold for 1 min.; 4 mL/min; inj. vol. = 5 ul; wavelength = 220 nm.

*Step 5:*

HATU (34.2 mg, 0.090 mmol) was added to a solution of (1S,2R)-2-amino-N-(cyclopropylsulfonyl)bi(cyclopropane)-2-carboxamide, HCl salt (20.18 mg, 0.072 mmol), the product from Step 4 (34 mg, 0.060 mmol) and Hunig's base (0.031 mL, 0.180 mmol) in dichloromethane (2 mL) and stirred at r.t. overnight. The reaction was concentrated and purified by HPLC (Xbridge C18 10u (30 x100 mm); flow = 42 mL/min; solvent gradient = 95:5 to 5:95 water/acetonitrile (with 10mM ammonium acetate)). The product fractions were concentrated to give Compound 40 (10.3 mg, 0.011 mmol, 19% yield) as a white solid. LCMS: r.t. = 2.13 min., $[M-OMe]^+$ = 763 Phenomenex Luna S 10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. = 10 ul; wavelength = 220 nm. $^1$H NMR (500 MHz, MeOD) δ ppm 0.24 (dddd, *J*=15.11, 8.77, 4.65, 4.43 Hz, 2 H) 0.50 - 0.58 (m, 1 H) 0.74 - 0.84 (m, 1 H) 0.94 (d, *J*=8.55 Hz, 6 H) 0.98 - 1.03 (m, 1 H) 1.04 - 1.11 (m, 3 H) 1.13 (s, 9 H) 1.17(dd, *J*=9.77, 5.19 Hz, 1 H) 1.22 - 1.31 (m, 3 H) 1.46 - 1.62 (m, 3 H) 1.71 (dd, *J*=8.24, 5.19 Hz, 1 H) 1.78 (ddd, *J*=13.12, 8.55, 4.27 Hz, 1 H) 1.83 - 1.91 (m, 1 H) 2.38 (d, *J*=9.16 Hz, 2 H) 2.68 (ddd, *J*=16.79, 8.09, 5.04 Hz, 1 H) 2.87 - 2.99 (m, 2 H) 3.01 - 3.09 (m, 1 H) 3.13 (s, 3 H) 3.35 (s, 1 H) 3.47 (td, *J*=11.98, 5.34 Hz, 1 H) 3.80 (t, *J*=9.00 Hz, 1 H) 3.85 (d, *J*=10.38 Hz, 1 H) 3.93 (s, 3 H) 4.64 (d, *J*=9.46 Hz, 1 H) 5.20 (d, *J*=10.38 Hz, 1 H) 6.18 - 6.26 (m, 2 H) 7.19 (s, 1 H) 7.51 (dd, *J*=8.55, 1.83 Hz, 1 H) 7.78 - 7.84 (m, 2 H) 7.90 (s, 1 H).

*Preparation of intermediate 14:*

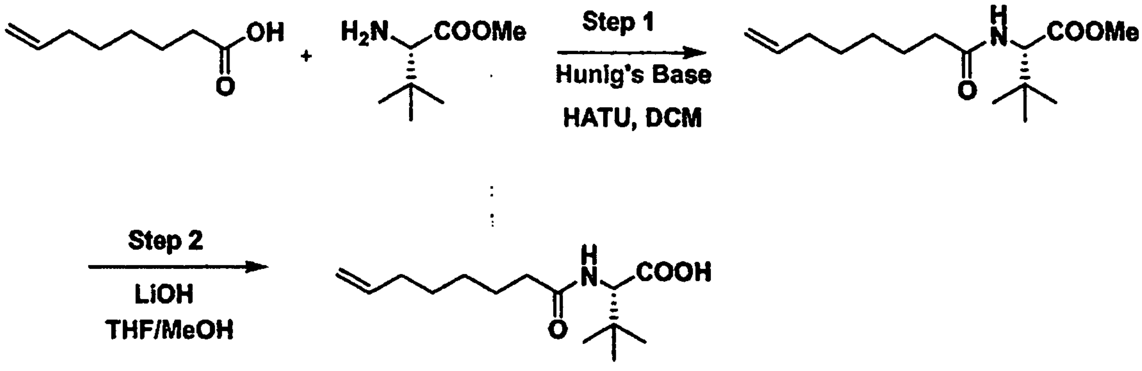


**Intermediate 14**

*Step 1:*

HATU (1.017 g, 4.22 mmol) was added to a solution of (S)-methyl 2-amino-3,3-dimethylbutanoate (0.511 g, 3.52 mmol), oct-7-enoic acid (0.5 g, 3.52 mmol) and Hunig's base (1.8 mL, 10.5 mmol) in DCM (50 mL) and stirred at room temperature overnight. After removal of solvent, the residue was purified by flash chromatography on the Biotage (20-40% EtOAc in Hexanes) to give (S)-methyl 3,3-dimethyl-2-oct-7-enamidobutanoate (763 mg, 2.83 mmol, 81% yield) as a colorless oil. $^1$H NMR (500 MHz, CHLOROFORM-*d*) $\delta$ ppm 0.98 (s, 9 H) 1.30 - 1.45 (m, 4 H) 1.63 - 1.70 (m, 2 H) 2.02 - 2.09 (m, 2 H) 2.24 (t, *J*=7.63 Hz, 2 H) 3.73 (s, 3 H) 4.50 (d, *J*=9.46 Hz, 1 H) 4.92 - 5.03 (m, 2 H) 5.74 - 5.85 (m, *J*=17.01, 10.22, 6.68, 6.68 Hz, 1 H) 5.95 (d, *J*=9.16 Hz, 1 H). LCMS: r.t. = 3.18 min., $[M+H]^+$ = 270 Phenomenex Luna C 18, 50 x 2, 3u; Solvent A =10% acetonitrile - 90% water - 0.1 % TFA, Solvent B = 90% acetonitrile - 10% water - 0.1% TFA; gradient = 0% to 100% solvent B over 4 min. and then hold for 1 min.; 0.8 mL/min; inj. vol. = 3 ul; wavelength = 220 nm.

*Step 2:*

2.0 M lithium hydroxide (7.1 mL, 14.2 mmol) was added to a solution of (S)-methyl 3,3-dimethyl-2-oct-7-enamidobutanoate (0.763 g, 2.83 mmol) in THF (10 mL) and MeOH (10 mL), and stirred at r.t for 5 hours. The reaction was quenched with 1.0 M HCl solution and extracted with ether. The organics were dried, filtered and concentrated to give (S)-3,3-dimethyl-2-oct-7-enamidobutanoic acid (620 mg, 2.43 mmol, 86% yield) as a white solid. $^1$H NMR (400 MHz, CHLOROFORM-*d*) $\delta$ ppm 1.04 (s, 9 H) 1.32 - 1.46 (m, 4 H) 1.67 (dt, *J*=14.87, 7.50 Hz, 2 H) 2.06 (q, *J*=6.78 Hz, 2 H) 2.22 - 2.31 (m, 2 H) 4.52 (d, *J*=9.29 Hz, 1 H) 4.91 - 5.05 (m, 2 H) 5.73 - 5.86 (m, *J*=17.03, 10.26, 6.68, 6.68 Hz, 1 H) 5.97 (d, *J*=9.03 Hz, 1 H). LCMS: r.t. = 1.49 min., $[M+H]^+$ = 256 Phenomenex Luna S10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. = 10 ul; wavelength = 220 nm.

*Preparation of Compound 41*

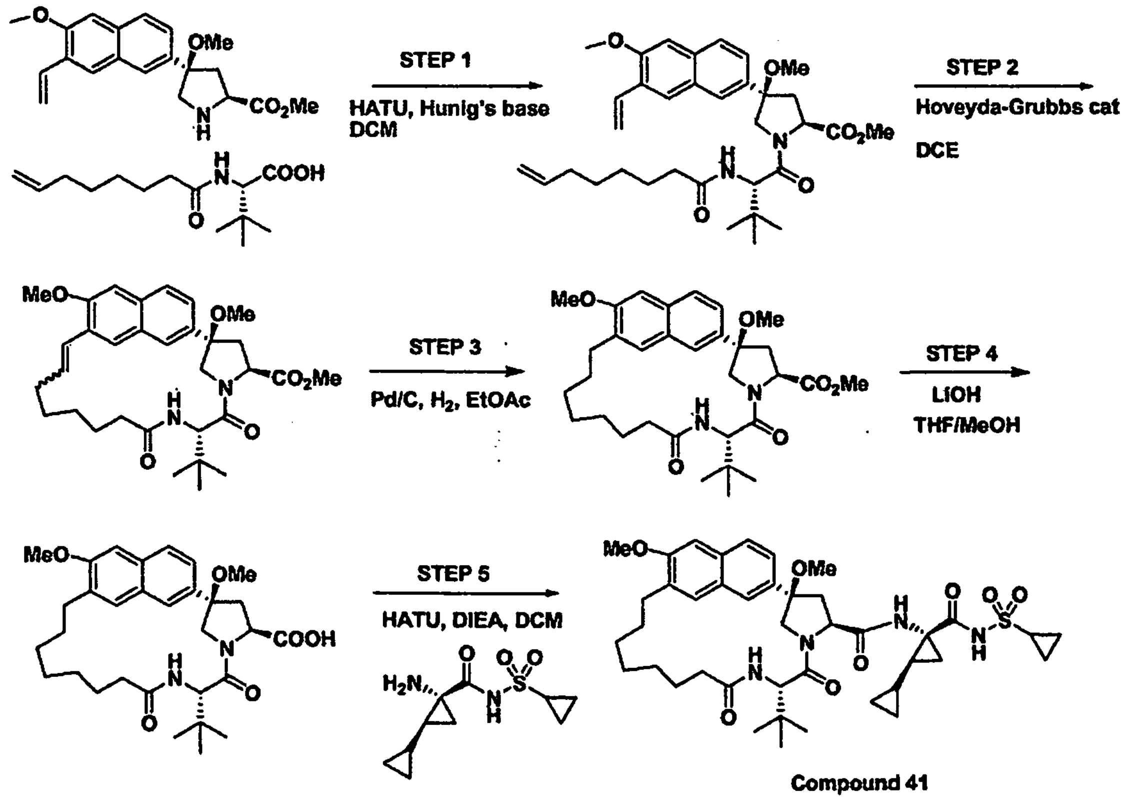


Compound 41

*Step 1:*

HATU (134 mg, 0.351 mmol) was added to a solution of (2S,4R)-methyl 4-methoxy-4-(6-methoxy-7-vinylnaphthalen-2-yl)pyrrolidine-2-carboxylate (Intermediate 3, 100 mg, 0.293 mmol), (S)-3,3-dimethyl-2-oct-7-enamidobutanoic acid (Intermediate 14, 74.8 mg, 0.293 mmol) and Hunig's base (0.15 mL, 0.88 mmol) in DCM (4 mL) and stirred at room temperature overnight. The reaction was concentrated and purified by flash chromatography on the Biotage (20-40% EtOAc in hexanes) to give (2S,4R)-methyl 1-((S)-3,3-dimethyl-2-oct-7-enamidobutanoyl)-4-methoxy-4-(6-methoxy-7-vinylnaphthalen-2-yl)pyrrolidine-2-carboxylate (53 mg, 0.092 mmol, 31% yield) as a white foam. $^1H$ NMR (500 MHz, CHLOROFORM-*d*) δ ppm 0.95 (br. s., 6 H) 1.04 - 1.16 (m, 3 H) 1.19 - 1.47 (m, 6 H) 1.55 - 1.66 (m., 2 H) 1.95 - 2.05 (m., 2 H) 2.07 - 2.25 (m., 2 H) 2.51 - 2.68 (m, 1 H) 2.93 (s, 3 H) 2.82 - 2.95 (m, 1 H) 3.70 - 3.79 (m, 3 H) 3.88 - 3.95 (m, 1 H) 3.97 (br. s., 3 H) 4.47 - 4.52 (m., 1 H) 4.75 - 4.85 (m, 1 H) 4.93 - 5.05 (m, 1 H) 5.38 - 5.42 (m, I H) 5.75 - 5.85 (m, 1 H) 5.92 (d, *J*=17.40 Hz, 1 H) 6.10 - 6.18 (m, 1 H) 7.05 - 7.19 (m, 2 H) 7.35 - 7.48 (m, 1 H) 7.59 - 7.78 (m, 2 H) 7.84 - 7.97 (m, I H). LCMS: r.t. = 4.24 min., $[M+H]^+$ = 579 Phenomenex Luna C18, 50 x 2, 3u; Solvent A = 10% acetonitrile - 90% water - 0.1% TFA, Solvent B = 90% acetonitrile - 10% water - 0.1% TFA; gradient = 0% to 100% solvent B over 4 min. and then hold for 1 min.; 0.8 mL/min; inj. vol. = 3 ul; wavelength = 220 nm.

*Step 2:*

A solution of (2S,4R)-methyl 1-((S)-3,3-dimethyl-2-oct-7-enamidobutanoyl)-4-methoxy-4-(6-methoxy-7-vinylnaphthalen-2-yl)pyrrolidine-2-carboxylate (53 mg, 0.092 mmol) in DCE (40 mL) was sparged with nitrogen for 30 min. and then Hoveyda-Grubbs Catalyst 2nd Generation (5.7 mg, 9.16 μmol) was added and the reaction sealed and heated to 80°C for overnight. The reaction was concentrated to give crude material. The crude material was purified by flash chromatography on the Biotage (20-40% EtOAc in hexanes) to give the purified product (20 mg, 0.036 mmol, 40% yield) as a white solid. $^1H$ NMR (400 MHz, CHLOROFORM-*d*) δ ppm 0.89 - 0.97 (m, 6 H) 1.01 (s, 3 H) 1.11 - 1.15 (m, 6 H) 1.63 - 1.71 (m, 2 H) 2.06 - 2.12 (m, 1 H) 2.33 - 2.50 (m, 2 H) 2.52 - 2.66 (m, 1 H) 3.08 - 3.15 (m, 3 H) 3.24 (s, 1 H) 3.68 - 3.73 (m, 3 H) 3.77 - 3.85 (m, 1 H) 3.92 - 4.03 (m, 4 H) 4.42 - 4.59 (m, 1 H) 6.28 (ddd, *J*=15.37, 7.59, 7.40 Hz, 1 H) 6.72 (dd, *J*=15.81, 4.77 Hz, 1 H) 7.04 (d, *J*=9.29 Hz, 1 H) 7.45 - 7.55 (m, 1 H) 7.55 - 7.62 (m, 1 H) 7.64 - 7.71 (m, 1 H) 7.71 - 7.80 (m, 2 H). LCMS: r.t. = 4.03 min., $[M+H]^+$ = 551 Phenomenex Luna C18, 50 x 2, 3u; Solvent A =10% acetonitrile - 90% water - 0.1 % TFA, Solvent B = 90% acetonitrile - 10% water - 0.1% TFA; gradient = 0% to 100% solvent B over 4 min. and then hold for 1 min.; 0.8 mL/min; inj. vol. = 3 ul; wavelength = 220 nm.

*Step 3:*

A solution of the product from Step 2 (20 mg, 0.036 mmol) and 10% Pd/C (4 mg, 3.63 μmol) in EtOAc (2 mL) was stirred under an atmosphere of hydrogen overnight. The reaction was filtered through a nylon frit and concentrated to give crude product (17 mg, 0.031 mmol, 87% yield) as a white solid. LCMS: r.t. = 2.19 min., $[M+H]^+$ = 553 Phenomenex Luna S10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. = 10 ul; wavelength = 220 nm.

*Step 4:*

2.0 M lithium hydroxide (0.079 mL, 0.157 mmol) was added to a solution of the product from Step 3 (17 mg, 0.031 mmol) in MeOH (0.5 mL) and THF (0.5 mL) and stirred at r.t. for 3 hours. The reaction was quenched with 1.0 M HCl solution and extracted with ether. The organics were dried, filtered and concentrated to give crude product (16 mg, 0.03 mmol, 94% yield) as a white foam. LCMS: r.t. = 1.82 min., $[M-OMe]^+$ = 507 Phenomenex Luna S10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. =10 ul; wavelength = 220 nm.

*Step 5:*

HATU (16.94 mg, 0.045 mmol) was added to a solution of (1S,2R)-2-amino-N-(cyclopropylsulfonyl)bi(cyclopropane)-2-carboxamide, HCl salt (10 mg, 0.036 mmol), the product from Step 4 (16 mg, 0.030 mmol) and Hunig's base (0.016 mL, 0.089 mmol) in dichloromethane (1 mL) and stirred at r.t. overnight The reaction was concentrated and purified by HPLC (Xbridge C18 10u (30 x100 mm); flow = 42 mL/min; solvent gradient = 95:5 to 5:95 water/acetonitrile (with 10mM ammonium acetate)). The product fractions were concentrated to give Compound 41 (2.2 mg, 2.76 μmol, 9% yield) as a white solid. $^1$H NMR (500 MHz, MeOD) δ ppm 0.29 - 0.43 (m,2 H) 0.50 - 0.63 (m, 2 H) 0.85 (br. s., 1 H) 0.95 - 1.16 (m, 9 H) 0.98 -1.22 (m, 1 H) 1.22 - 1.47 (m, 9 H) 1.54 - 1.85 (m, 4 H) 1.90 - 2.05 (m, 2 H) 2.09 (td, *J*=13.28, 4.58 Hz, 1 H) 2.28 - 2.38 (m, 1 H) 2.48 - 2.60 (m, 1 H) 2.75 - 2.83 (m, 1 H) 2.95 - 3.14 (m, 4 H) 3.59 - 3.89 (m, 1 H) 3.91 - 3.95 (m, 1 H) 3.95 - 4.29 (m, 1 H) 4.56 - 4.61 (m, 2 H) 4.83 - 5.09 (m, 2 H) 7.21 (d, *J*=3.97 Hz, 1 H) 7.47 - 7.52 (m, 1 H) 7.53 - 7.59 (m, 1 H) 7.61 - 7.74 (m, 1 H) 7.81 (dd, *J*=13.43, 8.55 Hz, 1 H) 7.92 (d, *J*=9.16 Hz, I H).. LCMS: r.t. =1.97 min., $[M-OMe]^+$ = 733 Phenomenex Luna S 10 (3 x50 mm); Solvent A = 95% water - 5% methanol-10mM ammonium acetate, Solvent B = 5% water - 95% methanol - 10 mM ammonium acetate; gradient = 0% to 100% solvent B over 2 min. and then hold for 1 min.; 4 mL/min; inj. vol. =10 ul; wavelength = 220 nm.

*Biological Studies*

HCV NS3/4A protease complex enzyme assays and cell-based HCV replicon assays were utilized in the present disclosure, and were prepared, conducted and validated as follows:

*Generation of recombinant HCV NS3/4A protease complex*

HCV NS3 protease complexes, derived from the BMS strain, H77 strain or J4L6S strain, were generated, as described below. These purified recombinant proteins were generated for use in a homogeneous assay (see below) to provide an indication of how effective compounds of the present disclosure would be in inhibiting HCV NS3 proteolytic activity.

Serum from an HCV-infected patient was obtained from Dr. T. Wright, San Francisco Hospital. An engineered full-length cDNA (compliment deoxyribonucleic acid) template of the HCV genome (BMS strain) was constructed from DNA fragments obtained by reverse transcription-PCR (RT-PCR) of serum RNA (ribonucleic acid) and using primers selected on the basis of homology between other genotype la strains. From the determination of the entire genome sequence, a genotype 1a was assigned to the HCV isolate according to the classification of Simmonds et al. (See P Simmonds, KA Rose, S Graham, SW Chan, F McOmish, BC Dow, EA Follett, PL Yap and H Marsden, J. Clin. Microbiol., 31(6), 1493-1503 (1993)). The amino acid sequence of the nonstructural region, NS2-5B, was shown to be >97% identical to HCV genotype 1a (H77) and 87% identical to genotype 1b (J4L6S). The infectious clones, H77 (1a genotype) and J4L6S (1b genotype) were obtained from R. Purcell (NIH) and the sequences are published in Genbank (AAB67036; see Yanagi,M., Purcell,R.H., Emerson, S.U. and Bukh, J. Proc. Natl. Acad. Sci. U.S.A. 94(16), 8738-8743 (1997); AF054247, see Yanagi,M., St Claire,M., Shapiro,M., Emerson,S.U., Purcell,R.H. and Bukh, J., Virology 244 (1), 161-172. (1998)).

The H77 and J4L6S strains were used for production of recombinant NS3/4A protease complexes. DNA encoding the recombinant HCV NS3/4A protease complex (amino acids 1027 to 1711) for these strains was manipulated as described by P. Gallinari et al. (see Gallinari P, Paolini C, Brennan D, Nardi C, Steinkuhler C, De Francesco R. Biochemistry. 38(17):5620-32, (1999)). Briefly, a three-lysine solubilizing tail was added at the 3'-end of the NS4A coding region. The cysteine in the P1 position of the NS4A-NS4B cleavage site (amino acid 1711) was changed to a glycine to avoid the proteolytic cleavage of the lysine tag. Furthermore, a cysteine to serine mutation was introduced by PCR at amino acid position 1454 to prevent the autolytic cleavage in the NS3 helicase domain. The variant DNA fragment was cloned in the pET21b bacterial expression vector (Novagen) and the NS3/4A complex was expressed in Escherichia. coli strain BL21 (DE3) (Invitrogen) following the protocol described by P. Gallinari et al. (see Gallinari P, Brennan D, Nardi C, Brunetti M, Tomei L, Steinkuhler C, De Francesco R., J Virol. 72(8):6758-69 (1998)) with modifications. Briefly, the NS3/4A protease complex expression was induced with 0.5 millimolar (mM) Isopropyl β-D-1-thiogalactopyranoside (IPTG) for 22 hours (h) at 20°C. A typical fermentation (1 Liter (L)) yielded approximately 10 grams (g) of wet cell paste. The cells were resuspended in lysis buffer (10 mL/g) consisting of 25 mM *N*-(2-Hydroxyethyl)Piperazine-*N'*-(2-Ethane Sulfonic acid) (HEPES), pH 7.5, 20% glycerol, 500 mM Sodium Chloride (NaCl), 0.5% Triton X-100, 1 microgram/milliliter ("μg/mL") lysozyme, 5 mM Magnesium Chloride ($MgCl_2$), 1 μg/ml DnaseI, 5 mM β-Mercaptoethanol (βME), Protease inhibitor-Ethylenediamine Tetraacetic acid (EDTA) free (Roche), homogenized and incubated for 20 minutes (min) at 4°C. The homogenate was sonicated and clarified by ultra-centrifugation at 235000 g for 1 hour (h) at 4°C. Imidazole was added to the supernatant to a final concentration of 15 mM and the pH adjusted to 8.0. The crude protein extract was loaded on a Nickel-Nitrilotriacetic acid (Ni-NTA) column pre-equilibrated with buffer B (25 mM HEPES, pH 8.0, 20% glycerol, 500 mM NaCl, 0.5% Triton X-100, 15 mM imidazole, 5 mM βME). The sample was loaded at a flow rate of 1 mL/min. The column was washed with 15 column volumes of buffer C (same as buffer B except with 0.2% Triton X-100). The protein was eluted with 5 column volumes of buffer D (same as buffer C except with 200 mM Imidazole).

NS3/4A protease complex-containing fractions were pooled and loaded on a desalting column Superdex-S200 pre-equilibrated with buffer D (25mM HEPES, pH 7.5, 20% glycerol, 300 mM NaCl, 0.2% Triton X-100, 10 mM βME). Sample was loaded at a flow rate of 1 mL/min. NS3/4A protease complex-containing fractions were pooled and concentrated to approximately 0.5 mg/ml. The purity of the NS3/4A protease complexes, derived from the BMS, H77 and J4L6S strains, were judged to be greater than 90% by SDS-PAGE and mass spectrometry analyses. The enzyme was stored at -80 °C, thawed on ice and diluted prior to use in assay buffer.

*FRET peptide assay to monitor HCV NS3/4A proteolytic activty*

The purpose of this in vitro assay was to measure the inhibition of HCV NS3 protease complexes, derived from the BMS strain, H77 strain or J4L6S strain, as described above, by compounds of the present disclosure. This assay provides an indication of how effective compounds of the present disclosure would be in inhibiting HCV NS3 proteolytic activity.

In order to monitor HCV NS3/4A protease activity, an NS3/4A peptide substrate was used. The substrate was RET S 1 (Resonance Energy Transfer Depsipeptide Substrate; AnaSpec, Inc. cat # 22991)(FRET peptide), described by Taliani et al. in Anal. Biochem. 240(2):60-67 (1996). The sequence of this peptide is loosely based on the NS4A/NS4B natural cleavage site for the HCV NS3 protease except there is an ester linkage rather than an amide bond at the cleavage site. The peptide also contains a fluorescence donor, EDANS, near one end of the peptide and an acceptor, DABCYL, near the other end. The fluorescence of the peptide is quenched by intermolecular resonance energy transfer (RET) between the donor and the acceptor, but as the NS3 protease cleaves the peptide the products are released from RET quenching and the fluorescence of the donor becomes apparent.

The peptide substrate was incubated with one of the three recombinant NS3/4A protease complexes, in the absence or presence of a compound of the present disclosure. The inhibitory effects of a compound were determined by monitoring the formation of fluorescent reaction product in real time using a Cytofluor Series 4000.

The reagents were as follow: HEPES and Glycerol (Ultrapure) were obtained from GIBCO-BRL. Dimethyl Sulfoxide (DMSO) was obtained from Sigma. β-Mercaptoethanol was obtained from Bio Rad.

Assay buffer: 50 mM HEPES, pH 7.5; 0.15 M NaCl; 0.1% Triton; 15% Glycerol; 10 mM βME. Substrate: 2 μM final concentration (from a 2 mM stock solution in DMSO stored at -20°C). HCV NS3/4A protease type la (1b), 2-3 nM final concentration (from a 5 μM stock solution in 25 mM HEPES, pH 7.5, 20% glycerol, 300 mM NaCl, 0.2% Triton-X100, 10 mM βME). For compounds with potencies approaching the assay limit, the assay was made more sensitive by adding 50 μg/ml Bovine Serum Albumin (Sigma) to the assay buffer and reducing the end protease concentration to 300 pM.

The assay was performed in a 96-well polystyrene black plate from Falcon. Each well contained 25 μl NS3/4A protease complex in assay buffer, 50 μl of a compound of the present disclosure in 10% DMSO/assay buffer and 25 μl substrate in assay buffer. A control (no compound) was also prepared on the same assay plate. The enzyme complex was mixed with compound or control solution for 1 min before initiating the enzymatic reaction by the addition of substrate.

The assay plate was read immediately using the Cytofluor Series 4000 (Perspective Biosystems). The instrument was set to read an emission of 340 nm and excitation of 490 nm at 25°C. Reactions were generally followed for approximately 15 min.

The percent inhibition was calculated with the following equation:

$$100-[(\delta F_{inh}/\delta F_{con})x100]$$

where $\delta F$ is the change in fluorescence over the linear range of the curve. A non-linear curve fit was applied to the inhibition-concentration data, and the 50% effective concentration ($IC_{50}$) was calculated by the use of Excel XLfit software using the equation, y=A+((B-A)/(1+((C/x)^D))).

Compounds of the present disclosure, which were tested against more than one type of NS3/4A complex, were found to have similar inhibitory properties though the compounds uniformly demonstrated greater potency against the 1b strains as compared to the 1a strains.

*Specificity Assays*

The specificity assays were performed to demonstrate the in vitro selectivity of the compounds of the present disclosure in inhibiting HCV NS3/4A protease complex as compared to other serine or cysteine proteases.

The specificities of compounds of the present disclosure were determined against a variety of serine proteases: human neutrophil elastase (HNE), porcine pancreatic elastase (PPE) and human pancreatic chymotrypsin and one cysteine protease: human liver cathepsin B. In all cases a 96-well plate format protocol using a fluorometric Amino-Methyl-Coumarin (AMC) substrate specific for each enzyme was used as described previously (PCT Patent Application No. WO 00/09543) with some modifications to the serine protease assays. All enzymes were purchased from Sigma, EMDbiosciences while the substrates were from Bachem, Sigma and EMDbiosciences.

Compound concentrations varied from 100 to 0.4 μM depending on their potency. The enzyme assays were each initiated by addition of substrate to enzyme-inhibitor pre-incubated for 10 min at room temperature and hydrolysis to 15% conversion as measured on cytofluor.

The final conditions for each assay were as follows:

50 mM Tris(hydroxymethyl) aminomethane hydrochloride (Tris-HCl) pH 8, 0.5 M Sodium Sulfate ($Na_2SO_4$), 50 mM NaCl, 0.1 mM EDTA, 3% DMSO, 0.01% Tween-20 with 5 μM LLVY-AMC and 1 nM Chymotrypsin.

50 M Tris-HCl, pH 8.0, 50 mM NaCl, 0.1mM EDTA, 3% DMSO, 0.02% Tween-20, 5 μM succ-AAPV-AMC and 20 nM HNE or 8 nM PPE;

100 mM NaOAC (Sodium Acetate) pH 5.5, 3% DMSO, 1 mM TCEP (Tris(2-carboxyethyl)phosphine hydrochloride), 5 nM Cathepsin B (enzyme stock activated in buffer containing 20 mM TCEP before use), and 2 μM Z-FR-AMC diluted in $H_2O$.

The percentage of inhibition was calculated using the formula:

$$[1-((UV_{inh}-UV_{blank})/(UV_{ctl}-UV_{blank}))] \times 100$$

A non-linear curve fit was applied to the inhibition-concentration data, and the 50% effective concentration ($IC_{50}$) was calculated by the use of Excel XLfit software.

*Generation of HCV Replicon*

An HCV replicon whole cell system was established as described by Lohmann V, Korner F, Koch J, Herian U, Theilmann L, Bartenschlager R., Science 285(5424):110-3 (1999). This system enabled us to evaluate the effects of our HCV Protease compounds on HCV RNA replication. Briefly, using the HCV strain 1b sequence described in the Lohmann paper (Assession number:AJ238799), an HCV cDNA was synthesized by Operon Technologies, Inc. (Alameda, CA), and the full-length replicon was then assembled in plasmid pGem9zf(+) (Promega, Madison, WI) using standard molecular biology techniques. The replicon consists of (i) the HCV 5' UTR fused to the first 12 amino acids of the capsid protein, (ii) the neomycin phosphotransferase gene (neo), (iii) the IRES from encephalomyocarditis virus (EMCV), and

(iv) HCV NS3 to NS5B genes and the HCV 3'UTR. Plasmid DNAs were linearized with ScaI and RNA transcripts were synthesized in vitro using the T7 MegaScript transcription kit (Ambion, Austin, TX) according to manufacturer's directions. In vitro transcripts of the cDNA were transfected into the human hepatoma cell line, HUH-7. Selection for cells constitutively expressing the HCV replicon was achieved in the presence of the selectable marker, neomycin (G418). Resulting cell lines were characterized for positive and negative strand RNA production and protein production over time.

*HCV Replicon FRET Assay*

The HCV replicon FRET assay was developed to monitor the inhibitory effects of compounds described in the disclosure on HCV viral replication. HUH-7 cells, constitutively expressing the HCV replicon, were grown in Dulbecco's Modified Eagle Media (DMEM) (Gibco-BRL) containing 10% Fetal calf serum (FCS) (Sigma) and 1 mg/ml G418 (Gibco-BRL). Cells were seeded the night before (1.5 x $10^4$ cells/well) in 96-well tissue-culture sterile plates. Compound and no compound controls were prepared in DMEM containing 4% FCS, 1:100 Penicillin/Streptomysin (Gibco-BRL), 1:100 L-glutamine and 5% DMSO in the dilution plate (0.5% DMSO final concentration in the assay). Compound/DMSO mixes were added to the cells and incubated for 4 days at 37°C. After 4 days, cells were first assessed for cytotoxicity using alamar Blue (Trek Diagnotstic Systems) for a $CC_{50}$ reading. The toxicity of compound ($CC_{50}$) was determined by adding 1/10$^{th}$ volume of alamar Blue to the media incubating the cells. After 4 h, the fluorescence signal from each well was read, with an excitation wavelength at 530 nm and an emission wavelength of 580 nm, using the Cytofluor Series 4000 (Perspective Biosystems). Plates were then rinsed thoroughly with Phosphate-Buffered Saline (PBS) (3 times 150 μl). The cells were lysed with 25 μl of a lysis assay reagent containing an HCV protease substrate (5X cell Luciferase cell culture lysis reagent (Promega #E153A) diluted to 1X with distilled water, NaCl added to 150 mM final, the FRET peptide substrate (as described for the enzyme assay above) diluted to 10 μM final from a 2 mM stock in 100% DMSO. The plate was then placed into the Cytofluor 4000 instrument which had been set to 340 nm excitation/490 nm emission, automatic mode for 21 cycles and the plate read in a kinetic mode. $EC_{50}$ determinations were carried out as described for the $IC_{50}$ determinations.

*HCV Replicon Luciferase Reporter Assay*

As a secondary assay, $EC_{50}$ determinations from the replicon FRET assay were confirmed in a replicon luciferase reporter assay. Utilization of a replicon luciferase reporter assay was first described by Krieger et al (Krieger N, Lohmann V, and Bartenschlager R, J. Virol. 75(10):4614-4624 (2001)). The replicon construct described for our FRET assay was modified by inserting cDNA encoding a humanized form of the Renilla luciferase gene and a linker sequence fused directly to the 3'-end of the luciferase gene. This insert was introduced into the replicon construct using an Asc1 restriction site located in core, directly upstream of the neomycin marker gene. The adaptive mutation at position 1179 (serine to isoleucine) was also introduced (Blight KJ, Kolykhalov, AA, Rice, CM, Science 290(5498):1972-1974). A stable cell line constitutively expressing this HCV replicon construct was generated as described above. The luciferase reporter assay was set up as described for the HCV replicon FRET assay with the following modifications. Following 4 days in a 37°C/5% $CO_2$ incubator, cells were analyzed for Renilla Luciferase activity using the Promega Dual-Glo Luciferase Assay System. Media (100 μl) was removed from each well containing cells. To the remaining 50 μl of media, 50 μl of Dual-Glo Luciferase Reagent was added, and plates rocked for 10 min to 2 h at room temperature. Dual-Glo Stop & Glo Reagent (50 μl) was then added to each well, and plates were rocked again for an additional 10 min to 2 h at room temperature. Plates were read on a Packard TopCount NXT using a luminescence program.

The percentage inhibition was calculated using the formula below:

$$\%\ \text{control} = \frac{\text{average luciferase signal in experimental wells (+ compound)}}{\text{average luciferase signal in DMSO control wells (- compound)}}$$

The values were graphed and analyzed using XLfit to obtain the $EC_{50}$ value.

The compound of the current disclosure was tested and found to have the activity as follows: IC50: A=1-10 nM; B=11-12 nM; C=21-3200 nM; EC50: A=1-10 nM; B=11-20 nM; C=21-150 nM.

*Table 2*

| *Example Number* | *IC50* | *EC50* |
|---|---|---|
| 1 | 31 nM | 120.5 nM |
| 2 | B | - |

(continued)

| Example Number | IC50 | EC50 |
|---|---|---|
| 3 | A | - |
| 4 | A | A |
| 5 | A | - |
| 6 | A | - |
| 7 | A | A |
| 8 | A | A |
| 9 | A | A |
| 10 | B | C |
| 11 | A | B |
| 12 | 1.0 nM | 2.84 nM |
| 13 | A | B |
| 14 | A | A |
| 15 | A | A |
| 16 | B | C |
| 17 | A | A |
| 18 | A | A |
| 19 | A | A |
| 20 | B | - |
| 21 | C | - |
| 22 | A | - |
| 23 | A | - |
| 24 | A | - |
| 25 | 240 nM | - |
| 26 | A | - |
| 27 | A | - |
| 28 | A | - |
| 29 | A | - |
| 30 | A | - |
| 31 | A | - |
| 32 | 25 nM | - |
| 33 | B | - |
| 34 | 1.0 nM | - |
| 35 | 413 nM | - |
| 36 | c | - |
| 37 | c | - |
| 38 | A | - |
| 39 | A | - |
| 40 | A | - |

(continued)

| *Example Number* | *IC50* | *EC50* |
|---|---|---|
| 41 | A | - |

It will be evident to one skilled in the art that the present disclosure is not limited to the foregoing illustrative examples, and that it can be embodied in other specific forms without departing from the essential attributes thereof. It is therefore desired that the examples be considered in all respects as illustrative and not restrictive, reference being made to the appended claims, rather than to the foregoing examples, and all changes which come within the meaning and range of equivalency of the claims are therefore intended to be embraced therein.

## Claims

**1.** A compound of formula (I)

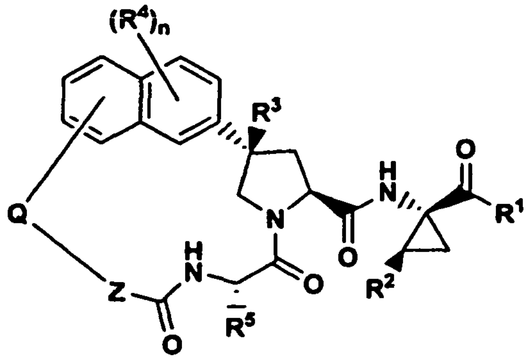

(I),

or a pharmaceutically acceptable salt thereof, wherein

n is 0, 1, 2, or 3;
$R^1$ is selected from hydroxy and-$NHSO_2R^6$;
$R^2$ is selected from hydrogen, alkenyl, alkyl, and cycloalkyl, wherein the alkenyl, the alkyl, and the cycloalkyl are each optionally substituted with one, two, three, or four halo groups;
$R^3$ is selected from hydrogen, alkoxy, alkoxyalkoxy, alkylsulfanyl, alkylsulfonyl, alkylsulfoxyl, hydroxy, and $(NR^cR^d)$carbonyloxy;
each $R^4$ is independently selected from alkoxy, alkyl, cyano, dialkylamino, halo, haloalkyl, haloalkoxy, a monocyclic heterocycle, hydroxy, and phenyl; wherein the moncyclic heterocycle and the phenyl are each optionally substituted with one, two, three, four, or five substituents independently selected from alkoxy, alkyl, dialkylamino, halo, haloalkoxy, and haloalkyl;
$R^5$ is selected from hydrogen, alkenyl, alkyl, aryl, arylalkyl, cycloalkyl, (cycloalkyl)alkyl, heterocyclyl, and heterocyclylalkyl; wherein the alkyl and cycloalkyl are each optionally substituted with one group selected from alkoxy, haloalkoxy, halo, haloalkyl, cyano, and dialkylamino;
$R^6$ is selected from alkyl, aryl, arylalkyl, cycloalkyl, (cycloalkyl)alkyl, heterocyclyl, and -$NR^aR^b$; wherein the alkyl and cycloalkyl are each optionally substituted with one group selected from alkyl, alkoxy, halo, haloalkyl, cyano, cyanoalkyl, and haloalkoxy;
$R^a$ and $R^b$ are independently selected from hydrogen, alkoxy, alkyl, aryl, arylalkyl, cycloalkyl, (cycloalkyl)alkyl, haloalkyl, heterocyclyl, and heterocyclylalkyl;
$R^c$ and $R^d$ are independently selected from hydrogen and alkyl;
Q is a $C_{4-8}$ saturated or unsaturated chain, wherein the chain is optionally substituted with one, two, three, or four groups independently selected from alkyl, halo, and haloalkyl, wherein the alkyl and haloalkyl groups can optionally form a 3-7 membered ring with the carbon atom to which they are attached; and
Z is selected from $CH_2$, O, and $NR^z$; wherein $R^z$ is selected from hydrogen and alkyl.

2. A compound of claim 1, or a pharmaceutically acceptable salt thereof, wherein R' is - $NHSO_2R^6$.

3. A compound of claim 2, or a pharmaceutically acceptable salt thereof, wherein n is 0, 1, or 2.

4. A compound of claim 3, or a pharmaceutically acceptable salt thereof, wherein Q is a $C_{4-8}$ saturated or unsubstituted chain optionally substituted with two alkyl groups which can optionally form a 3-membered ring with the carbon atom to which they are attached.

5. A compound of claim 4, or a pharmaceutically acceptable salt thereof, wherein $R^3$ is selected from alkoxy, alkoxyalkoxy, hydroxy, and $(NR^cR^d)$carbonyloxy.

6. A compound of claim 5, or a pharmaceutically acceptable salt thereof, wherein $R^2$ is selected from alkenyl, alkyl, and unsubstituted cycloalkyl; wherein the alkyl is optionally substituted with two halo groups.

7. A compound of claim 6, or a pharmaceutically acceptable salt thereof, wherein $R^5$ is selected from alkyl and heterocyclyl.

8. A compound of claim 1, or a pharmaceutically acceptable salt thereof, wherein
$R^1$ is $-NHSO_2R^6$;
$R^2$ is selected from alkenyl, alkyl, and unsubstituted cycloalkyl; wherein the alkyl is optionally substituted with two halo groups; and
$R^5$ is selected from alkyl and heterocyclyl.

9. A compound of claim 1, or a pharmaceutically acceptable salt thereof, wherein
n is 0, 1, or 2;
$R^1$ is $-NHSO_2R^6$; wherein $R^6$ is cycloalkyl;
$R^2$ is selected from alkenyl, alkyl, and unsubstituted cycloalkyl; wherein the alkyl is optionally substituted with two halo groups;
$R^3$ is alkoxy, alkoxyalkoxy, hydroxy, and $(NR^cR^d)$carbonyloxy;
each $R^4$ is selected from alkoxy, dialkylamino, and halo;
$R^5$ is selected from alkyl and heterocyclyl; and
Q is a $C_{4-8}$ saturated or unsubstituted chain optionally substituted with two alkyl groups which can optionally form a 3-membered ring with the carbon atom to which they are attached.

10. A compound of formula (II)

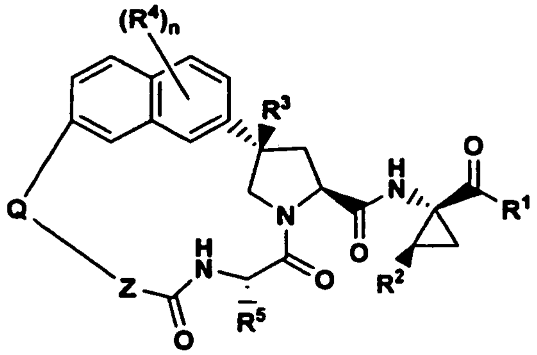

(II),

or a pharmaceutically acceptable salt thereof, wherein

n is 0, 1, 2, or 3;
$R^1$ is selected from hydroxy and $-NHSO_2R^6$;
$R^2$ is selected from hydrogen, alkenyl, alkyl, and cycloalkyl, wherein the alkenyl, the alkyl, and the cycloalkyl are each optionally substituted with one, two, three, or four halo groups;
$R^3$ is selected from hydrogen, alkoxy, alkylsulfanyl, alkylsulfonyl, alkylsulfoxyl, and hydroxy;

each $R^4$ is independently selected from alkoxy, alkyl, cyano, dialkylamino, halo, haloalkyl, haloalkoxy, a monocyclic heterocycle, hydroxy, and phenyl; wherein the moncyclic heterocycle and the phenyl are each optionally substituted with one, two, three, four, or five substituents independently selected from alkoxy, alkyl, dialkylamino, halo, haloalkoxy, and haloalkyl;
$R^5$ is selected from hydrogen, alkenyl, alkyl, aryl, arylalkyl, cycloalkyl, (cycloalkyl)alkyl, heterocyclyl, and heterocyclylalkyl; wherein the alkyl and cycloalkyl are each optionally substituted with one group selected from alkoxy, haloalkoxy, halo, haloalkyl, cyano, and dialkylamino;
$R^6$ is selected from alkyl, aryl, arylalkyl, cycloalkyl, (cycloalkyl)alkyl, heterocyclyl, and $-NR^aR^b$; wherein the alkyl and cycloalkyl are each optionally substituted with one group selected from alkyl, alkoxy, halo, haloalkyl, cyano, cyanoalkyl, and haloalkoxy;
$R^8$ and $R^b$ are independently selected from hydrogen, alkoxy, alkyl, aryl, arylalkyl, cycloalkyl, (cycloalkyl)alkyl, haloalkyl, heterocyclyl, and heterocyclylalkyl;
Q is a $C_{4-8}$ saturated or unsaturated chain, wherein the chain is optionally substituted with one, two, three, or four groups independently selected from alkyl, halo, and haloalkyl, wherein the alkyl and haloalkyl groups can optionally form a 3-7 membered ring with the carbon atom to which they are attached; and
Z is selected from $CH_2$, O, and $NR^z$; wherein $R^z$ is selected from hydrogen and alkyl.

**11.** A compound selected from

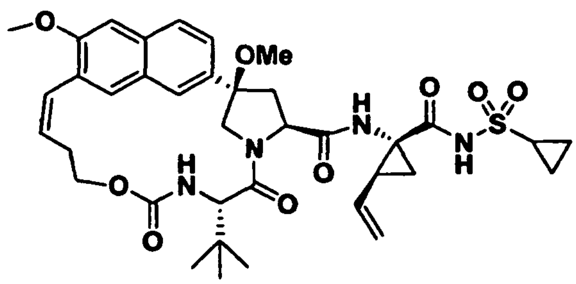

;

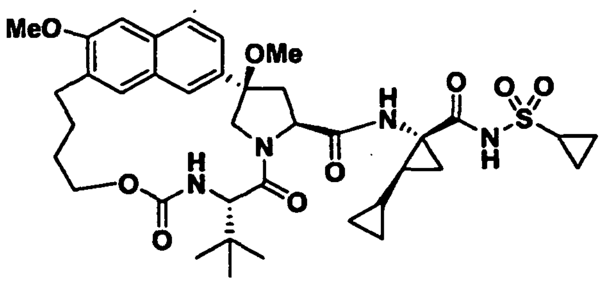

;

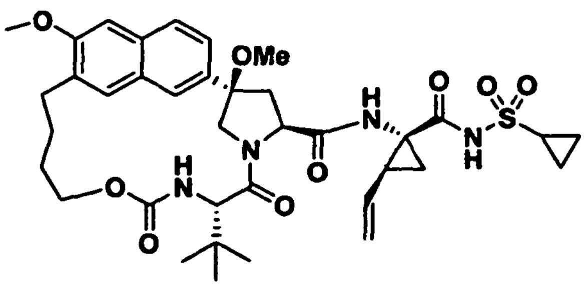

;

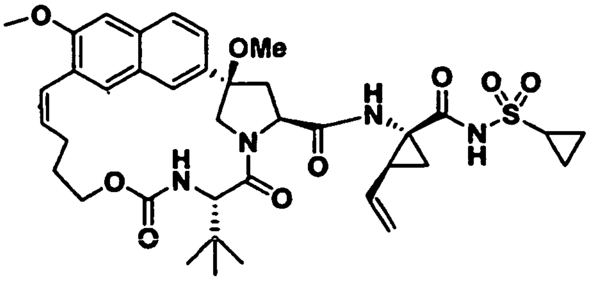

;

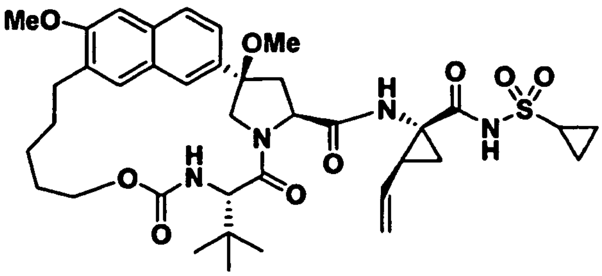

;

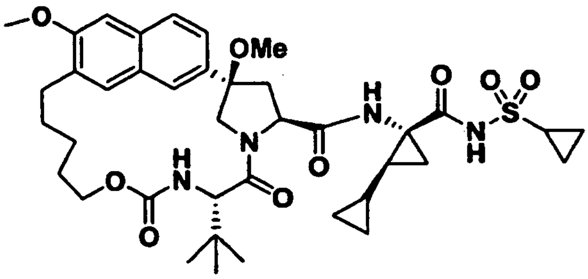

;

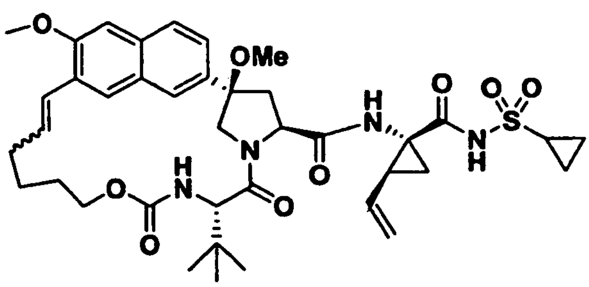

;

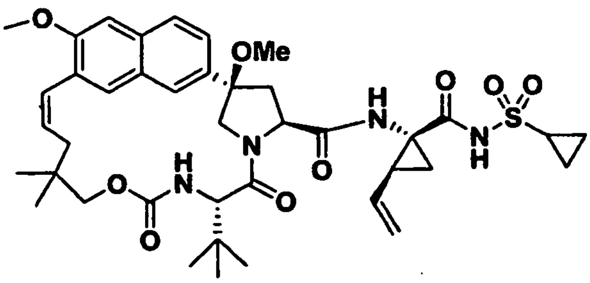

;

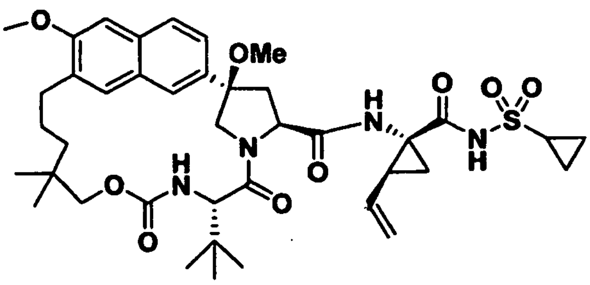

;

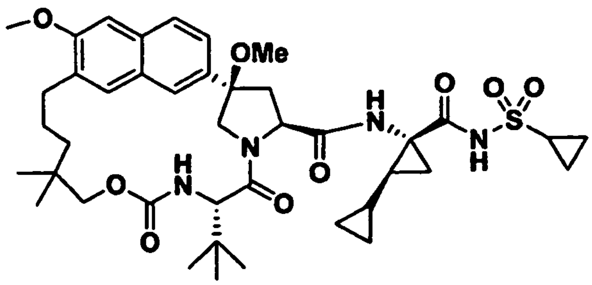

;

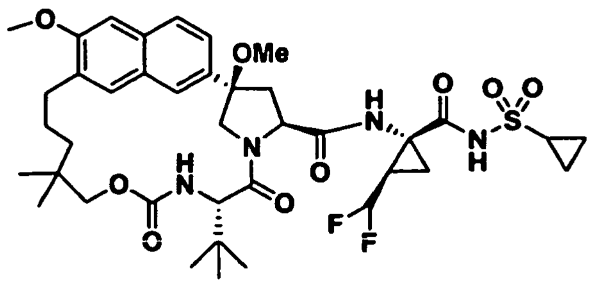

;

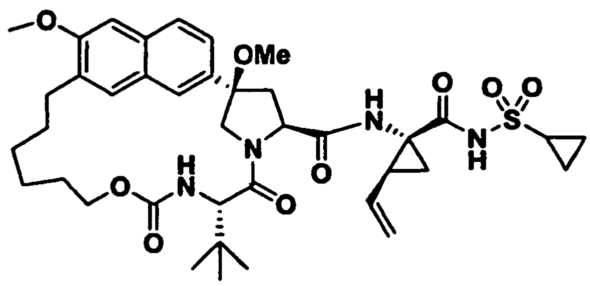

;

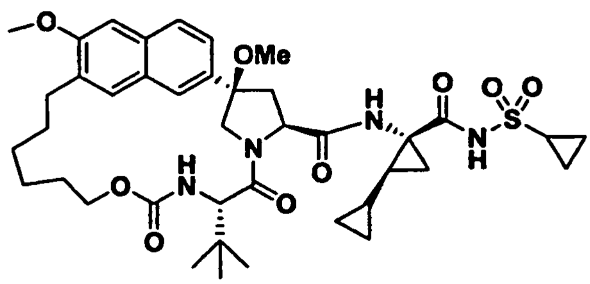

;

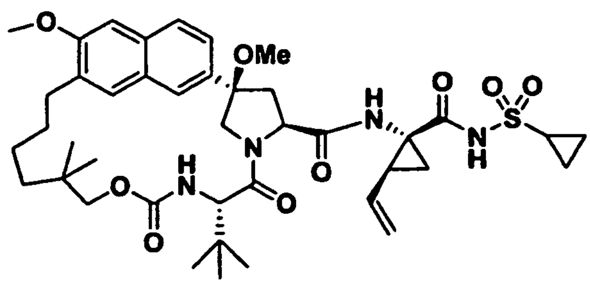

;

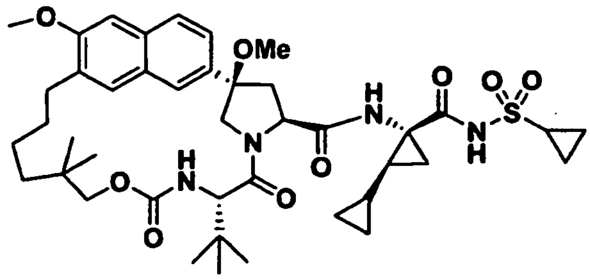

;

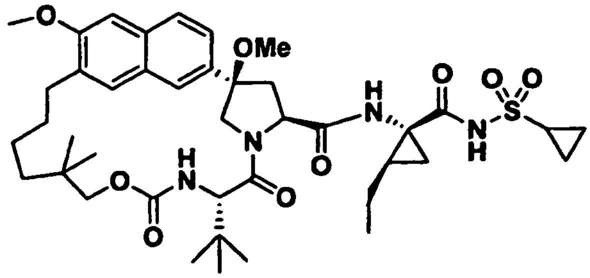

;

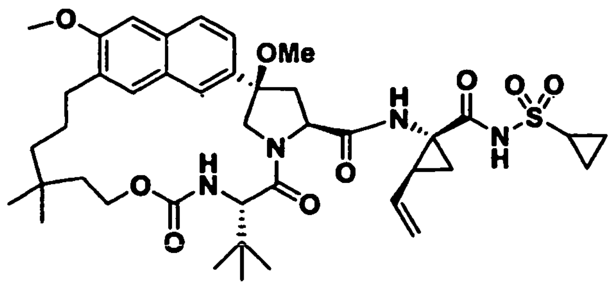

;

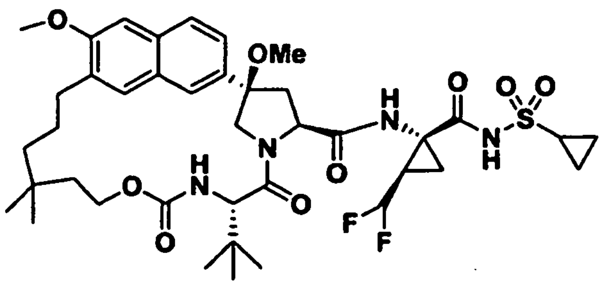

;

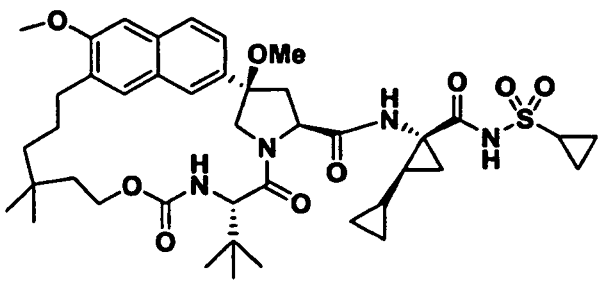

;

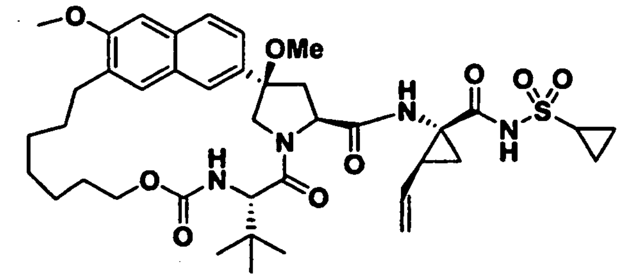

;

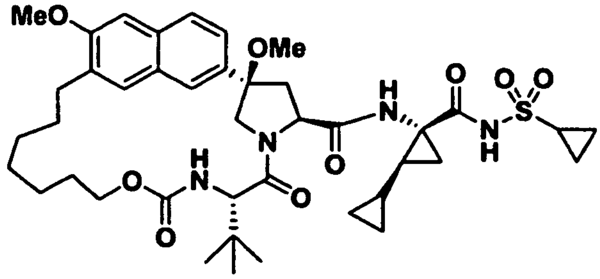

;

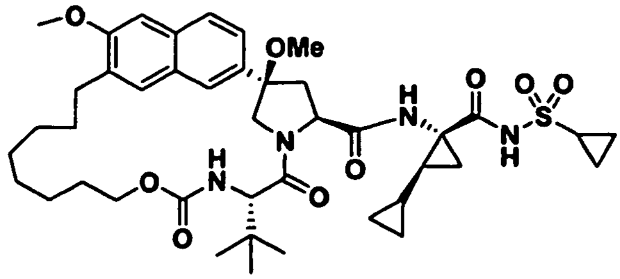

;

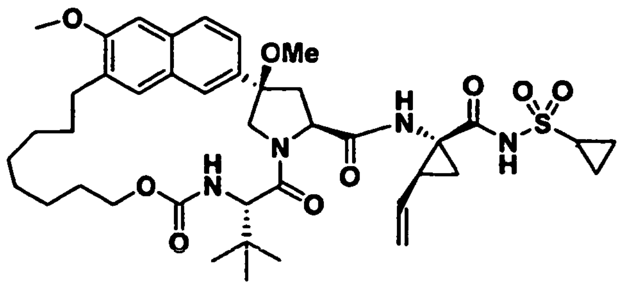

;

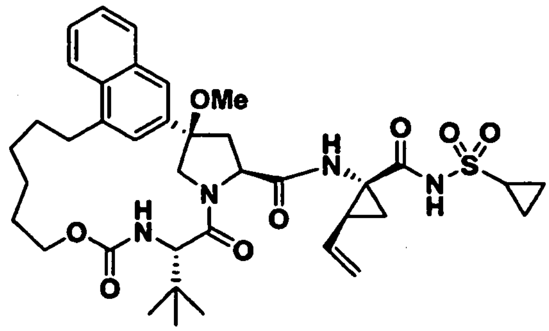

;

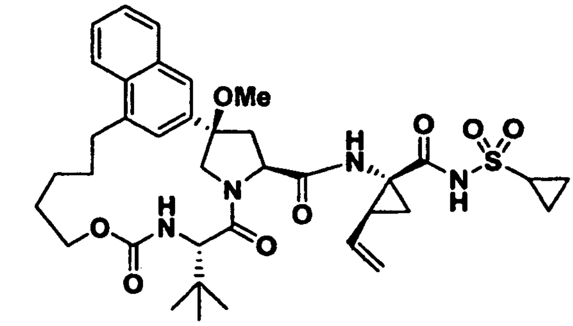

;

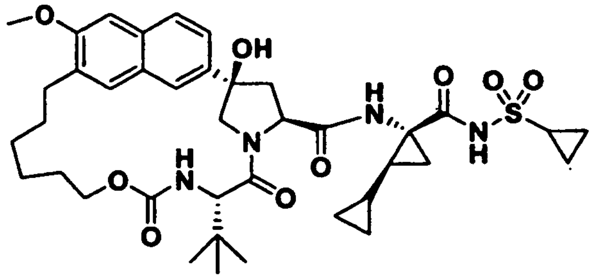

;

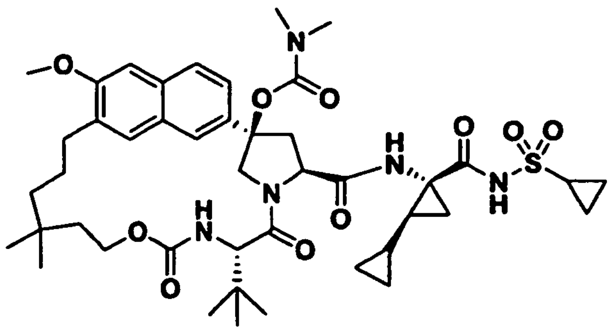

;

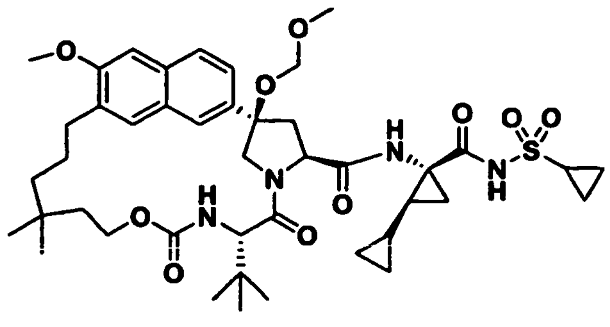

;

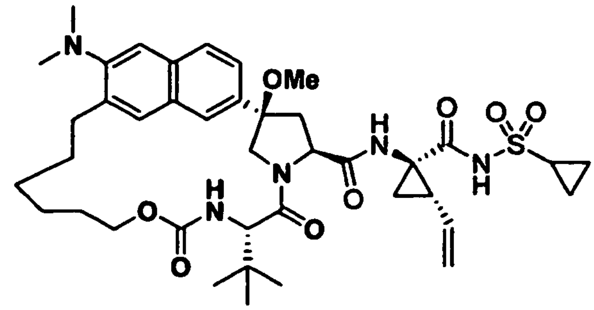

;

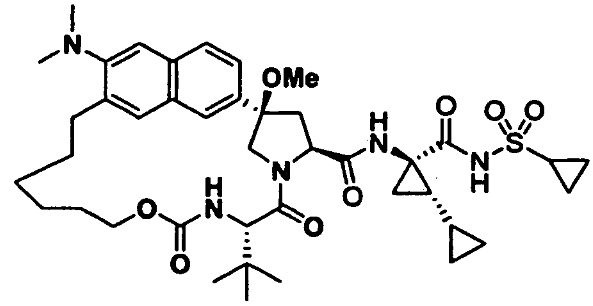

;

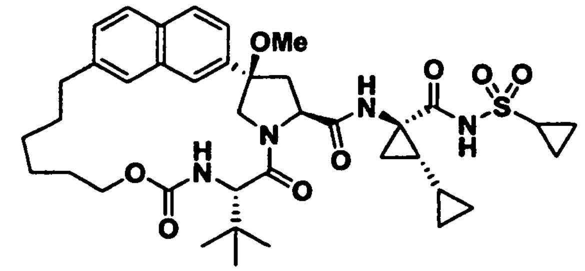

;

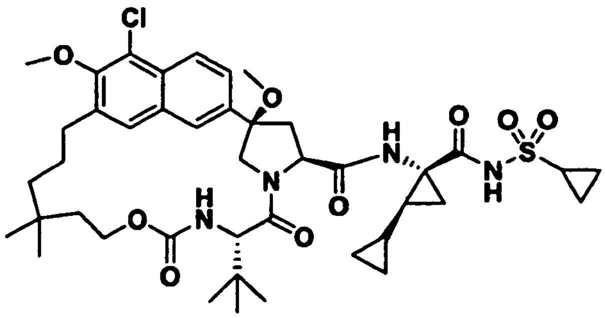

;

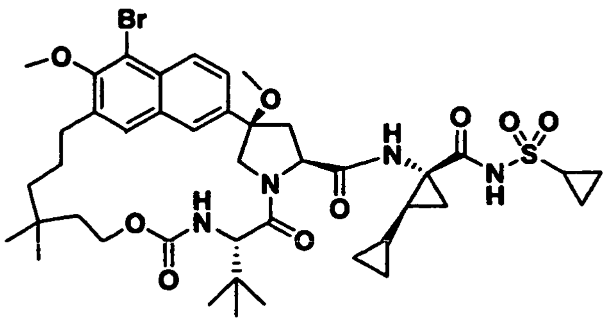

;

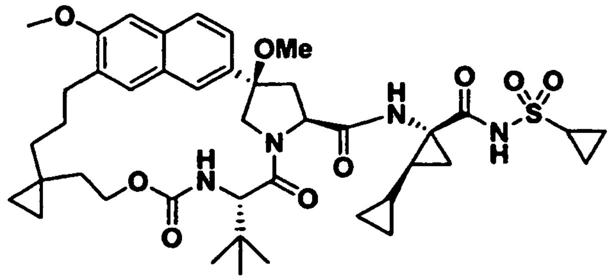

;

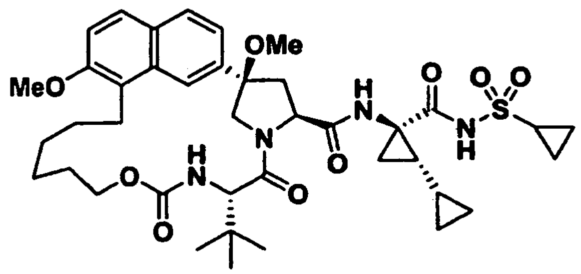

;

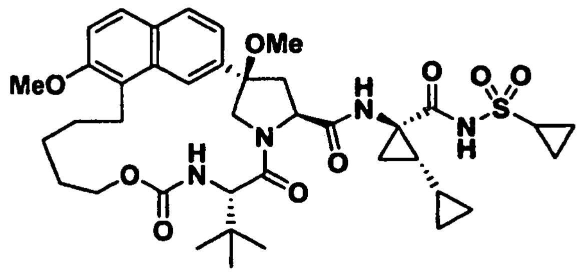

;

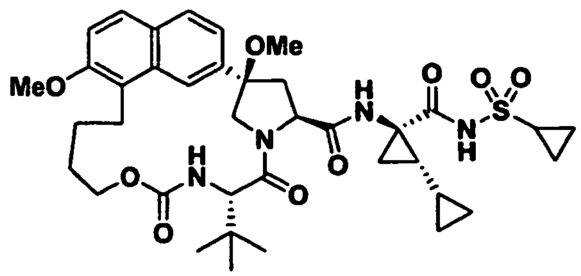

;

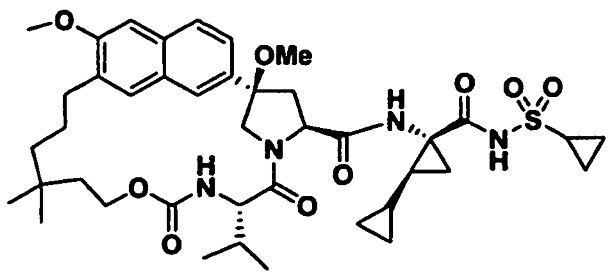

;

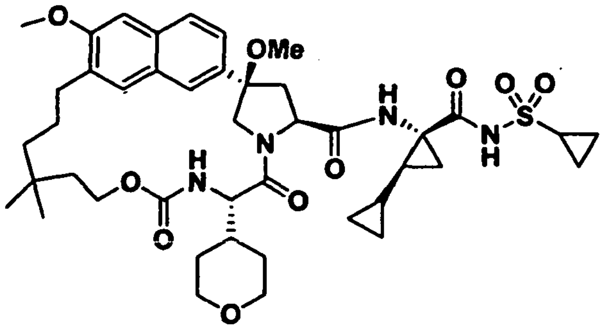

;

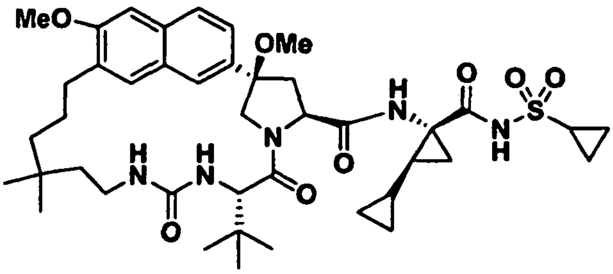

;

and

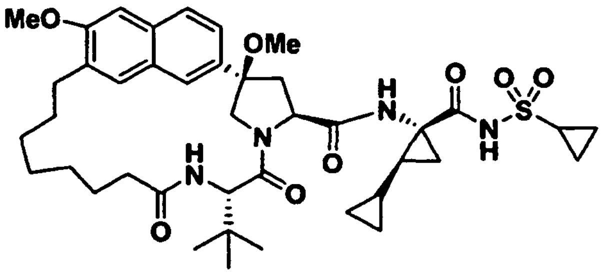

;

or a pharmaceutically acceptable salt thereof.

12. A composition comprising the compound of any one of claims 1 to 11, or a pharmaceutically acceptable salt thereof, and a pharmaceutically acceptable carrier.

13. The composition of claim 12 further comprising at least one additional compound having anti-HCV activity.

14. The composition of claim 13 wherein at least one of the additional compounds is an interferon or a ribavirin.

15. The composition of claim 14 wherein the interferon is selected from interferon alpha 2B, pegylated interferon alpha, consensus interferon, interferon alpha 2A, and lymphoblastiod interferon tau.

16. The composition of claim 13 wherein at least one of the additional compounds is selected from interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

17. The composition of claim 13 wherein at least one of the additional compounds is effective to inhibit the function of a target selected from HCV metalloprotease, HCV serine protease, HCV polymerase, HCV helicase, HCV NS4B protein, HCV entry, HCV assembly, HCV egress, HCV NS5A protein, and IMPDH for the treatment of an HCV

infection.

**18.** A compound of any one of claims 1 to 11, or a pharmaceutically acceptable salt thereof for use in a method of treating an HCV infection in a patient.

**19.** The compound for use according to claim 18 further comprising administering at least one additional compounds having anti-HCV activity as defined in claims 14 to 17 prior to, after, or simultaneously with the compound of any one of claims 1 to 11, or a pharmaceutically acceptable salt thereof.

**Patentansprüche**

**1.** Verbindung der Formel (I)

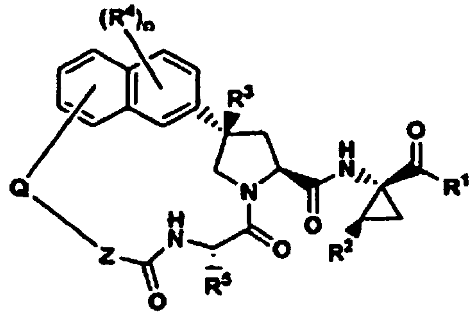

(I),

oder ein pharmazeutisch verträgliches Salz davon, wobei

n 0, 1, 2 oder 3 ist;
$R^1$ ausgewählt ist aus Hydroxy und $-NHSO_2R^6$;
$R^2$ ausgewählt ist aus Wasserstoff, Alkenyl, Alkyl und Cycloalkyl, wobei das Alkenyl, das Alkyl und das Cycloalkyl jeweils gegebenenfalls mit einer, zwei, drei oder vier Halogengruppe(n) substituiert sind;
$R^3$ ausgewählt ist aus Wasserstoff, Alkoxy, Alkoxyalkoxy, Alkylsulfanyl, Alkylsulfonyl, Alkylsulfoxyl, Hydroxy und $(NR^cR^d)$-Carbonyloxy;
jedes $R^4$ unabhängig ausgewählt ist aus Alkoxy, Alkyl, Cyano, Dialkylamino, Halogen, Halogenalkyl, Halogenalkoxy, einem monocyclischen Heterocyclus, Hydroxy und Phenyl; wobei der moncyclische Heterocyclus und das Phenyl jeweils gegebenenfalls mit einem, zwei, drei, vier oder fünf Substituenten substituiert sind, unabhängig ausgewählt aus Alkoxy, Alkyl, Dialkylamino, Halogen, Halogenalkoxy und Halogenalkyl;
$R^5$ ausgewählt ist aus Wasserstoff, Alkenyl, Alkyl, Aryl, Arylalkyl, Cycloalkyl, (Cycloalkyl)alkyl, Heterocyclyl und Heterocyclylalkyl; wobei das Alkyl und Cycloalkyl jeweils gegebenenfalls mit einer Gruppe substituiert sind, ausgewählt aus Alkoxy, Halogenalkoxy, Halogen, Halogenalkyl, Cyano und Dialkylamino;
$R^6$ ausgewählt ist aus Alkyl, Aryl, Arylalkyl, Cycloalkyl, (Cycloalkyl)alkyl, Heterocyclyl und $-NR^aR^b$; wobei das Alkyl und Cycloalkyl jeweils gegebenenfalls mit einer Gruppe substituiert sind, ausgewählt aus Alkyl, Alkoxy, Halogen, Halogenalkyl, Cyano, Cyanoalkyl und Halogenalkoxy;
$R^a$ und $R^b$ jeweils unabhängig ausgewählt sind aus Wasserstoff, Alkoxy, Alkyl, Aryl, Arylalkyl, Cycloalkyl, (Cycloalkyl)alkyl, Halogenalkyl, Heterocyclyl und Heterocyclylalkyl;
$R^c$ und $R^d$ unabhängig ausgewählt sind aus Wasserstoff und Alkyl;
Q eine gesättigte oder ungesättigte $C_{4-8}$-Kette ist, wobei die Kette gegebenenfalls mit einer, zwei, drei oder vier Gruppe(n) substituiert ist, unabhängig ausgewählt aus Alkyl, Halogen und Halogenalkyl, wobei die Alkyl- und Halogenalkylgruppen mit dem Kohlenstoffatom, an das sie gebunden sind, gegebenenfalls einen 3- bis 7-gliedrigen Ring bilden können; und
Z ausgewählt ist aus $CH_2$, O und $NR^z$; wobei $R^z$ ausgewählt ist aus Wasserstoff und Alkyl.

**2.** Verbindung nach Anspruch 1 oder ein pharmazeutisch verträgliches Salz davon, wobei $R^1$ $-NHSO_2R^6$ ist.

**3.** Verbindung nach Anspruch 2 oder ein pharmazeutisch verträgliches Salz davon, wobei n 0, 1 oder 2 ist.

4. Verbindung nach Anspruch 3 oder ein pharmazeutisch verträgliches Salz davon, wobei Q eine gesättigte oder ungesättigte $C_{4-8}$-Kette ist, gegebenenfalls substituiert mit zwei Alkylgruppen, die mit dem Kohlenstoffatom, an das sie gebunden sind, gegebenenfalls einen 3-gliedrigen Ring bilden können.

5. Verbindung nach Anspruch 4 oder ein pharmazeutisch verträgliches Salz davon, wobei $R^3$ ausgewählt ist aus Alkoxy, Alkoxyalkoxy, Hydroxy und ($NR^cR^d$)-Carbonyloxy.

6. Verbindung nach Anspruch 5 oder ein pharmazeutisch verträgliches Salz davon, wobei $R^2$ ausgewählt ist aus Alkenyl, Alkyl und unsubstituiertem Cycloalkyl; wobei das Alkyl gegebenenfalls mit zwei Halogengruppen substituiert ist.

7. Verbindung nach Anspruch 6 oder ein pharmazeutisch verträgliches Salz davon, wobei $R^5$ ausgewählt ist aus Alkyl und Heterocyclyl.

8. Verbindung nach Anspruch 1 oder ein pharmazeutisch verträgliches Salz davon, wobei
$R^1$ $-NHSO_2R^6$ ist;
$R^2$ ausgewählt ist aus Alkenyl, Alkyl und unsubstituiertem Cycloalkyl; wobei das Alkyl gegebenenfalls mit zwei Halogengruppen substituiert ist; und
$R^5$ ausgewählt ist aus Alkyl und Heterocyclyl.

9. Verbindung nach Anspruch 1 oder ein pharmazeutisch verträgliches Salz davon, wobei
n 0, 1 oder 2 ist;
$R^1$ $-NHSO_2R^6$ ist; wobei $R^6$ Cycloalkyl ist;
$R^2$ ausgewählt ist aus Alkenyl, Alkyl und unsubstituiertem Cycloalkyl; wobei das Alkyl gegebenenfalls mit zwei Halogengruppen substituiert ist;
$R^3$ Alkoxy, Alkoxyalkoxy, Hydroxy und ($NR^cR^d$)-Carbonyloxy ist;
jedes $R^4$ ausgewählt ist aus Alkoxy, Dialkylamino und Halogen;
$R^5$ ausgewählt ist aus Alkyl und Heterocyclyl; und
Q eine gesättigte oder ungesättigte $C_{4-8}$-Kette ist, gegebenenfalls substituiert mit zwei Alkylgruppen, die mit dem Kohlenstoffatom, an das sie gebunden sind, gegebenenfalls einen 3-gliedrigen Ring bilden können.

10. Verbindung der Formel (II)

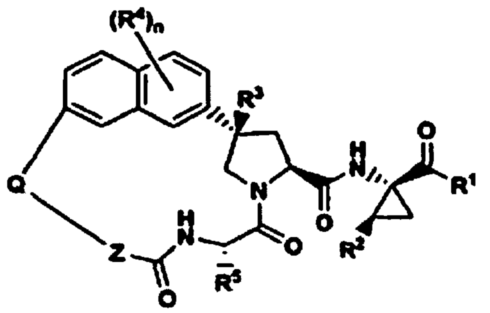

(II),

oder ein pharmazeutisch verträgliches Salz davon, wobei

n 0, 1, 2 oder 3 ist;
$R^1$ ausgewählt ist aus Hydroxy und $-NHSO_2R^6$;
$R^2$ ausgewählt ist aus Wasserstoff, Alkenyl, Alkyl und Cycloalkyl, wobei das Alkenyl, das Alkyl und das Cycloalkyl jeweils gegebenenfalls mit einer, zwei, drei oder vier Halogengruppe(n) substituiert sind;
$R^3$ ausgewählt ist aus Wasserstoff, Alkoxy, Alkylsulfanyl, Alkylsulfonyl, Alkylsulfoxyl, und Hydroxy;
jedes $R^4$ unabhängig ausgewählt ist aus Alkoxy, Alkyl, Cyano, Dialkylamino, Halogen, Halogenalkyl, Halogenalkoxy, einem monocyclischen Heterocyclus, Hydroxy und Phenyl; wobei der monocyclische Heterocyclus und das Phenyl jeweils gegebenenfalls mit einem, zwei, drei, vier oder fünf Substituenten substituiert sind, unabhängig ausgewählt aus Alkoxy, Alkyl, Dialkylamino, Halogen, Halogenalkoxy und Halogenalkyl;
$R^5$ ausgewählt ist aus Wasserstoff, Alkenyl, Alkyl, Aryl, Arylalkyl, Cycloalkyl, (Cycloalkyl)alkyl, Heterocyclyl und

Heterocyclylalkyl; wobei das Alkyl und Cycloalkyl jeweils gegebenenfalls mit einer Gruppe substituiert sind, ausgewählt aus Alkoxy, Halogenalkoxy, Halogen, Halogenalkyl, Cyano und Dialkylamino;
$R^6$ ausgewählt ist aus Alkyl, Aryl, Arylalkyl, Cycloalkyl, (Cycloalkyl)alkyl, Heterocyclyl und $-NR^aR^b$; wobei das Alkyl und Cycloalkyl jeweils gegebenenfalls mit einer Gruppe substituiert sind, ausgewählt aus Alkyl, Alkoxy, Halogen, Halogenalkyl, Cyano, Cyanoalkyl und Halogenalkoxy;
$R^a$ und $R^b$ unabhängig ausgewählt sind aus Wasserstoff, Alkoxy, Alkyl, Aryl, Arylalkyl, Cycloalkyl, (Cycloalkyl) alkyl, Halogenalkyl, Heterocyclyl und Heterocyclylalkyl;
Q eine gesättigte oder ungesättigte $C_{4-8}$-Kette ist, wobei die Kette gegebenenfalls mit einer, zwei, drei oder vier Gruppe(n) substituiert ist, unabhängig ausgewählt aus Alkyl, Halogen und Halogenalkyl, wobei die Alkyl- und Halogenalkylgruppen mit dem Kohlenstoffatom, an das sie gebunden sind, gegebenenfalls einen 3- bis 7-gliedrigen Ring bilden können; und
Z ausgewählt ist aus $CH_2$, O und $NR^z$; wobei $R^z$ ausgewählt ist aus Wasserstoff und Alkyl.

**11.** Verbindung, ausgewählt aus

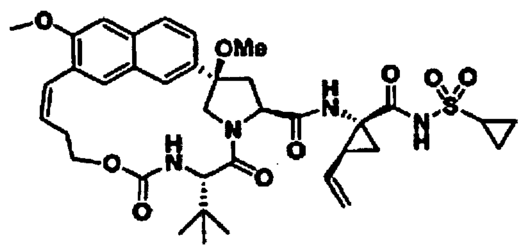

;

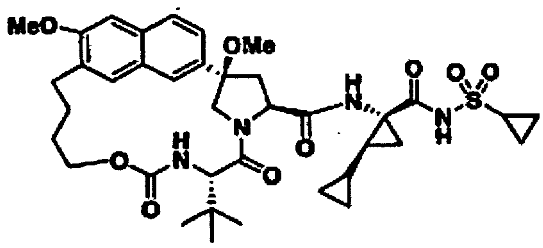

;

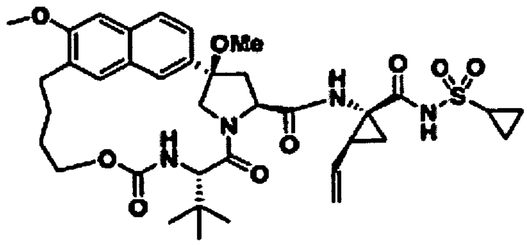

;

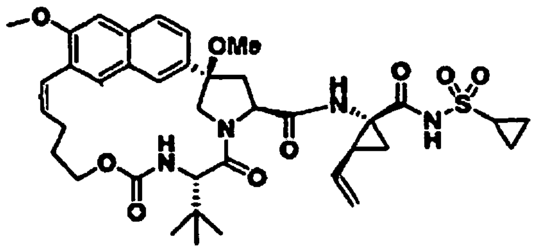

;

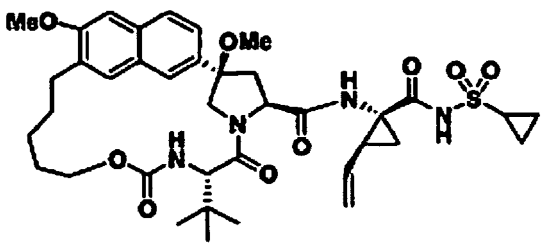

;

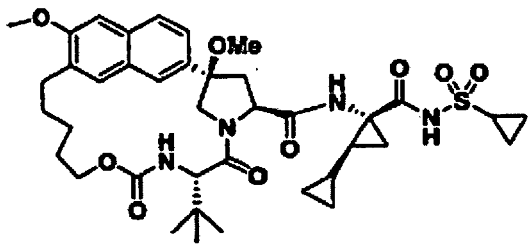

;

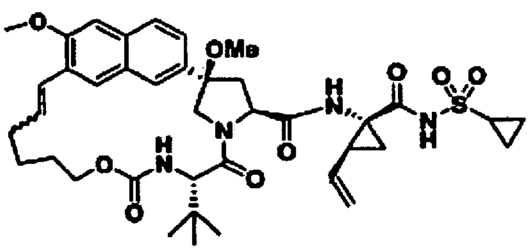

;

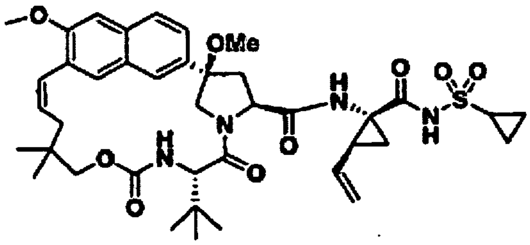

;

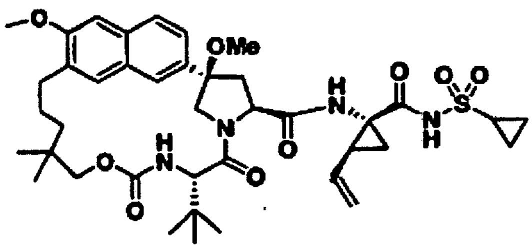

;

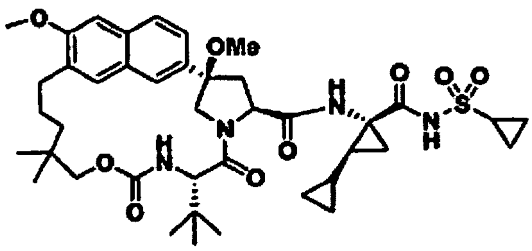

;

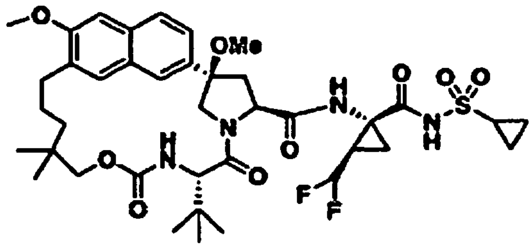

;

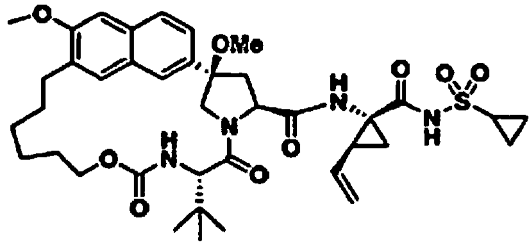

;

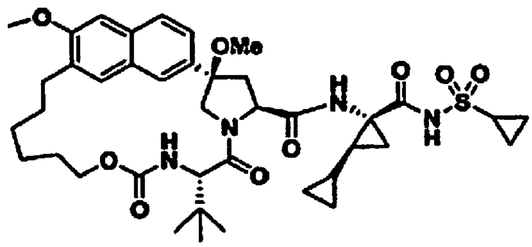

;

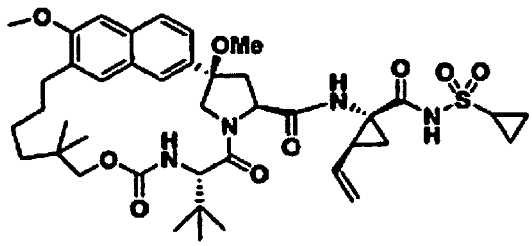

;

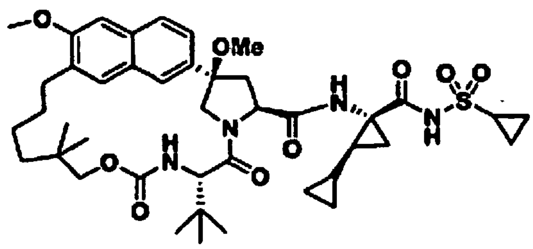

;

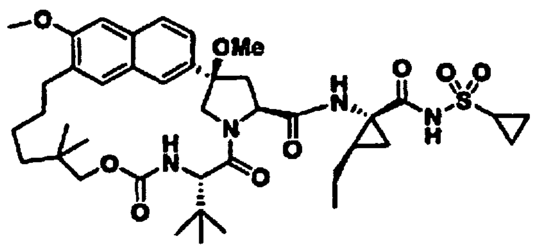

;

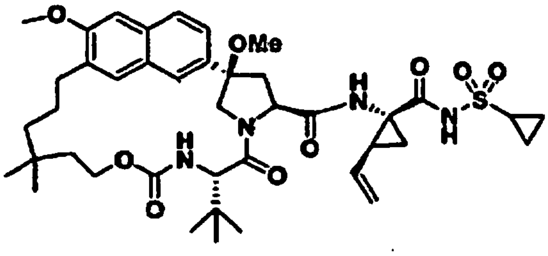

;

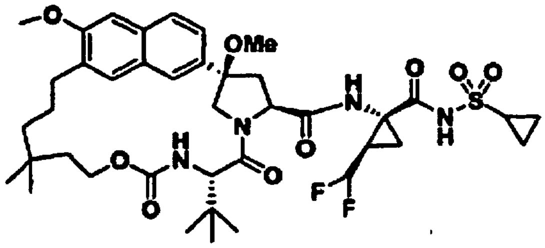

;

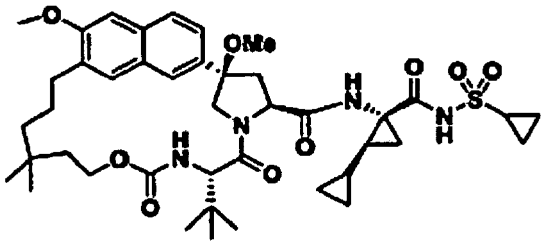

;

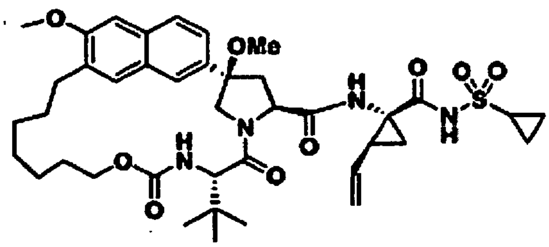

;

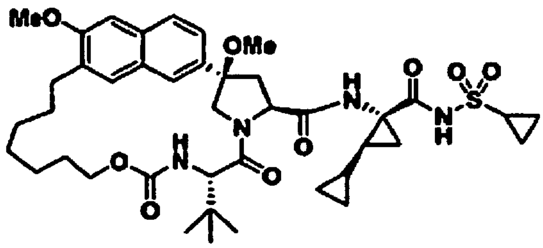

;

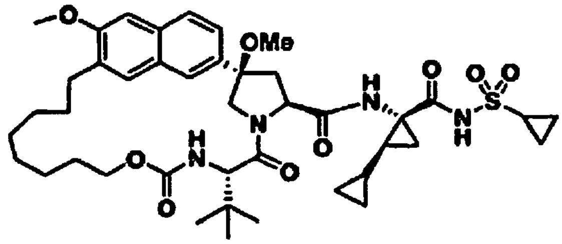

;

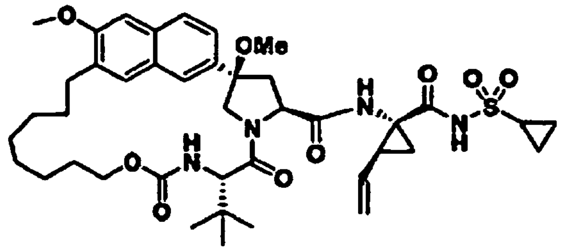

;

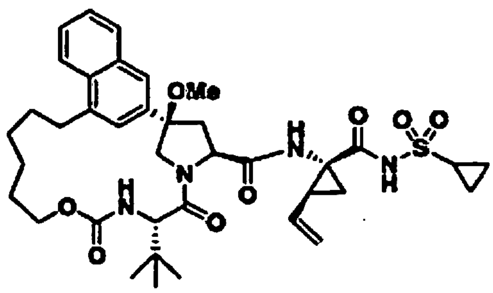

;

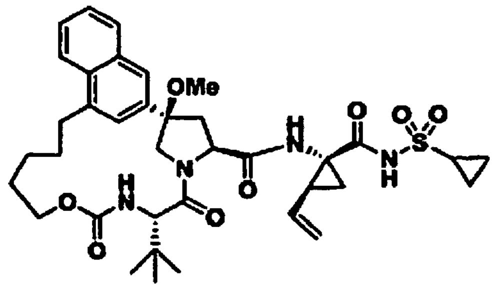

;

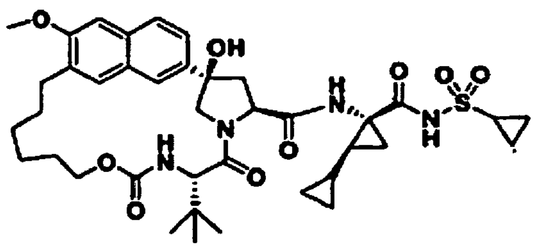

;

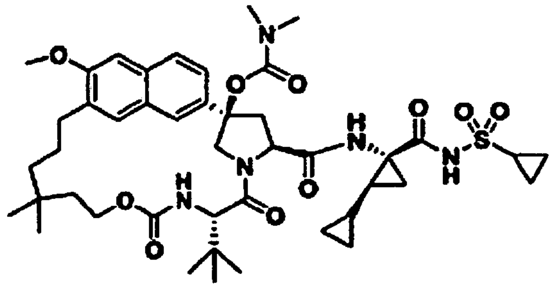

;

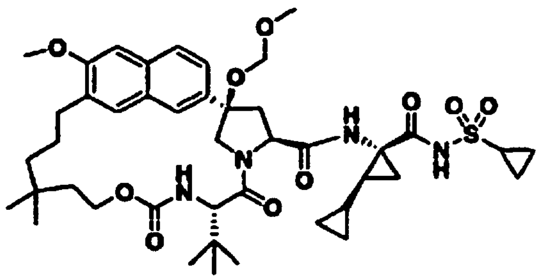

;

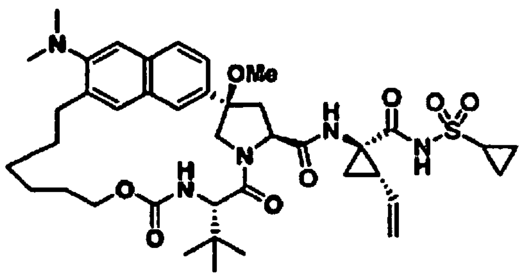

;

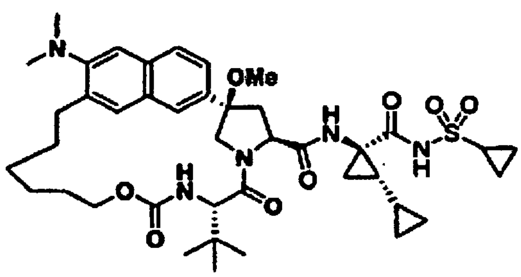

;

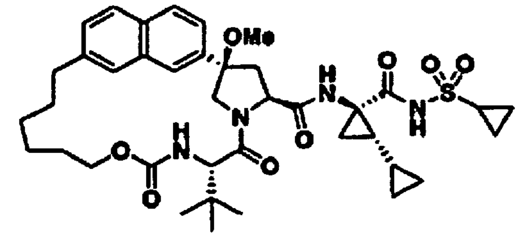

;

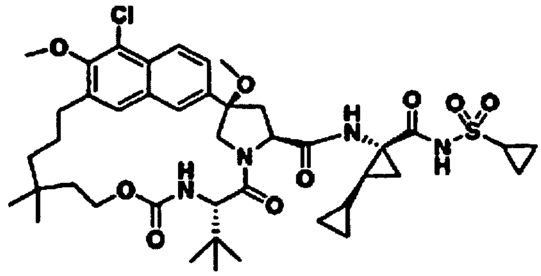

;

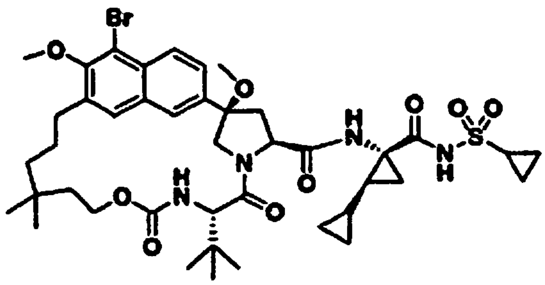

;

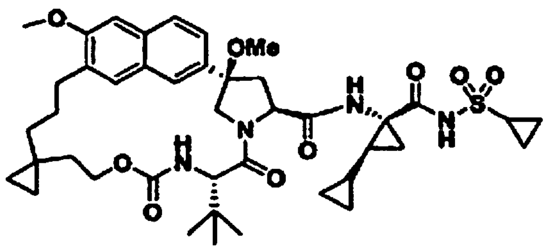

;

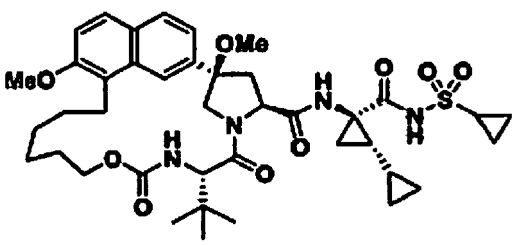

;

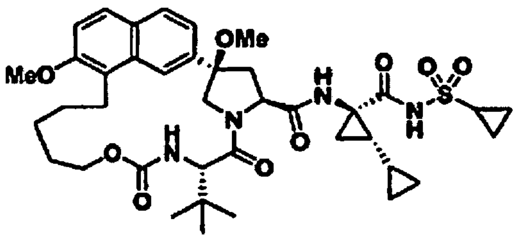

;

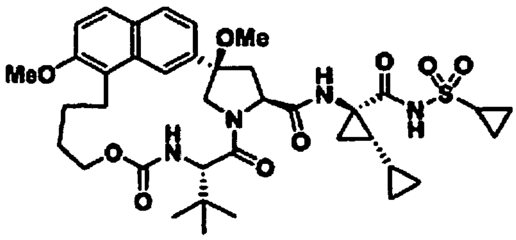

;

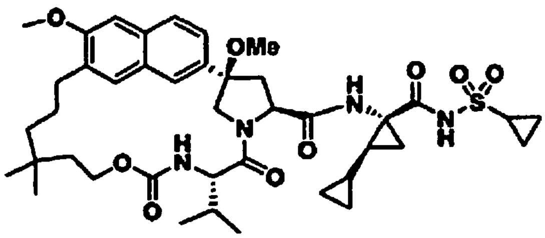

;

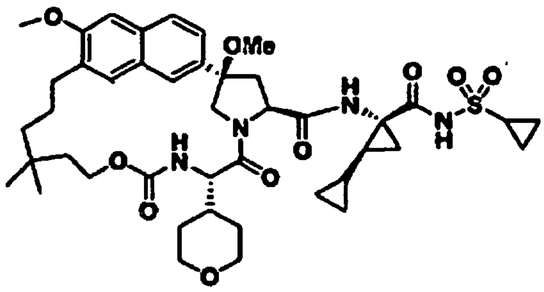

;

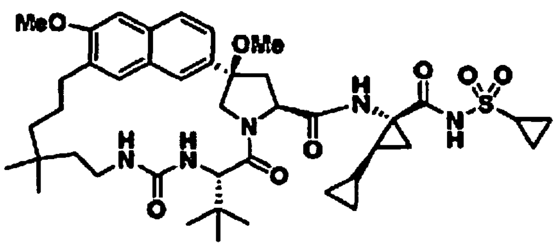

;

und

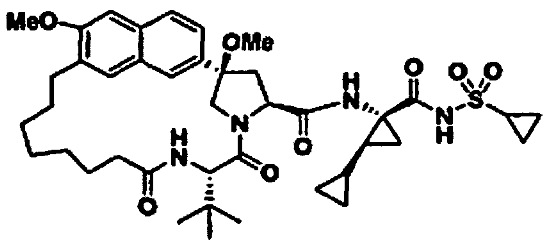

;

oder ein pharmazeutisch verträgliches Salz davon.

12. Zusammensetzung, umfassend die Verbindung nach einem der Ansprüche 1 bis 11 oder ein pharmazeutisch verträgliches Salz davon und einen pharmazeutisch verträglichen Träger.

13. Zusammensetzung nach Anspruch 12, ferner umfassend mindestens eine zusätzliche Verbindung mit anti-HCV-Aktivität.

14. Zusammensetzung nach Anspruch 13 wobei mindestens eine der zusätzlichen Verbindungen ein Interferon oder ein Ribavirin ist.

15. Zusammensetzung nach Anspruch 14, wobei das Interferon ausgewählt ist aus Interferon-alpha 2B, pegyliertem Interferon-alpha, Konsensus-Interferon, Interferon-alpha 2A und lymphoblastoidem Interferon-tau.

16. Zusammensetzung nach Anspruch 13, wobei mindestens eine der zusätzlichen Verbindungen ausgewählt ist aus Interleukin 2, Interleukin 6, Interleukin 12, einer Verbindung, welche die Entwicklung einer Antwort der Typ 1-T-Helferzellen fördert, interferierender RNA, Antisense-RNA, Imiqimod, Ribavirin, einem Inosin-5'-monophosphatdehydrogenase-Inhibitor, Amantadin und Rimantadin.

17. Zusammensetzung nach Anspruch 13, wobei mindestens eine der zusätzlichen Verbindungen bei der Inhibition der Funktion eines Targets wirksam ist, ausgewählt aus HCV-Metalloprotease, HCV-Serinprotease, HCV-Polymerase, HCV-Helicase, HCV-NS4B-Protein, HCV-Entry (Eintritt), HCV-Assembly (Assemblierung), HCV-Egress (Ausschleusung), HCV-NS5A-Protein und IMPDH zur Behandlung einer HCV-Infektion.

18. Verbindung nach einem der Ansprüche 1 bis 11 oder ein pharmazeutisch verträgliches Salz davon zur Verwendung in einem Verfahren zur Behandlung einer HCV-Infektion bei einem Patienten.

19. Verbindung zur Verwendung nach Anspruch 18, ferner umfassend mindestens eine der zusätzlichen Verbindungen mit anti-HCV-Aktivität, wie nach den Verbindungen 14 bis 17 definiert, vor, nach oder gleichzeitig mit der Verbindung nach einem der Ansprüche 1 bis 11 oder einem pharmazeutisch verträglichen Salz davon.

## Revendications

1. Composé de la formule (I)

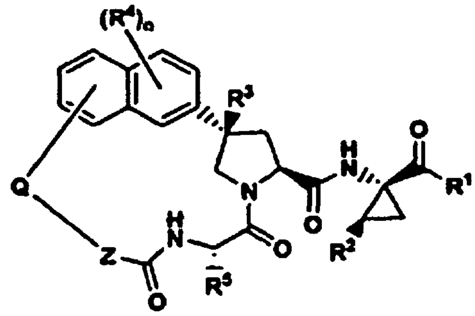

(I),

ou un sel pharmaceutiquement acceptable de celui-ci, dans lequel

n est 0, 1, 2 ou 3;
$R^1$ est sélectionné parmi hydroxy et $-NHSO_2R^6$;
$R^2$ est sélectionné parmi hydrogène, alcényle, alkyle et cycloalkyle, où l'alcényle, l'alkyle et le cycloalkyle sont chacun optionnellement substitués par un, deux, trois ou quatre groupes halo;
$R^3$ est sélectionné parmi hydrogène, alcoxy, alcoxyalcoxy, alkylsulfanyle, alkylsulfonyle, alkylsulfoxyle, hydroxy et $(NR^cR^d)$carbonyloxy;
chaque $R^4$ est sélectionné indépendamment parmi alcoxy, alkyle, cyano, dialkylamino, halo, haloalkyle, haloalcoxy, un hétérocycle monocyclique, hydroxy et phényle; où l'hétérocycle monocyclique et le phényle sont chacun optionnellement substitués par un, deux, trois, quatre ou cinq substituants sélectionnés indépendamment parmi alcoxy, alkyle, dialkylamino, halo, haloalcoxy et haloalkyle;
$R^5$ est sélectionné parmi hydrogène, alcényle, alkyle, aryle, arylalkyle, cycloalkyle, (cycloalkyl)alkyle, hétérocyclyle et hétérocyclylalkyle; où l'alkyle et le cycloalkyle sont chacun optionnellement substitués par un groupe sélectionné parmi alcoxy, haloalcoxy, halo, haloalkyle, cyano et dialkylamino;
$R^6$ est sélectionné parmi alkyle, aryle, arylalkyle, cycloalkyle, (cycloalkyl)alkyle, hétérocyclyle et $-NR^aR^b$; où l'alkyle et le cycloalkyle sont chacun optionnellement substitués par un groupe sélectionné parmi alkyle, alcoxy, halo, haloalkyle, cyano, cyanoalkyle et haloalcoxy;
$R^a$ et $R^b$ sont sélectionnés indépendamment parmi hydrogène, alcoxy, alkyle, aryle, arylalkyle, cycloalkyle, (cycloalkyl)alkyle, haloalkyle, hétérocyclyle et hétérocyclylalkyle;
$R^c$ et $R^d$ sont sélectionnés indépendamment parmi hydrogène et alkyle;
Q est une chaîne saturée ou insaturée en $C_{4-8}$, où la chaîne est optionnellement substituée par un, deux, trois ou quatre groupes sélectionnés indépendamment parmi alkyle, halo et haloalkyle, où les groupes alkyle et haloalkyle peuvent optionnellement former un cycle à 3-7 chaînons avec l'atome de carbone auquel ils sont attachés; et
Z est sélectionné parmi $CH_2$, O et $NR^z$; où $R^z$ est sélectionné parmi hydrogène et alkyle.

2. Composé selon la revendication 1 ou un sel pharmaceutiquement acceptable de celui-ci, dans lequel $R^1$ est $NHSO_2R^6$.

3. Composé selon la revendication 2 ou un sel pharmaceutiquement acceptable de celui-ci, dans lequel n est 0, 1 ou 2.

4. Composé selon la revendication 3 ou un sel pharmaceutiquement acceptable de celui-ci, dans lequel Q est une chaîne saturée ou insaturée en $C_{4-8}$ optionnellement substituée par deux groupes alkyle qui peuvent optionnellement former un cycle à 3 chaînons avec l'atome de carbone auquel ils sont attachés.

5. Composé selon la revendication 4 ou un sel pharmaceutiquement acceptable de celui-ci, dans lequel $R^3$ est sélectionné parmi alcoxy, alcoxyalcoxy, hydroxy et $(NR^cR^d)$carbonyloxy.

6. Composé selon la revendication 5 ou un sel pharmaceutiquement acceptable de celui-ci, dans lequel $R^2$ est sélectionné parmi alcényle, alkyle et cycloalkyle non substitué; où l'alkyle est optionnellement substitué par deux groupes halo.

7. Composé selon la revendication 6 ou un sel pharmaceutiquement acceptable de celui-ci, dans lequel $R^5$ est sélec-

tionné parmi alkyle et hétérocyclyle.

8. Composé selon la revendication 1 ou un sel pharmaceutiquement acceptable de celui-ci, dans lequel
$R^1$ est $-NHSO_2R^6$;
$R^2$ est sélectionné parmi alcényle, alkyle et cycloalkyle non substitué; où l'alkyle est optionnellement substitué par deux groupes halo; et
$R^5$ est sélectionné parmi alkyle et hétérocyclyle.

9. Composé selon la revendication 1 ou un sel pharmaceutiquement acceptable de celui-ci, dans lequel
n est 0, 1 ou 2;
$R^1$ est $-NHSO_2R^6$; où $R^6$ est cycloalkyle;
$R^2$ est sélectionné parmi alcényle, alkyle et cycloalkyle non substitué, où l'alkyle est optionnellement substitué par deux groupes halo;
$R^3$ est alcoxy, alcoxyalcoxy, hydroxy et ($NR^cR^d$)carbonyloxy;
chaque $R^4$ est sélectionné parmi alcoxy, dialkylamino et halo;
$R^5$ est sélectionné parmi alkyle et hétérocyclyle; et
Q est une chaîne saturée ou insaturée en $C_{4-8}$ optionnellement substituée par deux groupes alkyle qui peuvent optionnellement former un cycle à 3 chaînons avec l'atome de carbone auquel ils sont attachés.

10. Composé de la formule (II)

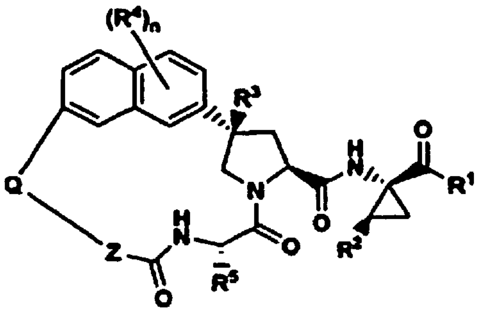

(II),

ou un sel pharmaceutiquement acceptable de celui-ci, dans lequel

n est 0, 1, 2 ou 3;
$R^1$ est sélectionné parmi hydroxy et $-NHSO_2R^6$;
$R^2$ est sélectionné parmi hydrogène, alcényle, alkyle et cycloalkyle, où l'alcényle, l'alkyle et le cycloalkyle sont chacun optionnellement substitués par un, deux, trois ou quatre groupes halo;
$R^3$ est sélectionné parmi hydrogène, alcoxy, alkylsulfanyle, alkylsulfonyle, alkylsulfoxyle et hydroxy;
chaque $R^4$ est sélectionné indépendamment parmi alcoxy, alkyle, cyano, dialkylamino, halo, haloalkyle, haloalcoxy, un hétérocycle monocyclique, hydroxy et phényle; où l'hétérocycle monocyclique et le phényle sont chacun optionnellement substitués par un, deux, trois, quatre ou cinq substituants sélectionnés indépendamment parmi alcoxy, alkyle, dialkylamino, halo, haloalcoxy et haloalkyle;
$R^5$ est sélectionné parmi hydrogène, alcényle, alkyle, aryle, arylalkyle, cycloalkyle, (cyclalkyl)alkyle, hétérocyclyle et hétérocyclylalkyle; où l'alkyle et le cycloalkyle sont chacun optionnellement substitués par un groupe sélectionné parmi alcoxy, haloalcoxy, halo, haloalkyle, cyano et dialkylamino;
$R^6$ est sélectionné parmi alkyle, aryle, arylalkyle, cycloalkyle, (cycloalkyl)alkyle, hétérocyclyle et $-NR^aR^b$; où l'alkyle et le cycloalkyle sont chacun optionnellement substitués par un groupe sélectionné parmi alkyle, alcoxy, halo, haloalkyle, cyano, cyanoalkyle et haloalcoxy;
$R^a$ et $R^b$ sont sélectionnés indépendamment parmi hydrogène, alcoxy, alkyle, aryle, arylalkyle, cycloalkyle, (cycloalkyl)alkyle, haloalkyle, hétérocyclyle et hétérocyclylalkyle;
Q est une chaîne saturée ou insaturée en $C_{4-8}$, où la chaîne est optionnellement substituée par un, deux, trois ou quatre groupes sélectionnés indépendamment parmi alkyle, halo et haloalkyle, où les groupes alkyle et haloalkyle peuvent optionnellement former un cycle à 3-7 chaînons avec l'atome de carbone auquel ils sont attachés; et
Z est sélectionné parmi $CH_2$, O et $NR^z$; où $R^z$ est sélectionné parmi hydrogène et alkyle.

**11.** Composé sélectionné parmi

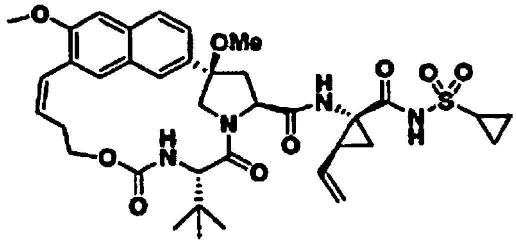

;

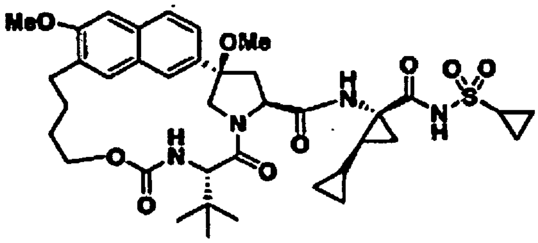

;

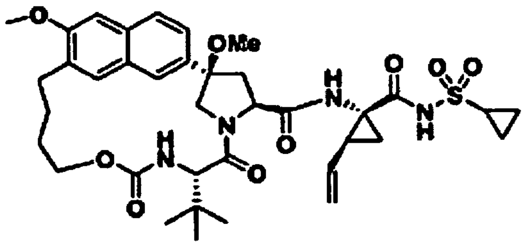

;

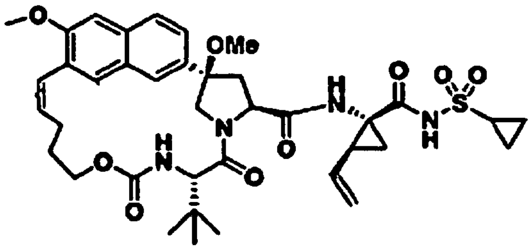

;

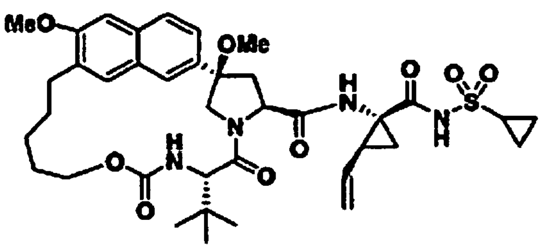

;

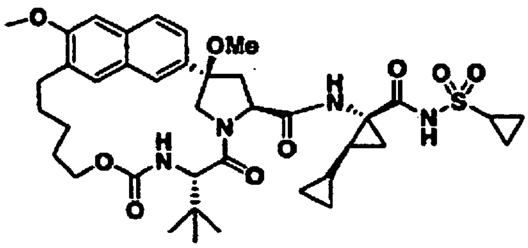

;

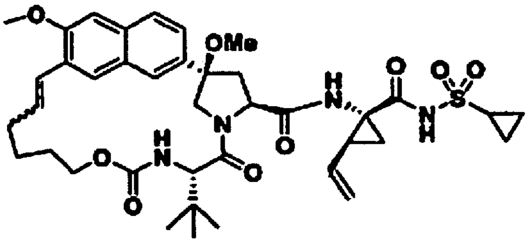

;

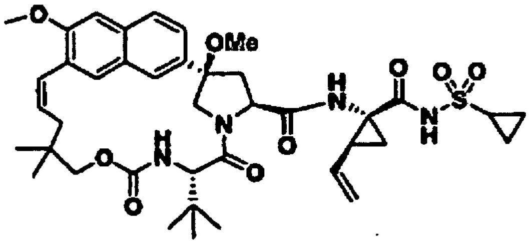

;

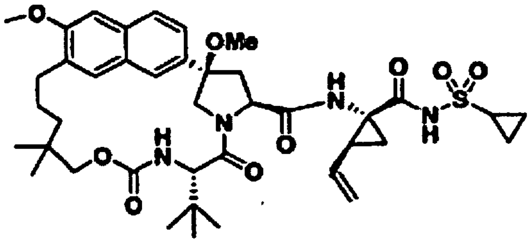

;

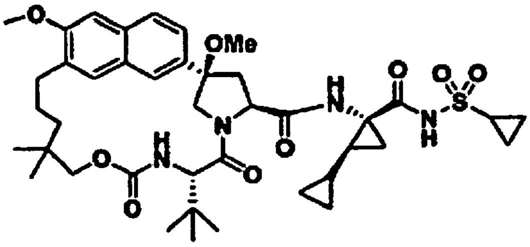

;

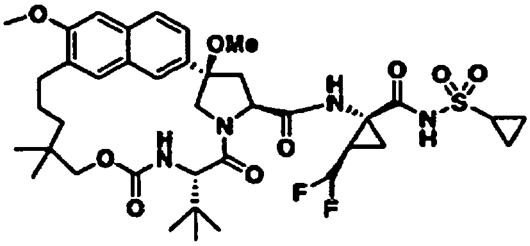

;

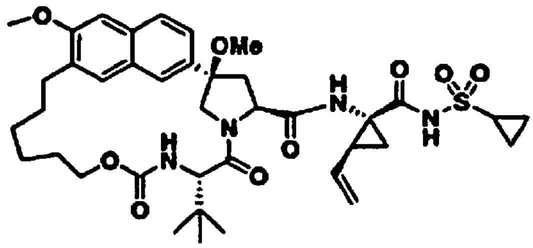

;

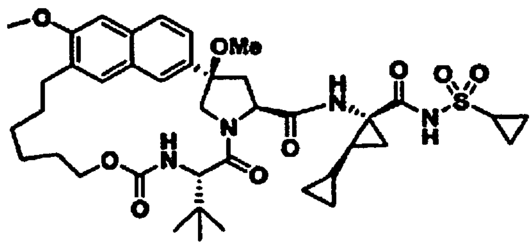

;

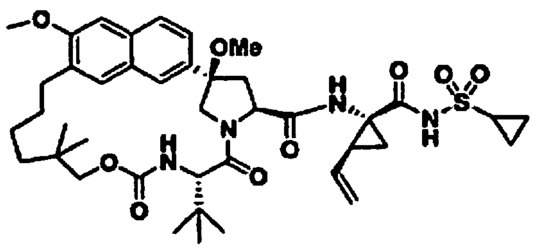

;

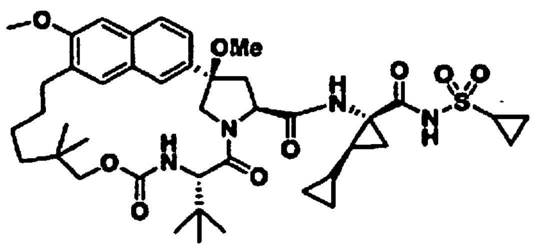

;

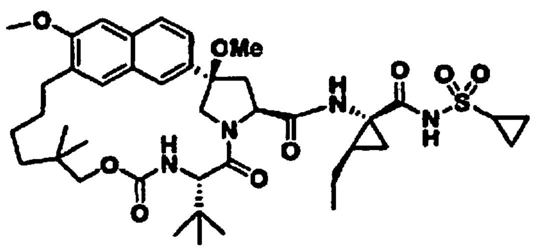

;

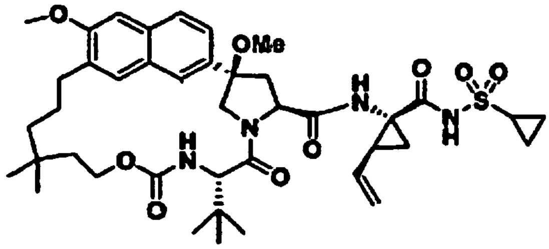

;

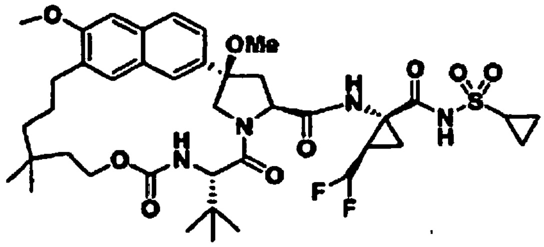

;

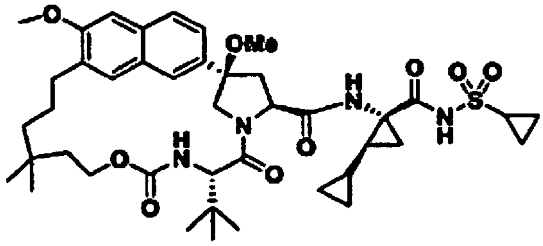

;

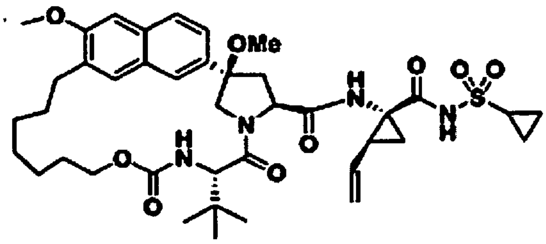

;

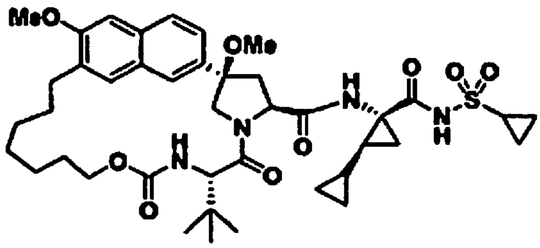

;

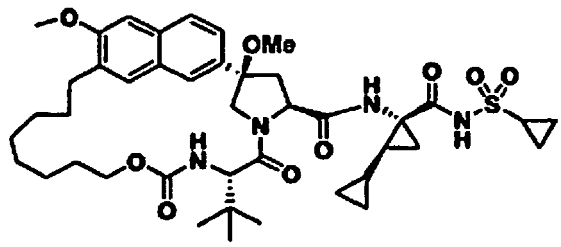

;

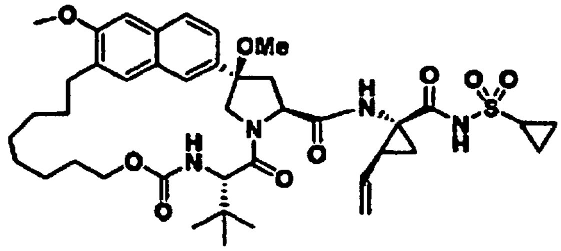

;

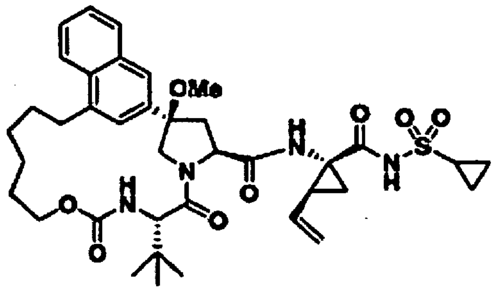

;

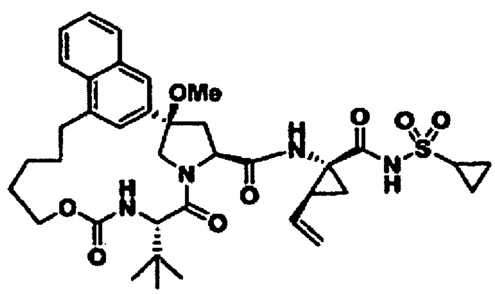

;

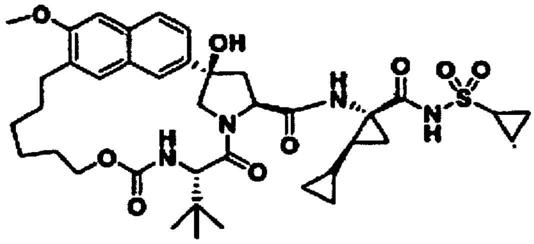

;

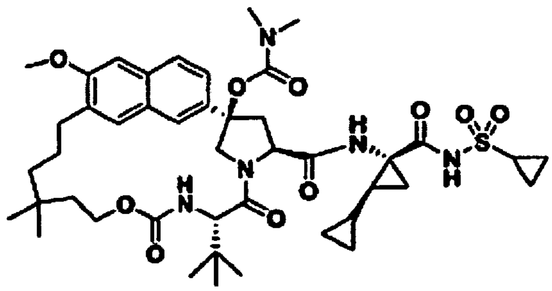

;

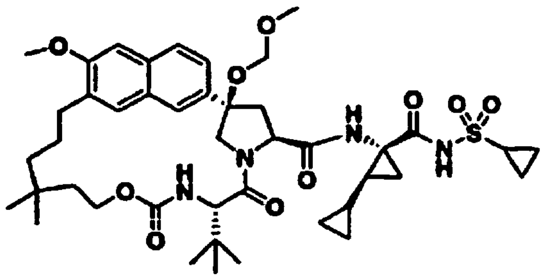

;

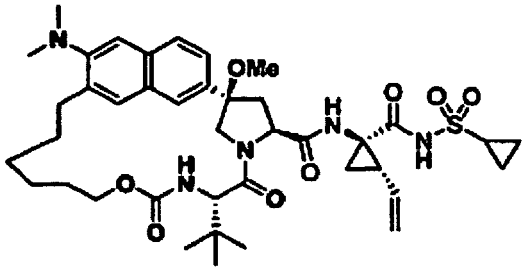

;

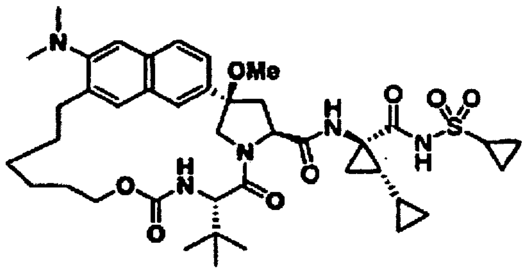

;

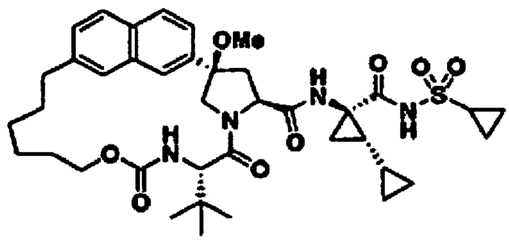

;

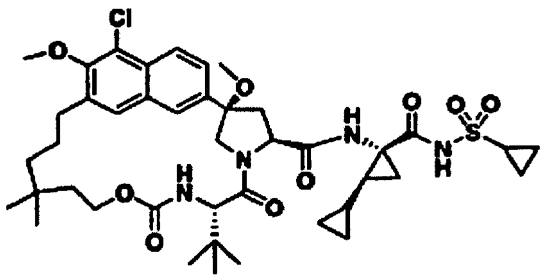

;

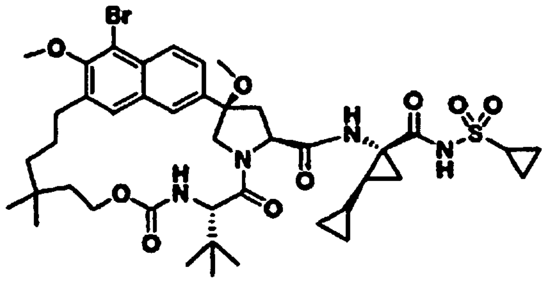

;

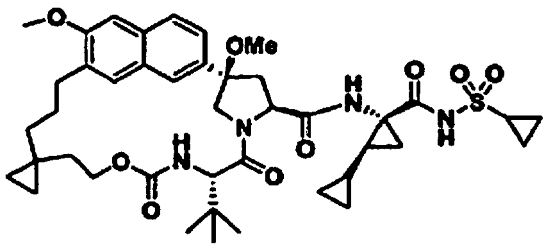

;

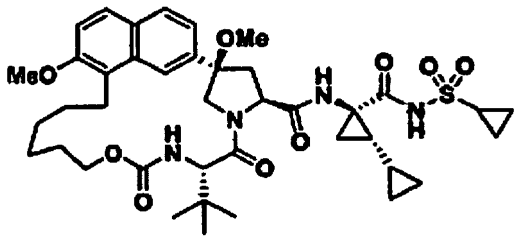

;

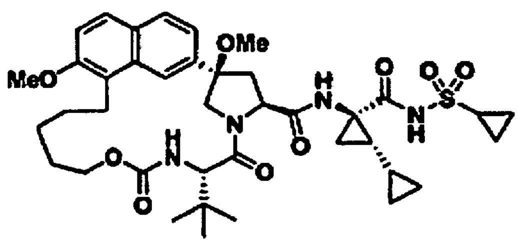

;

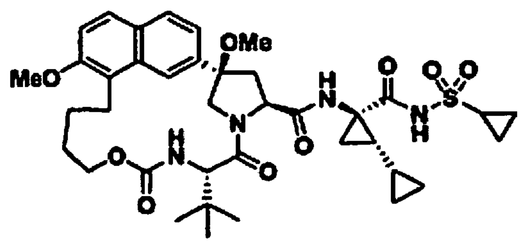

;

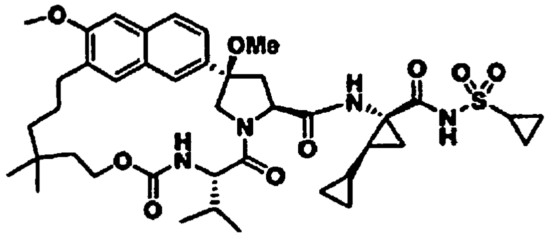

;

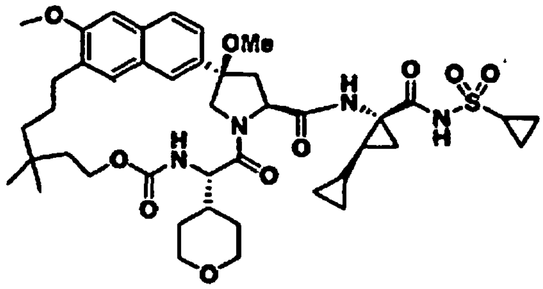

;

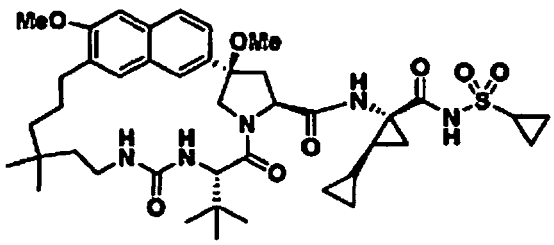

;

et

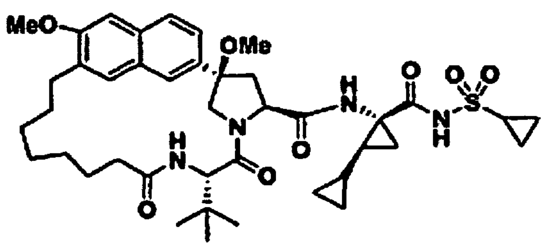

;

ou un sel pharmaceutiquement acceptable de ceux-ci.

**12.** Composition comprenant le composé selon l'une quelconque des revendications 1 à 11 ou un sel pharmaceutiquement acceptable de celui-ci et un véhicule pharmaceutiquement acceptable.

**13.** Composition selon la revendication 12, comprenant en plus au moins un composé supplémentaire ayant une activité anti-VHC.

**14.** Composition selon la revendication 13, dans laquelle au moins l'un des composés supplémentaires est un interféron ou une ribavirine.

**15.** Composition selon la revendication 14, dans laquelle l'interféron est sélectionné parmi l'interféron alpha 2B, l'interféron alpha pégylé, l'interféron consensus, l'interféron alpha 2A et l'interféron lymphoblastoïde tau.

**16.** Composition selon la revendication 13, dans laquelle au moins l'un des composés supplémentaires est sélectionné parmi l'interleukine 2, l'interleukine 6, l'interleukine 12, un composé qui renforce le développement de la réponse des lymphocytes T auxiliaires de type 1, l'ARN interférent, l'ARN anti-sens, l'imiquimod, la ribavirine, un inhibiteur d'inosine 5'-monophosphate déshydrogénase, l'amantadine et la rimantadine.

**17.** Composition selon la revendication 13, dans laquelle au moins l'un des composés supplémentaires est efficace pour inhiber la fonction d'une cible sélectionnée parmi la métalloprotéase du VHC, la sérine protéase du VHC, la polymérase du VHC, l'hélicase du VHC, la protéine NS4B du VHC, l'entrée du VHC, l'assemblage du VHC, la sortie du VHC, la protéine NS5A du VHC et l'IMPDH, dans le traitement d'une infection par le VHC.

**18.** Composé selon l'une quelconque des revendications 1 à 11 ou un sel pharmaceutiquement acceptable de celui-ci, à utiliser dans une méthode de traitement d'une infection par le VHC chez un patient.

**19.** Composé à utiliser selon la revendication 18, comprenant en plus administrer au moins un composé supplémentaire ayant une activité anti-VHC selon les revendications 14 à 17, avant, après ou en même temps que le composé selon l'une quelconque des revendications 1 à 11, ou un sel pharmaceutiquement acceptable de celui-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006119061 A
- WO 2008057209 A
- WO 2008051475 A
- WO 2008057208 A
- WO 2005047288 A
- WO 2004099143 A
- WO 0009543 A


### Non-patent literature cited in the description


- Remington's Pharmaceutical Sciences. Mack Publishing Company, 1999
- **BERGER A. ; SCHECHTER I.** *Transactions of the Royal Society London series,* 1970, vol. B257, 249-264
- *Pharmaceutical Research,* 1986, vol. 3 (6), 318
- *J. Med. Chem.,* 1990, vol. 33, 171
- *Eur. J. Org. Chem.,* 2004, 3992
- **P SIMMONDS ; KA ROSE ; S GRAHAM ; SW CHAN ; F MCOMISH ; BC DOW ; EA FOLLETT ; PL YAP ; H MARSDEN.** *J. Clin. Microbiol.,* 1993, vol. 31 (6), 1493-1503
- **YANAGI,M. ; PURCELL,R.H. ; EMERSON, S.U. ; BUKH, J.** *Proc. Natl. Acad. Sci. U.S.A.,* 1997, vol. 94 (16), 8738-8743
- **YANAGI,M. ; ST CLAIRE,M. ; SHAPIRO,M. ; EMERSON,S.U. ; PURCELL,R.H. ; BUKH, J.** *Virology,* 1998, vol. 244 (1), 161-172
- **GALLINARI P ; PAOLINI C ; BRENNAN D ; NARDI C ; STEINKUHLER C ; DE FRANCESCO R.** *Biochemistry,* 1999, vol. 38 (17), 5620-32
- **GALLINARI P ; BRENNAN D ; NARDI C ; BRUNETTI M ; TOMEI L ; STEINKUHLER C ; DE FRANCESCO R.** *J Virol.,* 1998, vol. 72 (8), 6758-69
- **TALIANI et al.** *Anal. Biochem.,* 1996, vol. 240 (2), 60-67
- **LOHMANN V ; KORNER F ; KOCH J ; HERIAN U ; THEILMANN L ; BARTENSCHLAGER R.** *Science,* 1999, vol. 285 (5424), 110-3
- **KRIEGER N ; LOHMANN V ; BARTENSCHLAGER R.** *J. Virol.,* 2001, vol. 75 (10), 4614-4624
- **BLIGHT KJ ; KOLYKHALOV, AA ; RICE, CM.** *Science,* vol. 290 (5498), 1972-1974